# EUROPEAN PATENT SPECIFICATION

(11) **EP 4 229 045 B1**
(45) Date of publication and mention of the grant of the patent: **12.11.2025**
(21) Application number: 21881085.1
(22) Date of filing: 14.10.2021
(51) Int. Cl.: C07D 307/85, C07D 405/04, C07D 405/12, A61K 31/343, A61K 31/4155, A61K 31/443, A61K 31/4725, A61P 35/00

(54) **SUBSTITUTED ACYL SULFONAMIDES FOR TREATING CANCER**
SUBSTITUIERTE ACYLSULFONAMIDE ZUR BEHANDLUNG VON KREBS
ACYLSULFONAMIDES SUBSTITUÉS POUR LE TRAITEMENT DU CANCER

(30) Priority: 16.10.2020 US 202063092972 P
(43) Date of publication of application: 23.08.2023
(73) Proprietor: The Broad Institute Inc., Cambridge, MA 02142 (US); Bayer Aktiengesellschaft, 51373 Leverkusen (DE)
(72) Inventor: BOUCHE, Léa, 13353 Berlin (DE); KORR, Daniel, 13353 Berlin (GB); TER LAAK, Antonius, 13353 Berlin (DE); HERBERT, Simon, 13353 Berlin (DE); BARAK, Naomi, 13353 Berlin (DE); HILLIG, Roman, 13353 Berlin (DE); NEUHAUS, Roland, 13353 Berlin (DE); GRADL, Stefan, 13353 Berlin (DE); WICHARD, Jörg, 13353 Berlin (DE); FERNANDEZ-MONTALVAN, Emesto, Amaury, 13353 Berlin (DE); GORJANACZ, Matyas, 13353 Berlin (DE); PÜTTER, Vera, 13353 Berlin (DE); MEIER, Robin, 13353 Berlin (DE); SUTTER, Andreas, 13353 Berlin (DE); FERRARA, Steven, James, Cambridge, MA 02142 (US)
(74) Representative: HGF
(86) International application number: PCT/US2021/054979
(87) International publication number: WO 2022/081842

(56) References cited:
- WO-A1-2016/198507
- WO-A1-2018/102419
- WO-A1-2019/108824
- WO-A1-2020/216701

## Description

### BACKGROUND

The present invention provides acyl sulfonamide compounds of general formula (I) which inhibit the activity of lysine acetyl transferase 6A (KAT6A) and lysine acetyl transferase 6B (KAT6B). In particular, the present invention provides compositions and methods for the treatment of lysine acetyl transferase 6A (KAT6A) activated cancers and lysine acetyl transferase 6B (KAT6B) activated cancers. More particularly, the present disclosure provides inhibitors of KAT6A and KAT6B for the treatment of breast cancer, lung cancer, ovarian cancer, endometrial cancer, esophageal cancer, bladder cancer, and acute myeloid leukemia. Even more particularly, the present disclosure provides inhibitors of KAT6A and KAT6B for the treatment of lung cancer, breast cancer, bladder cancer, uterine cancer, endometrial cancer, prostate cancer and leukemia.

Epigenetic regulation of gene expression is a complex process which confers stable, long term and, in some cases, heritable alterations in cellular gene expression. Rather than involving changes to the actual DNA sequence of the gene, epigenetic regulation is mediated through modifications to either the DNA backbone or to its associated chromatin proteins. These modifications facilitate interaction with the epigenetic gene regulation machinery, with the end result being stable gene activation/expression or stable gene silencing/repression. Alterations to the epigenetic gene regulation machinery underlie many different and diverse disease pathologies, and this is now recognized as a major driver in the oncogenic process for most cancers.

Histone proteins are the key chromatin proteins that facilitate the effector functions of the epigenetic gene regulation machinery. These are a family of five closely related proteins, denoted as histones H1, H2A, H2B, H3, and H4, that package and order the DNA into structural units called nucleosomes. Histones H3 and H4 both feature long amino-terminal polypeptide tails that protrude from the nucleosome, and that are rich in lysine and arginine amino acid residues which confer a net positive charge that enables the histone tails to interact with and bind to the negatively charged phosphate groups of the DNA backbone. Post-translational modification (PTM) of the lysine and arginine residues alters the nucleosome structure by altering the ability of the histones to bind DNA, and also by providing specific binding sites for the epigenetic gene regulation machinery. Such PTMs include methylation, acetylation, phosphorylation, ubiquitylation, and sumoylation.

Histone acetyltransferases (HATs) comprise a discrete family of enzymes which catalyze the transfer of an acetyl group from acetyl coenzyme A to a lysine residue in histones as well as other protein substrates. Lysine acetyl transferase 6A (KAT6A) and lysine acetyl transferase 6B (KAT6B) are the two HATs which are the focus of this disclosure. Both proteins have been implicated in cancer. *KAT6A* is the target of recurrent chromosomal translocations in acute myeloid leukemia, and it is focally amplified in lung, breast, ovarian, endometrial, bladder, and esophageal cancers. Similarly, *KAT6B* chromosomal translocations have been identified in a diverse range of cancers, and it is focally amplified in breast, ovarian, uterine, stomach, bladder, and lung cancer. Expression of *KAT6A* and *KAT6B* correlates well with gene copy number in tumors that have these focal amplifications, suggesting that there has been selective pressure to maintaining their activity during the oncogenic process. Moreover, cancer cell lines derived from tumors which bear these focal amplifications have high levels of *KAT6A* or KAT6B expression and are genetically dependent on this activity in long term proliferation experiments.

The biological pathways impacted by KAT6A and KAT6B activity are poorly defined, although there is some data which supports a gene activation function, notably the control of *ESR1* expression in breast cancer cell lines. Similarly, the histone substrates of KAT6A and KAT6B are also poorly defined, and are thought to include lysine 9 of histone H3 (H3K9), lysine 14 of histone 3 (H3K14) and lysine 23 of histone H3 (H3K23). Acetylation at these positions is associated with such diverse functions as gene activation and DNA damage recognition, and it remains unclear which of these PTMs are the critical effectors in tumor cells that are dependent on KAT6A or KAT6B.

WO2016198507 discloses aryl sulfonhydrazide derivatives are known as MOZ (KAT6A) inhibitors. Further, WO2019108824 relates to sulfonohydrazido compounds as histone acetyltransferase inhibitors. WO2018102419 discloses sulfonhydrazide derivatives as KAT inhibitors. However, there are currently no FDA-approved targeted therapeutics for KAT6A or KAT6B. Accordingly, there is an urgent need for compounds, compositions and methods for treating KAT6A- or KAT6B-activated cancers.

Further, the assessment of mutagenicity in a bacterial reverse gene mutation test (*i.e.* Ames test) is an integral component of the standard genotoxicity test battery for small molecular weight chemical entities as recommended by the International Council for Harmonisation of Technical Requirements for Pharmaceuticals for Human Use (ICH) in the guideline document ICH S2(R1). Compounds demonstrating activity in this test (positive Ames test result) may pose a risk to human health by initiating or promoting the development of neoplasia (cancer). Their potential therapeutic use is therefore restricted to late stage cancer patient with limited expected life-span and no other treatment options. In addition, manufacturing and handling of such compounds requires specific occupational safety measures. In contrast, compounds that do not demostrate a genotoxic potential offer a much broader range of potential therapeutic applications, even outside oncological indications. Moreover, occupational safety measures for manufacturing and handling can be less restrictive.

The present invention includes KAT6A and KAT6B inhibitors that are inactive in a bacterial reverse gene mutation test (negative Ames test result).

### SUMMARY

The present invention provides acyl sulfonamide compounds of general formula (I): 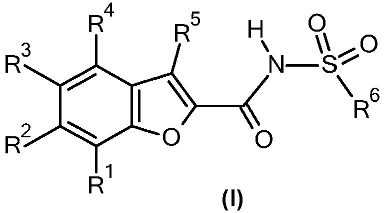 in which R¹, R², R³, R⁴, R⁵, and R⁶ are as described and defined herein, methods of preparing said compounds, intermediate compounds useful for preparing said compounds, pharmaceutical compositions and combinations comprising said compounds, and the use of said compounds for manufacturing pharmaceutical compositions for the treatment or prophylaxis of diseases, in particular of hyperproliferative disorders, as a sole agent or in combination with other active ingredients.

### DESCRIPTION OF THE INVENTION

It has now been found that the compounds of the present invention effectively inhibit the activity of lysine acetyl transferase 6A (KAT6A) and/or lysine acetyl transferase 6B (KAT6B) for which data are given in the biological experimental section, and may therefore be used for the treatment or prophylaxis of hyperproliferative disorders, such as cancer disorders. Further, the compounds of the present invention include KAT6A and KAT6B inhibitors that are inactive in a bacterial reverse gene mutation test (negative Ames test result).

In accordance with a first aspect, the present invention provides compounds of general formula (I): 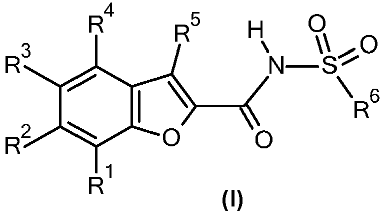 wherein
- R¹: is selected from a hydrogen atom and a halogen atom;
- R²: is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a C₁-C₆-alkyl group, a phenyl group, a naphthyl group, a heteroaryl group, a (phenyl)-(C₁-C₆-alkyl)- group, a (naphthyl)-(C₁-C₆-alkyl)-group, a (heteroaryl)-(C₁-C₆-alkyl)- group, a (heterocycloalkyl)-(C₁-C₆-alkyl)-group and a heterocycloalkyl group,
wherein said heterocycloalkyl group is connected to the rest of the molecule via a carbon atom of said heterocycloalkyl group,
wherein said phenyl, naphthyl, heteroaryl or heterocycloalkyl group is each optionally substituted with one or more substituents independently selected from a halogen atom, a cyano group, a nitro group, a hydroxy group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a (C₁-C₃-alkyl)-O-(C₁-C₂-alkyl)- group, a (C₁-C₃-alkyl)-O-(C₁-C₂-alkyl)-O-group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O-group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a C₁-C₆-thioalkyl group, a heterocycloalkyl group, a R⁷R⁸N- group, a (C₁-C₂-alkyl)-S(=O)₂- group, a (R⁷R⁸N)-S(=O)₂- group, a (R⁷R⁸N)-(C₁-C₆-alkyl)-(C=O)-NH- group, a (H₃C)-(C=O)- group, a (R⁷R⁸N)-(C=O)- group, a (R⁷R⁸N)-(C=O)-O- group, a (R⁷R⁸N)-(C₁-C₆-alkyl)- group, a R⁹OOC-(C₁-C₃-alkyl)- group, a R⁹OOC- group, a phenyl group, a naphthyl group, a (phenyl)-O- group and a heteroaryl group,
   wherein said heterocycloalkyl group is connected to the rest of the molecule via a carbon atom of said heterocycloalkyl group,
   wherein said phenyl, naphthyl, heteroaryl or heterocycloalkyl group is each optionally substituted with one or more substituents independently selected from a halogen atom, a cyano group, a nitro group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a heterocycloalkyl group, a phenyl group, a R⁷R⁸N- group, a (R⁷R⁸N)-S(=O)₂- group, a (R⁷R⁸N)-(C₁-C₆-alkyl)-(C=O)-NH- group, a (R⁷R⁸N)-(C₁-C₆-alkyl)- group, and a R⁹OOC- group,
      wherein said heterocycloalkyl group is connected to the rest of the molecule via a carbon atom of said heterocycloalkyl group;
- R⁷ and R⁸: are each independently selected from a hydrogen atom, a C₁-C₆-alkyl group, a C₃-C₈-cycloalkyl group, a C₁-C₆-haloalkyl group, a HC(=O)- group, a (C₁-C₆-alkyl)C(=O)- group, a (C₁-C₆-haloalkyl)C(=O)- group, a (C₃-C₈-cycloalkyl)-(C₁-C₆-alkyl)- group, a C₁-C₆-hydroxyalkyl group, a (C₁-C₆-alkoxy)-(C₁-C₆-alkyl)- group, a (phenyl)-(S=O)₂- group, a (C₁-C₆-alkyl)-(S=O)₂- group, a (C₁-C₆-haloalkyl)-(S=O)₂- group, a phenyl group, a heterocycloalkyl group and a heteroaryl group,
wherein said heterocycloalkyl group is connected to the rest of the molecule via a carbon atom of said heterocycloalkyl group,
wherein said phenyl, heteroaryl or heterocycloalkyl group is each optionally substituted with one or more substituents independently selected from a halogen atom, a cyano group, a nitro group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a C₁-C₆-thioalkyl group, a heterocycloalkyl group, and a R⁹OOC- group,
   wherein said heterocycloalkyl group is connected to the rest of the molecule via a carbon atom of said heterocycloalkyl group;
or R⁷ and R⁸ together with the nitrogen atom to which they are attached form a 4- to 7-membered nitrogen-containing heterocycloalkyl group, wherein said heterocycloalkyl group optionally contains one, two or three further heteroatoms independently selected from nitrogen, oxygen and sulfur and/or is optionally substituted with one or more substituents independently selected from a halogen atom, a cyano group, a nitro group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a C₁-C₆-thioalkyl group, a heterocycloalkyl group and a R⁹OOC-group,
wherein said heterocycloalkyl group is connected to the rest of the molecule via a carbon atom of said heterocycloalkyl group;
- R⁹: is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

### DETAILED DESCRIPTION

### DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3, 4 or 5, in particular 1, 2 or 3, more particularly 1 or 2, and even more particularly 1.

As used herein, the term "one or more", *e*.*g*., in the definition of the substituents of the compounds of general formula (I) of the present invention, means "1, 2, 3, 4 or 5, particularly 1, 2, 3 or 4, more particularly 1, 2 or 3, even more particularly 1 or 2". In the context of the present invention, when applied to the substituents of the compounds of general formula **(I),** the term "one or more" is to be understood as meaning preferably 1, 2 or 3 substituents, and more preferably 1 or 2 substituents.

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one, two or three substituents, more particularly with one substituent.

The terms "oxo", "an oxo group" or "an oxo substituent" mean a doubly bonded oxygen atom =O. Oxo may be attached to atoms of suitable valency, for example to a saturated carbon atom or to a sulfur atom. For example, but without limitation, one oxo group can be attached to a carbon atom, resulting in the formation of a carbonyl group C(=O), or two oxo groups can be attached to one sulfur atom, resulting in the formation of a sulfonyl group -S(=O)₂.

The term "ring substituent" means a substituent attached to an aromatic or nonaromatic ring which replaces an available hydrogen atom on the ring.

Should a composite substituent be composed of more than one parts, *e*.*g*. (C₁-C₄-alkoxy)-(C₁-C₄-alkyl)-, it is possible for the position of a given part to be at any suitable position of said composite substituent, *i.e.* the C₁-C₄-alkoxy part can be attached to any carbon atom of the C₁-C₄-alkyl part of said (C₁-C₄-alkoxy)-(C₁-C₄-alkyl)- group. A hyphen at the beginning or at the end of such a composite substituent indicates the point of attachment of said composite substituent to the rest of the molecule. Should a ring, comprising carbon atoms and optionally one or more heteroatoms, such as nitrogen, oxygen or sulfur atoms for example, be substituted with a substituent, it is possible for said substituent to be bound at any suitable position of said ring, be it bound to a suitable carbon atom and/or to a suitable heteroatom.

The term "comprising" when used in the specification includes "consisting of".

If within the present text any item is referred to as "as mentioned herein", it means that it may be mentioned anywhere in the present text.

If within the present text any item is referred to as *"supra"* within the description it indicates any of the respective disclosures made within the specification in any of the preceding pages, or above on the same page.

If within the present text any item is referred to as *"infra"* within the description it indicates any of the respective disclosures made within the specification in any of the subsequent pages, or below on the same page.

The terms as mentioned in the present text have the following meanings:
The term "halogen atom" means a fluorine, chlorine, bromine or iodine atom, particularly a fluorine, chlorine or bromine atom, more particularly a fluorine atom.

The term "C₁-C₆-alkyl" means a linear or branched, saturated, monovalent hydrocarbon group having 1, 2, 3, 4, 5 or 6 carbon atoms, *e*.*g*. a methyl-, ethyl-, propyl-, isopropyl-, butyl-, sec-butyl-, isobutyl-, *tert*-butyl-, pentyl-, isopentyl-, 2-methylbutyl-, 1-methylbutyl-, 1-ethylpropyl-, 1,2-dimethylpropyl-, *neo*-pentyl-, 1,1-dimethylpropyl-, hexyl-, 1-methylpentyl-, 2-methylpentyl-, 3-methylpentyl-, 4-methylpentyl-, 1-ethylbutyl-, 2-ethylbutyl-, 1,1-dimethylbutyl-, 2,2-dimethylbutyl-, 3,3-dimethylbutyl-, 2,3-dimethylbutyl-, 1,2-dimethylbutyl- or a 1,3-dimethylbutyl- group, or an isomer thereof. Particularly, said group has 1, 2, 3 or 4 carbon atoms ("C₁-C₄-alkyl"), *e.g*. a methyl-, ethyl-, propyl-, isopropyl-, butyl-, sec-butyl-, isobutyl- or a *tert*-butyl group, more particularly 1, 2 or 3 carbon atoms ("C₁-C₃-alkyl"), *e*.*g*. a methyl-, ethyl-, *n*-propyl- or an isopropyl group.

The term "C₁-C₆-hydroxyalkyl" means a linear or branched, saturated, monovalent hydrocarbon group in which the term "C₁-C₆-alkyl" is defined *supra,* and in which one or more hydrogen atoms are replaced with a hydroxy group, *e*.*g*. a hydroxymethyl-, 1-hydroxyethyl-, 2-hydroxyethyl-, 1,2-dihydroxyethyl-, 3-hydroxypropyl-, 2-hydroxypropyl-, 1-hydroxypropyl-, 1-hydroxypropan-2-yl-, 2-hydroxypropan-2-yl-, 2,3-dihydroxypropyl-, 1,3-dihydroxypropan-2-yl-, 3-hydroxy-2-methyl-propyl-, 2-hydroxy-2-methyl-propyl- or a 1-hydroxy-2-methyl-propyl- group.

The term "C₁-C₆-alkylsulfanyl" means a linear or branched, saturated, monovalent group of formula (C₁-C₆-alkyl)-S-, in which the term "C₁-C₆-alkyl" is as defined *supra, e.g.* a methylsulfanyl-, ethylsulfanyl-, propylsulfanyl-, isopropylsulfanyl-, butylsulfanyl-, *sec-*butylsulfanyl-, isobutylsulfanyl-, *tert*-butylsulfanyl-, pentylsulfanyl-, isopentylsulfanyl- or a hexylsulfanyl- group.

The term "C₁-C₆-haloalkyl" means a linear or branched, saturated, monovalent hydrocarbon group in which the term "C₁-C₆-alkyl" is as defined *supra* and in which one or more of the hydrogen atoms are replaced, identically or differently, with a halogen atom. Preferably, said halogen atom is a fluorine atom. Said C₁-C₆-haloalkyl, particularly a C₁-C₃-haloalkyl group is, for example, fluoromethyl-, difluoromethyl-, trifluoromethyl-, 2-fluoroethyl-, 2,2-difluoroethyl-, 2,2,2-trifluoroethyl-, pentafluoroethyl-, 3,3,3-trifluoropropyl- or a 1,3-difluoropropan-2-yl group.

The term "C₁-C₆-alkoxy" means a linear or branched, saturated, monovalent group of formula (C₁-C₆-alkyl)-O-, in which the term "C₁-C₆-alkyl" group is as defined *supra, e.g.* methoxy-, ethoxy-, *n*-propoxy-, isopropoxy-, *n*-butoxy-, *sec*-butoxy-, isobutoxy-, *tert*-butoxy-, pentyloxy-, isopentyloxy- or a *n*-hexyloxy group, or an isomer thereof.

The term "C₁-C₆-haloalkoxy" means a linear or branched, saturated, monovalent C₁-C₆-alkoxy group, as defined *supra,* in which one or more of the hydrogen atoms is replaced, identically or differently, with a halogen atom. Preferably, said halogen atom in "C₁-C₆-haloalkoxy-" is fluorine, resulting in a group referred herein as "C₁-C₆-fluoroalkoxy-". Representative C₁-C₆-fluoroalkoxy- groups include, for example, -OCF₃, -OCHF₂, - OCH₂F, -OCF₂CF₃ and -OCH₂CF₃.

The term "C₂-C₆-alkenyl-" means a linear or branched, monovalent hydrocarbon group, which contains one or more double bonds and which has 2, 3, 4, 5 or 6 carbon atoms, preferably 2, 3 or 4 carbon atoms ("C₂-C₄-alkenyl-") or 2 or 3 carbon atoms ("C₂-C₃-alkenyl-"), it being understood that in the case in which said alkenyl- group contains more than one double bond, then said double bonds may be isolated from, or conjugated with, each other. Representative alkenyl groups include, for example, an ethenyl-, prop-2-enyl-, (*E*)-prop-1-enyl-, (*Z*)-prop-1-enyl-, *iso*-propenyl-, but-3-enyl-, (*E*)-but-2-enyl-, (*Z*)-but-2-enyl-, (*E*)-but-1-enyl-, (Z)-but-1-enyl-, 2-methylprop-2-enyl-, 1-methylprop-2-enyl-, 2-methylprop-1-enyl-, (*E*)-1-methylprop-1-enyl-, (*Z*)-1-methyl prop-1-enyl-, buta-1,3-dienyl-, pent-4-enyl-, (*E*)-pent-3-enyl-, (*Z*)-pent-3-enyl-, (*E*)-pent-2-enyl-, (*Z*)-pent-2-enyl-, (*E*)-pent-1-enyl-, (*Z*)-pent-1-enyl-, 3-methylbut-3-enyl-, 2-methylbut-3-enyl-, 1-methylbut-3-enyl-, 3-methylbut-2-enyl-, (*E*)-2-methylbut-2-enyl-, (*Z*)-2-methylbut-2-enyl-, (*E*)-1-methylbut-2-enyl-, (*Z*)-1-methylbut-2-enyl-, (*E*)-3-methylbut-1-enyl-, (*Z*)-3-methylbut-1-enyl-, (*E*)-2-methylbut-1-enyl-, (*Z*)-2-methylbut-1-enyl-, (*E*)-1-methylbut-1-enyl-, (*Z*)-1-methylbut-1-enyl-, 1,1-dimethylprop-2-enyl-, 1-ethylprop-1-enyl-, 1-propylvinyl-, 1-isopropylvinyl-, (*E*)-3,3-dimethylprop-1-enyl-, (*Z*)-3,3-dimethylprop-1-enyl-, penta-1,4-dienyl-, hex-5-enyl-, (*E*)-hex-4-enyl-, (*Z*)-hex-4-enyl-, (*E*)-hex-3-enyl-, (*Z*)-hex-3-enyl-, (*E*)-hex-2-enyl-, (*Z*)-hex-2-enyl-, (*E*)-hex-1-enyl-, (*Z*)-hex-1-enyl-, 4-methyl pent-4-enyl-, 3-methylpent-4-enyl-, 2-methylpent-4-enyl-, 1-methyl pent-4-enyl-, 4-methylpent-3-enyl-, (*E*)-3-methylpent-3-enyl-, (*Z*)-3-methylpent-3-enyl-, (*E*)-2-methylpent-3-enyl-, (*Z*)-2-methylpent-3-enyl-, (*E*)-1-methylpent-3-enyl-, (*Z*)-1-methyl pent-3-enyl-, (*E*)-4-methylpent-2-enyl-, (*Z*)-4-methylpent-2-enyl-, (*E*)-3-methylpent-2-enyl-, (*Z*)-3-methylpent-2-enyl-, (*E*)-2-methylpent-2-enyl-, (*Z*)-2-methylpent-2-enyl-, (*E*)-1-methylpent-2-enyl-, (*Z*)-1-methylpent-2-enyl-, (*E*)-4-methylpent-1-enyl-, (*Z*)-4-methylpent-1-enyl-, (*E*)-3-methylpent-1-enyl-, (*Z*)-3-methylpent-1-enyl-, (*E*)-2-methylpent-1-enyl-, (*Z*)-2-methylpent-1-enyl-, (*E*)-1-methylpent-1-enyl-, (*Z*)-1-methylpent-1-enyl-, 3-ethylbut-3-enyl-, 2-ethylbut-3-enyl-, 1-ethylbut-3-enyl-, (*E*)-3-ethylbut-2-enyl-, (*Z*)-3-ethylbut-2-enyl-, (*E*)-2-ethylbut-2-enyl-, (*Z*)-2-ethylbut-2-enyl-, (*E*)-1-ethylbut-2-enyl-, (*Z*)-1-ethylbut-2-enyl-, (*E*)-3-ethylbut-1-enyl-, (*Z*)-3-ethylbut-1-enyl-, 2-ethylbut-1-enyl-, (*E*)-1-ethylbut-1-enyl-, (*Z*)-1-ethylbut-1-enyl-, 2-propylprop-2-enyl-, 1-propylprop-2-enyl-, 2-isopropylprop-2-enyl-, 1-isopropylprop-2-enyl-, (*E*)-2-propylprop-1-enyl-, (*Z*)-2-propylprop-1-enyl-, (*E*)-1-propylprop-1-enyl-, (*Z*)-1-propylprop-1-enyl-, (*E*)-2-isopropylprop-1-enyl-, (*Z*)-2-isopropylprop-1-enyl-, (*E*)-1-isopropylprop-1-enyl-, (*Z*)-1-isopropylprop-1-enyl-, hexa-1,5-dienyl- and a 1-(1,1-dimethylethyl-)ethenyl group. Particularly, said group is an ethenyl- or a prop-2-enyl group.

The same definitions can be applied should the alkenyl group be placed within a chain as a bivalent "C₂-C₆-alkenylene" moiety. All names as mentioned above then will bear a "ene" added to their end, thus *e*.*g*., a "pentenyl" becomes a bivalent "pentenylene" group.

The term "C₂-C₆-haloalkenyl-" means a linear or branched hydrocarbon group in which one or more of the hydrogen atoms of a "C₂-C₆-alkenyl-" as defined *supra* are each replaced, identically or differently, by a halogen atom. Preferably, said halogen atom is fluorine, resulting in a group referred herein as "C₂-C₆-fluoroalkenyl-". Representative C₂-C₆-fluoroalkenyl- groups include, for example, -CH=CF₂, -CF=CH₂, -CF=CF₂, - C(CH₃)=CF₂, -CH=C(F)-CH₃, -CH₂-CF=CF₂ and -CF₂-CH=CH₂.

The term "C₂-C₆-alkynyl-" means a linear or branched, monovalent hydrocarbon group which contains one or more triple bonds, and which contains 2, 3, 4, 5 or 6 carbon atoms, preferably 2, 3 or 4 carbon atoms ("C₂-C₄-alkynyl-") or 2 or 3 carbon atoms ("C₂-C₃-alkynyl-"). Representative C₂-C₆-alkynyl- groups include, for example, an ethynyl-, prop-1-ynyl-, prop-2-ynyl-, but-1-ynyl-, but-2-ynyl-, but-3-ynyl-, pent-1-ynyl-, pent-2-ynyl, pent-3-ynyl-, pent-4-ynyl-, hex-1-ynyl-, hex-2-ynyl-, hex-3-ynyl-, hex-4-ynyl-, hex-5-ynyl-, 1-methylprop-2-ynyl-, 2-methylbut-3-ynyl-, 1-methylbut-3-ynyl-, 1-methylbut-2-ynyl-, 3-methylbut-1-ynyl-, 1-ethylprop-2-ynyl-, 3-methylpent-4-ynyl-, 2-methylpent-4-ynyl-, 1-methylpent-4-ynyl-, 2-methylpent-3-ynyl-, 1-methylpent-3-ynyl-, 4-methylpent-2-ynyl-, 1-methylpent-2-ynyl-, 4-methylpent-1-ynyl-, 3-methylpent-1-ynyl-, 2-ethylbut-3-ynyl-, 1-ethylbut-3-ynyl-, 1-ethylbut-2-ynyl-, 1-propylprop-2-ynyl-, 1-isopropylprop-2-ynyl-, 2,2-dimethylbut-3-ynyl-, 1,1-dimethylbut-3-ynyl-, 1,1-dimethylbut-2-ynyl- and a 3,3-dimethylbut-1-ynyl- group. Particularly, said alkynyl- group is an ethynyl-, a prop-1-ynyl- or a prop-2-ynyl group.

The term "C₃-C₈-cycloalkyl" means a saturated, monovalent, mono- or bicyclic hydrocarbon ring which contains 3, 4, 5, 6, 7 or 8 carbon atoms ("C₃-C₈-cycloalkyl"). Said C₃-C₈-cycloalkyl group is for example, a monocyclic hydrocarbon ring, *e*.*g*. a cyclopropyl-, cyclobutyl-, cyclopentyl-, cyclohexyl-, cycloheptyl- or cyclooctyl- group, or a bicyclic hydrocarbon ring, *e*.*g*. a bicyclo[4.2.0]octyl- or an octahydropentalenyl- group.

The term "C₃-C₈-halocycloalkyl" means a saturated, monovalent, mono- or bicyclic hydrocarbon ring which contains 3, 4, 5, 6, 7 or 8 carbon atoms in which the term "C₃-C₈-cycloalkyl" is as defined *supra* and in which one or more of the hydrogen atoms of the hydrocarbon ring are replaced, identically or differently, with a halogen atom. Preferably, said halogen atom is a fluorine atom. The "C₃-C₈-cycloalkyl" group as defined *supra* in which one or more of the hydrogen atoms are replaced, identically or differently, with a halogen atom, preferably a fluorine atom, is for example, a monocyclic hydrocarbon ring, *e*.*g*. a cyclopropyl-, cyclobutyl-, cyclopentyl-, cyclohexyl-, cycloheptyl- or cyclooctyl-group, or a bicyclic hydrocarbon ring, *e*.*g*. a bicyclo[4.2.0]octyl- or an octahydropentalenyl- group.

The term "C₄-C₈-cycloalkenyl" means a monovalent, mono- or bicyclic hydrocarbon ring which contains 4, 5, 6, 7 or 8 carbon atoms and one double bond. Particularly, said ring contains 4, 5 or 6 carbon atoms ("C₄-C₆-cycloalkenyl"). Said C₄-C₈-cycloalkenyl group is for example, a monocyclic hydrocarbon ring, *e*.*g.*, a cyclobutenyl-, cyclopentenyl-, cyclohexenyl-, cycloheptenyl- or a cyclooctenyl group, or a bicyclic hydrocarbon ring, e*.g*., a bicyclo[2.2.1]hept-2-enyl- or a bicyclo[2.2.2]oct-2-enyl group.

The term "C₃-C₈-cycloalkoxy" means a saturated, monovalent, mono- or bicyclic group of formula (C₃-C₈-cycloalkyl)-O-, which contains 3, 4, 5, 6, 7 or 8 carbon atoms, in which the term "C₃-C₈-cycloalkyl" is defined *supra, e.g.* a cyclopropyloxy-, cyclobutyloxy-, cyclopentyloxy-, cyclohexyloxy-, cycloheptyloxy- or a cyclooctyloxy- group.

If the term "heterocycloalkyl" is used without specifying a number of atoms it is meant to be a "4- to 10-membered heterocycloalkyl-" group, more particularly a 5- to 6-membered heterocycloalkyl group. The terms "4- to 7-membered heterocycloalkyl", "4- to 6-membered heterocycloalkyl" and "5- to 7-membered heterocycloalkyl" mean a monocyclic, saturated heterocycle with "4, 5, 6 or 7" or, respectively, "4, 5 or 6" or "5, 6 or 7" ring atoms in total, which are saturated or partially unsaturated monocycles, bicycles or polycycles that contain one or two identical or different ring heteroatoms selected from nitrogen, oxygen and sulfur. It is possible for said heterocycloalkyl group to be attached to the rest of the molecule via any one of the carbon atoms or, if present, a nitrogen atom.

Exemplarily, without being limited thereto, said "4- to 7-membered heterocycloalkyl", can be a 4-membered ring, a "4-membered heterocycloalkyl-" group, such as an azetidinyl- or an oxetanyl group; or a 5-membered ring, a "5-membered heterocycloalkyl-" group, such as a tetrahydrofuranyl-, dioxolinyl-, pyrrolidinyl-, imidazolidinyl-, pyrazolidinyl- or a pyrrolinyl group; or a 6-membered ring, a "6-membered heterocycloalkyl-" group, such as a tetrahydropyranyl-, piperidinyl-, morpholinyl-, 3-oxomorpholin-4-yl, dithianyl-, thiomorpholinyl- or a piperazinyl group; or a 7-membered ring, a "7-membered heterocycloalkyl-" group, such as an azepanyl-, diazepanyl- or an oxazepanyl group, for example. The heterocycloalkyl groups may be substituted one or more times independently with C₁-C₃-alkyl, C₁-C₃-alkoxy, hydroxy, halogen or a carbonyl group.

Particularly, "4- to 6-membered heterocycloalkyl" means a 4- to 6-membered heterocycloalkyl as defined *supra* containing one ring nitrogen atom and optionally one further ring heteroatom selected from nitrogen, oxygen and sulfur. Particularly, "5- to 7-membered heterocycloalkyl" means a 5- to 7-membered heterocycloalkyl as defined *supra* containing one ring nitrogen atom and optionally one further ring heteroatom selected from nitrogen, oxygen and sulfur. More particularly, "5- or 6-membered heterocycloalkyl" means a monocyclic, saturated heterocycle with 5 or 6 ring atoms in total, containing one ring nitrogen atom and optionally one further ring heteroatom selected from nitrogen and oxygen.

The term "heteroaryl-" means a monocyclic, bicyclic or tricyclic aromatic ring system having 5, 6, 7, 8, 9, 10, 11, 12, 13 or 14 ring atoms (a "5- to 14-membered heteroaryl-" group), preferably 5, 6, 9 or 10 ring atoms and which contains 1, 2, 3 or 4 heteroatoms which may be identical or different, said heteroatoms being selected from oxygen, nitrogen and sulfur. Said heteroaryl- group can be a 5-membered heteroaryl group, such as, for example, a thienyl-, furanyl-, pyrrolyl-, oxazolyl-, thiazolyl-, imidazolyl-, pyrazolyl-, isoxazolyl-, isothiazolyl-, oxadiazolyl-, triazolyl-, thiadiazolyl- or a tetrazolyl group; or a 6-membered heteroaryl group, such as, for example, a pyridyl-, pyridazinyl-, pyrimidyl-, pyrazinyl- or a triazinyl group; or a benzo-fused 5-membered heteroaryl- group, such as, for example, a benzofuranyl-, benzothienyl-, benzoxazolyl-, benzisoxazolyl-, benzimidazolyl-, benzothiazolyl-, benzotriazolyl-, indazolyl-, indolyl- or a isoindolyl group; or a benzo-fused 6-membered heteroaryl group, such as, for example, a quinolinyl-, quinazolinyl-, isoquinolinyl-, cinnolinyl-, phthalazinyl- or quinoxalinyl-; or another bicyclic group, such as, for example, indolizinyl-, purinyl- or a pteridinyl group.

Preferably, "heteroaryl-" is a monocyclic aromatic ring system having 5 or 6 ring atoms and which contains at least one heteroatom, if more than one, they may be identical or different, said heteroatom being selected from oxygen, nitrogen and sulfur, a ("5- to 6-membered monocyclic heteroaryl-") group, such as, for example, a thienyl-, furanyl-, pyrrolyl-, oxazolyl-, thiazolyl-, imidazolyl-, pyrazolyl-, isoxazolyl-, isothiazolyl-, oxadiazolyl-, triazolyl-, thiadiazolyl-, tetrazolyl-, pyridyl-, pyridazinyl-, pyrimidyl-, pyrazinyl- or a triazinyl group.

In particular, in the context of the present invention, when applied to any of the substituents of the compounds of general formula **(I)** such as R², R³, R⁷ and/or R⁸, the term "heteroaryl" is to be understood as meaning preferably a monocyclic aromatic ring system having 5 or 6 ring atoms and which contains one or two heteroatoms, which may be identical or different, said heteroatom being selected from oxygen and nitrogen, *i*.*e*. a ("5- to 6-membered monocyclic heteroaryl-") group.

In general, and unless otherwise mentioned, said heteroaryl- groups include all the possible isomeric forms thereof, *e*.*g*., the positional isomers thereof. Thus, for some illustrative non-restricting example, the term pyridyl- includes pyridin-2-yl-, pyridin-3-yl- and pyridin-4-yl-; the term thienyl- includes thien-2-yl- and thien-3-yl-, and a heteroarylene group may be inserted into a chain also in the inverse way such as e.g. a 2,3-pyridinylene includes pyridine-2,3-yl as well as pyridine-3,2-yl. Furthermore, said heteroaryl- groups can be attached to the rest of the molecule via any one of the carbon atoms, or, if applicable, a nitrogen atom, *e*.*g*., a pyrrol-1-yl-, a pyrazol-1-yl- or an imidazol-1-yl- group.

Particularly, the heteroaryl group is a pyridyl- or pyrimidyl group or a imidazolyl group, including a hydroxy substitution of the pyridyl group leading, e.g., to a 2-hydroxy-pyridine which is the tautomeric form to a 2-oxo-2(1H)-pyridine. In some embodiments, the heteroaryl group is an oxazolyl group.

Further, as used herein, the term "C₃-C₈", as used throughout this text, *e*.*g*., in the context of the definition of "C₃-C₈-cycloalkyl-", is to be understood as meaning *e*.*g*. a cycloalkyl-group having a whole number of carbon atoms of 3 to 8, *i.e.,* 3, 4, 5, 6, 7 or 8 carbon atoms. It is to be understood further that said term "C₃-C₈" is to be interpreted as disclosing any sub-range comprised therein, *e*.*g*., C₃-C₆, C₄-C₅, C₃-C₅, C₃-C₄, C₄-C₆, C₅-C₇, preferably C₃-C₆.

Similarly, as used herein, the term "C₂-C₆", as used throughout this text, e.g., in the context of the definitions of "C₂-C₆-alkenyl-" and "C₂-C₆-alkynyl-", is to be understood as meaning an alkenyl- group or an alkynyl- group having a whole number of carbon atoms from 2 to 6, *i.e.,* 2, 3, 4, 5 or 6 carbon atoms. It is to be understood further that said term "C₂-C₆" is to be interpreted as disclosing any sub-range comprised therein, *e*.*g*., C₂-C₆, C₃-C₅, C₃-C₄, C₂-C₃, C₂-C₄, C₂-C₅, preferably C₂-C₃.

The term "C₁-C₆", as used throughout this text, e.g., in the context of the definition of "C₁-C₆-alkyl-", "C₁-C₆-haloalkyl-", "C₁-C₆-alkoxy-" or "C₁-C₆-haloalkoxy-" is to be understood as meaning an alkyl group having a whole number of carbon atoms from 1 to *6, i.e.,* 1, 2, 3, 4, 5 or 6 carbon atoms. It is to be understood further that said term "C₁-C₆" is to be interpreted as disclosing any sub-range comprised therein, e.g. C₁-C₆, C₂-C₅, C₃-C₄, C₁-C₂, C₁-C₃, C₁-C₄, C₁-C₅, C₁-C₆; preferably C₁-C₂, C₁-C₃, C₁-C₄, C₁-C₅, C₁-C₆; more preferably C₁-C₄ in the case of "C₁-C₆-haloalkyl-" or "C₁-C₆-haloalkoxy-" even more preferably C₁-C₂.

When a range of values is given, said range encompasses each value and sub-range within said range.

For example:
"C₁-C₆" encompasses C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆;
"C₂-C₆" encompasses C₂, C₃, C₄, C₅, C₆, C₂-C₆, C₂-C₃, C₂-C₄, C₂-C₃, C₃-C₆, C₃-C₅, C₃-C₄, C₄-C₆, C₄-C₅, and C₅-C₆;
"C₃-C₁₀" encompasses C₃, C₄, C₅, C₆, C₇, C₈, C₉, C₁₀, C₃-C₁₀, C₃-C₉, C₃-C₈, C₃-C₇, C₃-C₆, C₃-C₅, C₃-C₄, C₄-C₁₀, C₄-C₉, C₄-C₈, C₄-C₇, C₄-C₆, C₄-C₅, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₃-C₈" encompasses C₃, C₄, C₅, C₆, C₇, C₈, C₃-C₅, C₃-C₇, C₃-C₆, C₃-C₅, C₃-C₄, C₄-C₈, C₄-C₇, C₄-C₆, C₄-C₅, C₅-C₈, C₅-C₇, C₅-C₆, C₆-C₈, C₆-C₇ and C₇-C₈;
"C₃-C₆" encompasses C₃, C₄, C₅, C₆, C₃-C₆, C₃-C₅, C₃-C₄, C₄-C₆, C₄-C₅, and C₅-C₆;
"C₄-C₈" encompasses C₄, C₅, C₆, C₇, C₈, C₄-C₈, C₄-C₇, C₄-C₆, C₄-C₅, C₅-C₈, C₅-C₇, C₅-C₆, C₆-C₈, C₆-C₇ and C₇-C₈;
"C₄-C₇" encompasses C₄, C₅, C₆, C₇, C₄-C₇, C₄-C₆, C₄-C₅, C₅-C₇, C₅-C₆ and C₆-C₇;
"C₄-C₆" encompasses C₄, C₅, C₆, C₄-C₆, C₄-C₅ and C₅-C₆;
"C₅-C₁₀" encompasses C₅, C₆, C₇, C₈, C₉, C₁₀, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉ and C₉-C₁₀;
"C₆-C₁₀" encompasses C₆, C₇, C₈, C₉, C₁₀, C₆-C₁₀, C₆-C₉, C₆-C₈, C₆-C₇, C₇-C₁₀, C₇-C₉, C₇-C₈, C₈-C₁₀, C₈-C₉ and C₉-C₁₀.

In particular embodiments for the compounds of formula (I) of the present invention, reference is made to the fact that, when substituted, a particular phenyl group is preferably substituted in one or two of the ortho-positions with respect to the point of attachment of said phenyl group to the rest of the molecule. This is shown below, where the ortho-positions in which the phenyl group is preferably substituted with respect to the point of attachment to the rest of the molecule are marked (*) and where "R" denotes any possible substituents on the phenyl ring, either in the marked and preferred ortho-positions or in any other position(s):

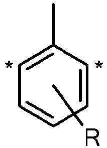

In particular, preferred embodiments of the compounds of formula (I) of the present invention may comprise a phenyl group as substituent R⁶ which is, when substituted, preferably substituted in one or two of the ortho-positions (marked with * below, where "R" denotes any possible substituents of the phenyl ring, either in the marked and preferred ortho-positions or in any other position(s)) with respect to the point of attachment of said phenyl group to the rest of the molecule:

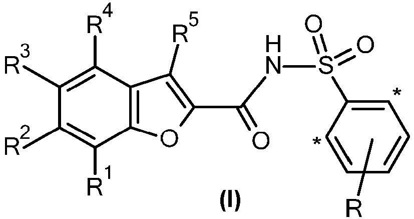

As used herein, the term "leaving group" refers to an atom or a group of atoms that is displaced in a chemical reaction as stable species taking with it the bonding electrons, e.g., typically forming an anion. Preferably, a leaving group is selected from the group comprising: halo, in particular a chloro, bromo or iodo, (methylsulfonyl)oxy-, [(4-methylphenyl)sulfonyl]oxy-, [(trifluoromethyl)sulfonyl]oxy-, [(nonafluorobutyl)sulfonyl]oxy-, [(4-bromophenyl)sulfonyl]oxy-, [(4-nitrophenyl)sulfonyl]oxy-, [(2-nitrophenyl)sulfonyl]oxy-, [(4-isopropylphenyl)sulfonyl]oxy-, [(2,4,6-triisopropylphenyl)sulfonyl]oxy-, [(2,4,6-trimethylphenyl)sulfonyl]oxy-, [(4-*tert-*butylphenyl)sulfonyl]oxy-, (phenylsulfonyl)oxy-, and a [(4-methoxyphenyl)sulfonyl]oxy group.

As used herein, the term "protective group" is a protective group attached to an oxygen or nitrogen atom in intermediates used for the preparation of compounds of the general formula (I). Such groups are introduced *e*.*g*., by chemical modification of the respective hydroxy or amino group in order to obtain chemoselectivity in a subsequent chemical reaction. Protective groups for hydroxy and amino groups are described for example in T.W. Greene and P.G.M. Wuts in Protective Groups in Organic Synthesis, 4th edition, Wiley 2006; more specifically, protective groups for amino groups can be selected from substituted sulfonyl groups, such as a mesyl-, tosyl- or a phenylsulfonyl group, acyl groups such as a benzoyl-, acetyl- or a tetrahydropyranoyl group, or carbamate based groups, such as a tert-butoxycarbonyl group (Boc). Protective groups for hydroxy groups can be selected from acyl groups such as a benzoyl-, acetyl-, pivaloyl- or a tetrahydropyranoyl group, or can include silicon, as in *e*.*g*., a tert-butyldimethylsilyl-, tert-butyldiphenylsilyl-, triethylsilyl- or a triisopropylsilyl group.

The term "substituent" refers to a group "substituted" on, e.g., an alkyl-, haloalkyl-, cycloalkyl-, heterocyclyl-, heterocycloalkenyl-, cycloalkenyl-, aryl-, or a heteroaryl group at any atom of that group, replacing one or more hydrogen atoms therein. In one aspect, the substituent(s) on a group are independently any one single, or any combination of two or more of the permissible atoms or groups of atoms delineated for that substituent. In another aspect, a substituent may itself be substituted with any one of the above substituents. Further, as used herein, the phrase "optionally substituted" means unsubstituted (e.g., substituted with an H) or substituted.

It will be understood that the description of compounds herein is limited by principles of chemical bonding known to those skilled in the art. Accordingly, where a group may be substituted by one or more of a number of substituents, such substitutions are selected so as to comply with principles of chemical bonding with regard to valencies, etc., and to give compounds which are not inherently unstable. For example, any carbon atom will be bonded to two, three, or four other atoms, consistent with the four valence electrons of carbon.

By "subject" is meant a mammal, including, but not limited to, a human or non-human mammal, such as a bovine, equine, canine, ovine, rodent, or feline.

It is possible for the compounds of general formula **(I)** to exist as isotopic variants. The invention therefore includes one or more isotopic variant(s) of the compounds of general formula **(I),** particularly deuterium-containing compounds of general formula **(I).**

The invention also includes all suitable isotopic variations of a compound of the invention.

The term "isotopic variant" of a compound or a reagent is defined as a compound exhibiting an unnatural proportion of one or more of the isotopes that constitute such a compound.

The expression "unnatural proportion" in relation to an isotope means a proportion of such isotope which is higher than its natural abundance. The natural abundances of isotopes to be applied in this context are described in "Isotopic Compositions of the Elements 1997", Pure Appl. Chem., 70(1), 217-235, 1998.

An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I and ¹³¹I, respectively. Accordingly, recitation of "hydrogen" or "H" should be understood to encompass ¹H (protium), ²H (deuterium), and ³H (tritium) unless otherwise specified. Certain isotopic variations of a compound of the invention, for example, those in which one or more radioactive isotopes such as ³H or¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

With respect to the treatment and/or prophylaxis of the disorders specified herein, the isotopic variant(s) of the compounds of general formula **(I)** preferably contain deuterium ("deuterium-containing compounds of general formula **(I)").** Isotopic variants of the compounds of general formula **(I)** in which one or more radioactive isotopes, such as ³H or ¹⁴C, are incorporated are useful, *e.g*., in drug and/or substrate tissue distribution studies. These isotopes are particularly preferred for the ease of their incorporation and detectability. Positron-emitting isotopes such as ¹⁸F or ¹¹C may be incorporated into a compound of general formula **(I).** These isotopic variants of the compounds of general formula **(I)** are useful for in vivo imaging applications. Deuterium-containing and ¹³C-containing compounds of general formula **(I)** can be used in mass spectrometry analyses in the context of preclinical or clinical studies.

Isotopic variants of the compounds of general formula **(I)** can generally be prepared by methods known to a person skilled in the art, such as those described in the schemes and/or examples herein, by substituting a reagent for an isotopic variant of said reagent, preferably for a deuterium-containing reagent. Depending on the desired sites of deuteration, in some cases deuterium from D₂O can be incorporated either directly into the compounds or into reagents that are useful for synthesizing such compounds. Deuterium gas is also a useful reagent for incorporating deuterium into molecules. Catalytic deuteration of olefinic bonds and acetylenic bonds is a rapid route for incorporation of deuterium. Metal catalysts (i.e. Pd, Pt, and Rh) in the presence of deuterium gas can be used to directly exchange deuterium for hydrogen in functional groups containing hydrocarbons. A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA.

The term "deuterium-containing compound of general formula **(I)"** is defined as a compound of general formula **(I),** in which one or more hydrogen atom(s) is/are replaced by one or more deuterium atom(s) and in which the abundance of deuterium at each deuterated position of the compound of general formula **(I)** is higher than the natural abundance of deuterium, which is about 0.015%. Particularly, in a deuterium-containing compound of general formula **(I)** the abundance of deuterium at each deuterated position of the compound of general formula **(I)** is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).

The selective incorporation of one or more deuterium atom(s) into a compound of general formula **(I)** may alter the physicochemical properties (such as for example acidity [C. L. Perrin, et al., J. Am. Chem. Soc., 2007, 129, 4490], basicity [C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula **(I).** In some cases deuterium substitution reduces or eliminates the formation of an undesired or toxic metabolite and enhances the formation of a desired metabolite (*e*.*g*., Nevirapine: A. M. Sharma et al., Chem. Res. Toxicol., 2013, 26, 410; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. ML-337 (C. J. Wenthur et al., J. Med. Chem., 2013, 56, 5208) and Odanacatib (K. Kassahun et al., WO2012/112363) are examples for this deuterium effect. Still other cases have been reported in which reduced rates of metabolism result in an increase in exposure of the drug without changing the rate of systemic clearance (*e*.*g*., Rofecoxib: F. Schneider et al., Arzneim. Forsch. / Drug. Res., 2006, 56, 295; Telaprevir: F. Maltais et al., J. Med. Chem., 2009, 52, 7993). Deuterated drugs showing this effect may have reduced dosing requirements (e.g., lower number of doses or lower dosage to achieve the desired effect) and/or may produce lower metabolite loads.

A compound of general formula **(I)** may have multiple potential sites of attack for metabolism. To optimize the above-described effects on physicochemical properties and metabolic profile, deuterium-containing compounds of general formula **(I)** having a certain pattern of one or more deuterium-hydrogen exchange(s) can be selected. Particularly, the deuterium atom(s) of deuterium-containing compound(s) of general formula **(I)** is/are attached to a carbon atom and/or is/are located at those positions of the compound of general formula **(I),** which are sites of attack for metabolizing enzymes such as e.g. cytochrome P₄₅₀.

Where the plural form of the word compounds, salts, polymorphs, hydrates, solvates and the like, is used herein, this is taken to mean also a single compound, salt, polymorph, isomer, hydrate, solvate or the like.

By "stable compound' or "stable structure" is meant a compound that is sufficiently robust to survive isolation to a useful degree of purity from a reaction mixture, and formulation into an efficacious therapeutic agent.

The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds.

Preferred compounds are those which produce the more desirable biological activity. Separated, pure or partially purified isomers and stereoisomers or racemic or diastereomeric mixtures of the compounds of the present invention are also included within the scope of the present invention. The purification and the separation of such materials can be accomplished by standard techniques known in the art.

Preferred isomers are those which produce the more desirable biological activity. These separated, pure or partially purified isomers or racemic mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e*.*g*., HPLC columns using a chiral phase), with or without conventional derivatisation, optimally chosen to maximise the separation of the enantiomers. Suitable HPLC columns using a chiral phase are commercially available, such as those manufactured by Daicel, *e*.*g*., Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.

In order to distinguish different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e*.*g*. (R)- or (S)- isomers, in any ratio. Isolation of a single stereoisomer, *e*.*g*. a single enantiomer or a single diastereomer, of a compound of the present invention is achieved by any suitable state of the art method, such as chromatography, especially chiral chromatography, for example.

Further, it may be possible for the compounds of the present invention to exist as tautomers. For example, any compound of the present invention which contains an imidazopyridine moiety as a heteroaryl group for example can exist as a 1H tautomer, or a 3H tautomer, or even a mixture in any amount of the two tautomers, namely :

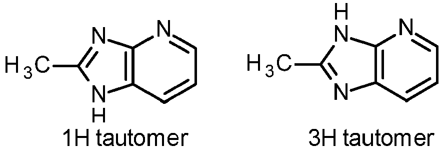

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also provides useful forms of the compounds of the present invention, such as hydrates, solvates, salts, in particular pharmaceutically acceptable salts, and/or co-precipitates.

The compounds of the present invention can exist as a hydrate, or as a solvate, wherein the compounds of the present invention contain polar solvents, in particular water, methanol or ethanol for example, as structural element of the crystal lattice of the compounds. It is possible for the amount of polar solvents, in particular water, to exist in a stoichiometric or non-stoichiometric ratio. In the case of stoichiometric solvates, *e*.*g*. a hydrate, hemi-, (semi-), mono-, sesqui-, di-, tri-, tetra-, penta- *etc.* solvates or hydrates, respectively, are possible. The present invention includes all such hydrates or solvates.

Further, it is possible for the compounds of the present invention to exist in free form, *e*.*g*. as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.

The term "pharmaceutically acceptable salt" refers to an inorganic or organic acid addition salt of a compound of the present invention. For example, see S. M. Berge, et al. "Pharmaceutical Salts," J. Pharm. Sci. 1977, 66, 1-19.

Physiologically acceptable salts of the compounds according to the invention encompass acid addition salts of mineral acids, carboxylic acids and sulfonic acids, for example salts of hydrochloric acid, hydrobromic acid, hydroiodic acid, sulfuric acid, bisulfuric acid, phosphoric acid, nitric acid or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, paratoluenesulfonic, methansulfonic, 2-naphthalenesulfonic, naphthalenedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric, or thiocyanic acid, for example.

A "pharmaceutically acceptable anion" refers to the deprotonated form of a conventional acid, such as, for example, a hydroxide, a carboxylate, a sulfate, a halide, a phosphate, or a nitrate.

Physiologically acceptable salts of the compounds according to the invention also comprise salts of conventional bases, such as, by way of example and by preference, alkali metal salts (for example lithium, sodium and potassium salts), alkaline earth metal salts (for example calcium, strontium and magnesium salts) and ammonium salts derived from ammonia or organic amines with 1 to 16 C atoms, such as, by way of example and by preference, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine, N-methylpiperidine, N-methylglucamine, dimethylglucamine, ethylglucamine, 1,6-hexadiamine, glucosamine, sarcosine, serinol, tris(hydroxymethyl)aminomethane, aminopropanediol, Sovak base, and 1-amino-2,3,4-butanetriol.

Additionally, the compounds according to the invention may form salts with a quaternary ammonium ion obtainable, e.g., by quaternisation of a basic nitrogen-containing group with agents such as lower alkylhalides such as methyl-, ethyl-, propyl-, and butylchlorides, -bromides and -iodides; dialkylsulfates such as dimethyl-, diethyl-, dibutyl- and diamylsulfates, long chain halides such as decyl-, lauryl-, myristyl- and stearylchlorides, -bromides and -iodides, aralkylhalides such as benzyl- and phenethylbromides and others. Examples of suitable quaternary ammonium ions are tetramethylammonium, tetraethylammonium, tetra(n-propyl)ammonium, tetra (*n-*butyl)ammonium, or *N*-benzyl-*N*,*N*,*N*-trimethylammonium.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.

This applies analogously to cases in which synthesis intermediates or example compounds or salts thereof have been obtained, by the preparation and/or purification processes described, as solvates, such as hydrates, with (if defined) unknown stoichiometric composition.

Unless specified otherwise, suffixes to chemical names or structural formulae relating to salts, such as "hydrochloride", "trifluoroacetate", "sodium salt", or "x HCl", "x CF₃COOH", "x Na⁺", for example, mean a salt form, the stoichiometry of which salt form not being specified.

Solvates and hydrates of disclosed intermediates or example compounds, or salts thereof, which have been obtained, by the preparation and/or purification processes described herein, may be formed in any ratio.

Furthermore, the present invention includes all possible crystalline forms, or polymorphs, of the compounds of the present invention, either as a single polymorph, or as a mixture of more than one polymorph, in any ratio.

### DESCRIPTION

### Further embodiments of the first aspect of the present invention

In accordance with further embodiments, the present invention provides compounds of general formula **(I),** *supra,* wherein:
- R¹: is selected from a hydrogen atom and a halogen atom;
- R²: is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
wherein said phenyl group is optionally substituted with one or more substituents independently selected from a halogen atom and a C₁-C₄-alkoxy group;
- R⁷ and R⁸: are each independently selected from a hydrogen atom, a C₁-C₄-alkyl group and a C₃-C₈-cycloalkyl group;
- R⁹: is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₄-alkyl group, a C₁-C₄-haloalkyl group, a C₁-C₄-alkoxy group, a (C₃-C₆-cycloalkyl)-(C₁-C₂-alkyl)-O- group, a (C₃-C₆-halocycloalkyl)-(C₁-C₂-alkyl)-O- group, a (phenyl)-(C₁-C₂-alkyl)-O- group, a C₁-C₄-haloalkoxy group, a C₃-C₆-cycloalkoxy group, a phenyl group and a (phenyl)-O- group,
wherein said phenyl group is optionally substituted with one or more halogen atoms;
- R⁷ and R⁸: are each independently selected from a hydrogen atom, a C₁-C₄-alkyl group and a C₃-C₈-cycloalkyl group;
- R⁹: is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group,a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
- R⁷ and R⁸: are each independently selected from a hydrogen atom, a C₁-C₄-alkyl group and a C₃-C₈-cycloalkyl group;
- R⁹: is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
- R³: is a hydrogen atom;
- R⁴: is selected from a hydrogen atom, a halogen atom, a C₁-C₆-alkyl group and a C₁-C₆-haloalkyl group;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
- R⁷ and R⁸: are each independently selected from a hydrogen atom, a C₁-C₄-alkyl group and a C₃-C₈-cycloalkyl group;
- R⁹: is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is a hydrogen atom;
- R²: is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
- R³: is a hydrogen atom;
- R⁴: is selected from a hydrogen atom, a halogen atom, and a C₁-C₆-haloalkyl group;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
- R⁷ and R⁸: are each independently selected from a hydrogen atom, a C₁-C₄-alkyl group and a C₃-C₈-cycloalkyl group;
- R⁹: is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is a hydrogen atom;
- R²: is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
- R³: is a hydrogen atom;
- R⁴: is a fluorine atom;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
- R⁷ and R⁸: are each independently selected from a hydrogen atom, a C₁-C₄-alkyl group and a C₃-C₈-cycloalkyl group;
- R⁹: is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a C₁-C₄-alkyl group, a C₃-C₅-cycloalkyl group, and a C₁-C₄-haloalkyl group;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
wherein said phenyl group is optionally substituted with one or more substituents independently selected from a halogen atom and a C₁-C₄-alkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a C₁-C₄-alkyl group, a C₃-C₅-cycloalkyl group, and a C₁-C₄-haloalkyl group;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N- group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₄-alkyl group, a C₁-C₄-haloalkyl group, a C₁-C₄-alkoxy group, a (C₃-C₆-cycloalkyl)-(C₁-C₂-alkyl)-O- group, a (C₃-C₆-halocycloalkyl)-(C₁-C₂-alkyl)-O- group, a (phenyl)-(C₁-C₂-alkyl)-O- group, a C₁-C₄-haloalkoxy group, a C₃-C₆-cycloalkoxy group, a phenyl group and a (phenyl)-O- group,
wherein said phenyl group is optionally substituted with one or more halogen atoms;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a C₁-C₄-alkyl group, a C₃-C₅-cycloalkyl group, and a C₁-C₄-haloalkyl group;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a C₁-C₄-alkyl group, a C₃-C₅-cycloalkyl group, and a C₁-C₄-haloalkyl group;
- R³: is a hydrogen atom;
- R⁴: is selected from a hydrogen atom, a halogen atom, a C₁-C₆-alkyl group and a C₁-C₆-haloalkyl group;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a C₁-C₄-alkyl group, a C₃-C₅-cycloalkyl group, and a C₁-C₄-haloalkyl group;
- R³: is a hydrogen atom;
- R⁴: is selected from a hydrogen atom, a halogen atom and a C₁-C₆-haloalkyl group;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is selected from a C₁-C₄-alkyl group, a C₃-C₅-cycloalkyl group, and a C₁-C₄-haloalkyl group;
- R³: is a hydrogen atom;
- R⁴: is a fluorine atom;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is a C₃-C₅-cycloalkyl group;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
wherein said phenyl group is optionally substituted with one or more substituents independently selected from a halogen atom and a C₁-C₄-alkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is a C₃-C₅-cycloalkyl group;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₄-alkyl group, a C₁-C₄-haloalkyl group, a C₁-C₄-alkoxy group, a (C₃-C₆-cycloalkyl)-(C₁-C₂-alkyl)-O- group, a (C₃-C₆-halocycloalkyl)-(C₁-C₂-alkyl)-O- group, a (phenyl)-(C₁-C₂-alkyl)-O- group, a C₁-C₄-haloalkoxy group, a C₃-C₆-cycloalkoxy group, a phenyl group and a (phenyl)-O- group,
wherein said phenyl group is optionally substituted with one or more halogen atoms;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is a C₃-C₅-cycloalkyl group;
- R³: is a hydrogen atom;
- R⁴: is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N-group and an imidazole group,
wherein said imidazole group is connected to the rest of the molecule via a nitrogen atom of said imidazole group;
- R⁵: is selected from a hydrogen atom and a methyl group;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is a C₃-C₅-cycloalkyl group;
- R³: is a hydrogen atom;
- R⁴: is selected from a hydrogen atom, a halogen atom, a C₁-C₆-alkyl group and a C₁-C₆-haloalkyl group;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is a C₃-C₅-cycloalkyl group;
- R³: is a hydrogen atom;
- R⁴: is selected from a hydrogen atom, a halogen atom and a C₁-C₆-haloalkyl group;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In accordance with further embodiments, the present invention provides compounds of general formula **(I),** *supra,* wherein:
- R¹: is selected from a hydrogen atom and a halogen atom;
- R²: is a C₃-C₅-cycloalkyl group;
- R³: is a hydrogen atom;
- R⁴: is a fluorine atom;
- R⁵: is a hydrogen atom;
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

The present invention provides the compounds of general formula (I) which are disclosed in the Example Section of this text, *infra.*

In some embodiments, the present invention includes compounds of general formula (I) selected from:
N-([1,1'-biphenyl]-2-sulfonyl)-6-(4-fluorophenyl)-1-benzofuran-2-carboxamide,
N-([1,1'-biphenyl]-2-sulfonyl)-6-methyl-1-benzofuran-2-carboxamide,
N-([1,1'-biphenyl]-2-sulfonyl)-6-cyano-1-benzofuran-2-carboxamide,
N-([1,1'-biphenyl]-2-sulfonyl)-6-(pyridin-3-yl)-1-benzofuran-2-carboxamide,
2-[([1,1'-biphenyl]-2-sulfonyl)carbamoyl]-1-benzofuran-6-carboxylic acid,
4,6-Dimethyl-N-(2'-methyl[1,1'-biphenyl]-2-sulfonyl)-1-benzofuran-2-carboxamide,
4,6-Dimethyl-N-(5-methyl[1,1'-biphenyl]-2-sulfonyl)-1-benzofuran-2-carboxamide,
N2-([1,1'-biphenyl]-2-sulfonyl)-N6,N6-dimethyl-1-benzofuran-2,6-dicarboxamide,
N-([1,1'-biphenyl]-2-sulfonyl)-4,6-dimethyl-1-benzofuran-2-carboxamide,
N-([1,1'-biphenyl]-2-sulfonyl)-4-methoxy-6-methyl-1-benzofuran-2-carboxamide,
N-(3'-chloro[1,1'-biphenyl]-2-sulfonyl)-4,6-dimethyl-1-benzofuran-2-carboxamide,
4-Methoxy-6-methyl-N-(5-methyl[1,1'-biphenyl]-2-sulfonyl)-1-benzofuran-2-carboxamide,
N-(2-Ethoxybenzene-1-sulfonyl)-4-methoxy-6-methyl-1-benzofuran-2-carboxamide,
N-([1,1'-biphenyl]-2-sulfonyl)-3,6-dimethyl-1-benzofuran-2-carboxamide,
N-([1,1'-biphenyl]-2-sulfonyl)-6-cyclopropyl-7-fluoro-1-benzofuran-2-carboxamide,
N-([1,1'-bi phenyl]-2-sulfonyl)-6-cyclopropyl-1-benzofuran-2-carboxamide,
N-([1,1'-Biphenyl]-2-sulfonyl)-6-(difluoromethyl)-1-benzofuran-2-carboxamide,
N-(2-Ethoxybenzene-1-sulfonyl)-3,6-dimethyl-1-benzofuran-2-carboxamide,
N-(2-Methylquinoline-8-sulfonyl)-6-(trifluoromethyl)-1-benzofuran-2-carboxamide,
N-([1,1'-Biphenyl]-2-sulfonyl)-6-(pyridin-4-yl)-1-benzofuran-2-carboxamide,
4,6-Dimethyl-N-(2-methylquinoline-8-sulfonyl)-1-benzofuran-2-carboxamide,
6-Cyclopropyl-4-fluoro-N-(2-methylquinoline-8-sulfonyl)-1-benzofuran-2-carboxamide,
N-([1,1'-biphenyl]-2-sulfonyl)-6-(prop-1-en-2-yl)-1-benzofuran-2-carboxamide,
6-Cyclopropyl-N-[2-(cyclopropyloxy)benzene-1-sulfonyl]-4-fluoro-1-benzofuran-2-carboxamide,
6-Cyclopropyl-N-(2-ethoxybenzene-1-sulfonyl)-4-fluoro-1-benzofuran-2-carboxamide,
N-([1,1'-biphenyl]-2-sulfonyl)-6-(2-methoxyethyl)-1-benzofuran-2-carboxamide,
N-([1,1'-biphenyl]-2-sulfonyl)-6-(butan-2-yl)-1-benzofuran-2-carboxamide,
6-Cyclopropyl-N-(2-ethoxy-3-fluoro-phenyl)sulfonyl-4-fluoro-benzofuran-2-carboxamide,
6-Cyclopropyl-N-(2-ethoxy-5-sec-butyl-phenyl)sulfonyl-4-fluoro-benzofuran-2-carboxamide,
6-Cyclopropyl-N-(2-ethoxy-4-fluoro-phenyl)sulfonyl-4-fluoro-benzofuran-2-carboxamide,
6-Cyclopropyl-N-[2-ethoxy-5-(trifluoromethyl)phenyl]sulfonyl-4-fluoro-benzofuran-2-carboxamide,
6-Cyclopropyl-N-(2-ethoxy-5-fluoro-phenyl)sulfonyl-4-fluoro-benzofuran-2-carboxamide,
6-Cyclopropyl-N-(2-ethoxy-5-phenoxy-phenyl)sulfonyl-4-fluoro-benzofuran-2-carboxamide,
6-Cyclopropyl-N-[2-ethoxy-5-(trifluoromethoxy)phenyl]sulfonyl-4-fluoro-benzofuran-2-carboxamide,
N-(2-Benzyloxy-5-isopropyl-phenyl)sulfonyl-6-cyclopropyl-4-fluoro-benzofuran-2-carboxamide,
6-Cyclopropyl-N-[2-(cyclopropylmethoxy)-5-isopropyl-phenyl]sulfonyl-4-fluoro-benzofuran-2-carboxamide,
6-Cyclopropyl-N-(2,4-dichlorophenyl)sulfonyl-4-fluoro-benzofuran-2-carboxamide,
6-Cyclopropyl-4-fluoro-N-(2-phenylphenyl)sulfonyl-benzofuran-2-carboxamide,
N-(Benzenesulfonyl)-6-cyclopropyl-4-fluoro-benzofuran-2-carboxamide,
6-Cyclopropyl-4-fluoro-N-(2-propoxyphenyl)sulfonyl-benzofuran-2-carboxamide,
6-Cyclopropyl-4-fluoro-N-[5-isopropyl-2-(2,2,2-trifluoroethoxy)phenyl]sulfonyl-benzofuran-2-carboxamide,
N-(Benzenesulfonyl)-6-cyclopropyl-4-(dimethylamino)benzofuran-2-carboxamide,
6-Cyclopropyl-4-(dimethylamino)-N-[(2-methyl-8-quinolyl)sulfonyl]benzofuran-2-carboxamide,
6-Cyclopropyl-4-(dimethylamino)-N-(2-phenylphenyl)sulfonyl-benzofuran-2-carboxamide,
6-Cyclopropyl-4-(dimethylamino)-N-(2-ethoxyphenyl)sulfonyl-benzofuran-2-carboxamide,
N-(benzenesulfonyl)-6-cyclopropyl-4-(difluoromethoxy)benzofuran-2-carboxamide,
6-Cyclopropyl-4-(difluoromethoxy)-N-[(2-methyl-8-quinolyl)sulfonyl]benzofuran-2-carboxamide,
6-Cyclopropyl-4-(difluoromethoxy)-N-(2-phenylphenyl)sulfonyl-benzofuran-2-carboxamide,
6-Cyclopropyl-4-(difluoromethoxy)-N-(2-ethoxyphenyl)sulfonyl-benzofuran-2-carboxamide,
6-Cyclopropyl-N-[(2-methyl-8-quinolyl)sulfonyl]-4-(trifluoromethyl)benzofuran-2-carboxamide,
6-Cyclopropyl-N-(2-ethoxyphenyl)sulfonyl-4-(trifluoromethyl)benzofuran-2-carboxamide,
N-(Benzenesulfonyl)-6-cyclopropyl-4-(trifluoromethyl)benzofuran-2-carboxamide,
6-Cyclopropyl-N-(2-phenylphenyl)sulfonyl-4-(trifluoromethyl)benzofuran-2-carboxamide,
N-{[1,1'-Biphenyl]-2-sulfonyl}-6-(propan-2-yl)-1-benzofuran-2-carboxamide,
N-(2-Ethoxybenzenesulfonyl)-6-(propan-2-yl)-1-benzofuran-2-carboxamide,
N-(Benzenesulfonyl)-6-(propan-2-yl)-1-benzofuran-2-carboxamide,
N-(2,4-Dichlorobenzenesulfonyl)-6-(propan-2-yl)-1-benzofuran-2-carboxamide,
6-Cyclopropyl-N-(2-ethoxyphenyl)sulfonyl-4-imidazol-1-yl-benzofuran-2-carboxamide,
N-((5-(tert-butyl)-2-cyclopropoxyphenyl)sulfonyl)-6-cyclopropyl-4-fluorobenzofuran-2-carboxamide,
N-[(2-(Benzyloxy)-5-(tert-butyl)phenyl)sulfonyl]-6-cyclopropyl-4-fluorobenzofuran-2-carboxamide,
N-((5-(tert-butyl)-2-(cyclopropylmethoxy)phenyl)sulfonyl)-6-cyclopropyl-4-fluorobenzofuran-2-carboxamide,
N-((5-(tert-butyl)-2-(2,2,2-trifluoroethoxy)phenyl)sulfonyl)-6-cyclopropyl-4-fluorobenzofuran-2-carboxamide,
N-((5-(tert-Butyl)-2-cyclobutoxyphenyl)sulfonyl)-6-cyclopropyl-4-fluorobenzofuran-2-carboxamide,
N-((5-(tert-Butyl)-2-isopropoxyphenyl)sulfonyl)-6-cyclopropyl-4-fluorobenzofuran-2-carboxamide,
N-[(2-Cyclobutoxy-5-isopropylphenyl)sulfonyl]-6-cyclopropyl-4-fluorobenzofuran-2-carboxamide,
N-((5-(tert-butyl)-2-methoxyphenyl)sulfonyl)-6-cyclopropyl-4-fluorobenzofuran-2-carboxamide,
6-Cyclopropyl-N-{(2-[(2,2-difluorocyclopropyl)methoxy)-5-isopropylphenyl]sulfonyl}-4-fluorobenzofuran-2-carboxamide,
6-Cyclopropyl-N-[(2-ethoxy-4,5-difluorophenyl)sulfonyl]-4-fluorobenzofuran-2-carboxamide,
6-Cyclopropyl-N-[(5-cyclopropyl-2-methoxyphenyl)sulfonyl]-4-fluorobenzofuran-2-carboxamide,
6-Cyclopropyl-N-((5-cyclopropyl-2-ethoxyphenyl)sulfonyl)-4-fluorobenzofuran-2-carboxamide,
6-Cyclopropyl-4-fluoro-N-{[2-methoxy-5-(2-oxopropyl)phenyl]sulfonyl}benzofuran-2-carboxamide,
6-Cyclopropyl-N-(2-ethoxy-5-isopropyl-phenyl)sulfonyl-4-fluoro-benzofuran-2-carboxamide and
N-(2-chlorobenzenesulfonyl)-6-(propan-2-yl)-1-benzofuran-2-carboxamide.

In some embodiments, the present invention includes compounds of general formula **(I)** selected from:
6-Cyclopropyl-N-[2-(cyclopropyloxy)benzene-1-sulfonyl]-4-fluoro-1-benzofuran-2-carboxamide,
6-Cyclopropyl-4-fluoro-N-(2-methylquinoline-8-sulfonyl)-1-benzofuran-2-carboxamide, 6-Cyclopropyl-N-(2-ethoxy-5-sec-butyl-phenyl)sulfonyl-4-fluoro-benzofuran-2-carboxamide,
6-Cyclopropyl-N-(2-ethoxy-4-fluoro-phenyl)sulfonyl-4-fluoro-benzofuran-2-carboxamide,
6-Cyclopropyl-N-[2-ethoxy-5-(trifluoromethyl)phenyl]sulfonyl-4-fluoro-benzofuran-2-carboxamide,
N-(2-Benzyloxy-5-isopropyl-phenyl)sulfonyl-6-cyclopropyl-4-fluoro-benzofuran-2-carboxamide,
6-Cyclopropyl-N-[2-(cyclopropylmethoxy)-5-isopropyl-phenyl]sulfonyl-4-fluoro-benzofuran-2-carboxamide,
6-Cyclopropyl-4-fluoro-N-[5-isopropyl-2-(2,2,2-trifluoroethoxy)phenyl]sulfonyl-benzofuran-2-carboxamide,
N-[(2-(Benzyloxy)-5-(tert-butyl)phenyl)sulfonyl]-6-cyclopropyl-4-fluorobenzofuran-2-carboxamide,
N-((5-(tert-butyl)-2-(cyclopropylmethoxy)phenyl)sulfonyl)-6-cyclopropyl-4-fluorobenzofuran-2-carboxamide,
N-((5-(tert-butyl)-2-(2,2,2-trifluoroethoxy)phenyl)sulfonyl)-6-cyclopropyl-4-fluorobenzofuran-2-carboxamide,
N-((5-(tert-Butyl)-2-cyclobutoxyphenyl)sulfonyl)-6-cyclopropyl-4-fluorobenzofuran-2-carboxamide,
N-((5-(tert-Butyl)-2-isopropoxyphenyl)sulfonyl)-6-cyclopropyl-4-fluorobenzofuran-2-carboxamide,
N-[(2-Cyclobutoxy-5-isopropylphenyl)sulfonyl]-6-cyclopropyl-4-fluorobenzofuran-2-carboxamide,
N-((5-(tert-butyl)-2-methoxyphenyl)sulfonyl)-6-cyclopropyl-4-fluorobenzofuran-2-carboxamide,
6-Cyclopropyl-N-{(2-[(2,2-difluorocyclopropyl)methoxy)-5-isopropylphenyl]sulfonyl}-4-fluorobenzofuran-2-carboxamide and
6-Cyclopropyl-N-(2-ethoxy-5-isopropyl-phenyl)sulfonyl-4-fluoro-benzofuran-2-carboxamide.

In some embodiments, the present invention includes compounds of general formula **(I)** selected from:
N-(3'-chloro[1,1'-biphenyl]-2-sulfonyl)-4,6-dimethyl-1-benzofuran-2-carboxamide, 4-methoxy-6-methyl-N-(5-methyl[1,1'-biphenyl]-2-sulfonyl)-1-benzofuran-2-carboxamide,
N-(2-Ethoxybenzene-1-sulfonyl)-4-methoxy-6-methyl-1-benzofuran-2-carboxamide,
N-([1,1'-biphenyl]-2-sulfonyl)-3,6-dimethyl-1-benzofuran-2-carboxamide,
N-([1,1'-biphenyl]-2-sulfonyl)-6-cyclopropyl-1-benzofuran-2-carboxamide,
N-(2-Ethoxybenzene-1-sulfonyl)-3,6-dimethyl-1-benzofuran-2-carboxamide,
N-(2-Methylquinoline-8-sulfonyl)-6-(trifluoromethyl)-1-benzofuran-2-carboxamide,
4,6-Dimethyl-N-(2-methylquinoline-8-sulfonyl)-1-benzofuran-2-carboxamide,
6-Cyclopropyl-N-(2-ethoxybenzene-1-sulfonyl)-4-fluoro-1-benzofuran-2-carboxamide,
6-Cyclopropyl-N-(2-ethoxy-3-fluoro-phenyl)sulfonyl-4-fluoro-benzofuran-2-carboxamide,
6-Cyclopropyl-N-(2-ethoxy-5-fluoro-phenyl)sulfonyl-4-fluoro-benzofuran-2-carboxamide,
6-Cyclopropyl-N-(2-ethoxy-5-phenoxy-phenyl)sulfonyl-4-fluoro-benzofuran-2-carboxamide,
6-Cyclopropyl-N-[2-ethoxy-5-(trifluoromethoxy)phenyl]sulfonyl-4-fluoro-benzofuran-2-carboxamide,
6-Cyclopropyl-4-fluoro-N-(2-phenylphenyl)sulfonyl-benzofuran-2-carboxamide,
N-(Benzenesulfonyl)-6-cyclopropyl-4-fluoro-benzofuran-2-carboxamide,
6-Cyclopropyl-4-fluoro-N-(2-propoxyphenyl)sulfonyl-benzofuran-2-carboxamide,
6-Cyclopropyl-4-(difluoromethoxy)-N-[(2-methyl-8-quinolyl)sulfonyl]benzofuran-2-carboxamide,
6-Cyclopropyl-4-(difluoromethoxy)-N-(2-phenylphenyl)sulfonyl-benzofuran-2-carboxamide,
6-Cyclopropyl-N-[(2-methyl-8-quinolyl)sulfonyl]-4-(trifluoromethyl)benzofuran-2-carboxamide,
6-Cyclopropyl-N-(2-ethoxyphenyl)sulfonyl-4-(trifluoromethyl)benzofuran-2-carboxamide,
6-Cyclopropyl-N-(2-phenylphenyl)sulfonyl-4-(trifluoromethyl)benzofuran-2-carboxamide,
N-{[1,1'-Biphenyl]-2-sulfonyl}-6-(propan-2-yl)-1-benzofuran-2-carboxamide,
N-(2-Ethoxybenzenesulfonyl)-6-(propan-2-yl)-1-benzofuran-2-carboxamide,
4,6-Dimethyl-N-(2'-methyl[1,1'-biphenyl]-2-sulfonyl)-1-benzofuran-2-carboxamide,
4,6-Dimethyl-N-(5-methyl[1,1'-biphenyl]-2-sulfonyl)-1-benzofuran-2-carboxamide and
N-([1,1'-biphenyl]-2-sulfonyl)-4,6-dimethyl-1-benzofuran-2-carboxamide.

In some embodiments, the present invention includes compounds of general formula **(I)** selected from:
N-([1,1'-biphenyl]-2-sulfonyl)-6-(4-fluorophenyl)-1-benzofuran-2-carboxamide,
N-([1,1'-biphenyl]-2-sulfonyl)-4-methoxy-6-methyl-1-benzofuran-2-carboxamide,
N-([1,1'-biphenyl]-2-sulfonyl)-6-cyclopropyl-7-fluoro-1-benzofuran-2-carboxamide,
N-([1,1'-Biphenyl]-2-sulfonyl)-6-(difluoromethyl)-1-benzofuran-2-carboxamide,
N-([1,1'-biphenyl]-2-sulfonyl)-6-methyl-1-benzofuran-2-carboxamide,
N-([1,1'-Biphenyl]-2-sulfonyl)-6-(pyridin-4-yl)-1-benzofuran-2-carboxamide,
N-([1,1'-biphenyl]-2-sulfonyl)-6-(prop-1-en-2-yl)-1-benzofuran-2-carboxamide,
N-([1,1'-biphenyl]-2-sulfonyl)-6-(2-methoxyethyl)-1-benzofuran-2-carboxamide,
N-([1,1'-biphenyl]-2-sulfonyl)-6-(butan-2-yl)-1-benzofuran-2-carboxamide,
N-([1,1'-biphenyl]-2-sulfonyl)-6-cyano-1-benzofuran-2-carboxamide,
6-Cyclopropyl-N-(2,4-dichlorophenyl)sulfonyl-4-fluoro-benzofuran-2-carboxamide,
N-([1,1'-biphenyl]-2-sulfonyl)-6-(pyridin-3-yl)-1-benzofuran-2-carboxamide,
N-(Benzenesulfonyl)-6-cyclopropyl-4-(dimethylamino)benzofuran-2-carboxamide,
6-Cyclopropyl-4-(dimethylamino)-N-[(2-methyl-8-quinolyl)sulfonyl]benzofuran-2-carboxamide,
6-Cyclopropyl-4-(dimethylamino)-N-(2-phenylphenyl)sulfonyl-benzofuran-2-carboxamide,
6-Cyclopropyl-4-(dimethylamino)-N-(2-ethoxyphenyl)sulfonyl-benzofuran-2-carboxamide,
N-(benzenesulfonyl)-6-cyclopropyl-4-(difluoromethoxy)benzofuran-2-carboxamide,
6-Cyclopropyl-4-(difluoromethoxy)-N-(2-ethoxyphenyl)sulfonyl-benzofuran-2-carboxamide,
2-[([1,1'-biphenyl]-2-sulfonyl)carbamoyl]-1-benzofuran-6-carboxylic acid,
N-(Benzenesulfonyl)-6-cyclopropyl-4-(trifluoromethyl)benzofuran-2-carboxamide,
N-(Benzenesulfonyl)-6-(propan-2-yl)-1-benzofuran-2-carboxamide,
N-(2,4-Dichlorobenzenesulfonyl)-6-(propan-2-yl)-1-benzofuran-2-carboxamide,
6-Cyclopropyl-N-(2-ethoxyphenyl)sulfonyl-4-imidazol-1-yl-benzofuran-2-carboxamide,
N-(2-chlorobenzenesulfonyl)-6-(propan-2-yl)-1-benzofuran-2-carboxamide and
N2-([1,1'-biphenyl]-2-sulfonyl)-N6,N6-dimethyl-1-benzofuran-2,6-dicarboxamide.

### Further embodiments of the first aspect of the present invention:

In some embodiments, the present invention provides compounds of formula **(I),** *supra,* in which R¹ is selected from a hydrogen atom and a halogen atom or a tautomer, or an N-oxide, or a salt thereof, or a salt of a tautomer, or a salt of an N-oxide, or a mixture of same.

In some embodiments, the present invention provides compounds of formula (I), *supra,* in which R¹ is a hydrogen atom or a tautomer, or an N-oxide, or a salt thereof, or a salt of a tautomer, or a salt of an N-oxide, or a mixture of same.

In some embodiments, the present invention provides compounds of formula (I), *supra,* in which R¹ is a halogen atom or a tautomer, or an N-oxide, or a salt thereof, or a salt of a tautomer, or a salt of an N-oxide, or a mixture of same.

In some embodiments, the present invention provides compounds of formula (I), *supra,* in which R¹ is a fluorine atom or a tautomer, or an N-oxide, or a salt thereof, or a salt of a tautomer, or a salt of an N-oxide, or a mixture of same.

In some embodiments, the present invention provides compounds of formula (I), *supra,* in which R² is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
or a tautomer, or an N-oxide, or a salt thereof, or a salt of a tautomer, or a salt of an N-oxide, or a mixture of same.

In some embodiments, the present invention provides compounds of formula (I), *supra,* in which R² is selected from a C₁-C₄-alkyl group, a C₃-C₅-cycloalkyl group, and a C₁-C₄-haloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R² is a C₃-C₅-cycloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R² is a cyclopropyl group or a tautomer, or an N-oxide, or a salt thereof, or a salt of a tautomer, or a salt of an N-oxide, or a mixture of same.

In some embodiments, the present invention provides compounds of formula (I), *supra,* in which R³ is a hydrogen atom or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N- group and an imidazole group,
wherein said imidazole group is connected to the rest of the molecule via a nitrogen atom of said imidazole group;
or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is selected from a hydrogen atom, a halogen atom, a C₁-C₆-alkyl group, a C₁-C₆-haloalkyl group, a C₁-C₆-alkoxy group and a C₁-C₆-haloalkoxy group or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is selected from a hydrogen atom, a halogen atom, a C₁-C₆-alkyl group and a C₁-C₆-haloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is selected from a hydrogen atom, a halogen atom and a C₁-C₆-haloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is selected from a halogen atom and a C₁-C₆-haloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is selected from a halogen atom and a C₁-C₄-haloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is a halogen atom or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is a fluorine atom or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is a hydrogen atom or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is a C₁-C₄-haloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is a C₁-haloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁴ is a trifluoromethyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁵ is selected from a hydrogen atom and a methyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁵ is a hydrogen atom or a tautomer, or an N-oxide, or a salt thereof, or a salt of a tautomer, or a salt of an N-oxide, or a mixture of same.

In some embodiments, the present invention provides compounds of formula (I), *supra,* in which R⁵ is a methyl group or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a C₁-C₆-alkyl group, a phenyl group, a naphthyl group, a heteroaryl group, a (phenyl)-(C₁-C₆-alkyl)- group, a (naphthyl)-(C₁-C₆-alkyl)-group, a (heteroaryl)-(C₁-C₆-alkyl)- group, a (heterocycloalkyl)-(C₁-C₆-alkyl)-group and a heterocycloalkyl group,
wherein said heterocycloalkyl group is connected to the rest of the molecule via a carbon atom of said heterocycloalkyl group,
wherein said phenyl, naphthyl, heteroaryl or heterocycloalkyl group is each optionally substituted with one or more substituents independently selected from a halogen atom, a cyano group, a nitro group, a hydroxy group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a (C₁-C₃-alkyl)-O-(C₁-C₂-alkyl)- group, a (C₁-C₃-alkyl)-O-(C₁-C₂-alkyl)-O-group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O-group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a C₁-C₆-thioalkyl group, a heterocycloalkyl group, a R⁷R⁸N- group, a (C₁-C₂-alkyl)-S(=O)₂- group, a (R⁷R⁸N)-S(=O)₂- group, a (R⁷R⁸N)-(C₁-C₆-alkyl)-(C=O)-NH- group, a (H₃C)-(C=O)- group, a (R⁷R⁸N)-(C=O)- group, a (R⁷R⁸N)-(C=O)-O- group, a (R⁷R⁸N)-(C₁-C₆-alkyl)- group, a R⁹OOC-(C₁-C₃-alkyl)- group, a R⁹OOC- group, a phenyl group, a naphthyl group, a (phenyl)-O- group and a heteroaryl group,
   wherein said heterocycloalkyl group is connected to the rest of the molecule via a carbon atom of said heterocycloalkyl group,
   wherein said phenyl, naphthyl, heteroaryl or heterocycloalkyl group is each optionally substituted with one or more substituents independently selected from a halogen atom, a cyano group, a nitro group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a heterocycloalkyl group, a phenyl group, a R⁷R⁸N- group, a (R⁷R⁸N)-S(=O)₂- group, a (R⁷R⁸N)-(C₁-C₆-alkyl)-(C=O)-NH- group, a (R⁷R⁸N)-(C₁-C₆-alkyl)- group, and a R⁹OOC- group,
      wherein said heterocycloalkyl group is connected to the rest of the molecule via a carbon atom of said heterocycloalkyl group;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
wherein said phenyl group is optionally substituted with one or more substituents independently selected from a halogen atom and a C₁-C₄-alkoxy group;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
and wherein, when substituted, said phenyl group is preferably substituted in one or two of the ortho-positions with respect to the point of attachment of said phenyl group to the rest of the molecule,
   wherein said phenyl group is optionally substituted with one or more substituents independently selected from a halogen atom and a C₁-C₄-alkoxy group;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
and wherein, when substituted, said phenyl group is preferably substituted with a C₁-C₄-alkoxy group in one or two of the ortho-positions with respect to the point of attachment of said phenyl group to the rest of the molecule,
   wherein said phenyl group is optionally substituted with one or more substituents independently selected from a halogen atom and a C₁-C₄-alkoxy group;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₄-alkyl group, a C₁-C₄-haloalkyl group, a C₁-C₄-alkoxy group, a (C₃-C₆-cycloalkyl)-(C₁-C₂-alkyl)-O- group, a (C₃-C₆-halocycloalkyl)-(C₁-C₂-alkyl)-O- group, a (phenyl)-(C₁-C₂-alkyl)-O- group, a C₁-C₄-haloalkoxy group, a C₃-C₆-cycloalkoxy group, a phenyl group and a (phenyl)-O- group,
wherein said phenyl group is optionally substituted with one or more halogen atoms;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₄-alkyl group, a C₁-C₄-haloalkyl group, a C₁-C₄-alkoxy group, a (C₃-C₆-cycloalkyl)-(C₁-C₂-alkyl)-O- group, a (C₃-C₆-halocycloalkyl)-(C₁-C₂-alkyl)-O- group, a (phenyl)-(C₁-C₂-alkyl)-O- group, a C₁-C₄-haloalkoxy group, a C₃-C₆-cycloalkoxy group, a phenyl group and a (phenyl)-O- group,
and wherein, when substituted, said phenyl group is preferably substituted in one or two of the ortho-positions with respect to the point of attachment of said phenyl group to the rest of the molecule,
   wherein said phenyl group is optionally substituted with one or more halogen atoms;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₄-alkyl group, a C₁-C₄-haloalkyl group, a C₁-C₄-alkoxy group, a (C₃-C₆-cycloalkyl)-(C₁-C₂-alkyl)-O- group, a (C₃-C₆-halocycloalkyl)-(C₁-C₂-alkyl)-O- group, a (phenyl)-(C₁-C₂-alkyl)-O- group, a C₁-C₄-haloalkoxy group, a C₃-C₆-cycloalkoxy group, a phenyl group and a (phenyl)-O- group,
and wherein, when substituted, said phenyl group is preferably substituted with a C₁-C₄-alkoxy group in one or two of the ortho-positions with respect to the point of attachment of said phenyl group to the rest of the molecule,
   wherein said phenyl group is optionally substituted with one or more halogen atoms;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
and wherein, when substituted, said phenyl group is preferably substituted in one or two of the ortho-positions with respect to the point of attachment of said phenyl group to the rest of the molecule;
or a tautomer, or an N-oxide, or a salt thereof, or a salt of a tautomer, or a salt of an N-oxide, or a mixture of same.

In some embodiments, the present invention provides compounds of formula (I), *supra,* in which
- R⁶: is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
and wherein, when substituted, said phenyl group is preferably substituted with a C₁-C₄-alkoxy group in one or two of the ortho-positions with respect to the point of attachment of said phenyl group to the rest of the molecule;
or a tautomer, or an N-oxide, or a salt thereof, or a salt of a tautomer, or a salt of an N-oxide, or a mixture of same.

In some embodiments, the present invention provides compounds of formula (I), *supra,* in which
R⁷ and R⁸ are each independently selected from a hydrogen atom, a C₁-C₆-alkyl group, a C₃-C₈-cycloalkyl group, a C₁-C₆-haloalkyl group, a HC(=O)- group, a (C₁-C₆-alkyl)C(=O)- group, a (C₁-C₆-haloalkyl)C(=O)- group, a (C₃₋C₈-cycloalkyl)-(C₁-C₆₋alkyl)- group, a C₁-C₆-hydroxyalkyl group, a (C₁-C₆-alkoxy)-(C₁-C₆-alkyl)- group, a (phenyl)-(S=O)₂- group, a (C₁-C₆-alkyl)-(S=O)₂- group, a (C₁-C₆-haloalkyl)-(S=O)₂- group, a phenyl group, a heterocycloalkyl group and a heteroaryl group,
   wherein said heterocycloalkyl group is connected to the rest of the molecule via a carbon atom of said heterocycloalkyl group,
   wherein said phenyl, heteroaryl or heterocycloalkyl group is each optionally substituted with one or more substituents independently selected from a halogen atom, a cyano group, a nitro group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a C₁-C₆-thioalkyl group, a heterocycloalkyl group, and a R⁹OOC- group,
      wherein said heterocycloalkyl group is connected to the rest of the molecule via a carbon atom of said heterocycloalkyl group;
or R⁷ and R⁸ together with the nitrogen atom to which they are attached form a 4- to 7-membered nitrogen-containing heterocycloalkyl group, wherein said heterocycloalkyl group optionally contains one, two or three further heteroatoms independently selected from nitrogen, oxygen and sulfur and/or is optionally substituted with one or more substituents independently selected from a halogen atom, a cyano group, a nitro group, a C₁-C₆-alkyl group, a C₁-C₆-hydroxyalkyl group, a C₁-C₆-alkylsulfanyl group, a C₁-C₆-haloalkyl group, a C₁-C₆-alkoxy group, a C₁-C₆-haloalkoxy group, a C₂-C₆-alkenyl group, a C₂-C₆-alkynyl group, a C₃-C₈-cycloalkyl group, a C₄-C₈-cycloalkenyl group, a C₃-C₈-cycloalkoxy group, a C₁-C₆-thioalkyl group, a heterocycloalkyl group and a R⁹OOC-group,
wherein said heterocycloalkyl group is connected to the rest of the molecule via a carbon atom of said heterocycloalkyl group;
or a tautomer, or an N-oxide, or a salt thereof, or a salt of a tautomer, or a salt of an N-oxide, or a mixture of same.

In some embodiments, the present invention provides compounds of formula (I), *supra,* in which R⁷ and R⁸ are each independently selected from a hydrogen atom, a C₁-C₄-alkyl group and a C₃-C₈-cycloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁷ and R⁸ are each independently selected from a hydrogen atom and a C₁-C₄-alkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁷ and R⁸ are each independently selected from a hydrogen atom and a methyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁹ is selected from a hydrogen atom, a C₁-C₃-alkyl group and a C₃-C₅-cycloalkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁹ is selected from a hydrogen atom and a C₁-C₃-alkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁹ is a hydrogen atom or a tautomer, or an N-oxide, or a salt thereof, or a salt of a tautomer, or a salt of an N-oxide, or a mixture of same.

In some embodiments, the present invention provides compounds of formula (I), *supra,* in which R⁹ is a C₁-C₃-alkyl group or a tautomer, or an N-oxide, or a salt thereof, or a salt of a tautomer, or a salt of an N-oxide, or a mixture of same.

In some embodiments, the present invention provides compounds of formula (I), *supra,* in which R⁹ is a methyl group or a tautomer, or an N-oxide, or a salt thereof, or a salt of a tautomer, or a salt of an N-oxide, or a mixture of same.

In further embodiments, the present invention includes compounds of formula (I), or a tautomer, an N-oxide, or a salt thereof, or a salt of a tautomer or an N-oxide, or a mixture of same.

In further embodiments, the present invention includes compounds of formula (I), or a salt thereof.

In further embodiments, the present invention includes compounds of formula (I), or a tautomer, or a salt thereof, or a salt of a tautomer, or a mixture of same.

In further embodiments, the present invention includes compounds of formula (I), which are a salt.

In further embodiments, the present invention includes compounds of formula (I), which are a tautomer or a salt thereof, or a salt of a tautomer, or a mixture of same.

In further embodiments, the present invention includes compounds of formula (I), which are an N-oxide, or a salt thereof, or a salt of an N-oxide, or a mixture of same.

In further embodiments of the first aspect, the present invention provides combinations of two or more of the above mentioned embodiments under the heading "further embodiments of the first aspect of the present invention".

Furthermore it is understood that the invention includes any subcombination of the disclosed single embodiments herein for certain residues or subcombination of residues of formula (I).

The present invention includes any sub-combination within any embodiments or aspects of the present invention of compounds of general formula **(I)**, *supra.*

The present invention includes any sub-combination within any embodiments or aspects of the present invention of compounds of general formula **(I)** or intermediate compounds. The present invention includes the compounds of general formula **(I)** which are disclosed in the Example Section of this text, *infra.*

### General synthesis of compounds of general formula (I) of the present invention

The compounds of general formula **(I)** according to the invention as well as relevant intermediate and/or precursor compounds can be prepared according to the following schemes 1, 2, 3, 4 and 5. The schemes and procedures described below illustrate synthetic routes to the compounds of general formula **(I)** of the invention as well as to relevant intermediate and/or precursor compounds and are not intended to be limiting. It is clear to the person skilled in the art that the order of transformations as exemplified in schemes 1, 2, 3, 4 and 5 can be modified in various ways. The order of transformations exemplified in these schemes is therefore not intended to be limiting. In addition, interconversion of any of the substituents, R¹, R², R³, R⁴, R⁵, and R⁶ can often be achieved before and/or after the exemplified transformations. These modifications can include the introduction of protecting groups, cleavage of protecting groups, reduction or oxidation of functional groups, halogenation, metallation, substitution or other suitable reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs and in the Experimental Section.

Scheme 1 describes one possible route for the preparation of compounds of general formula **(I)**, in which R¹, R², R³, R⁴, R⁵ and R⁶ have the meaning as given for the compounds of general formula **(I)**, *supra.*

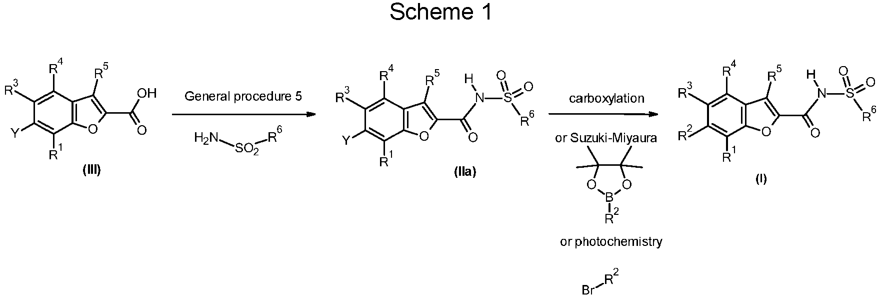

As depicted in Scheme 1, compounds of general formula **(I)** can be synthesized *via* either carboxylation, Suzuki-Miyaura cross-coupling or photochemistry of a derivative of formula **(IIa)**, wherein R¹, R², R³, R⁴, R⁵ and R⁶ have the meaning as given for general formula **(I)**, *supra* and Y is a halogen atom, such as *e.g.* fluorine, chlorine, bromine or iodine, preferably chlorine, bromine or iodine, more preferably bromine. See, for example, Chem. Rev. 1995, 95, 7, 2457-2483.

In turn, derivatives of formula **(IIa)** can be prepared *via* the coupling of an intermediate of formula **(III)** with a primary sulfonamide of formula H₂N-S(=O)₂-R⁶, wherein R⁶ has the meaning as given for the compounds of general formula **(I)**, *supra.* For the coupling of sulfonamides in the context of the present invention see, for example: Org. Proc. Res. Dev., 2009, 13, 255-262; Angew. Chem. Int. Ed. 2018, 57, 3488-3492. Specific examples are described in the Experimental Section.

Depending *inter alia* on the nature of the substituents R¹, R², R³, R⁴, R⁵ and R⁶, compounds of general formula **(I)** can also be prepared via intermediates of formula (**IIb**), in which R² is introduced before the coupling with a primary sulfonamide of formula H₂N-S(=O)₂-R⁶, wherein R⁶ has the meaning as given for the compounds of general formula **(I)**, *supra* (Scheme 2). Said compounds of formula (**IIb**) are either commercially available or can be prepared *via* either carboxylation, Suzuki-Miyaura cross-coupling or photochemistry starting from compounds of formula (**III**), wherein Y is a halogen atom, such as e.g. fluorine, chlorine, bromine or iodine, preferably chlorine, bromine or iodine, more preferably bromine. See, for example, Chem. Rev. 1995, 95, 7, 2457-2483. Specific examples are described in the Experimental Section.

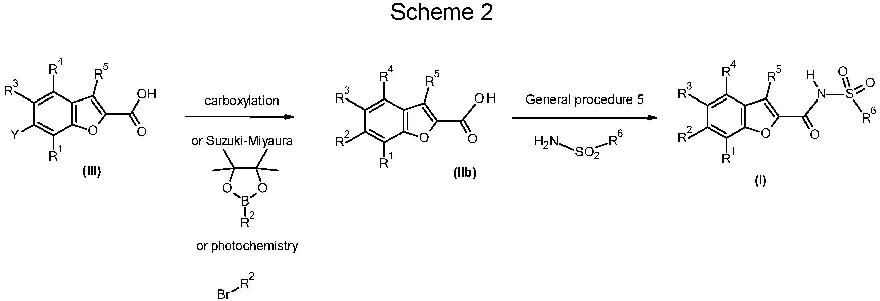

Depending inter alia on the nature of the substituents R¹, R², R³, R⁴, R⁵ and R⁶, compounds of formula (**IIb**) can be prepared as described herein via cyclopropanation using a Zinc organometallic compound of formula R²-Zn-Br and starting from compounds of formula (III), wherein R² has the meaning as given for the compounds of general formula (**I**), *supra* and wherein Y is a halogen atom, such as e.g. chlorine, bromine or iodine, preferably bromine or iodine, more preferably bromine (Scheme 3). The compounds of formula (**IIb**) can then be coupled with a primary sulfonamide of formula H2N-S(=O)₂-R⁶, wherein R⁶ has the meaning as given for the compounds of general formula (**I**), *supra,* to furnish the compounds of formula (I) as described herein.

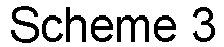

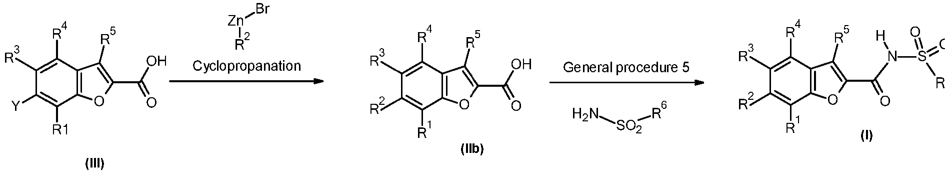

In general, the synthesis of the compounds of formula (**I**) of the present invention is preferably carried out following the sequence depicted in Scheme 2.

The compounds of formula (**III**) are either commercially available or can be prepared according to procedures available from the public domain, as understandable to the person skilled in the art. Specific examples are described in the Experimental Section.

For example, the benzofurane derivatives of formula (**III**) in which R¹, R³, R⁴ and R⁵ have the meaning as given for the compounds of general formula (**I**), *supra* and Y is a halogen atom, such as e.g. fluorine, chlorine, bromine or iodine, preferably chlorine, bromine or iodine, more preferably chlorine or bromine can be prepared via the reaction between a hydroxybenzaldehyde of formula (**IV**) and for example ethyl chloro- or bromoacetate or any other suitable reagent of similar nature (Scheme 4). It can happen that mixtures of the free carboxylic acid and the corresponding ester are obtained. In this case, a saponification step might be necessary, which can be carried out using standard methods known in the art. For the cyclization reactions described herein, see, for example: Chem. Biol. Drug. Des., 2018, 92, 1497-1503; J. of enzyme inh. Med. Chem. 2018, 33, 1, 1212-1224. Specific examples are described in the Experimental Section.

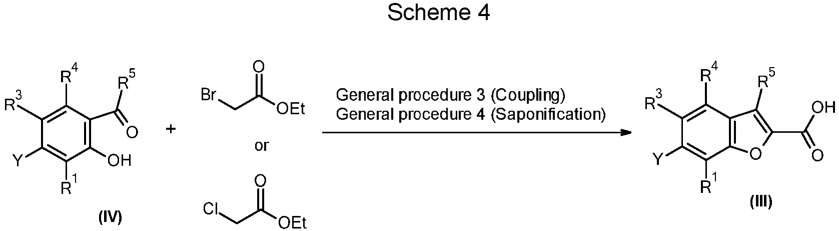

The sulfonamides of formula H₂N-S(=O)₂-R⁶ are either commercially available or can be prepared according to procedures available from the public domain, as understandable to the person skilled in the art. Specific examples are described in the Experimental Section.

For example, suitable sulfonamides comprising a (phenyl)-(phenyl)- moiety can be prepared via the sequence depicted in Scheme 5, comprising the protection of an aryl sulfonamide with 1,1-dimethoxy-*N,N-*dimethylmethanamine, a coupling reaction with a suitable boronic acid derivative and optional deprotection, if necessary. The phenyl moieties can each be individually unsubstituted or substituted as defined for the compounds of general formula **(I),** *supra.* [B] indicates a boron-containing functionality suitable for the carbon-carbon coupling of the two phenyl moieties, e.g. a group -B(OR^{B})₂ wherein OR^{B} is a substituent that, when attached to the boron atom, can be used as coupling partner in a Suzuki reaction. For the reaction of aryl sulfonamides with 1,1-dimethoxy-*N,N*-dimethylmethanamine see, for example: Organic Preparations and Procedures International 2002, 34(5), 545-549; WO2005/26158; Green Chemistry 2013, 15(8), 2294-2301. Specific examples are described in the Experimental Section.

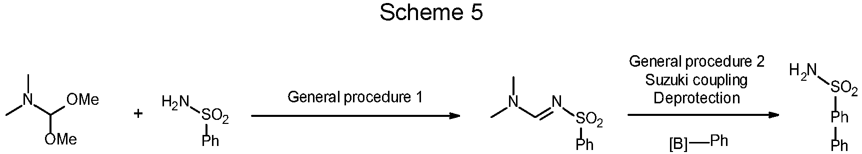

Further, for the compounds of formula **(I)** wherein R⁴ is as defined for the compounds of general formula **(I)**, *supra* but not a hydrogen atom, the corresponding substituent can be already be incorporated in one of the starting materials or if necessary it can be introduced at different stages during the reaction sequence leading to the compounds of formula **(I)**. This can be achieved using methods, reagents and conditions well-known to the person skilled in the art. Specific examples are described in the Experimental Section.

In accordance with a second aspect, the present invention provides methods of preparing compounds of general formula (**I**) as defined *supra,* said method comprising the reaction of an intermediate compound of formula (**IIa**) 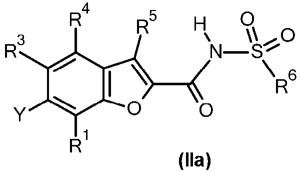 with an R²-containing coupling partner suitable for C-C bond formation, preferably wherein the coupling partner is a boron-containing compound, a Zinc organometallic compound or an alkyl halide, wherein R¹, R², R³, R⁴, R⁵ and R⁶ are as defined for the compounds of general formula (**I**) *supra,* or a tautomer, or an N-oxide, or a salt thereof, or a salt of a tautomer, or a salt of an N-oxide, or a mixture of same and Y is a halogen atom selected from fluorine, chlorine, bromine or iodine, preferably chlorine, bromine or iodine, more preferably chlorine or bromine, thereby giving a compound of general formula (**I**) 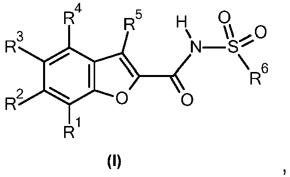 wherein R¹, R², R³, R⁴, R⁵ and R⁶ are as defined *supra,* or a tautomer, or an N-oxide, or a salt thereof, or a salt of a tautomer, or a salt of an N-oxide, or a mixture of same.

Further, the present invention provides intermediate compounds of general formula (**IIa**): 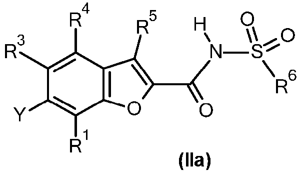 wherein R¹, R³, R⁴, R⁵ and R⁶ are as defined for the compounds of general formula (**I**) *supra* supra and Y is a halogen atom selected from chlorine and bromine or a tautomer, or an N-oxide, or a salt thereof, or a salt of a tautomer, or a salt of an N-oxide, or a mixture of same.

Particularly, the present invention provides the use of intermediate compounds of general formula (**IIa**) : 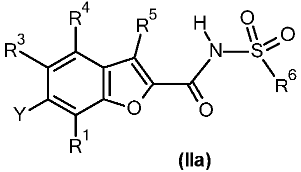 wherein R¹, R³, R⁴, R⁵ and R⁶ are as defined for the compounds of general formula (**I**) *supra,* or a tautomer, or an N-oxide, or a salt thereof, or a salt of a tautomer, or a salt of an N-oxide, or a mixture of same and Y is a halogen atom selected from fluorine, chlorine, bromine or iodine, preferably chlorine, bromine or iodine, more preferably chlorine or bromine, for the preparation of a compound of general formula (**I**) as defined supra.

In accordance with a third aspect, the present invention provides methods of preparing compounds of general formula (**I**) as defined *supra,* said method comprising the reaction of an intermediate compound of formula (**IIb**) 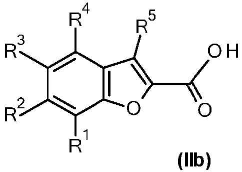 with a sulphonamide of formula H₂N-SO₂-R⁶, wherein R¹, R², R³, R⁴, R⁵ and R⁶ are as defined for the compounds of general formula (**I**) *supra,* or a tautomer, or an N-oxide, or a salt thereof, or a salt of a tautomer, or a salt of an N-oxide, or a mixture of same, thereby giving a compound of general formula (I) 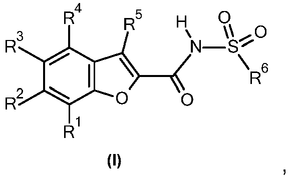 wherein R¹, R², R³, R⁴, R⁵ and R⁶ are as defined *supra,* or a tautomer, or an N-oxide, or a salt thereof, or a salt of a tautomer, or a salt of an N-oxide, or a mixture of same.

Further, the present invention provides intermediate compounds of general formula (**IIb**): 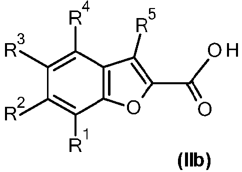 wherein R¹, R², R³, R⁴, R⁵ and R⁶ are as defined for the compounds of general formula (**I**) *supra* or a tautomer, or an N-oxide, or a salt thereof, or a salt of a tautomer, or a salt of an N-oxide, or a mixture of same.

Particularly, the present invention provides the use of intermediate compounds of general formula (**IIb**) : 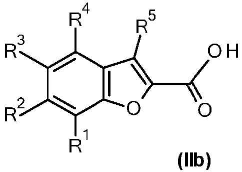 wherein R¹, R², R³, R⁴, R⁵ and R⁶ are as defined for the compounds of general formula (I) *supra,* or a tautomer, or an N-oxide, or a salt thereof, or a salt of a tautomer, or a salt of an N-oxide, or a mixture of same, for the preparation of a compound of general formula (I) as defined supra.

The present invention includes the intermediate compounds which are disclosed in the Example Section of this text, infra.

The present invention includes any sub-combination within any embodiments or aspects of the present invention of intermediate compounds of general formula (**II**), of general formula (**IIa**), of general formula (**IIb**), of general formula (**III**), of general formula (**IV**), *supra.*

The compounds of general formula **(I)** of the present invention can be converted to any salt, preferably pharmaceutically acceptable salts, by any method which is known to the person skilled in the art. Similarly, any salt of a compound of general formula **(I)** of the present invention can be converted into the free compound, by any method which is known to the person skilled in the art.

Compounds of general formula **(I)** of the present invention demonstrate a valuable pharmacological spectrum of action which could not have been predicted. The compounds of the present invention effectively inhibit the activity of lysine acetyl transferase 6A (KAT6A) and/or lysine acetyl transferase 6B (KAT6B) for which data are given in the biological experimental section and may therefore be used for the treatment or prophylaxis of hyperproliferative disorders, such as cancer disorders in humans and animals.

### Methods and administration

Compounds of general formula **(I)** of the present invention demonstrate a valuable pharmacological spectrum of action and pharmacokinetic profile, both of which could not have been predicted. Compounds of the present invention have surprisingly been found to effectively inhibit the activity of lysine acetyl transferase 6A (KAT6A) and lysine acetyl transferase 6B (KAT6B), and it is possible therefore that said compounds can be used for the treatment or prophylaxis of diseases, preferably hyperproliferative disorders in humans and animals.

As used herein, "prophylaxis" includes a use of the compound that, in a statistical sample, reduces the occurrence of the disorder or condition in the treated sample relative to an untreated control sample, or delays the onset or reduces the severity of one or more symptoms of the disorder or condition relative to the untreated control sample, when administered to prior to the onset of the disorder or condition.

Compounds of the present invention can be utilized to inhibit, block, reduce, decrease, *etc.,* cell proliferation and/or cell division, and/or produce apoptosis, which are all types of "treatment". This method comprises administering to a mammal in need thereof, including a human, an amount of a compound of general formula **(I)** of the present invention, or a pharmaceutically acceptable salt, isomer, polymorph, metabolite, hydrate, solvate or ester thereof, which is effective to treat the disorder.

Hyperproliferative disorders include, but are not limited to, for example: psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include multiple myeloma, lymphomas, sarcomas, and leukaemias.

Examples of breast cancers include, but are not limited to, invasive ductal carcinoma, invasive lobular carcinoma, ductal carcinoma *in situ,* and lobular carcinoma *in situ.*

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalamic glioma, cerebellar and cerebral astrocytoma, medulloblastoma, ependymoma, as well as neuroectodermal and pineal tumour.

Tumours of the male reproductive organs include, but are not limited to, prostate and testicular cancer.

Tumours of the female reproductive organs include, but are not limited to, endometrial, cervical, ovarian, vaginal, and vulvar cancer, as well as sarcoma of the uterus.

Tumours of the digestive tract include, but are not limited to, anal, colon, colorectal, oesophageal, gallbladder, gastric, pancreatic, rectal, small-intestine, and salivary gland cancers.

Tumours of the urinary tract include, but are not limited to, bladder, penile, kidney, renal pelvis, ureter, urethral and human papillary renal cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, basal cell carcinoma, squamous cell carcinoma, Kaposi's sarcoma, malignant melanoma, Merkel cell skin cancer and non-melanoma skin cancer.

Head-and-neck cancers include, but are not limited to, laryngeal, hypopharyngeal, nasopharyngeal, oropharyngeal cancer, lip and oral cavity cancer and squamous cell.

Lymphomas include, but are not limited to, AIDS-related lymphoma, chronic lymphocytic lymphoma (CLL), non-Hodgkin's lymphoma (NHL), T-non-Hodgkin lymphoma (T-NHL), subtypes of NHL such as Diffuse Large Cell Lymphoma (DLBCL), activated B-cell DLBCL, germinal center B-cell lymphoma DLBCL, double-hit lymphoma and double-expressor lymphoma; anaplastic large cell lymphoma, B-cell lymphoma, cutaneous T-cell lymphoma, Burkitt's lymphoma, follicular lymphoma, hairy cell lymphoma, Hodgkin's disease, mantle cell lymphoma (MCL), lymphoma of the central nervous system, small lymphocytic lymphoma and chronic lymphocytic lymphoma and Sezary syndrome.

Sarcomas include, but are not limited to, sarcoma of the soft tissue, osteosarcoma, malignant fibrous histiocytoma, lymphosarcoma, and rhabdomyosarcoma.

Leukemias include, but are not limited to acute lymphoblastic leukemia, acute myeloid leukemia, (acute) T-cell leukemia, acute lymphoblastic leukemia, acute lymphocytic leukemia (ALL) , acute monocytic leukemia (AML), acute promyelocytic leukemia (APL), bisphenotypic B myelomonocytic leukemia, chronic lymphocytic leukemia (CLL), chronic myelogenous leukemia, chronic myeloid leukemia (CML), chronic myelomonocytic leukemia (CMML), large granular lymphocytic leukemia, plasma cell leukemia and also myelodysplastic syndrome (MDS), which can develop into an acute myeloid leukemia.

The present invention also provides methods of treating angiogenic disorders including diseases associated with excessive and/or abnormal angiogenesis.

Inappropriate and ectopic expression of angiogenesis can be deleterious to an organism. A number of pathological conditions are associated with the growth of extraneous blood vessels. These include, for example, diabetic retinopathy, ischemic retinal-vein occlusion, and retinopathy of prematurity [Aiello et al., New Engl. J. Med., 1994, 331, 1480; Peer et al., Lab. Invest., 1995, 72, 638], age-related macular degeneration (AMD) [Lopez et al., Invest. Ophthalmol. Vis. Sci., 1996, 37, 855], neovascular glaucoma, psoriasis, retrolental fibroplasias, angiofibroma, inflammation, rheumatoid arthritis (RA), restenosis, in-stent restenosis, vascular graft restenosis, *etc.* In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, compounds of general formula **(I)** of the present invention can be utilized to treat and/or prevent any of the aforementioned angiogenesis disorders, for example by inhibiting and/or reducing blood vessel formation; by inhibiting, blocking, reducing, decreasing, *etc.* endothelial cell proliferation, or other types involved in angiogenesis, as well as causing cell death or apoptosis of such cell types.

These disorders have been well characterized in humans, but also exist with a similar etiology in other mammals, and can be treated by administering pharmaceutical compositions of the present invention.

The term "treating" or "treatment" as stated throughout this document is used conventionally, for example the management or care of a subject for the purpose of combating, alleviating, reducing, relieving and/or improving the condition of a disease or disorder, such as a carcinoma.

The compounds of the present invention can be used in particular in therapy and prevention, *i.e.* prophylaxis, of tumour growth and metastases, especially in solid tumours of all indications and stages with or without pre-treatment of the tumour growth.

Generally, the use of chemotherapeutic agents and/or anti-cancer agents in combination with a compound or pharmaceutical composition of the present invention will serve to:
1. yield better efficacy in reducing the growth of a tumour or even eliminate the tumour as compared to administration of either agent alone,
2. provide for the administration of lesser amounts of the administered chemotherapeutic agents,
3. provide for a chemotherapeutic treatment that is well tolerated in the patient with fewer deleterious pharmacological complications than observed with single agent chemotherapies and certain other combined therapies,
4. provide for treating a broader spectrum of different cancer types in mammals, especially humans,
5. provide for a higher response rate among treated patients,
6. provide for a longer survival time among treated patients compared to standard chemotherapy treatments,
7. provide a longer time for tumour progression, and/or
8. yield efficacy and tolerability results at least as good as those of the agents used alone, compared to known instances where other cancer agent combinations produce antagonistic effects.

In addition, the compounds of general formula **(I)** of the present invention can also be used in combination with radiotherapy and/or surgical intervention.

In a further embodiment of the present invention, the compounds of general formula **(I)** of the present invention may be used to sensitize a cell to radiation, *i.e.* treatment of a cell with a compound of the present invention prior to radiation treatment of the cell renders the cell more susceptible to DNA damage and cell death than the cell would be in the absence of any treatment with a compound of the present invention. In one aspect, the cell is treated with at least one compound of general formula **(I)** of the present invention.

Thus, the present invention also provides a method of killing a cell, wherein a cell is administered one or more compounds of the present invention in combination with conventional radiation therapy.

The present invention also provides a method of rendering a cell more susceptible to cell death, wherein the cell is treated with one or more compounds of general formula **(I)** of the present invention prior to the treatment of the cell to cause or induce cell death. In one aspect, after the cell is treated with one or more compounds of general formula **(I)** of the present invention, the cell is treated with at least one compound, or at least one method, or a combination thereof, in order to cause DNA damage for the purpose of inhibiting the function of the cell or killing the cell.

In other embodiments of the present invention, a cell is killed by treating the cell with at least one DNA damaging agent, *i.e.* after treating a cell with one or more compounds of general formula **(I)** of the present invention to sensitize the cell to cell death, the cell is treated with at least one DNA damaging agent to kill the cell. DNA damaging agents useful in the present invention include, but are not limited to, chemotherapeutic agents (*e.g.* cis platin), ionizing radiation (X-rays, ultraviolet radiation), carcinogenic agents, and mutagenic agents.

In other embodiments, a cell is killed by treating the cell with at least one method to cause or induce DNA damage. Such methods include, but are not limited to, activation of a cell signalling pathway that results in DNA damage when the pathway is activated, inhibiting of a cell signalling pathway that results in DNA damage when the pathway is inhibited, and inducing a biochemical change in a cell, wherein the change results in DNA damage. By way of a non-limiting example, a DNA repair pathway in a cell can be inhibited, thereby preventing the repair of DNA damage and resulting in an abnormal accumulation of DNA damage in a cell.

In some embodiments, a compound of general formula **(I)** of the present invention is administered to a cell prior to the radiation or other induction of DNA damage in the cell. In some embodiments of the invention, a compound of general formula **(I)** of the present invention is administered to a cell concomitantly with the radiation or other induction of DNA damage in the cell. In yet some embodiments of the invention, a compound of general formula **(I)** of the present invention is administered to a cell after radiation or other induction of DNA damage in the cell has begun. In yet some embodiments of the invention, a compound of general formula **(I)** of the present invention is administered to a cell immediately after radiation or other induction of DNA damage in the cell has begun.

In some embodiments, the cell is *in vitro.* In another embodiment, the cell is *in vivo.*

Thus in some embodiments, the present invention includes a method of inhibiting proliferation of a cell and/or the induction of apoptosis in a cell, comprising contacting the cell with a compound of formula **(I)**.

Another aspect of the invention is a method for treating, preventing or prophylaxing cancer (i.e. a method for the treatment, prevention or prophylaxis of cancer) in a subject (e.g., human, other mammal, such as rat, etc.) by administering an effective amount of at least one compound of general formula **(I),** or a pharmaceutically acceptable salt, polymorph, metabolite, hydrate, solvate or ester thereof to the subject.

In some embodiments, the subject may be administered a medicament, comprising at least one compound of general formula **(I)** and one or more pharmaceutically acceptable carriers, excipients and/or diluents.

Furthermore in some embodiments, the present invention includes a method of using a compound of general formula **(I)** for the treatment of diseases.

Particularly in some embodiments, the present invention includes a method of treating a hyperproliferative disease, more particularly cancer, comprising administering an effective amount of at least one compound of general formula **(I)** to a subject in need thereof.

In some embodiments, the method of treatment and/or prophylaxis of a hyperproliferative disorder in a subject may comprise administering to the subject an effective amount of a compound of general formula **(I)**. The hyperproliferative disorder may be, for example, cancer (e.g., lung cancer, acute myeloid leukemia, lymphoma, glioblastoma, prostate cancer, etc.).

Furthermore in some embodiments, the present invention includes a method of treating cancer, particularly lymphoma, non-Hodgkin-lymphoma type, diffuse large B-cell lymphoma subtype, acute leukemia, acute myeloid leukemia type, multiple myeloma, ovarian cancer, comprising administering an effective amount of at least one compound of formula **(I)** to a subject in need thereof.

Furthermore in some embodiments, the present invention includes a method of treating cancer, particularly multiple myeloma, ovarian carcinoma, acute monocytic leukemia, melanoma and lung cancer, comprising administering an effective amount of at least one compound of formula **(I)** to a subject in need thereof.

Furthermore in some embodiments, the present invention includes a method of treating cancer, particularly breast cancer; lung cancer; lymphoma including non-Hodgkin-lymphoma type, diffuse large B-cell lymphoma subtype including GC-DLBCL* and ABC-DLBCL** subtypes, and mantle cell lymphoma; acute leukemia, acute myeloid leukemia type, acute monocytic leukemia; melanoma; multiple myeloma; ovarian cancer; and pancreas cancer, comprising administering an effective amount of at least one compound of formula **(I)** to a subject in need thereof. GC-DLBCL means Germinal B-cell Diffuse Large B-Cell Lymphoma and ** ABC-DLBCL means Activated B-cell Diffuse Large B-Cell Lymphoma.

Furthermore in some embodiments, the present invention includes a method of treating cancer, particularly breast cancer, lung cancer, diffuse large B-cell lymphoma subtype including GC-DLBCL* and ABC-DLBCL** subtypes, mantle cell lymphoma, acute monocytic leukemia, melanoma, ovarian cancer, and pancreas cancer comprising administering an effective amount of at least one compound of formula **(I)** to a subject in need thereof. Furthermore in some embodiments, the present invention provides a compound of formula **(I)** for use of treating diseases.

Furthermore in some embodiments, the present invention includes a method of treating cancer, particularly breast cancer; lymphoma, leukemia, multiple myeloma; and ovarian cancer, comprising administering an effective amount of at least one compound of formula **(I)** to a subject in need thereof.

Furthermore in some embodiments, the present invention includes a method of treating cancer, particularly lymphoma, non-Hodgkin-lymphoma type, diffuse large B-cell lymphoma subtype, acute leukemia, acute myeloid leukemia type, multiple myeloma, and ovarian cancer, comprising administering an effective amount of at least one compound of formula **(I)** to a subject in need thereof.

Furthermore in some embodiments, the present invention includes a method of treating cancer, particularly breast cancer, lymphoma (including non-Hodgkin-lymphoma type, diffuse large B-cell lymphoma subtype, mantle cell lymphoma), leukemia (including acute monocytic leukemia), liver cancer, multiple myeloma, melanoma, non-small cell lung cancer, small cell lung cancer, ovarian cancer, ovarian carcinoma, stomach cancer, and squamous cell carcinoma, comprising administering an effective amount of at least one compound of formula **(I)** to a subject in need thereof.

Furthermore in some embodiments, the present invention includes a method of treating cancer, particularly breast cancer, diffuse large B-cell lymphoma subtype, mantle cell lymphoma, acute monocytic leukemia, liver cancer, multiple myeloma, melanoma, non-small cell lung cancer, small cell lung cancer, ovarian cancer, ovarian carcinoma, prostate cancer, stomach cancer, and squamous cell carcinoma, comprising administering an effective amount of at least one compound of formula **(I)** to a subject in need thereof.

Furthermore in some embodiments, the present invention includes a method of treating cancer, particularly bladder cancer, bone cancer, brain cancer, breast cancer, colon cancer (colorectal cancer), endometrial (uterine) cancer, gastric cancer, head and neck cancer, kidney cancer, leukemia, liver cancer, lung cancer, lymphoma, lung cancer, myeloma, neuroblastoma, ovarian cancer, pancreatic cancer, rhabdoid tumor, sarcoma and skin cancer, comprising administering an effective amount of at least one compound of formula **(I)** to a subject in need thereof.

Furthermore in some embodiments, the present invention includes a method of treating cancer, particularly lung cancer, breast cancer, bladder cancer, uterine cancer, endometrial cancer, prostate cancer and leukemia comprising administering an effective amount of at least one compound of formula **(I)** to a subject in need thereof.

Furthermore in some embodiments, the present invention includes a method of treating cancer, particularly cancer in which KAT6A and/or KAT6B is focally amplified, said method comprising administering an effective amount of at least one compound of formula **(I)** to a subject in need thereof.

Furthermore in some embodiments, the present invention includes a method of treating cancer, particularly cancer in which KAT6A is focally amplified, said method comprising administering an effective amount of at least one compound of formula **(I)** to a subject in need thereof.

Furthermore in some embodiments, the present invention includes a method of treating cancer, particularly cancer in which KAT6B is focally amplified, said method comprising administering an effective amount of at least one compound of formula **(I)** to a subject in need thereof.

Furthermore in some embodiments, the present invention includes a method of treating cancer, particularly breast cancer, lung cancer, endometrial cancer, ovarian cancer, bladder cancer, esophageal cancer, uterine cancer and epithelial cancer comprising administering an effective amount of at least one compound of formula **(I)** to a subject in need thereof.

Furthermore in some embodiments, the present invention includes a method of treating cancer, particularly breast cancer, lung cancer, endometrial cancer, ovarian cancer, bladder cancer and esophageal cancer comprising administering an effective amount of at least one compound of formula **(I)** to a subject in need thereof.

Furthermore in some embodiments, the present invention includes a method of treating cancer, particularly breast cancer, uterine cancer and epithelial cancer comprising administering an effective amount of at least one compound of formula **(I)** to a subject in need thereof.

In accordance with some embodiments, the present invention provides compounds of general formula **(I),** as described *supra,* or stereoisomers, tautomers, N-oxides, hydrates, solvates, and salts thereof, particularly pharmaceutically acceptable salts thereof, or mixtures of same, for use in the treatment or prophylaxis of diseases, in particular hyperproliferative disorders.

Furthermore in accordance with a further aspect, the present invention provides a compound of formula **(I)** for use of treating diseases.

In in accordance with a further aspect, the present invention includes a compound of general formula **(I)** for use in a method of inhibiting proliferation of a cell and/or the induction of apoptosis in a cell, comprising contacting the cell with a compound of formula **(I)**.

Particularly in some embodiments, the present invention includes compounds of general formula **(I)** for use in a method of treating a hyperproliferative disease, more particularly wherein the hyperproliferative disease is cancer, and yet even more particularly wherein the cancer disease is selected from lymphoma, non-Hodgkin-lymphoma type, diffuse large B-cell lymphoma subtype, ovarian cancer, multiple myeloma, acute leukemia, and acute myeloid leukemia.

More particularly in some embodiments, the present invention includes compounds of general formula **(I)** for use in a method of treating a hyperproliferative disease, more particularly wherein the hyperproliferative disease is cancer, and yet even more particularly wherein the cancer disease is selected from breast cancer; lymphoma, leukemia, multiple myeloma; and ovarian cancer.

Particularly in some embodiments, the present invention includes compounds of general formula **(I)** for use in a method of treating a hyperproliferative disease, more particularly wherein the hyperproliferative disease is cancer, and yet even more particularly wherein the cancer is selected from breast cancer; esophageal cancer; liver cancer; lung cancer; lymphoma including non-Hodgkin-lymphoma type, diffuse large B-cell lymphoma subtype including GC-DLBCL* and ABC-DLBCL** subtypes, and mantle cell lymphoma; acute leukemia, acute myeloid leukemia type, acute monocytic leukemia; melanoma; multiple myeloma; melanoma; ovarian cancer; or pancreas cancer.

More particularly in some embodiments, the present invention includes compounds of general formula **(I)** for use in a method of treating cancer wherein the cancer disease is selected from breast cancer; lymphoma, leukemia, multiple myeloma; and ovarian cancer.

More particularly in some embodiments, the present invention includes compounds of general formula **(I)** for use in a method of treating cancer wherein the cancer disease is selected from lung cancer, breast cancer, bladder cancer, uterine cancer, endometrial cancer, prostate cancer and leukemia.

More particularly in some embodiments, the present invention includes compounds of general formula **(I)** for use in a method of treating cancer in which KAT6A and/or KAT6B is focally amplified.

More particularly in some embodiments, the present invention includes compounds of general formula **(I)** for use in a method of treating cancer in which KAT6A is focally amplified.

More particularly in some embodiments, the present invention includes compounds of general formula **(I)** for use in a method of treating cancer in which KAT6B is focally amplified.

More particularly in some embodiments, the present invention includes compounds of general formula **(I)** for use in a method of treating cancer wherein the cancer disease is selected from breast cancer, lung cancer, endometrial cancer, ovarian cancer, bladder cancer, esophageal cancer, uterine cancer and epithelial cancer.

More particularly in some embodiments, the present invention includes compounds of general formula **(I)** for use in a method of treating cancer wherein the cancer disease is selected from breast cancer, lung cancer, endometrial cancer, ovarian cancer, bladder cancer and esophageal cancer.

More particularly in some embodiments, the present invention includes compounds of general formula **(I)** for use in a method of treating cancer wherein the cancer disease is selected from breast cancer, uterine cancer and epithelial cancer.

In some embodiments, the present invention includes the use of the compounds of general formula **(I)** for the manufacture of a medicament for the treatment and/or prophylaxis of a hyperproliferative disease.

In some embodiments, the present invention includes the use of the compounds of general formula **(I)** for the manufacture of a medicament for the treatment and/or prophylaxis of a hyperproliferative disease, wherein the hyperproliferative disease is cancer.

In some embodiments, the present invention includes the use of the compounds of general formula **(I)** for the manufacture of a medicament for the treatment of a hyperproliferative disease, particularly cancer and more particularly lymphoma, non-Hodgkin-lymphoma type, diffuse large B-cell lymphoma subtype, ovarian cancer, multiple myeloma, acute leukemia, and acute myeloid leukemia type.

In some embodiments, the present invention includes the use of the compounds of general formula **(I)** for the manufacture of a medicament for the treatment of a hyperproliferative disease, particularly cancer and more particularly lung cancer, breast cancer, bladder cancer, uterine cancer, endometrial cancer, prostate cancer and leukemia.

In some embodiments, the present invention includes the use of the compounds of general formula **(I)** for the manufacture of a medicament for the treatment of a hyperproliferative disease, particularly cancer and more particularly cancer in which KAT6A and/or KAT6B is focally amplified.

In some embodiments, the present invention includes the use of the compounds of general formula **(I)** for the manufacture of a medicament for the treatment of a hyperproliferative disease, particularly cancer and more particularly cancer in which KAT6A is focally amplified.

In some embodiments, the present invention includes the use of the compounds of general formula **(I)** for the manufacture of a medicament for the treatment of a hyperproliferative disease, particularly cancer and more particularly cancer in which KAT6B is focally amplified.

In some embodiments, the present invention includes the use of the compounds of general formula **(I)** for the manufacture of a medicament for the treatment of a hyperproliferative disease, particularly cancer and more particularly breast cancer, lung cancer, endometrial cancer, ovarian cancer, bladder cancer, esophageal cancer, uterine cancer and epithelial cancer.

In some embodiments, the present invention includes the use of the compounds of general formula **(I)** for the manufacture of a medicament for the treatment of a hyperproliferative disease, particularly cancer and more particularly breast cancer, lung cancer, endometrial cancer, ovarian cancer, bladder cancer and esophageal cancer.

In some embodiments, the present invention includes the use of the compounds of general formula **(I)** for the manufacture of a medicament for the treatment of a hyperproliferative disease, particularly cancer and more particularly breast cancer, uterine cancer and epithelial cancer.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly lung cancer, breast cancer, bladder cancer, uterine cancer, endometrial cancer, prostate cancer and leukemia.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly cancer in which KAT6A and/or KAT6B is focally amplified.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly cancer in which KAT6A is focally amplified.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly cancer in which KAT6B is focally amplified.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly breast cancer, lung cancer, endometrial cancer, ovarian cancer, bladder cancer, esophageal cancer, uterine cancer and epithelial cancer.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly breast cancer, lung cancer, endometrial cancer, ovarian cancer, bladder cancer and esophageal cancer.

In some embodiments, the present invention provides use of a compound of general formula **(I),** as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular hyperproliferative disorders, particularly cancer, more particularly breast cancer, uterine cancer and epithelial cancer.

In some embodiments, the present invention provides a method of treatment or prophylaxis of diseases, in particular hyperproliferative disorders, particularly cancer, comprising administering an effective amount of a compound of general formula **(I)**, as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides a method of treatment or prophylaxis of diseases, in particular hyperproliferative disorders, particularly cancer, more particularly lung cancer, breast cancer, bladder cancer, uterine cancer, endometrial cancer, prostate cancer and leukemia comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides a method of treatment or prophylaxis of diseases, in particular hyperproliferative disorders, particularly cancer, more particularly cancer in which KAT6A and/or KAT6B is focally amplified comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides a method of treatment or prophylaxis of diseases, in particular hyperproliferative disorders, particularly cancer, more particularly cancer in which KAT6A is focally amplified comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides a method of treatment or prophylaxis of diseases, in particular hyperproliferative disorders, particularly cancer, more particularly cancer in which KAT6B is focally amplified comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides a method of treatment or prophylaxis of diseases, in particular hyperproliferative disorders, particularly cancer, more particularly breast cancer, lung cancer, endometrial cancer, ovarian cancer, bladder cancer, esophageal cancer, uterine cancer and epithelial cancer comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides a method of treatment or prophylaxis of diseases, in particular hyperproliferative disorders, particularly cancer, more particularly breast cancer, lung cancer, endometrial cancer, ovarian cancer, bladder cancer and esophageal cancer comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides a method of treatment or prophylaxis of diseases, in particular hyperproliferative disorders, particularly cancer, more particularly breast cancer, uterine cancer and epithelial cancer comprising administering an effective amount of a compound of general formula **(I),** as described *supra,* or stereoisomers, tautomers, N-oxides, hydrates, solvates, and salts thereof, particularly pharmaceutically acceptable salts thereof, or mixtures of same to a subject in need thereof.

In some embodiments, the present invention provides pharmaceutical compositions, in particular a medicament, comprising a compound of general formula **(I),** as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.

The present invention furthermore provides pharmaceutical compositions, in particular medicaments, which comprise at least one compound according to the invention, conventionally together with one or more pharmaceutically suitable excipients, and to their use for the above mentioned purposes.

It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette^{®}), sorbitan fatty acid esters (such as, for example, Span^{®}), polyoxyethylene sorbitan fatty acid esters (such as, for example, Tween^{®}), polyoxyethylene fatty acid glycerides (such as, for example, Cremophor^{®}), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®})),
- capsule materials (for example gelatine, hydroxypropylmethylcellulose),
- synthetic polymers (for example polylactides, polyglycolides, polyacrylates, polymethacrylates (such as, for example, Eudragit^{®}), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprise at least one compound according to the invention, conventionally together with one or more pharmaceutically suitable excipient(s), and to their use according to the present invention.

In some embodiments, the present invention provides pharmaceutical combinations, in particular medicaments, comprising at least one compound of general formula **(I)** of the present invention and at least one or more further active ingredients, in particular for the treatment and/or prophylaxis of a hyperproliferative disorder, particularly cancer.

Particularly, the present invention provides a pharmaceutical combination, which comprises:
- one or more first active ingredients, in particular compounds of general formula (I) as defined *supra,* and
- one or more further active ingredients, in particular for the treatment and/or prophylaxis of a hyperproliferative disorder, particularly cancer.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula **(I)** of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

The compounds of the present invention can be administered as the sole pharmaceutical agent or in combination with one or more other pharmaceutically active ingredients where the combination causes no unacceptable adverse effects. The present invention also provides such pharmaceutical combinations. For example, the compounds of the present invention can be combined with known anti-cancer agents.

Examples of anti-cancer agents include:
131I-chTNT, abarelix, abemaciclib, abiraterone, acalabrutinib, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, apalutamide, aprepitant, arcitumomab, arglabin, arsenic trioxide, asparaginase, atezolizumab, avelumab, axicabtagene ciloleucel, axitinib, azacitidine, basiliximab, belotecan, bendamustine, besilesomab, belinostat, bevacizumab, bexarotene, bicalutamide, bisantrene, bleomycin, blinatumomab, bortezomib, bosutinib, buserelin, brentuximab vedotin, brigatinib, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, durvalumab, eculizumab, edrecolomab, elliptinium acetate, elotuzumab, eltrombopag, enasidenib, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, 1-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, inotuzumab ozogamicin, interferon alfa, interferon beta, interferon gamma, iobitridol, iobenguane (123I), iomeprol, ipilimumab, irinotecan, ltraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, lutetium Lu 177 dotatate, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, midostaurin, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, mvasi, nabilone, nabiximols, nafarelin, naloxone + pentazocine, naltrexone, nartograstim, necitumumab, nedaplatin, nelarabine, neratinib, neridronic acid, netupitant/palonosetron, nivolumab, pentetreotide, nilotinib, nilutamide, nimorazole, nimotuzumab, nimustine, nintedanib, niraparib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib , regorafenib, ribociclib, risedronic acid, rhenium-186 etidronate, rituximab, rolapitant, romidepsin, romiplostim, romurtide, rucaparib, samarium (153Sm) lexidronam, sargramostim, sarilumab, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tisagenlecleucel, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib , valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid and zorubicin.

Based upon standard laboratory techniques known to evaluate compounds useful for the treatment of hyperproliferative disorders, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### EXPERIMENTAL SECTION

### General remarks:

In the text, the chemical names and the numbers of the compounds are given in bold. The intermediates are defined by INT-.

Chemical names were generated using the ACD/Name software from ACD/Labs. In some cases generally accepted names of commercially available reagents were used in place of ACD/Name generated names.

If the purity of the obtained example product is not mentioned, the compounds are 90 to 100% pure.

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1: Abbreviations**

| The following table lists the abbreviations used herein. | |
|---|---|
| **Abbreviation** | **Meaning** |
| h | hour(s) |
| rt | room temperature |
| aq. | aqueous |
| sat. | saturated |
| HCl | hydrochloric acid |
| NaHCO₃ | sodium bicarbonate |
| DMF | N,N-dimethylformamide |
| DMSO | dimethyl sulfoxide |
| THF | tetrahydrofuran |
| DIPEA | N,N-diisopropylamine |
| PyBOP | [(1H-benzotriazol-1-yl)oxy]tri(pyrrolidin-1-yl)phosphanium hexafluoridophosphate(1-) |
| XPhos | 2-dicyclohexylphosphino-2',4',6'-triisopropylbiphenyl |
| XPhos Pd G3 | (2-dicyclohexylphosphino-2',4',6'-triisopropyl-1,1'-biphenyl)[2-(2'-amino-1,1'-biphenyl)]palladium(II) methanesulfonate |
| [Ir(dF(CF3)ppy2)2(dtbbpy)]PF6 | [4,4'-bis(1,1-dimethylethyl)-2,2'-bipyridine-N1,N1']bis[3,5-difluoro-2-[5-(trifluoromethyl)-2-pyridinyl-N]phenyl-C]lridium(III) hexafluorophosphate |
| TTMS | 1,1,1,3,3,3-hexamethyl-2-trimethylsilyl-trisilane |
| LC | liquid chromatography |
| MS | mass spectrostrometry |
| HPLC | high performance liquid chromatography |
| UPLC | ultra performance liquid chromatography |
| DAD | diode array detector |

| **Abbreviation** | **Meaning** |
|---|---|
| NMR | nuclear magnetic resonance spectroscopy: chemical |
| PyBOP | Benzotriazol-1-yl-oxytripyrrolidinophosphonium hexafluorophosphate |
| RT | room temperature |
| Rt | retention time (as measured either with HPLC or UPLC) |
| rac | racemic |
| THF | tetrahydrofuran |
| TPP | Triphenylphospine |
| UPLC | ultra performance liquid chromatography |

shifts (δ) are given in ppm. The chemical shifts were corrected by setting the DMSO signal to 2.50 ppm unless otherwise stated.

Other abbreviations have their meanings customary per se to the skilled person.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartridges KP-Sil^{®} or KP-NH^{®} in combination with a Biotage autopurifier system (SP4^{®} or Isolera Four^{®}) and eluents such as gradients of hexane/ethyl acetate or DCM/methanol. In some cases, the compounds may be purified by preparative HPLC using for example a Waters autopurifier equipped with a diode array detector and/or online electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### Solvents

The following solvents were purchased from commercial sources and were used without purification:
N,N-dimethylformamide (CAS: 68-12-2)
Tetrahydrofuran (CAS: 109-99-9)
Dichloromethane (CAS: 75-09-2)
Toluene (CAS: 108-88-3)
Dimethyl sulfoxide (CAS: 67-68-5)
Ethyl acetate (CAS: 141-78-6)
Hydrochloric acid (CAS: 7647-01-0
Hexane (CAS: 110-54-3)
Acetonitrile (CAS: 75-05-8)
Formic acid (CAS: 64-18-6)
Isopropanol (CAS: 67-63-0)
Diethyl ether (CAS: 60-29-7)
Methanol (CAS: 67-56-1)
N,N-dimethylacetamide (CAS: 127-19-5)
Ammonium hydroxide (CAS: 1336-21-6)
1,4-Dioxane (CAS: 123-91-1)
1,2-Dimethoxyethane (CAS: 110-71-4)

### Reagents

All reagents, for which the synthesis is not described in the experimental part, are either commercially available or synthesized according to literature procedures.

The following reactants were purchased from commercial sources and were used without further purification:
4-Bromo-2-fluoro-6-hydroxybenzaldehyde (CAS: 1427438-90-1)
Ethyl chloroacetate (CAS: 105-39-5)
Ethyl bromoacetate (CAS: 105-36-2)
Potassium carbonate (CAS: 584-08-7)
2-hydroxy-6-methoxy-4-methylbenzaldehyde (CAS: 39503-23-6)
Methyl 6-bromo-1-benzofuran-2-carboxylate (CAS: 425675-94-1)
Lithium hydroxide (CAS: 1310-65-2)
[1,1'-Biphenyl]-2-sulfonamide (CAS: 40182-06-7)
PyBOP (CAS: 128625-52-5)
*N,N*-diisopropylethylamine (CAS: 7087-68-5)
Di-MU-iodobis(tri-t-butylphosphino)dipalladium(I) (CAS: 166445-62-1)
Bromido(cyclopropyl)zinc (CAS: 126403-68-7)
1,1'-Bis(diphenylphosphanyl)ferrocene (CAS: 12150-46-8)
Palladium(ll) acetate (CAS: 3375-31-3)
Potassium acetate (CAS: 127-08-2)
Carbon monoxide (CAS: 630-08-0)
6-Bromo-7-fluoro-1-benzofuran-2-carboxylic acid (CAS: 1823358-68-4)
Tripotassium phosphate (CAS: 7778-53-2)
Cyclopropylboronic acid (CAS: 411235-57-9)
4-bromo-2-fluoro-6-methoxy-benzaldehyde (CAS: 856767-09-4)
Tricyclohexylphosphine tetrafluoroborate (CAS: 58656-04-5)
Boron tribromide (CAS: 10294-33-4)
Sodium hydroxide (CAS: 1310-73-2)
2-Fluoro-6-iodo-phenol (CAS: 28177-50-6)
lodoethane (CAS: 75-03-6)
CataCXium^{®} A (CAS: 321921-71-5)
1,4-Diazabicyclo[2.2.2]octane bis(sulfur dioxide) adduct (CAS: 119752-83-9)
Triethylamine (CAS: 121-44-8)
Sulfuryl chloride (CAS: 7791-25-5)
Ammonium chloride (CAS: 12125-02-9)
4-sec-Butylphenol (CAS: 99-71-8)
N-iodosuccinimide (CAS: 516-12-1)
Sodium thiosulfate (CAS: 7772-98-7)
5-Fluoro-2-iodo-phenol (CAS: 186589-87-7)
2-lodo-4-(trifluoromethyl)phenol (CAS: 463976-21-8)
4-Fluoro-2-iodo-phenol (CAS: 2713-29-3)
4-Phenoxyphenol (CAS: 831-82-3)
Chloromethyl methyl ether (CAS: 107-30-2)
*N*-Butyllithium (CAS: 109-72-8)
Iodine (CAS: 7553-56-2
Sodium iodide (CAS: 7681-82-5)
Trimethylsilyl chloride (CAS: 75-77-4)
4-(Trifluoromethoxy)phenol (CAS: 828-27-3)
p-Toluenesulfonic acid monohydrate (CAS: 6192-52-5)
Sodium sulfite (CAS: 7757-83-7)
4-lsopropylphenol (CAS: 99-89-8)
Benzyl bromide (CAS: 100-39-0)
2-Iodo-4-isopropylphenol (CAS: 58456-88-5)
(lodomethyl)cyclopropane (CAS: 33574-02-6)
1,1,1-Trifluoro-2-iodoethane (CAS: 353-83-3)
4-Bromo-2,6-difluoro-benzaldehyde (CAS: 537013-51-7)
Dimethylamine hydrochloride (CAS: 506-59-2)
Potassium hydroxide (CAS: 1310-58-3)
4-Bromo-2-fluoro-6-hydroxy-benzaldehyde (CAS: 1427438-90-1)
Sodium chlorodifluoroacetate (CAS: 1895-39-2)
1-Bromo-3-methoxy-5-(trifluoromethyl)benzene (CAS: 627527-23-5)
*N,N,N',N'*-tetramethylethylenediamine (CAS: 110-18-9)
2-Hydroxy-4-(propan-2-yl)benzaldehyde (CAS: 536-32-3)
Imidazole (CAS: 288-32-4)
4-*tert*-Butyl-2-iodo-phenol (CAS: 38941-98-9)
Bromocyclopropane (CAS: 4333-56-6)
Bromomethylcyclopropane (CAS: 7051-34-5)
1,1,1-Trifluoro-2-iodo-ethane (CAS: 353-83-3)
Bromocyclobutane (CAS: 4399-47-7)
2-Bromopropane (CAS: 75-26-3)
5-*tert*-butyl-2-methoxy-benzenesulfonyl chloride (CAS: 88041-83-2)
2-(Bromomethyl)-1,1-difluorocyclopropane (CAS: 77613-65-1)
Potassium iodide (CAS: 7681-11-0)
3,4-Difluorophenol (CAS: 2713-33-9)
4-(Hydroxymethyl)phenol (CAS: 623-05-2)
lodomethane (CAS: 74-88-4)
Sodium hydride (CAS: 7646-69-7
4-Cyclopropylphenol (CAS: 10292-61-2)
4-Cyclopropyl-2-iodo-phenol (CAS:
   *tert*-Butyl 4-(bromomethyl)piperidine-1-carboxylate (CAS: 158407-04-6)
   Methylboronic acid (CAS: 13061-96-6)
   [1,1'-Bis(diphenylphosphino)ferrocene]palladium(II)dichloride dichloromethane complex (CAS: 95464-05-4)
   Di-mu-chloro[bis(1-phenylprop-2-en-1-yl)]dipalladium (CAS: 12131-44-1)
   Zinc cyanide (CAS: 557-21-1)
   Pyridin-3-ylboronic acid (CAS: 1692-25-7)
   4,6-Dimethyl-1-benzofuran-2-carboxylic acid (CAS: 77037-40-2)
   2'-Methyl[biphenyl]-2-sulfonamide (CAS: 217498-86-7)
   5-Methyl[biphenyl]-2-sulfonamide (CAS: 936841-54-2)
   *N*-Methylmethanamin (CAS: 124-40-3)
   3'-Chloro[biphenyl]-2-sulfonamide (CAS: 1350725-94-8)
   2-Ethoxybenzene-1-sulfonamide (CAS: 58734-61-5)
   3,6-Dimethyl-1-benzofuran-2-carboxylic acid (CAS: 16820-37-4)
   XPhos (CAS: 564438-18-7)
   XPhos Pd G3 (CAS: 1445085-55-1)
   [1,3-Bis[2,6-bis(i-propyl)phenyl]-2-imidazolidinylidene]difluoromethylsilver(I) (CAS: 1643366-13-5)
   6-(Trifluoromethyl)-1-benzofuran-2-carboxylic acid (CAS: 575469-39-5)
   2-Methylquinoline-8-sulfonamide (CAS: 157686-27-6)
   Pyridin-4-ylboronic acid (CAS: 1692-15-5)
   4,4,5,5-Tetramethyl-2-(prop-1-en-2-yl)-1,3,2-dioxaborolane (CAS: 126726-62-3)
   2-(Cyclopropyloxy)benzene-1-sulfonamide (CAS: 1243451-33-3)
   2,6-Dimethylpyridine (CAS: 108-48-5)
   [Ir(dF(CF3)ppy2)2(dtbbpy)]PF6 (CAS: 870987-63-6)
   Nickel(ll) chloride dimethoxyethane adduct (CAS: 29046-78-4)
   4,4'-Di-*tert*-butyl-2,2'-bipyridine (CAS: 72914-19-3)
   TTMS (CAS: 1873-77-4)
   1-Bromo-2-methoxyethane (CAS: 6482-24-2)
   2-Bromobutane (CAS: 78-76-2)
   Carbonyldiimidazole (CAS: 530-62-1)

### UPLC-MS Standard Procedures

Analytical UPLC-MS was performed as described below. The masses (m/z) are reported from the positive mode electrospray ionization unless the negative mode is indicated (ESI-).

Analytical UPLC methods:
Method 1:
   Instrument: Waters Acquity UPLCMS SingleQuad; Column: Acquity UPLC BEH C₁₈ 1.7 µm, 50x2.1 mm; eluent A: water + 0.1 vol% formic acid (99%), eluent B: acetonitrile; gradient: 0-1.6 min 1-99% B, 1.6-2.0 min 99% B; flow 0.8 mL/min; temperature: 60 °C; DAD scan: 210-400 nm.
Method 2:
   Instrument: Waters Acquity UPLCMS SingleQuad; Column: Acquity UPLC BEH C₁₈ 1.7 µm, 50x2.1 mm; eluent A: water + 0.2 vol % aq. ammonia (32%), eluent B: acetonitrile; gradient: 0-1.6 min 1-99% B, 1.6-2.0 min 99% B; flow 0.8 mL/min; temperature: 60 °C; DAD scan: 210-400 nm.
Method 3:
   Instrument: Waters Acquity UPLCMS SingleQuad; Column: Acquity UPLC BEH C₁₈ 1.7 50x2.1 mm; eluent A: water + 0.2 vol% aq. ammonia (32%), eluent B: acetonitrile; gradient: 0-1.6 min 1-99% B, 1.6-2.0 min 99% B; flow 0.8 mL/min; temperature: 60 °C; DAD scan: 210-400 nm.
Method 4:
   Instrument: Waters Acquity UPLCMS SingleQuad; Column: Waters Acquity BEH C₁₈ 50 mm x 2.1 mm x 1.7 µm; eluent A: Water (MilliQ) + 0.01 vol% formic acid, eluent B: acetonitrile + 0.01 vol% formic acid; gradient: 0-0.3 min 3-4% B, 0.3-1.5 min 4-95% B, 1.5-1.9 min 95% B; 1.9-2.0 min 5% B; flow: 0.65 mL/min; temperature: 50 °C; DAD scan: 200-500 nm.
Method 5:
   Instrument: Waters Autopurification MS SingleQuad; Column: Waters XBrigde C₁₈ 5 µm 100x30 mm; eluent A: water + 0.1 vol% formic acid (99%), Eluent B: acetonitrile; Gradient: 0-5.5 min 5-100% B; flow 70 mL/min; temperature: 25 °C; DAD scan: 210-400 nm.
Method 6:
   Instrument: Waters Acquity UPLCMS SingleQuad; Column: Acquity UPLC BEH C₁₈ 1.7 50x2. 1 mm; eluent A: water + 0.1 vol % formic acid (99%), eluent B: acetonitrile; gradient: 0-1.6 min 1-99% B, 1.6-2.0 min 99% B; flow 0.8 ml/min; temperature: 60 °C; DAD scan: 210-400 nm.

Analytical LC-MS methods
**Method 1**
   Instrument: Waters Acquity UPLCMS SingleQuad; Column: Acquity UPLC BEH C18 1.7 µm, 50x2.1mm; eluent A: water + 0.1 vol % formic acid (99%), eluent B: acetonitrile; gradient: 0-1.6 min 1-99% B, 1.6-2.0 min 99% B; flow 0.8 ml/min; temperature: 60 °C; DAD scan: 210-400 nm.
**Method 2**
   Instrument: Waters Acquity UPLCMS SingleQuad; Column: Acquity UPLC BEH C18 1.7 µm, 50x2.1mm; eluent A: water + 0.2 vol % aqueous ammonia (32%), eluent B: acetonitrile; gradient: 0-1.6 min 1-99% B, 1.6-2.0 min 99% B; flow 0.8 ml/min; temperature: 60 °C; DAD scan: 210-400 nm.
**Method 3**
   Instrument: Waters Acquity UPLCMS SingleQuad; Column: Acquity UPLC BEH C18 1.7 50x2.1mm; eluent A: water + 0.1 vol % formic acid (99%), eluent B: acetonitrile; gradient: 0-1.6 min 1-99% B, 1.6-2.0 min 99% B; flow 0.8 ml/min; temperature: 60 °C; DAD scan: 210-400 nm
**Method 4**
   Instrument: Waters Acquity; Column: Waters Acquity BEH C18 50 mm x 2.1 mm x 1.7 µm; eluent A: Water (MilliQ) + 0.01 vol % formic acid, eluent B: acetonitrile + 0.01 vol % formic acid; gradient: 0-0.3 min 3-4% B, 0.3-1.5 min 4-95% B, 1.5-1.9 min 95% B; 1.9-2.0 min 5% B; flow: 0.65 mL/min; temperature: 50°C; DAD scan: 200-500 nm.
**Method 5**
   Instrument: Waters Acquity; Column: Waters Acquity BEH C₁₈ 50 mm x 2.1 mm x 1.7 µm; eluent A: Water (MilliQ) + 0.01 vol % formic acid, eluent B: acetonitrile + 0.01 vol % formic acid; gradient: 0-0.5 min 5% B, 0.5-4.0 min 5-95% B, 4.0-4.5 min 95% B; 4.5-5.0 min 5% B; flow: 0.65 mL/min; temperature: 50°C; DAD scan: 200-500 nm.
**Method 6**
   Instrument: Waters Acquity; Column: Waters Acquity BEH C₁₈ 50 mm x 2.1 mm x 1.7 µm; eluent A: Water (MilliQ) + 0.01 vol % formic acid, eluent B: acetonitrile + 0.01 vol % formic acid; gradient: 0-0.5 min 5% B, 0.5-9.0 min 5-95% B, 9.0-9.5 min 95% B; 9.5-10.0 min 5% B; flow: 0.65 mL/min; temperature: 50°C; DAD scan: 200-500 nm.

### EXPERIMENTAL SECTION - INTERMEDIATES

### Intermediate 1

### 6-Bromo-4-fluoro-1-benzofuran-2-carboxylic acid

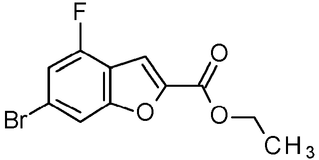

Commercially available 4-bromo-2-fluoro-6-hydroxybenzaldehyde (CAS: 1427438-90-1, 4.90 g, 22.4 mmol), ethyl chloroacetate (2.4 ml, 22 mmol) and potassium carbonate (30.9 g, 224 mmol) were suspended in DMF (40 ml) and stirred at 160 °C for 2 h. The reaction mixture was poured into water and the solution was acidified with aq. HCl (1 M). The resulting precipitate was collected by filtration and dried in vacuum at 40 °C to give 4.00 g (69% yield) of the product as a mixture of ester and free acid which was used in the next step without further purification. ¹H-NMR (400 MHz, DMSO-d₆) δ [ppm]: 2.332 (0.48), 2.518 (2.57), 2.523 (1.67), 2.673 (0.51), 2.726 (3.69), 2.728 (3.49), 2.887 (4.58), 3.942 (0.50), 4.899 (0.60), 7.006 (2.03), 7.010 (2.11), 7.032 (2.03), 7.036 (2.11), 7.146 (3.11), 7.150 (4.45), 7.154 (3.10), 7.426 (0.73), 7.439 (9.75), 7.443 (11.02), 7.450 (14.45), 7.452 (14.69), 7.463 (10.35), 7.466 (10.05), 7.890 (10.36), 7.893 (16.00), 7.896 (10.04), 7.949 (0.55), 8.102 (0.42), 8.108 (0.48), 10.217 (4.86), 10.219 (4.47); LC-MS (method 1): Rₜ = 1.05 min; MS (ESlneg): m/z = 256 [M-H]⁻

### Intermediate 2

### 6-Bromo-1-benzofuran-2-carboxylic acid

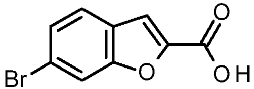

To a stirring solution of commercially available methyl 6-bromo-1-benzofuran-2-carboxylate (CAS: 425675-94-1, 5.00 g, 19.6 mmol) in THF (167 ml) was added water (83 ml) and lithium hydroxide (2.35 g, 98.0 mmol) and the mixture was stirred for 18h at rt. After evaporating the organic solvent the aqueous residue was acidified with aq. HCl (50 % w/w) and the resulting precipitate was collected by filtration, washed with water and dried in vaccum at 40 °C to give 4.50 g (90% yield) of the title compound as a white solid. ¹H-NMR (400 MHz, DMSO-d6) δ [ppm]: 2.518 (0.57), 7.509 (6.19), 7.513 (7.11), 7.530 (7.31), 7.534 (8.19), 7.674 (15.06), 7.677 (16.00), 7.733 (10.24), 7.754 (8.30), 8.051 (6.16), 8.053 (8.06), 8.056 (6.16); LC-MS (method 1): Rt = 0.98 min; MS (ESlneg): m/z = 239 [M-H]⁻

### Intermediate 3

### 6-Bromo-4-fluoro-1-benzofuran-2-carboxylic acid

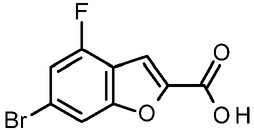

Synthesized analogously to **Intermediate 2** using **Intermediate 1** (4.00 g, 4.18 mmol) instead of methyl 6-bromo-1-benzofuran-2-carboxylate to give 402 mg (35% yield) of the title compound. ¹H-NMR (400 MHz, DMSO-d₆) δ [ppm]: 2.326 (0.44), 2.518 (1.54), 2.522 (1.06), 2.539 (1.59), 2.668 (0.44), 7.522 (5.95), 7.526 (5.96), 7.545 (5.50), 7.549 (6.06), 7.766 (14.52), 7.769 (16.00), 7.985 (6.67), 7.988 (9.81), 7.990 (6.07); LC-MS (method 1): Rt = 1.05 min; MS (ESlpos): m/z = 259 [M+H]⁺

### Intermediate 4

### N-([1,1'-Biphenyl]-2-sulfonyl)-6-bromo-1-benzofuran-2-carboxamide

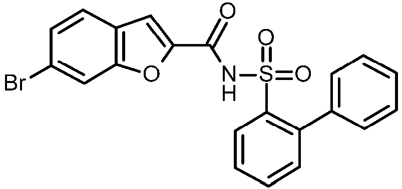

Intermediate 2 (4.50 g, 18.7 mmol), [1,1'-biphenyl]-2-sulfonamide (10 ml, 21 mmol) and PyBOP (11.7 g, 22.4 mmol) were suspended in dichloromethane (1.2 I), then DIPEA was added and the resulting mixture was stirred for 3 days at rt. The reaction mixture was diluted with water and extracted with dichloromethane twice. The combined organic layers were washed with brine, dried over sodium sulfate, concentrated and purified by preparative HPLC to give 4.80 g (90% purity, 51% yield) of the title compound as a white solid. ¹H-NMR (400 MHz, DMSO-d₆) δ [ppm]: 1.218 (0.42), 1.234 (0.51), 2.074 (1.54), 2.083 (1.47), 2.518 (1.70), 2.522 (1.20), 7.244 (6.27), 7.249 (8.49), 7.254 (2.89), 7.265 (14.43), 7.269 (13.56), 7.273 (2.48), 7.285 (6.27), 7.288 (9.14), 7.293 (2.47), 7.306 (16.00), 7.310 (6.61), 7.312 (7.57), 7.316 (7.37), 7.320 (4.75), 7.323 (7.56), 7.331 (7.98), 7.335 (7.55), 7.340 (4.53), 7.343 (7.47), 7.347 (3.63), 7.354 (2.45), 7.361 (6.77), 7.369 (1.40), 7.375 (1.26), 7.379 (1.92), 7.383 (1.03), 7.523 (9.16), 7.528 (8.67), 7.544 (9.62), 7.549 (10.46), 7.648 (2.99), 7.651 (3.07), 7.667 (6.39), 7.670 (6.14), 7.687 (6.20), 7.691 (7.24), 7.694 (13.16), 7.696 (13.08), 7.718 (5.19), 7.722 (5.75), 7.737 (7.52), 7.741 (7.25), 7.755 (2.97), 7.759 (2.97), 7.773 (12.98), 7.793 (10.87), 8.007 (10.99), 8.174 (7.22), 8.178 (7.69), 8.195 (6.71), 8.198 (6.62); LC-MS (method 1): Rt = 1.34 min; MS (ESlpos): m/z = 456 [M+H]⁺

### Intermediate 5

### 6-Cyclopropyl-4-fluoro-1-benzofuran-2-carboxylic acid

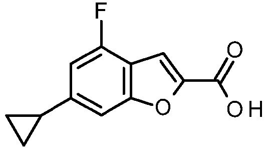

**Intermediate 3** (200 mg, 772 µmol) and Di-MU-iodobis(tri-t-butylphosphino)dipalladium(I) (67 mg, 77 µmol) were sealed in a vessel and flushed with argon. Toluene (3 mL) was added and the mixture was stirred at rt for 1 h followed by the dropwise addition of bromido(cyclopropyl)zinc (4.6 ml, 0.5 M in THF, 2.3 mmol) and the mixture was stirred at rt for 1h. The reation mixture was separated between water and dichloromethane, the organic layer was dried over sodium sulfate, concentrated and purified by preparative HPLC to give 48.0 mg (25% yield) of the title compound as an offwhite solid. ¹H-NMR (400 MHz, DMSO-d₆) δ [ppm]: -0.008 (0.94), 0.008 (0.91), 0.792 (3.08), 0.803 (10.34), 0.809 (8.91), 0.816 (9.62), 0.821 (9.43), 0.832 (4.11), 1.017 (3.84), 1.027 (8.62), 1.033 (9.28), 1.037 (4.68), 1.044 (4.38), 1.048 (9.23), 1.054 (8.81), 1.065 (3.25), 1.121 (14.45), 1.155 (15.43), 2.060 (1.13), 2.072 (2.29), 2.081 (2.46), 2.087 (2.09), 2.093 (4.41), 2.106 (2.31), 2.114 (2.17), 2.127 (0.98), 2.339 (0.42), 2.521 (4.73), 2.526 (3.22), 2.543 (0.42), 2.682 (0.42), 6.929 (6.72), 6.931 (6.77), 6.957 (6.60), 6.959 (6.62), 7.332 (14.42), 7.648 (16.00), 7.651 (15.43); LC-MS (method 1): Rₜ = 1.08 min; MS (ESlneg): m/z = 219 [M-H]⁻

### Intermediate 6

### Ethyl 4-methoxy-6-methyl-1-benzofuran-2-carboxylate

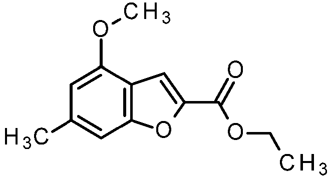

Synthesized analogously to Intermediate 1 using 1.00 g (6.02 mmol) of 2-hydroxy-6-methoxy-4-methylbenzaldehyde instead of 4-bromo-2-fluoro-6-hydroxybenzaldehyde to give 880 mg (64% yield) of the product as a mixture of ester and free acid. ¹H-NMR (400 MHz, DMSO-d₆) delta [ppm]: 2.433 (10.37), 3.869 (0.72), 3.903 (16.00), 6.703 (3.10), 7.079 (1.32), 7.081 (2.75), 7.084 (2.73), 7.086 (1.29), 7.488 (4.45), 7.490 (4.21); LC-MS (method 1): Rt = 1.08 min; MS (ESlpos): m/z = 235 [M+H]⁺

### Intermediate 7

### 2-[([1,1'-Biphenyl]-2-sulfonyl)carbamoyl]-1-benzofuran-6-carboxylic acid

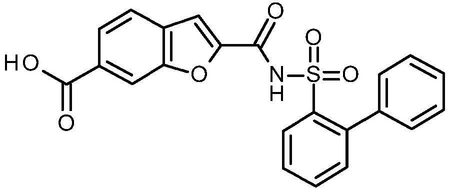

To a stirring solution of **Intermediate 4** (600 mg, 1.31 mmol) in DMSO (25 ml) in an autoclave was added 1,1'-ferrocenediyl-bis(diphenylphosphine) (150 mg, 263 µmol), palladium(ll) acetate (14.8 mg, 65.7 µmol) and potassium acetate (516 mg, 5.26 mmol). The resulting mixture was flushed with carbon monoxide and stirred overnight at 100°C at a maximum pressure of 19.3 bar. The reaction mixture was purified by preparative HPLC to give 120 mg (80% purity, 17% yield) of the title compound as an ochre solid. ¹H-NMR (400 MHz, DMSO-d₆) δ [ppm]: 1.218 (0.89), 1.237 (5.07), 1.252 (4.56), 1.266 (3.17), 2.323 (0.45), 2.327 (0.65), 2.332 (0.50), 2.539 (1.29), 2.665 (0.48), 2.669 (0.67), 2.673 (0.50), 3.125 (0.48), 3.136 (0.49), 3.143 (0.51), 3.154 (0.50), 3.166 (1.33), 3.363 (0.46), 3.611 (0.40), 3.620 (0.40), 7.074 (0.43), 7.261 (0.53), 7.286 (5.54), 7.294 (16.00), 7.333 (1.25), 7.347 (0.69), 7.608 (1.16), 7.627 (1.77), 7.646 (1.28), 7.671 (1.08), 7.689 (1.31), 7.870 (0.96), 7.890 (3.72), 7.901 (4.13), 7.904 (4.09), 7.922 (1.06), 7.924 (1.07), 8.123 (4.57), 8.133 (2.98), 8.166 (2.30), 8.183 (2.08); LC-MS (method 1): Rt = 1.08 min; MS (ESlneg): m/z = 420 [M-H]⁻

### Intermediate 8

### 6-Cyclopropyl-7-fluoro-1-benzofuran-2-carboxylic acid

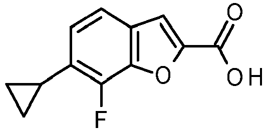

Synthesized analogously to Intermediate 5 using commercially available 6-bromo-7-fluoro-1-benzofuran-2-carboxylic acid (CAS: 1823358-68-4 , 150 mg, 0.58 mmol) instead of 6-bromo-4-fluoro-1-benzofuran-2-carboxylic acid to yield 150 mg as a crude product which was used without purification in the next step. ¹H-NMR (400 MHz, DMSO-d₆) δ [ppm]: 0.761 (0.83), 0.773 (2.60), 0.778 (2.46), 0.785 (2.63), 0.790 (2.52), 0.801 (0.97), 1.010 (1.08), 1.014 (3.27), 1.020 (2.35), 1.026 (2.30), 1.032 (1.36), 1.037 (1.41), 1.042 (4.84), 1.047 (2.38), 1.058 (0.86), 1.110 (1.08), 1.136 (7.09), 1.159 (1.02), 1.163 (0.80), 1.171 (7.70), 1.257 (7.09), 1.261 (16.00), 1.270 (14.73), 1.286 (15.06), 1.300 (13.48), 1.355 (1.99), 1.358 (1.36), 1.439 (6.59), 1.498 (0.80), 1.513 (0.58), 1.526 (4.82), 1.533 (1.02), 1.548 (0.58), 1.561 (4.84), 2.086 (0.47), 2.155 (0.61), 2.163 (0.66), 2.176 (1.11), 2.184 (0.69), 2.189 (0.64), 2.300 (9.47), 2.334 (1.00), 2.521 (5.23), 2.525 (3.52), 2.676 (1.02), 6.835 (1.00), 6.851 (1.25), 6.855 (1.27), 6.871 (1.16), 7.144 (0.83), 7.163 (1.58), 7.165 (1.88), 7.167 (1.55), 7.183 (2.08), 7.186 (1.61), 7.188 (1.27), 7.233 (1.88), 7.238 (0.66), 7.251 (1.94), 7.270 (0.94), 7.300 (1.02), 7.375 (1.97), 7.396 (1.77); LC-MS (method 1): Rt = 1.07 min; MS (ESlneg): m/z = 219 [M-H]⁻

### Intermediate 9

### 4-Cyclopropyl-2-fluoro-6-methoxy-benzaldehyde

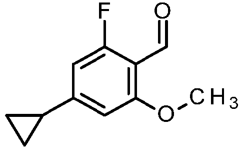

To a mixture of tripotassium phosphate (12.7 g, 60.0 mmol, 2.00 eq.) in argon degassed tetrhydrofuran (30.0 mL) and water (7.50 mL) was added 4-bromo-2-fluoro-6-methoxybenzaldehyde (6.99 g, 30.0 mmol, 1.00 eq.), cyclopropylboronic acid (3.87 g, 45.0 mmol, 1.50 eq.), palladium(ll) acetate (337 mg, 1.50 mmol, 10 mol%) and tricyclohexylphosphine tetrafluoroborate (1.10 g, 3.00 mmol, 20 mol%). The resulting mixture was stirred at 80 °C for 18 h and then cooled to room temperature. The mixture was diluted with water (200 mL) and extracted with ethyl acetate (3 x 100 mL). The combined organic extracts were washed with water (100 mL), brine (100 mL), dried (magnesium sulfate), filtered and dry loaded onto Celite under reduced pressure. The residue was purified by flash column chromatography (330 g Silica, 0-50% ethyl acetate/hexanes gradient) to give the title compound as a yellow solid (4.78 g). ¹H NMR (400 MHz, Chloroform-d) δ 10.34 (t, 1H), 6.49 (s, 1H), 6.38 - 6.29 (m, 1H), 3.92 (d), 1.96 - 1.86 (m, 1H), 1.15 - 1.03 (m, 2H), 0.84 - 0.75 (m, 2H); LC-MS (method 5): Rt = 2.38 min; MS (ESlpos): m/z = 195 [M+H]⁺

### Intermediate 10

### 4-Cyclopropyl-2-fluoro-6-hydroxy-benzaldehyde

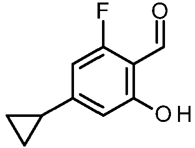

To a -5 °C stirred solution of 4-cyclopropyl-2-fluoro-6-methoxy-benzaldehyde (4.40 g, 22.7 mmol, 1.00 eq.) in anhydrous dichloromethane (30.2 mL, 0.75 M) was added boron tribromide (1.00 M in dichloromethane, 34.0 mL, 24.0 mmol, 1.50 eq.) dropwise. Following complete addition, the dark solution was warmed to room temperature, stirred for 30 minutes and then poured onto ice. The mixture was diluted with water (100 mL) and extracted with dichloromethane (3 x 100 mL). The combined organic extracts were washed with hydrochloric acid (1.00 M in water, 100 mL), brine (100 mL), dried (magnesium sulfate), filtered and dry loaded onto Celite under reduced pressure. The residue was purified by flash column chromatography (330 g Silica, 0-30% ethyl acetate/hexanes gradient) to give the title compound as a yellow oil (3.81 g, 98% purity). ¹H NMR (400 MHz, Chloroform-d) δ 11.51 - 11.44 (m, 1H), 10.14 (d, 1H), 6.43 (s, 1H), 6.30 (dt, 1H), 1.93 - 1.81 (m, 1H), 1.16 - 1.04 (m, 2H), 0.88 - 0.74 (m, 2H); LC-MS (method 5.): Rt = 2.67min; MS (ESlpos): m/z = 181 [M+H]⁺

### Intermediate 11

### 6-Cyclopropyl-4-fluoro-benzofuran-2-carboxylic acid

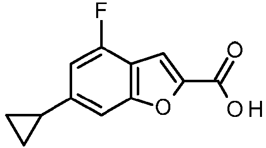

To a rt stirred solution of 4-cyclopropyl-2-fluoro-6-hydroxy-benzaldehyde (4.00 g, 22.2 mmol, 1.00 eq.) in anhydrous *N,N*-dimethylformamide (127 mL, 0.15 M) was sequentially added potassium carbonate (4.60 g, 33.3 mmol, 1.50 eq.) and ethyl bromoacetate (2.70 mL, 24.4 mmol, 1.10 eq.). The resulting yellow suspension was heated at 160°C for 2 h, after which sodium hydroxide (2.00 M in water, 27.8 mL, 55.5 mmol, 2.50 eq.) was added. After cooling to room temperature, the mixture was acidified to pH 2.0 with hydrochloric acid (1.00 M in water, ~ 100 mL), diluted with water (100 mL) and extracted with ethyl acetate (3 x 100 mL). The combined organic extracts were washed with water (2 x 100 mL), brine (100 mL), dried (magnesium sulfate) and concentrated under reduced pressure. The residue was purified by reverse phase flash column chromatography (275 g HP C₁₈, 10-100% acetonitrile/water gradient buffered with 0.1% formic acid) to give the title compound as a light yellow solid (3.63 g). ¹H NMR (400 MHz, DMSO-d₆) δ 13.63 (s, 1H), 7.64 (s, 1H), 7.32 (s, 1H), 6.93 (dd, 1H), 2.15 - 2.03 (m, 1H), 1.10 - 0.97 (m, 2H), 0.88 - 0.74 (m, 2H); LC-MS (method 5): Rₜ = 2.42 min; MS (ESlpos): m/z = 221 [M+H]⁺

### Intermediate 12

### 2-Ethoxy-1-fluoro-3-iodo-benzene

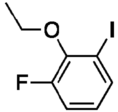

To a stirred solution of 2-fluoro-6-iodo-phenol (1.00 g, 4.20 mmol, 1.00 eq) in anhydrous DMF (8.40 mL, 0.50 M) was added potassium carbonate (1.16 g, 8.40 mmol, 2.00 eq) and stirred at room temperature. After five minutes, iodoethane (510 µL, 6.30 mmol, 1.50 eq) was added, and the reaction was left to stir at 70 °C for 20 h. The reaction was washed with water (20 mL), and the aqueous layer was extracted with dichloromethane (3 x 20 mL). The combined organics washed with brine (20 mL), dried over magnesium sulfate, filtered, and dry loaded onto Celite under reduced pressure. The crude was purified by flash column chromatography (80 g HP Silica, 0-20 %, hexane/ethyl acetate) to afford the title compound as a clear oil (1.06 g). ¹H NMR (400 MHz, Chloroform-d) δ 7.57 - 7.49 (m, 1H), 7.12 - 7.02 (m, 1H), 6.82 - 6.72 (m, 1H), 4.21 - 4.11 (m, 2H), 1.46 (t, 3H); LC-MS (method 4): Rₜ = 1.65 min; MS (ESlpos): m/z = not detected

### Intermediate 13

### 2-Ethoxy-3-fluoro-benzenesulfonamide

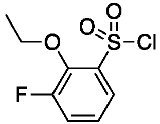

To an oven dried 40 mL vial under an argon atmosphere was added palladium(ll) acetate (44.3 mg, 0.20 mmol, 5.00 mol%), CataCXium^{®} A (113 mg, 0.32 mmol, 7.50 mol%), and 1,4-Diazabicyclo[2.2.2]octane bis(sulfur dioxide) adduct (569 mg, 2.37 mmol, 0.60 eq.) were sequentially added argon degassed anhydrous isopropanol (19.7 mL, 0.20 M), 2-ethoxy-1-fluoro-3-iodo-benzene (1.05 g, 3.95 mmol, 1.00 eq.), and triethylamine (1.64 mL, 11.8 mmol, 3.00 eq.). The resulting red solution was heated at 75 °C for 7 d, cooled to room temperature and concentrated under reduced pressure. The residue was resuspended in anhydrous tetrahydrofuran (17.5 mL) and cooled to 0 °C in a ice/water bath. To this mixture was then added sulfuryl chloride (960 µL, 11.8 mmol, 3.00 eq.) dropwise, after which the resulting mixture was warmed to room temperature and stirred for a further 30 minutes. The reaction was quenches with ammonium chloride (20 mL), and the aqueous layer was extracted with dichloromethane (3 x 20 mL). The combined organics were washed with brine (20 mL), dried over magnesium sulfate, filtered, and dry loaded onto Celite under reduced pressure. The crude was purified by flash column chromatography (80 g HP Silica, 0-20 %, ethyl acetate/hexanes) to afford the title compound as an oil (498 mg, >60% purity). LC-MS (method 4): Rt = 1.57 min; MS (ESlpos): m/z = not detected

### Intermediate 14

### 2-Ethoxy-3-fluoro-benzenesulfonamide

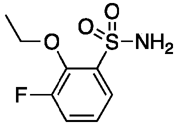

To a stirred solution of 2-ethoxy-3-fluoro-benzenesulfonyl chloride (525 mg, 2.20 mmol, 1.00 eq) in anhydrous tetrahydrofuran (3.67 mL, 0.60 mmol) was added ammonium hydroxide solution (20 % in water, 8.57 mL, 44.0 mmol, 20.0 eq) and stirred at room temperature for 30 minutes. The reaction was quenched with hydrochloric acid (1.00 M, 20 mL), and the aqueous layer was extracted with ethyl acetate (3 x 20 mL). The combined organics were washed with brine (20 mL), dried over magnesium sulfate, filtered, and dry loaded onto Celite under reduced pressure. The crude was purified by flash column chromatography (40 g HP silica, 0-60 %, ethyl acetate/hexnaes) to afford the title compound as a white solid (192 mg). ¹H NMR (400 MHz, Chloroform-d) δ 7.72 - 7.64 (m, 1H), 7.37 - 7.26 (m, 1H), 7.19 - 7.09 (m, 1H), 5.07 (s, 2H), 4.43 - 4.32 (m, 2H), 1.50 (t, 3H); LC-MS (method 4): Rₜ = 1.25 min; MS (ESlpos): m/z = not detected

### Intermediate 15

### 2-Iodo-4-sec-butyl-phenol

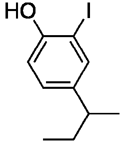

To a stirred solution of 4-sec-butylphenol (5.00 g, 33.3 mmol, 1.00 eq) in acetonitrile (16.6 mL, 2.00 M) was added *N*-iodosuccinimide (7.49 g, 33.3 mmol, 1.00 eq) and stirred at room temperature for 22 h. Additional N-iodosuccinimide (3.75 g, 16.7 mmol, 0.50 eq) was added and stirred at room temperature for 112 h (five nights). The reaction was quenched with sodium thiosulfate solution (10 % in water, 50 mL), and the aqueous layer was extracted with ethyl acetate (3 x 30 mL). The combined organics were washed with brine (30 mL), dried over magnesium sulfate, filtered, and dry loaded onto Celite under reduced pressure. The crude was purified by flash column chromatography (120 g Silica, 0-30 %, ethyl acetate/hexanes) to afford the title compound as an oil (2.35 g). ¹H NMR (400 MHz, Chloroform-d) δ 7.50 - 7.42 (m, 1H), 7.11 - 6.99 (m, 1H), 6.95 - 6.86 (m, 1H), 5.09 (s, 1H), 2.55 - 2.41 (m, 1H), 1.64 - 1.46 (m, 2H), 1.33 - 1.15 (m, 3H), 0.92 - 0.77 (m, 3H); LC-MS (method 4): Rₜ = 1.63 min; MS (ESlneg): m/z = 275 [M-H]⁻

### Intermediate 16

### 1-Ethoxy-2-iodo-4-sec-butyl-benzene

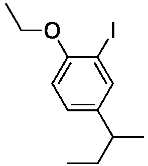

To a stirred solution of 2-iodo-4-sec-butyl-phenol (2.35 g, 8.51 mmol, 1.00 eq) in anhydrous DMF (17.0 mL, 0.50 M) was added potassium carbonate (2.35 g, 17.0 mmol, 2.00 eq) and stirred at room temperature. After five minutes, iodoethane (1.03 mL, 12.8 mmol, 1.50 eq) was added, and the reaction was left to stir at 75 °C for 16 h. The reaction was washed with water (30 mL), and the aqueous layer was extracted with dichloromethane (3 x 30 mL). The combined organics washed with brine (30 mL), dried over magnesium sulfate, filtered, and dry loaded onto Celite under reduced pressure. The crude was purified by flash column chromatography (120 g Silica, 0-20 %, hexane/ethyl acetate) to afford the title compound as an oil (1.57 g). 1H NMR (400 MHz, Chloroform-d) δ 7.61 - 7.53 (m, 1H), 7.11 - 7.04 (m, 1H), 6.76 - 6.69 (m, 1H), 4.12 - 3.98 (m, 2H), 2.55 - 2.44 (m, 1H), 1.60 - 1.42 (m, 5H), 1.23 - 1.15 (m, 3H), 0.89 - 0.77 (m, 3H); LC-MS (method 4): Rt = 1.84 min; MS (ESlpos): m/z = not detected

### Intermediate 17

### 2-Ethoxy-5-sec-butyl-benzenesulfonyl chloride

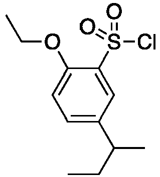

To an oven dried 40 mL vial under an argon atmosphere was added palladium(ll) acetate (57.8 mg, 0.26 mmol, 5.00 mol%), CataCXium^{®} A (148 mg, 0.41 mmol, 7.50 mol%), and 1,4-Diazabicyclo[2.2.2]octane bis(sulfur dioxide) adduct (742 mg, 3.09 mmol, 0.60 eq.) were sequentially added argon degassed anhydrous isopropanol (25.7 mL, 0.20 M), 1-ethoxy-2-iodo-4-sec-butyl-benzene (1.57 g, 5.15 mmol, 1.00 eq.), and triethylamine (2.14 mL, 15.4 mmol, 3.00 eq.). The resulting red solution was heated at 75 °C for 16 h, cooled to room temperature and concentrated under reduced pressure. The residue was resuspended in anhydrous tetrahydrofuran (25.7 mL) and cooled to 0 °C in a ice/water bath. To this mixture was then added sulfuryl chloride (1.25 mL, 15.4 mmol, 3.00 eq.) dropwise, after which the resulting mixture was warmed to room temperature and stirred for a further 30 minutes. The reaction was quenches with ammonium chloride (30 mL), and the aqueous layer was extracted with dichloromethane (3 x 30 mL). The combined organics were washed with brine (30 mL), dried over magnesium sulfate, filtered, and dry loaded onto Celite under reduced pressure. The crude was purified by flash column chromatography (80 g, 0-20 %, ethyl acetate/hexanes) to afford the title compound as a black oil (1.17 g, >90% purity). LC-MS (method 4): Rt = 1.73 min; MS (ESlneg): m/z = not detected

### Intermediate 18

### 2-Ethoxy-5-sec-butyl-benzenesulfonamide

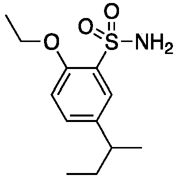

To a stirred solution of 2-ethoxy-5-sec-butyl-benzenesulfonyl chloride (1.17 g, 4.22 mmol, 1.00 eq) in anhydrous tetrahydrofuran (7.03 mL, 0.60 mmol) was added ammonium hydroxide solution (20 % in water, 16.4 mL, 84.3 mmol, 20.0 eq) and stirred at room temperature for 30 minutes. The reaction was quenched with hydrochloric acid (1.00 M, 20 mL), and the aqueous layer was extracted with ethyl acetate (3 x 20 mL). The combined organics were washed with brine (20 mL), dried over magnesium sulfate, filtered, and dry loaded onto Celite under reduced pressure. The crude was purified by flash column chromatography (80 g HP silica, 0-60 %, ethyl acetate/hexnaes) to afford the title compound as a clear oil (398 mg). ¹H NMR (400 MHz, Chloroform-d) δ 7.75 - 7.69 (m, 1H), 7.31 (dd, 1H), 6.95 (d, 1H), 5.03 (s, 2H), 4.28 - 4.18 (m, 2H), 2.61 (hept, 1H), 1.65 - 1.47 (m, 5H), 1.30 - 1.18 (m, 3H), 0.85 - 0.76 (m, 3H); LC-MS (method 4): Rt = 1.45 min; MS (ESlpos): m/z = 258 [M+H]⁺

### Intermediate 19

### 2-Ethoxy-4-fluoro-1-iodo-benzene

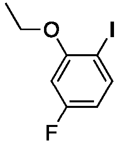

To a stirred solution of 5-fluoro-2-iodo-phenol (1.00 g, 4.20 mmol, 1.00 eq) in anhydrous N,N-dimethylformamide (8.40 mL, 0.50 M) was added potassium carbonate (1.16 g, 8.40 mmol, 2.00 eq) and stirred at room temperature. After five minutes, iodoethane (510 µL, 6.30 mmol, 1.50 eq) was added, and the reaction was left to stir at 70 °C for 16 h. The reaction was washed with water (30 mL), and the aqueous layer was extracted with dichloromethane (3 x 20 mL). The combined organics were washed with brine (20 mL), dried over magnesium sulfate, filtered, and dry loaded onto Celite under reduced pressure. The crude was purified by flash column chromatrography (80 g, 0-20 %, ethyl acetate/hexanes) to afford the title compound as a clear oil (931 mg). ¹H NMR (400 MHz, Chloroform-d) δ 7.69 (dd, 1H), 6.55 (dd, 1H), 6.53 - 6.43 (m, 1H), 4.12 - 4.00 (m, 2H), 1.49 (t, 3H); LC-MS (method 4): Rₜ = 1.65 min; MS (ESlpos): m/z = not detected

### Intermediate 20

### 2-Ethoxy-4-fluoro-benzenesulfonyl chloride

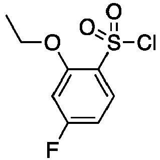

To an oven dried 40 mL vial under an argon atmosphere was added palladium(ll) acetate (39.3 mg, 0.17 mmol, 5.00 mol%), CataCXium^{®} A (100 mg, 0.28 mmol, 7.50 mol%), and 1,4-Diazabicyclo[2.2.2]octane bis(sulfur dioxide) adduct (505 mg, 2.10 mmol, 0.60 eq.) were sequentially added argon degassed anhydrous isopropanol (17.5 mL, 0.20 M), 2-ethoxy-4-fluoro-1-iodo-benzene (931 g, 3.50 mmol, 1.00 eq.), and triethylamine (1.46 mL, 10.5 mmol, 3.00 eq.). The resulting red solution was heated at 75 °C for 16 h, cooled to room temperature and concentrated under reduced pressure. The residue was resuspended in anhydrous tetrahydrofuran (17.5 mL) and cooled to 0 °C in a ice/water bath. To this mixture was then added sulfuryl chloride (1.46 mL, 10.5 mmol, 3.00 eq.) dropwise, after which the resulting mixture was warmed to room temperature and stirred for a further 30 minutes. The reaction was quenches with ammonium chloride (20 mL), and the aqueous layer was extracted with dichloromethane (3 x 20 mL). The combined organics were washed with brine (20 mL), dried over magnesium sulfate, filtered, and dry loaded onto Celite under reduced pressure. The crude was purified by flash column chromatography (80 g, 0-20 %, ethyl acetate/hexanes) to afford the title compound as an oil (498 mg). ¹H NMR (400 MHz, Chloroform-d) δ 8.02 - 7.93 (m, 1H), 6.83 - 6.72 (m, 2H), 4.26 (q, 2H), 1.59 - 1.53 (m, 3H); LC-MS (method 4): Rt = 1.21 min; MS (ESlneg): m/z = not detected

### Intermediate 21

### 2-Ethoxy-4-fluoro-benzenesulfonamide

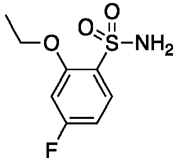

To a stirred solution of 2-ethoxy-4-fluoro-benzenesulfonyl chloride (498 mg, 2.09 mmol, 1.00 eq) in anhydrous tetrahydrofuran (3.48 mL, 0.60 mmol) was added ammonium hydroxide solution (20 % in water, 8.13 mL, 41.7 mmol, 20.0 eq) and stirred at room temperature for 30 minutes. The reaction was quenched with hydrochloric acid (1.00 M, 10 mL), and the aqueous layer was extracted with ethyl acetate (3 x 10 mL). The combined organics were washed with brine (20 mL), dried over magnesium sulfate, filtered, and dry loaded onto Celite under reduced pressure. The crude was purified by flash column chromatography (24 g HP Silica, 0-60 %, ethyl acetate/hexnaes) to afford the title compound as a white solid (336 mg). ¹H NMR (400 MHz, Chloroform-d) δ 7.96 - 7.88 (m, 1H), 6.80 - 6.70 (m, 2H), 4.97 (s, 2H), 4.23 (q, 2H), 1.58 - 1.50 (m, 3H); LC-MS (method 4): Rₜ = 1.20 min; MS (ESlneg): m/z = 218 [M-H]⁻

### Intermediate 22

### 1-Ethoxy-2-iodo-4-(trifluoromethyl)benzene

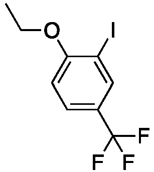

To a stirred solution of 2-iodo-4-(trifluoromethyl)phenol (1.00 g, 3.47 mmol, 1.00 eq) in anhydrous N,N-dimethylformamide (6.94 mL, 0.50 M) was added potassium carbonate (960 mg, 6.94 mmol, 2.00 eq) and stirred at room temperature. After five minutes, iodoethane (420 µL, 5.21 mmol, 1.50 eq) was added, and the reaction was left to stir at 70 °C for 16 h. The reaction was washed with water (30 mL), and the aqueous layer was extracted with dichloromethane (3 x 20 mL). The combined organic layers were washed with brine (20 mL), dried over magnesium sulfate, filtered, and dry loaded onto Celite under reduced pressure. The residue was purified by flash column chromatrography (80 g, 0-20 %, ethyl acetate/hexanes) to afford the title compound as a clear oil (886 mg). 1H NMR (400 MHz, Chloroform-d) δ 8.04 - 7.99 (m, 1H), 7.59 - 7.51 (m, 1H), 6.82 (d, 1H), 4.14 (q, 2H), 1.55 - 1.47 (m, 3H); LC-MS (method 4): Rt = 1.73 min; MS (ESlpos): m/z = not detected

### Intermediate 23

### 2-Ethoxy-5-(trifluoromethyl)benzenesulfonyl chloride

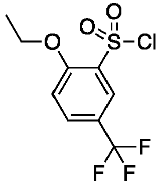

To an oven dried 40 mL vial under an argon atmosphere was added palladium(ll) acetate (31.5 mg, 0.14 mmol, 5.00 mol%), CataCXium^{®} A (80.4 mg, 0.22 mmol, 7.50 mol%), and 1,4-Diazabicyclo[2.2.2]octane bis(sulfur dioxide) adduct (404 mg, 1.68 mmol, 0.60 eq.) were sequentially added argon degassed anhydrous isopropanol (14.0 mL, 0.20 M), 1-ethoxy-2-iodo-4-(trifluoromethyl)benzene (886 g, 2.80 mmol, 1.00 eq.), and triethylamine (1.17 mL, 8.41 mmol, 3.00 eq.). The resulting red solution was heated at 75 °C for 16 h, cooled to room temperature and concentrated under reduced pressure. The residue was resuspended in anhydrous tetrahydrofuran (14.0 mL) and cooled to 0 °C in a ice/water bath. To this mixture was then added sulfuryl chloride (1.67 mL, 8.41 mmol, 3.00 eq.) dropwise, after which the resulting mixture was warmed to room temperature and stirred for a further 30 minutes. The reaction was quenched with ammonium chloride (20 mL), and the aqueous layer was extracted with dichloromethane (3 x 20 mL). The combined organics were washed with brine (20 mL), dried over magnesium sulfate, filtered, and dry loaded onto Celite under reduced pressure. The crude was purified by flash column chromatography (40 g HP Silica, 0-20 %, ethyl acetate/hexanes) to afford the title compound as an oil (175 mg). ¹H NMR (400 MHz, Chloroform-d) δ 8.26 - 8.21 (m, 1H), 7.94 - 7.86 (m, 1H), 7.20 (d, 1H), 4.36 (q, 2H), 1.63 - 1.54 (m, 3H); LC-MS (method 4): Rₜ = 1.63 min; MS (ESlneg): m/z = not detected

### Intermediate 24

### 2-Ethoxy-5-(trifluoromethyl)benzenesulfonamide

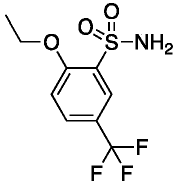

To a stirred solution of 2-ethoxy-5-(trifluoromethyl)benzenesulfonyl chloride (175 mg, 0.60 mmol, 1.00 eq) in anhydrous tetrahydrofuran (1.00 mL, 0.60 mmol) was added ammonium hydroxide solution (20 % in water, 2.32 mL, 11.9 mmol, 20.0 eq) and stirred at room temperature for 30 minutes. The reaction was quenched with hydrochloric acid (1.00 M, 10 mL), and the aqueous layer was extracted with ethyl acetate (3 x 10 mL). The combined organics were washed with brine (10 mL), dried over magnesium sulfate, filtered, and dry loaded onto Celite under reduced pressure. The crude was purified by flash column chromatography (12 g HP Silica, 0-60 %, ethyl acetate/hexanes) to afford the title compound as a white solid (45 mg). ¹H NMR (400 MHz, Chloroform-d) δ 8.24 - 8.18 (m, 1H), 7.82 - 7.75 (m, 1H), 7.16 - 7.09 (m, 1H), 5.03 (s, 2H), 4.33 (q, 2H), 1.61 - 1.52 (m, 3H); LC-MS (method 4): Rₜ = 1.31 min; MS (ESlneg): m/z = 268 [M-H]⁻

### Intermediate 25

### 1-Ethoxy-4-fluoro-2-iodo-benzene

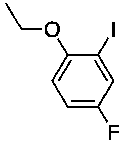

To a stirred solution of 4-fluoro-2-iodo-phenol (1.00 g, 4.20 mmol, 1.00 eq) in anhydrous N,N-dimethylformamide (8.40 mL, 0.50 M) was added potassium carbonate (1.16 g, 8.40 mmol, 2.00 eq) and stirred at room temperature. After five minutes, iodoethane (510 µL, 6.30 mmol, 1.50 eq) was added, and the reaction was left to stir at 70 °C for 17 h. The reaction was washed with water (20 mL), and the aqueous layer was extracted with dichloromethane (3 x 20 mL). The combined organics washed with brine (20 mL), dried over magnesium sulfate, filtered, and dry loaded onto Celite under reduced pressure. The crude was purified by flash column chromatography (80 g HP Silica, 0-20 %, hexanes/ethyl acetate) to afford the title compound as a clear oil (1.02 g). ¹H NMR (400 MHz, Chloroform-d) δ 7.50 (dd, 1H), 7.05 - 6.95 (m, 1H), 6.73 (dd, 1H), 4.04 (q, 2H), 1.46 (t, 3H); LC-MS (method 4): Rₜ = 1.65 min; MS (ESlpos): m/z = not detected

### Intermediate 26

### 2-Ethoxy-5-fluoro-benzenesulfonyl chloride

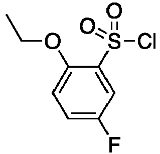

To an oven dried 40 mL vial under an argon atmosphere was added palladium(ll) acetate (43.2 mg, 0.19 mmol, 5.00 mol%), CataCXium^{®} A (110 mg, 0.31 mmol, 7.50 mol%), and 1,4-Diazabicyclo[2.2.2]octane bis(sulfur dioxide) adduct (554 mg, 2.31 mmol, 0.60 eq.) were sequentially added argon degassed anhydrous isopropanol (19.2 mL, 0.20 M), 1-ethoxy-4-fluoro-2-iodo-benzene (1023 g, 3.85 mmol, 1.00 eq.), and triethylamine (1.60 mL, 11.5 mmol, 3.00 eq.). The resulting red solution was heated at 75 °C for 16 h, cooled to room temperature and concentrated under reduced pressure. The residue was resuspended in anhydrous tetrahydrofuran (19.2 mL) and cooled to 0 °C in a ice/water bath. To this mixture was then added sulfuryl chloride (930 µL, 11.5 mmol, 3.00 eq.) dropwise, after which the resulting mixture was warmed to room temperature and stirred for a further 30 minutes. The reaction was quenches with ammonium chloride (20 mL), and the aqueous layer was extracted with dichloromethane (3 x 20 mL). The combined organics were washed with brine (20 mL), dried over magnesium sulfate, filtered, and dry loaded onto Celite under reduced pressure. The crude was purified by flash column chromatography (80 g HP Silica, 0-20 %, ethyl acetate/hexanes) to afford the title compound as an oil (295 mg). 1H NMR (400 MHz, Chloroform-d) δ 7.69 (dd, 1H), 7.43 - 7.33 (m, 1H), 7.07 (dd, 1H), 4.26 (q, 2H), 1.59 - 1.49 (m, 3H); LC-MS (method 4): Rt = 1.55 min; MS (ESlneg): m/z = not detected

### Intermediate 27

### 2-Ethoxy-5-fluoro-benzenesulfonamide

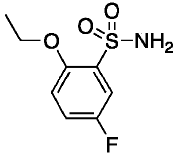

To a stirred solution of 2-ethoxy-5-fluoro-benzenesulfonyl chloride (295 mg, 1.22 mmol, 1.00 eq) in anhydrous tetrahydrofuran (2.03 mL, 0.60 mmol) was added ammonium hydroxide solution (20 % in water, 4.73 mL, 24.3 mmol, 20.0 eq) and stirred at room temperature for 30 minutes. The reaction was quenched with hydrochloric acid (1.00 M, 10 mL), and the aqueous layer was extracted with ethyl acetate (3 x 10 mL). The combined organics were washed with brine (10 mL), dried over magnesium sulfate, filtered, and dry loaded onto Celite under reduced pressure. The crude was purified by flash column chromatography (12 g HP Silica, 0-60 %, ethyl acetate/hexanes) to afford the title compound as a white solid (233 mg). ¹H NMR (400 MHz, Chloroform-d) δ 7.65 (dd, 1H), 7.22 (ddd, 1H), 7.04 - 6.95 (m, 1H), 5.05 (s, 2H), 4.23 (q, 2H), 1.54 - 1.48 (m, 2H); LC-MS (method 4): Rₜ = 1.21 min; MS (ESlneg): m/z = 218 [M-H]⁻

### Intermediate 28

### 1-(Methoxymethoxy)-4-phenoxy-benzene

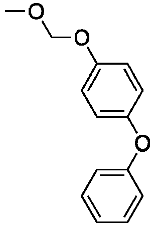

To a 0 °C stirred solution of 4-phenoxyphenol (7.27 g, 39.0 mmol, 1.00 eq.) in anhydrous dichloromethane (130 mL, 0.30 M) was added chloromethyl methyl ether (4.45 mL, 58.6 mmol, 1.50 eq.) dropwise. The resulting solution was warmed to room temperature and stirred for 24 h, after which the mixture was diluted with water (200 mL) and extracted with dichloromethane (3 x 100 mL). The combined organic extracts were washed with hydrochloric acid (1.00 M in water, 200 mL), sodium hydroxide (1.00 M in water, 200 mL), brine (200 mL), dried (magnesium sulfate), filtered, and dry loaded onto Celite under reduced pressure. The residue was purified by flash column chromatography (330 g Silica, 0-20% ethyl acetate/hexanes gradient) to give the title compound as a colourless oil (6.76 g). ¹H NMR (400 MHz, Chloroform-d) δ 7.34 - 7.27 (m, 2H), 7.08 - 6.93 (m, 7H), 5.15 (s, 2H), 3.50 (s, 3H); LC-MS (method 5): Rt= 2.99 min; MS (ESlneg): m/z = 229 [M-H]⁻

### Intermediate 29

### 2-lodo-1-(methoxymethoxy)-4-phenoxy-benzene

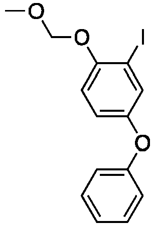

To a -78 °C stirred solution of 1-(methoxymethoxy)-4-phenoxy-benzene (6.76 g, 29.4 mmol, 1.00 eq.) in anhydrous tetrahydrofuran (147 mL, 0.20 M) was added n-butyllithium solution (2.50 M in hexanes, 13.5 mL, 33.8 mmol, 1.15 eq.) dropwise, afterwhich the yellow solution was warmed to room temperature and stirred for 30 minutes. The mixture was then cooled back to -78 °C and a solution of iodine (8.64 g, 34.1 mmol, 1.16 eq.) in anhydrous tetrahydrofuran (169 mL, 0.20 M) was added dropwise via cannula. After complete addition, the mixture was warmed to room temperature and stirred for 30 minutes. The mixture was then quenched with sodium thiosulfate (10 wt.% in water, 200 mL), partially concentrated under reduced pressure and extracted with diethyl ether (3 x 200 mL). The combined organic extracts were washed with brine (200 mL), dried (magnesium sulfate), filtered and concentrated under reduced pressure. The residue was purified by flash reverse phase column chromatography (275 g HP C₁₈, 40-100% acetonitrile/water bufferd with 0.1% formic acid) to give the title compound as a colourless oil (6.68 g). ¹H NMR (400 MHz, Chloroform-d) δ 7.46 (d, 1H), 7.39 - 7.27 (m, 2H), 7.14 - 7.01 (m, 2H), 7.04 - 6.92 (m, 3H), 5.20 (s, 2H), 3.54 (s, 3H); LC-MS (method 5): Rₜ = 3.34 min; MS (ESlpos): m/z = not found.

### Intermediate 30

### 2-lodo-4-phenoxy-phenol

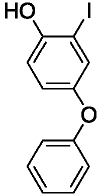

To a stirred solution of sodium iodide (5.62 g, 37.5 mmol, 2.00 eq) and 2-iodo-1-(methoxymethoxy)-4-phenoxy-benzene (6.68 g, 18.8 mmol, 1.00 eq) in anhydrous acetonitrile (94.0 mL, 0.20 M) was added trimethylsilyl chloride (3.55 mL, 28.1 mmol, 1.50 eq) dropwise at 0 °C and stirred for 10 minutes then warmed to room temperature. After 3 h, the reaction was quenched with aqueous sodium thiosulfate (10 % w/w, 50 mL), and the aqueous layer was extracted with diethyl ether (3 x 30 mL). The combined organics were dried over sodium sulfate, filtered, and dry loaded onto Celite under reduced pressure. The crude was purified using flash column chromatography (120 g, 0-30 %, hexane/ethyl acetate) to afford the title compound as an orange oil (5.53 g). ¹H NMR (400 MHz, Chloroform-d) δ 7.37 - 7.27 (m, 3H), 7.13 - 7.03 (m, 1H), 7.00 - 6.91 (m, 4H), 5.12 (s, 1H); LC-MS (method 5): Rt = 2.87 min; MS (ESlneg): m/z = 310 [M-H]⁻

### Intermediate 31

### 1-Ethoxy-2-iodo-4-phenoxy-benzene

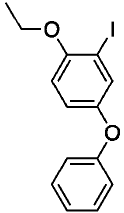

To a stirred solution of 2-iodo-4-phenoxy-phenol (1.50 g, 4.81 mmol, 1.00 eq) in anhydrous N,N-dimethylformamide (9.61 mL, 0.50 M) was added potassum carbonate (1.33 g, 9.61 mmol, 2.00 eq) and stirred at room temperature. After five minutes, iodoethane (410 µL, 5.29 mmol, 1.10 eq) was added and stirred at 100 °C for 16 h. The reaction was washed with water (30 mL), and the aqueous layer was extracted with dichloromethane (3 x 30 mL). The combined organic layers were washed with brine (30 mL), dried over magnesium sulfate, filtered, and dry loaded onto Celite under reduced pressure. The crude was purified using flash column chromatography (80 g HP Silica, 0-20 %, hexane/ethyl acetate) to afford the title compound as an oil (1.49 g). ¹H NMR (400 MHz, Chloroform-d) δ 7.47 (d, 1H), 7.36 - 7.27 (m, 2H), 7.12 - 7.03 (m, 1H), 7.03 - 6.90 (m, 3H), 6.81 - 6.74 (m, 1H), 4.07 (q, 2H), 1.48 (t, J = 6.9 Hz, 3H); LC-MS (method 4): Rt = 1.79 min; MS (ESlpos): m/z = not detected

### Intermediate 32

### 2-Ethoxy-5-phenoxy-benzenesulfonyl chloride

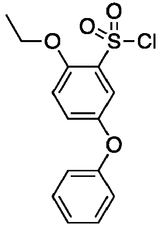

To an oven dried 40 mM vial under an argon atmosphere was added palladium(ll) acetate (49.2 mg, 0.22 mmol, 5.00 mol%), CataCXium^{®} A (126 mg, 0.35 mmol, 7.50 mol%), and 1,4-Diazabicyclo[2.2.2]octane bis(sulfur dioxide) adduct (632 mg, 2.63 mmol, 0.60 eq.) were sequentially added argon degassed anhydrous isopropanol (21.9 mL, 0.20 M), 1-ethoxy-2-iodo-4-phenoxy-benzene (1.49 g, 4.38 mmol, 1.00 eq.), and triethylamine (1.82 mL, 13.2 mmol, 3.00 eq.). The resulting red solution was heated at 75 °C for 16 h, cooled to room temperature and concentrated under reduced pressure. The residue was resuspended in anhydrous tetrahydrofuran (21.9 mL) and cooled to 0 °C in a ice/water bath. To this mixture was then added sulfuryl chloride (1.06 mL, 13.2 mmol, 3.00 eq.) dropwise, after which the resulting mixture was warmed to room temperature and stirred for a further 30 minutes. The mixture was diluted with saturated ammonium chloride (30 mL) and extracted with dichloromethane (3 x 30 mL). The combined organic extracts were dried over magnesium sulfate, filterd, and dry loaded onto Celite under reduced pressure. The residue was purified by flash column chromatography (80 g HP Silica, 0-20% ethyl acetate/hexanes gradient) to give the title compound as an off white solid (687 mg). ¹H NMR (400 MHz, Chloroform-d) δ 7.62 (d, 1H), 7.40 - 7.29 (m, 3H), 7.14 (t, 1H), 7.07 (d, 1H), 7.01 - 6.93 (m, 2H), 4.27 (q, 2H), 1.56 - 1.50 (m, 3H); LC-MS (method 4): Rt = 1.69 min; MS (ESlpos): m/z = not detected

### Intermediate 33

### 2-Ethoxy-5-phenoxy-benzenesulfonamide

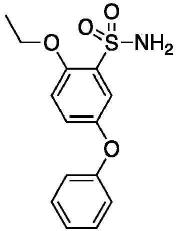

To a stirred solution of 2-ethoxy-5-phenoxy-benzenesulfonyl chloride (684 mg, 2.19 mmol, 1.00 eq) in anhydrous tetrahydrofuran (2.64 mL, 0.6 M) was added ammonium hydroxide (20 % in water, 8.52 mL, 43.7 mmol, 20.0 eq) and stirred at room temperature for 30 minutes. The reaction was washed with hydrochloric acid (1.00 M, 15 mL), and the aqueous layer was extracted with ethyl acetate (3 x 20 mL). The combined organic layers were washed with brine (20 mL), dried over magnesium sulfate, filtered, and dry loaded onto Celite under reduced pressure. The crude was purified by flash column chromatography (40 g HP Silica, 0-60 %, ethyl acetate/hexane) to give the title compound as a white solid (590 mg). ¹H NMR (400 MHz, Chloroform-d) δ 7.60 (d, 1H), 7.38 - 7.28 (m, 2H), 7.24 - 7.16 (m, 1H), 7.15 - 7.06 (m, 1H), 7.04 - 6.99 (m, 1H), 6.96 (dd, 2H), 5.04 (s, 2H), 4.24 (q, 2H), 1.57 - 1.48 (m, 3H); LC-MS (method 4): R^{t} = 1.42 min; MS (ESlpos): m/z = 294 [M+1]⁺

### Intermediate 34

### 2-lodo-4-(trifluoromethoxy)phenol

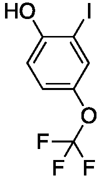

To a stirred solution of 4-(trifluoromethoxy)phenol (4.90 g, 27.5 mmol, 1.00 eq) in acetonitrile (13.8 mL, 2.00 M) was added p-toluenesulfonic acid monohydrate (4.74 g, 27.5 mmol, 1.00 eq) at room temperature. After ten minutes, N-iodosuccinimide (6.19 g, 27.5 mmol, 1.00 eq) was added to the reaction mixture. The reaction stirred for 6 h before being quenched with aqueous sodium sulfite (10 % w/w, 30 mL). The mixture was acidified using aqueous hydrochloric acid (1.00 M, 30 mL), and the aqueous layer was extracted with ethyl acetate (3 x 30 mL). The combined organic layers were dried over magnesium sulfate, filtered, and dry loaded onto Celite under reduced pressure. The crude was purified by flash column chromatography (120 g HP Silica, 0-20%, ethyl acetate/hexanes) to afford the desired product as an orange oil (6.03 g). ¹H NMR (400 MHz, Chloroform-d) δ 7.56 - 7.50 (m, 1H), 7.18 - 7.10 (m, 1H), 7.03 - 6.95 (m, 1H), 5.40 - 5.29 (m, 1H); LC-MS (method 4): Rt = 1.75 min; MS (ESlpos): m/z = 303 [M-H]⁻

### Intermediate 35

### 1-Ethoxy-2-iodo-4-(trifluoromethoxy)benzene

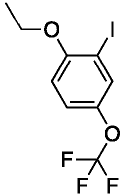

To a solution of 2-iodo-4-(trifluoromethoxy)phenol (1.50 g, 4.93 mmol, 1.00 eq) in anhydrous N,N-dimethylformamide (9.87 mL, 0.50 M) was added potassium carbonate (1.36 g, 9.87 mmol, 2.00 eq) and stirred at room temperature. After five minutes, iodoethane (440 µL, 5.43 mmol, 1.10 eq) was added, and the reaction stirred for 16 h at 70 °C. The reaction was washed with water (20 mL), and the aqueous was extracted with dichloromethane (3 x 20 mL). The combined organic layers were washed with brine (20 mL), dried over magnesium sulfate, filtered, and dry loaded onto Celite under reduced pressure. The crude was purified using flash column chromatography (80 g HP Silica, 0-30 %, hexane/ethyl acetate) to afford the title compound as an oil (922 mg). 1H NMR (400 MHz, Chloroform-d) δ 7.68 - 7.62 (m, 1H), 7.21 - 7.13 (m, 1H), 6.80 - 6.72 (m, 1H), 4.08 (q, 2H), 1.48 (t, 3H); LC-MS (method 4): Rt = 1.75 min; MS (ESlpos): m/z = not detected

### Intermediate 36

### 2-Ethoxy-5-(trifluoromethoxy)benzenesulfonyl chloride

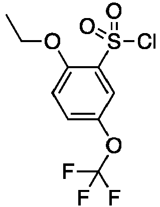

To an oven dried 40 mL vial under an argon atmosphere were added palladium(ll) acetate (31.4 mg, 0.14 mmol, 5.00 mol%), CataCXium^{®} A (75.1 mg, 0.21 mmol, 7.50 mol%), and 1,4-Diazabicyclo[2.2.2]octane bis(sulfur dioxide) adduct (403 mg, 1.68 mmol, 0.60 eq.) were sequentially added argon degassed anhydrous isopropanol (14.0 mL, 0.20 M), 1-benzyloxy-2-iodo-4-isopropyl-benzene (928 mg, 2.79 mmol, 1.00 eq.), and triethylamine (1.17 mL, 8.38 mmol, 3.00 eq.). The resulting red solution was heated at 75 °C for 16 h, cooled to room temperature and concentrated under reduced pressure. The residue was resuspended in anhydrous tetrahydrofuran (14.0 mL) and cooled to 0 °C in a ice/water bath. To this mixture was then added sulfuryl chloride (680 µL, 8.38 mmol, 3.00 eq.) dropwise, after which the resulting mixture was warmed to room temperature and stirred for a further 30 minutes. The mixture was diluted with saturated ammonium chloride (30 mL) and extracted with dichloromethane (3 x 30 mL). The combined organic extracts were dried over magnesium sulfate, filterd, and dry loaded onto Celite under reduced pressure. The residue was purified by flash column chromatography (80 g HP Silica, 0-20% ethyl acetate/hexanes gradient) to give the title compound as a white solid (192 mg). 1H NMR (400 MHz, Chloroform-d) δ 7.84 (d, 1H), 7.57 - 7.48 (m, 1H), 7.15 - 7.08 (m, 1H), 4.30 (q, 2H), 1.56 (t, 3H). LC-MS (method 4): Rₜ = 1.64 min; MS (ESlpos): m/z = not detected

### Intermediate 37

### 2-Ethoxy-5-(trifluoromethoxy)benzenesulfonamide

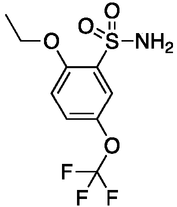

To a room temperature stirred solution of 2-ethoxy-5-(trifluoromethoxy)benzenesulfonyl chloride (192 mg, 0.63 mmol, 1.00 eq.) in anhydrous tetrahydrofuran (1.05 mL, 0.60 M) was added ammonium hydroxide solution (20% in water, 2.45 mL, 12.6 mmol, 20.0 eq.). The resulting suspension was stirred at room temperature for 30 mintues and then diluted with hydrochloric acid (1.00 M in water, 10 mL). The mixture was extracted with ethyl acetate (3 x 10 mL) and the combined organic extracts were washed with brine (10 mL), dried over magnesium sulfate, filtered, and dry loaded onto Celite. The residue was purifed by flash column chromatography (12 g HP Silica, 0-60% ethyl acetate/hexanes gradient) to give the title compound as a white solid (154 mg). 1H NMR (400 MHz, Chloroform-d) δ 7.82 (d, 1H), 7.43 - 7.35 (m, 1H), 7.04 (d, 1H), 5.04 (s, 2H), 4.27 (q, 2H), 1.58 - 1.50 (m, 3H); LC-MS (method 5): Rₜ = 1.37 min; MS (ESlneg): m/z = 284 [M-H]⁻

### Intermediate 38

### 2-lodo-4-isopropyl-phenol

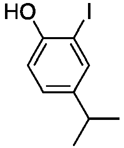

To a stirred solution of 4-isopropylphenol (10.0 g, 73.4 mmol, 1.00 eq) in acetonitrile (36.7 mL) was added p-toluenesulfonic acid monohydrate (12.6 g, 36.7 mmol, 1.00 eq) at room temperature. After twenty minutes, N-iodosuccinimide (16.5 g, 73.43 mmol, 1.00 eq) was added to the reaction mixture. The reaction stirred for 45 minutes before being quenched with aqueous sodium sulfite (10 % w/w). The mixture was acidified using aqueous hydrochloric acid (1.00 M), and the aqueous layer was extracted ethyl acetate (3 x 50 mL). The combined organic layers were dried over magnesium sulfate, filtered, and dry loaded onto Celite under reduced pressure. The crude was purified by flash column chromatography (330 g silica, 0-20% ethyl acetate/hexanes) to afford the desired product as an orange oil. ¹H NMR (400 MHz, Chloroform-d) δ 7.50 (d, 1H), 7.10 (dd, 1H), 6.92 (d, 1H), 5.12 (s, 1H), 2.82 (hept, 1H), 1.21 (d, 7H); LC-MS (method 5): Rt = 2.90 min; MS (ESlneg): m/z = 261 [M-H]⁻

### Intermediate 39

### 1-Benzyloxy-2-iodo-4-isopropyl-benzene

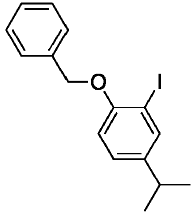

To a stirred solution of 2-iodo-4-isopropyl-phenol (1.00 g, 3.82 mmol, 1.00 eq) in anhydrous DMF (7.63 mL, 0.50 M) was added potassium carbonate (1.05 g, 7.63 mmol, 2.00 eq), and the reaction stirred for 5 minutes at room temperature. Benzyl bromide (540 µL, 4.58 mmol, 1.20 eq) was added, and the reaction was left stirring at 100 °C for 7 h. The reaction was cooled to room temperature, the mixture was washed with water (20 mL), and the aqueous layer was extracted with dichloromethane (3 x 20 mL). The combined organic layers were washed with brine (20 mL), dried over magnesium sulfate, filtered, and dry loaded onto Celite under reduced pressure. The crude was purified by flash column chromatography (80 g HP Silica, 0-10% ethyl acetate/hexanes) to afford the desired product as a clear oil (1.182 g). ¹H NMR (400 MHz, Chloroform-d) δ 7.66 (d, 1H), 7.54 - 7.46 (m, 2H), 7.44 - 7.35 (m, 2H), 7.35 - 7.28 (m, 1H), 7.12 (dd, 1H), 6.82 - 6.75 (m, 1H), 5.13 (s, 2H), 2.82 (hept, 1H), 1.24 - 1.16 (m, 6H); LC-MS (method 5): Rt = 3.80 min; MS (ESlpos): m/z = not detected

### Intermediate 40

### 2-benzyloxy-5-isopropyl-benzenesulfonyl chloride

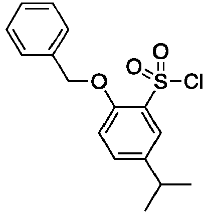

To a flame dried 40 mL vial under an argon atmosphere was added palladium(ll) acetate (17.2 mg, 0.08 mmol, 5.00 mol%), CataCXium^{®} A (41.2 mg, 0.11 mmol, 7.50 mol%), and 1,4-diazabicyclo[2.2.2]octane bis(sulfur dioxide) adduct (221 mg, 0.92 mmol, 0.60 eq.) was sequentially added argon degassed anhydrous isopropanol (7.67 mL, 0.20 M), 1-benzyloxy-2-iodo-4-isopropyl-benzene (540 mg, 1.53 mmol, 1.00 eq.), and triethylamine (0.64 mL, 4.60 mmol, 3.00 eq.). The resulting red solution was heated at 75 °C for 18 h, cooled to room temperature and concentrated under reduced pressure. The residue was resuspended in anhydrous tetrahydrofuran (7.67 mL) and cooled to 0 °C in a ice/water bath. To this mixture was then added sulfuryl chloride (0.37 mL, 4.60 mmol, 3.00 eq.) dropwise, after which the resulting mixture was warmed to room temperature and stirred for a further 30 minutes. The mixture was diluted with saturated ammonium chloride (50 mL) and extracted with dichloromethane (3 x 20 mL). The combined organic extracts were dried (magnesium sulfate), filterd and dry loaded onto Celite under reduced pressure. The residue was purified by flash column chromatography (24 g HP Silica, 0-20% ethyl acetate/hexanes gradient) to give the title compound as an off white solid (345 mg). ¹H NMR (400 MHz, Chloroform-d) δ 7.82 (d, 1H), 7.55 - 7.50 (m, 2H), 7.48 (dd, 1H), 7.44 - 7.37 (m, 2H), 7.37 - 7.30 (m, 1H), 7.06 (d, 1H), 5.33 (s, 2H), 2.93 (hept, 1H), 1.25 (d, 6H); LC-MS (method 5): Rₜ = 3.48 min; MS (ESlpos): m/z = not detected

### Intermediate 41

### 2-Benzyloxy-5-isopropyl-benzenesulfonamide

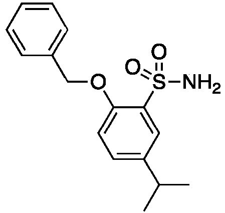

To a room temperature stirred solution of 2-benzyloxy-5-isopropyl-benzenesulfonyl chloride (340 mg, 1.05 mmol, 1.00 eq.) in anhydrous tetrahydrofuran (1.74 mL, 0.60 M) was added ammonium hydroxide solution (28% in water, 2.91 mL, 20.9 mmol, 20.0 eq.). The resulting suspension was stirred at room temperature for 30 mintues and then diluted with hydrochloric acid (1.00 M in water, 20 mL). The mixture was extracted with ethyl acetate (3 x 20 mL) and the combined organic extracts were washed with brine (20 mL), dired (magnesium sulfate) filtered and dry loaded onto Celite. The residue was purifed by flash column chromatography (40 g HP Silica, 0-60% ethyl acetate/hexanes gradient) to give the title compound as a colourless oil (305 mg). 1H NMR (400 MHz, Chloroform-d) δ 7.80 (d, 1H), 7.53 - 7.46 (m, 2H), 7.48 - 7.32 (m, 4H), 7.03 (d, 1H), 5.23 (s, 2H), 4.95 (s, 2H), 2.92 (hept, 1H), 1.24 (d, 6H). LC-MS (method 5): Rₜ = 2.73 min; MS (ESlneg): m/z = 304 [M-H]⁻

### Intermediate 42

### 1-(Cyclopropylmethoxy)-2-iodo-4-isopropyl-benzene

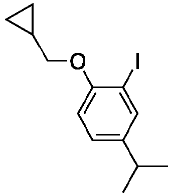

To a stirred solution of 2-iodo-4-isopropyl-phenol (1.31 g, 4.99 mmol, 1.00 eq) in anhydrous DMF (9.98 mL) was added potassium carbonate (1.38 g, 9.98 mmol, 2.00 eq), and the reaction stirred for 5 minutes at room temperature. (lodomethyl)cyclopropane (0.510 mL, 5.49 mmol, 1.10 eq) was added, and the reaction was left stirring at 100 °C for 20 h. The reaction was cooled to room temperature, the mixture was washed with water (20 mL), and the aqueous layer was extracted with methylene chloride (3 x 20 mL). The combined organic layers were washed with brine (20 mL), dried over magnesium sulfate, filtered, and dry loaded onto Celite under reduced pressure. The crude was purified by flash column chromatography (80 g silica, 0-10% ethyl acetate/hexanes) to afford the desired product as a clear oil (606 mg). ¹H NMR (400 MHz, Chloroform-d) δ 7.63 (d, 1H), 7.11 (dd, 1H), 6.73 (d, 1H), 3.86 (d, 2H), 2.81 (hept, 1H), 1.35 - 1.24 (m, 1H), 1.21 (d, 6H), 0.68 - 0.56 (m, 2H), 0.47 - 0.34 (m, 2H); LC-MS (method 4): Rt = 1.76 min; MS (ESlpos): m/z = not detected

### Intermediate 43

### 2-(Cyclopropylmethoxy)-5-isopropyl-benzenesulfonyl chloride

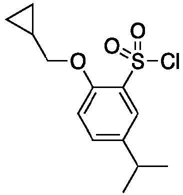

To an oven dried 40 mL vial under an argon atmosphere was added palladium(ll) acetate (21.3 mg, 0.0900 mmol, 5.00 mol%), CataCXium^{®} A (51.0 mg, 0.140 mmol, 7.50 mol%), and 1,4-diazabicyclo[2.2.2]octane bis(sulfur dioxide) adduct (274 mg, 1.14 mmol, 0.600 eq.) were sequentially added argon degassed anhydrous isopropanol (9.49 mL, 0.20 M), 1-(cyclopropylmethoxy)-2-iodo-4-isopropyl-benzene (600 mg, 1.90 mmol, 1.00 eq.), and triethylamine (790 µL, 5.69 mmol, 3.00 eq.). The resulting red solution was heated at 75 °C for 15 h, cooled to room temperature and concentrated under reduced pressure. The residue was resuspended in anhydrous tetrahydrofuran (9.49 mL) and cooled to 0 °C in a ice/water bath. To this mixture was then added sulfuryl chloride (460 µL, 5.69 mmol, 3.00 eq.) dropwise, after which the resulting mixture was warmed to room temperature and stirred for a further 30 minutes. The mixture was diluted with saturated ammonium chloride (20 mL) and extracted with dichloromethane (3 x 20 mL). The combined organic extracts were dried over magnesium sulfate, filterd, and dry loaded onto Celite under reduced pressure. The residue was purified by flash column chromatography (40 g HP Silica, 0-20% ethyl acetate/hexanes gradient) to give the title compound as a white solid (425 mg). ¹H NMR (400 MHz, Chloroform-d) δ 7.81 - 7.76 (m, 1H), 7.52 - 7.45 (m, 1H), 7.04 - 6.97 (m, 1H), 4.07 (d, 2H), 3.01 - 2.85 (m, 1H), 1.42 - 1.29 (m, 1H), 1.29 - 1.22 (m, 6H), 0.74 - 0.61 (m, 2H), 0.47 (dt, 2H); LC-MS (method 4): Rt = 1.73 min; MS (ESlpos): m/z = not detected

### Intermediate 44

### 2-(Cyclopropylmethoxy)-5-isopropyl-benzenesulfonamide

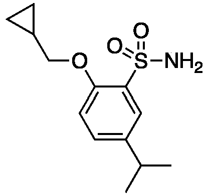

To a room temperature stirred solution of 2-(cyclopropylmethoxy)-5-isopropyl-benzenesulfonyl chloride (425 mg, 1.47 mmol, 1.00 eq.) in anhydrous tetrahydrofuran (2.45 mL, 0.60 M) was added ammonium hydroxide solution (20% in water, 5.73 mL, 29.4 mmol, 20.0 eq.). The resulting suspension was stirred at room temperature for 30 mintues and then diluted with hydrochloric acid (1.00 M in water, 20 mL). The mixture was extracted with ethyl acetate (3 x 20 mL) and the combined organic extracts were washed with brine (20 mL), dried over magnesium sulfate, filtered, and dry loaded onto Celite. The residue was purifed by flash column chromatography (40 g HP Silica, 0-60% ethyl acetate/hexanes gradient) to give the title compound as a white solid (335 mg). ¹H NMR (400 MHz, Chloroform-d) δ 7.77 (d, 1H), 7.35 (dd, 1H), 6.96 - 6.88 (m, 1H), 5.10 (s, 2H), 3.98 (d, 2H), 2.91 (hept, 1H), 1.45 - 1.30 (m, 1H), 1.30 - 1.20 (m, 6H), 0.76 - 0.63 (m, 2H), 0.46 - 0.32 (m, 2H); LC-MS (method 4): Rₜ = 1.46 min; MS (ESlneg): m/z = 268 [M-H]⁻

### Intermediate 45

### 2-iodo-4-isopropyl-1-(2,2,2-trifluoroethoxy)benzene

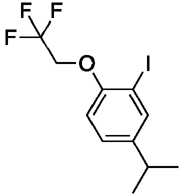

To a stirred solution of 2-iodo-4-isopropyl-phenol (1.13 g, 4.33 mmol, 1.00 eq) in anhydrous N,N-dimethylformamide (8.66 mL) was added potassium carbonate (1.20 g, 8.66 mmol, 2.00 eq), and the reaction stirred for 5 minutes at room temperature. 1,1,1-trifluoro-2-iodoethane (0.470 mL, 4.76 mmol, 1.10 eq) was added, and the reaction was left stirring at 100 °C for 20 h. The reaction was cooled to room temperature, the mixture was washed with water (20 mL), and the aqueous layer was extracted with methylene chloride (3 x 20 mL). The combined organic layers were washed with brine (20 mL), dried over magnesium sulfate, filtered, and dry loaded onto Celite under reduced pressure. The crude was purified by flash column chromatography (80 g, 0-10% ethyl acetate/hexanes) to afford the desired product as a clear oil (647 mg). ¹H NMR (400 MHz, Chloroform-d) δ 7.66 (d, 1H), 7.16 (dd, 1H), 6.79 (d1H), 4.36 (q, 2H), 2.84 (hept, 1H), 1.22 (d, 6H); LC-MS (method 4): Rₜ = 1.76 min; MS (ESlpos): m/z = not detected.

### Intermediate 46

### 5-isopropyl-2-(2,2,2-trifluoroethoxy)benzenesulfonyl chloride

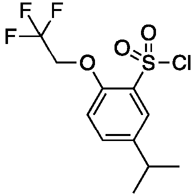

To a flame dried 40 mL vial under an argon atmosphere was added palladium(ll) acetate (21.1 mg, 0.09 mmol, 5.00 mol%), CataCXium^{®} A (50.6 mg, 0.14 mmol, 7.50 mol%), and 1,4-diazabicyclo[2.2.2]octane bis(sulfur dioxide) adduct (271 mg, 1.13 mmol, 0.600 eq.) were sequentially added argon degassed anhydrous isopropanol (9.40 mL, 0.20 M), 2-iodo-4-isopropyl-1-(2,2,2-trifluoroethoxy)benzene (647 mg, 1.88 mmol, 1.00 eq.), and triethylamine (790 µL, 5.64 mmol, 3.00 eq.). The resulting red solution was heated at 75 °C for 18 h, cooled to room temperature and concentrated under reduced pressure. The residue was resuspended in anhydrous tetrahydrofuran (9.40 mL) and cooled to 0 °C in a ice/water bath. To this mixture was then added sulfuryl chloride (460 µL, 5.64 mmol, 3.00 eq.) dropwise, after which the resulting mixture was warmed to room temperature and stirred for a further 30 minutes. The mixture was diluted with saturated ammonium chloride (20 mL) and extracted with dichloromethane (3 x 20 mL). The combined organic extracts were dried over magnesium sulfate, filtered, and dry loaded onto Celite under reduced pressure. The residue was purified by flash column chromatography (40 g HP Silica, 0-20% ethyl acetate/hexanes gradient) to give the title compound as an off white solid (334 mg). ¹H NMR (400 MHz, Chloroform-d) δ 7.85 (d, 1H), 7.60 - 7.52 (m, 1H), 7.08 - 7.01 (m, 1H), 4.55 (q, 2H), 2.98 (hept, 1H), 1.32 - 1.23 (m, 6H); LC-MS (method 4): Rt = 1.62 min; MS (ESlpos): m/z = not detected

### Intermediate 47

### 5-isopropyl-2-(2,2,2-trifluoroethoxy)benzenesulfonamide

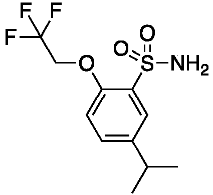

To a room temperature stirred solution of 5-isopropyl-2-(2,2,2-trifluoroethoxy)benzenesulfonyl chloride (330 mg, 1.04 mmol, 1.00 eq.) in anhydrous tetrahydrofuran (1.74 mL, 0.60 M) was added ammonium hydroxide solution (20 % in water, 4.06 mL, 20.8 mmol, 20.0 eq.). The resulting suspension was stirred at room temperature for 30 mintues and then diluted with hydrochloric acid (1.00 M in water, 20 mL). The mixture was extracted with ethyl acetate (3 x 20 mL) and the combined organic extracts were washed with brine (20 mL), dried over magnesium sulfate, filtered, and dry loaded onto Celite under reduced pressure. The residue was purifed by flash column chromatography (24 g HP Silica, 0-60% ethyl acetate/hexanes gradient) to give the title compound as a white solid (195 mg). ¹H NMR (400 MHz, Chloroform-d) δ 7.82 (d, 1H), 7.45 - 7.38 (m, 1H), 7.02 - 6.95 (m, 1H), 4.96 (s, 2H), 4.52 (q, 2H), 3.03 - 2.87 (m, 1H), 1.30 - 1.22 (m, 6H); LC-MS (method 4): Rt = 1.42 min; MS (ESlneg): m/z = 296 [M-H]⁻

### Intermediate 48

### 4-Bromo-2-(dimethylamino)-6-fluoro-benzaldehyde

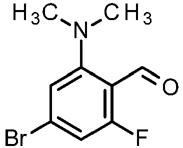

To a room temperature stirred suspension of 4-bromo-2,6-difluoro-benzaldehyde (4.42 g, 20.0 mmol, 1.00 eq.) and potassium carbonate (6.08 g, 44.0 mmol, 2.20 eq.) in anhydrous N,N-dimethylformamide (20.0 mL, 1.00 M) was added dimethylamine hydrochloride (1.79 g, 22.0 mmol, 1.10 eq.) in one portion. The resulting yellow suspension was heated at 50 °C for 18 h and then cooled to room temperature. The mixture was diluted with water (300 mL) and extrated with ethyl acetate (3 x 100 mL). The combined organic extracts were washed with water (2 x 100 mL), brine (100 mL), dried (magnesium sulfate), filterd and dry loaded onto Celite under reduced pressure. The residue was purifeid by flash column chromatography (330 g Silica, 0-30% ethyl acetate/hexanes gradient) to give the title compound as a yellow oil (4.09 g). ¹H NMR (400 MHz, Chloroform-d) δ 10.18 (s, 1H), 6.90 (s, 1H), 6.75 (dd, 1H), 2.92 (s, 6H); LC-MS (method 5): Rt = 2.66 min; MS (ESlpos): m/z = 246 [M+H]⁺

### Intermediate 49

### 4-Cyclopropyl-2-(dimethylamino)-6-fluoro-benzaldehyde

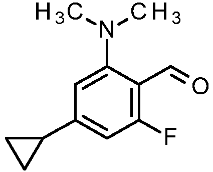

To a mixture of 4-bromo-2-(dimethylamino)-6-fluoro-benzaldehyde (3.57 g, 14.5 mmol, 1.00 eq.) and tripotassium phosphate (6.16 g, 29.0 mmol, 2.00 eq.) in argon degassed tetrahydrofuran (14.5 mL) and water (3.63 mL) was added cyclopropylboronic acid (1.87 g, 21.8 mmol, 1.50 eq.), palladium(ll) acetate (163 mg, 0.73 mmol, 5.00 mol%) and tricyclohexylphosphine tetrafluoroborate (534 mg, 1.45 mmol, 10.0 mol%). The resulting mixture was stirred at 80 °C for 4 h and then cooled to room temperature. The dark mixture was diluted with water (100 mL) and extracted with ethyl acetate (3 x 100 mL). The combined organic extracts were washed with brine (100 mL), dried (magnesium sulfate), filtered and dry loaded onto Celite under reduced pressure. The residue was purified by flash column chromatography (330 g silica, 0-30% ethyl acetate/hexanes gradient) to give the title compound as a orange oil (2.66 g, 98% purity). ¹H NMR (400 MHz, Chloroform-d) δ 10.18 (s, 1H), 6.51 (s, 1H), 6.19 (dd, 1H), 2.91 (s, 6H), 1.87 (tt, 1H), 1.12 - 0.99 (m, 2H), 0.82 - 0.70 (m, 2H); LC-MS (method 5): Rt = 2.57 min; MS (ESlpos): m/z = 208 [M+H]⁺

### Intermediate 50

### 4-Cyclopropyl-2-(dimethylamino)-6-hydroxy-benzaldehyde

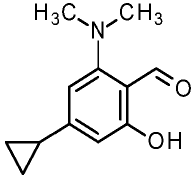

To a stirred solution of 4-cyclopropyl-2-(dimethylamino)-6-fluoro-benzaldehyde (2.65 g, 12.8 mmol, 1.00 eq.) in dimethylsulfoxide (6.39 mL, 2.00 M) was added potassium hydroxide (5.00 M in water, 6.39 mL, 32.0 mmol, 2.50 eq) dropwise. The resulting mixture was heated at 120 °C for 4 h and then cooled to room temperature. The mixture was diluted with water (100 mL), acidified to pH 2.0 with hydrochloric acid (1.00 M in water) and extracted with ethyl acetate (3 x 100 mL). The combined organic extracts were washed with water (2 x 100 mL), dired (magnesium sulfate), filtered and dry loaded onto Celite under reduced pressure. The residue was purified by flash column chromatography (80 g HP Silica, 0-30% ethyl acetate/hexanes gradient) to give the title compound as a yellow oil (1.32 g). ¹H NMR (400 MHz, Chloroform-d) δ 11.90 (s, 1H), 10.06 (s, 1H), 6.23 (d, J = 1.5 Hz, 1H), 6.13 (d, J = 1.4 Hz, 1H), 2.89 (s, 6H), 1.82 (tt, 1H), 1.09 - 0.97 (m, 2H), 0.83 - 0.72 (m, 2H); LC-MS (method 5): Rₜ = 2.73 min; MS (ESlpos): m/z = 206 [M+H]⁺

### Intermediate 51

### 6-Cyclopropyl-4-(dimethylamino)benzofuran-2-carboxylic acid

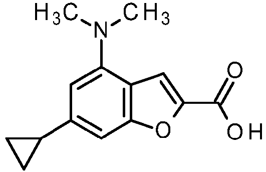

To a room temperature stirred solution of 4-cyclopropyl-2-(dimethylamino)-6-hydroxybenzaldehyde (1.24 g, 6.04 mmol, 1.00 eq.) in anhydrous N,N-dimethylformamide (34.5 mL, 0.15 M) was sequentially added potassium carbonate (1.25 g, 9.06 mmol, 1.50 eq.) and ethyl bromoacetate (0.73 mL, 6.65 mmol, 1.10 eq.). The resulting yellow suspension was heated at 160 °C for 2 h, after which sodium hydroxide (2.00 M in water, 7.55 mL, 15.1 mmol, 2.50 eq.) was added. After cooling to room temperature, the mixture was acidified to pH 2.0 with hydrochloric acid (1.00 M in water), diluted with water (100 mL) and extracted with ethyl acetate (3 x 100 mL). The combined organic extracts were washed with water (2 x 100 mL), brine (100 mL), dried (magnesium sulfate) and concentrated under reduced pressure. The residue was purified by reverse phase flash column chromatography (275 g HP C18, 10-100% acetonitrile/water gradient buffered with 0.1% formic acid) to give the title compound as a yellow solid (1.27 g). ¹H NMR (400 MHz, DMSO-d₆) δ 13.16 (s, 1H), 7.67 (d, 1H), 6.66 (s, 1H), 6.25 (d, 1H), 3.05 (s, 6H), 1.97 (tt, 1H), 1.00 - 0.89 (m, 2H), 0.78 - 0.67 (m, 2H); LC-MS (method 5): Rt = 2.30 min; MS (ESlpos): m/z = 246 [M+H]⁺

### Intermediate 52

### 4-Bromo-2-(difluoromethoxy)-6-fluoro-benzaldehyde

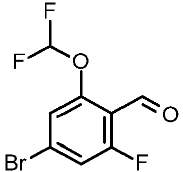

To a room temperature stirred solution of 4-bromo-2-fluoro-6-hydroxy-benzaldehyde (4.00 g, 18.3 mmol, 1.00 eq.) in DMF (30.4 mL, 0.60 M) was sequentially added water (3.29 mL, 183 mmol, 10.0 eq.), potassium carbonate (3.03 g, 21.9 mmol, 1.20 eq.) and sodium chlorodifluoroacetate (5.57 g, 36.5 mmol, 2.00 eq.). The resulting suspension was heated at 85 °C for 2 h and then cooled to 0 °C. Water (3.00 mL) and concentrated hydrochloric acid (2.00 mL) was added to the mixture after which the solution was warmed to room temperature and stirred for a further 30 minutes, followed by extraction with ethyl acetate (3 x 50.0 mL). The combined organic extracts were washed with water (2 x 50.0 mL), brine (50.0 mL), dried (magnesium sulfate) and dry loaded onto Celite under reduced pressure. The residue was purified by flash column chromatography (330 g Silica, 0-40% ethyl acetate/hexanes gradient) to give the title compound as a light yellow oil (2.42 g, 95% purity). ¹H NMR (400 MHz, Chloroform-d) δ 10.30 (d, 1H), 7.31 - 7.23 (m, 2H), 6.63 (t, 1H); LC-MS (method 5): Rt = 2.54 min; MS (ESlpos): m/z = not present

### Intermediate 53

### 4-Cyclopropyl-2-(difluoromethoxy)-6-fluoro-benzaldehyde

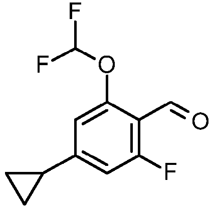

To a stirred solution of 4-bromo-2-(difluoromethoxy)-6-fluoro-benzaldehyde (108 mg, 0.40 mmol, 1.00 eq.) in tetrahydrofuran (0.40 mL, 1.00 M) was added water (0.04 mL), cyclopropylboronic acid (68.7 mg, 0.80 mmol, 2.00 eq.), tricyclohexylphosphine tetrafluoroborate (14.7 mg, 0.04 mmol, 10.0 mol%) and tripotassium phosphate (297 mg, 1.40 mmol, 3.50 eq.). The mixture was then degassed with argon, after which palladium(ll) acetate (4.49 mg, 0.05 mmol, 5.00 mol%) was added and the resulting yellow mixture was heated at 80 °C for 5 h. The mixture was diluted with hydrochloric acid (1.00 M in water, 50.0 mL) and extracted with ethyl acetate (3 x 50.0 mL). The combined organic extracts were washed with brine (50.0 mL), dried (magnesium sulfate), filtered and concentrated under reduced pressure. The residue was purified by flash column chromatography (330 g Silica, 0-30% ethyl acetate/hexanes gradient) to give the title compound as a yellow solid (1.08 g). ¹H NMR (400 MHz, Chloroform-d) δ 10.29 (d, 1H), 6.75 - 6.72 (m, 1H), 6.67 (dd, 1H), 6.61 (t, 1H), 1.93 (tt, 1H), 1.21 - 1.10 (m, 2H), 0.84 - 0.79 (m, 2H); LC-MS (method 5): Rₜ = 2.63 min; MS (ESlpos): m/z = not detected

### Intermediate 54

### 4-Cyclopropyl-2-(difluoromethoxy)-6-hydroxy-benzaldehyde

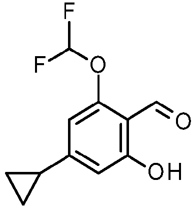

To a stirred solution of 4-cyclopropyl-2-(difluoromethoxy)-6-fluoro-benzaldehyde (711 mg, 3.09 mmol, 1.00 eq.) in dimethylsulfoxide (7.72 mL, 0.40 M) was added potassium hydroxide (1.00 M in water, 6.18 mL, 6.18 mmol, 2.00 eq.) dropwise. The resulting yellow solution was heated at 110 °C for 30 minutes and then cooled to room temperature. The reaction mixture was acidified to pH 2.0 with 1.0 M hydrochloric acid and extracted with ethyl acetate (3 x 50.0 mL). The combined organic extracts were washed with water (50.0 mL), brine (50.0 mL), dried (magnesium sulfate), filtered and dry loaded onto Celite under reduced pressure. The residue was purified by flash column chromatography (80 g HP Silica, 0-20% ethyl acetate/hexanes gradient) to give the title compound as a white solid (241 mg). ¹H NMR (400 MHz, Chloroform-d) δ 11.85 (s, 1H), 10.18 (s, 1H), 6.62 (t, 1H), 6.45 - 6.42 (m, 1H), 6.35 - 6.30 (m, 1H), 1.87 (tt, 1H), 1.19 - 1.04 (m, 2H), 0.88 - 0.75 (m, 2H); LC-MS (method 5): Rₜ = 2.80 min; MS (ESlneg): m/z =227 [M-H]⁻

### Intermediate 55

### 6-Cyclopropyl-4-(difluoromethoxy)benzofuran-2-carboxylic acid

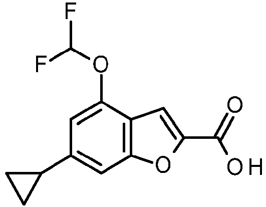

To a room temperature stirred solution of 4-cyclopropyl-2-(difluoromethoxy)-6-hydroxybenzaldehyde (238 mg, 1.04 mmol, 1.00 eq.) in anhydrous DMF (6.95 mL, 0.15 M) was sequentially added potassium carbonate (216 mg, 1.56 mmol, 1.50 eq.) and ethyl bromoacetate (127 µL, 1.15 mmol, 1.10 eq.). The resulting yellow suspension was heated at 160 °C for 2 h, after which sodium hydroxide (2.00 M in water, 1.30 mL, 2.61 mmol, 2.50 eq.) was added. After cooling to room temperature, the mixture was acidified to pH 2.0 with hydrochloric acid (1.00 M in water, - 4.20 mL), diluted with water (20.0 mL) and extracted with ethyl acetate (3 x 20.0 mL). The combined organic extracts were washed with water (20.0 mL), brine (20.0 mL), dried (magnesium sulfate) and concentrated under reduced pressure. The residue was purified by reverse phase flash column chromatography (100 g HP C18, 10-100% acetonitrile/ water gradient buffered with 0.1 % formic acid) to give the title compound as a white solid (235 mg). ¹H NMR (400 MHz, DMSO-d₆) δ 13.64 (s, 1H), 7.52 (s, 1H), 7.40 (t, 1H), 7.33 (s, 1H), 6.90 (s, 1H), 2.10 (tt, 1H), 1.10 - 1.00 (m, 2H), 0.85 - 0.78 (m, 2H); LC-MS (method 5): Rt = 2.49 min; MS (ESlneg): m/z = 267 [M-H]⁻

### Intermediate 56

### 1-Cyclopropyl-3-methoxy-5-(trifluoromethyl)benzene

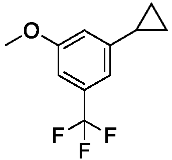

To a stirred solution of 1-bromo-3-methoxy-5-(trifluoromethyl)benzene (8.60 g, 33.7 mmol, 1.00 eq.) in tetrahydrofuran (67.4 mL, 0.50 M) was added water (6.74 mL), cyclopropylboronic acid (5.79 g, 67.4 mmol, 2.00 eq.), tricyclohexylphosphine tetrafluoroborate (1.24 g, 3.37 mmol, 10.0 mol%) and tripotassium phosphate (25.1 g, 118 mmol, 3.50 eq.). The mixture was then degassed with argon, after which palladium(ll) acetate (379 mg, 1.69 mmol, 5.00 mol%) was added and the resulting yellow mixture was heated at 80 °C for 16 h. The mixture was diluted with hydrochloric acid (1.00 M in water, 100 mL) and extracted with diethyl ether (3 x 100 mL). The combined organic extracts were washed with brine (100 mL), dried (magnesium sulfate), filtered and concentrated under reduced pressure. The residue was purified by flash column chromatography (330 g Silica, 0-30% ethyl acetate/hexanes gradient) to give the title compound as a colourless oil (6.01 g). ¹H NMR (400 MHz, Chloroform-d) δ 6.93 - 6.90 (m, 1H), 6.91 - 6.89 (m, 1H), 6.78 - 6.75 (m, 1H), 3.82 (s, 3H), 1.92 (tt, 1H), 1.05 - 0.97 (m, 2H), 0.76 - 0.69 (m, 2H); LC-MS (method 5): Rt = 3.09 min; MS (ESlpos): m/z = Not detected

### Intermediate 57

### 4-Cyclopropyl-2-methoxy-6-(trifluoromethyl)benzaldehyde

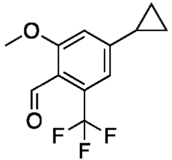

To a -5 °C stirred solution of 1-cyclopropyl-3-methoxy-5-(trifluoromethyl)benzene (6.01 g, 27.8 mmol, 1.00 eq.) in anhydrous diethyl ether (92.5 mL, 0.30 M) was added N,N,N',N'-tetramethylethylenediamine (5.20 mL, 34.7 mmol, 1.25 eq.) followed by dropwise addition of n-butyllithium (2.50 M in hexanes, 13.3 mL, 33.3 mmol, 1.20 eq.). The resulting solution was warmed to room temperature, stirred for 1 h and then cooled to -78 °C. Anhydrous N,N-dimethylformamide (3.01 mL, 38.9 mmol, 1.40 eq.) was added rapidly, after which the solution was stirred at -78 °C for 30 minutes and then warmed to room temperature. After a further 30 minutes at room temperature the mixture was poured onto ice water (100 mL) and extracted with diethyl ether (3 x 100 mL). The combined organic extracts were washed with brine (100 mL), dried (magnesium sulfate), filtered and dry loaded onto Celite under reduced pressure. The residue was purified by flash column chromatography (330 g silica, 0-40% ethyl acetate/hexanes gradient) to give the title compound as a white solid (4.45 g). ¹H NMR (400 MHz, Chloroform-d) δ 10.44 (q, 1H), 7.01 - 6.97 (m, 1H), 6.91 - 6.87 (m, 1H), 3.93 (s, 3H), 1.98 (tt, 1H), 1.16 - 1.10 (m, 2H), 0.86 - 0.80 (m, 2H); LC-MS (method 5): Rt = 2.73 min; MS (ESlpos): m/z = 245 [M+H]⁺

### Intermediate 58

### 4-Cyclopropyl-2-hydroxy-6-(trifluoromethyl)benzaldehyde

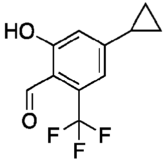

To a -5 °C stirred solution of 4-cyclopropyl-2-methoxy-6-(trifluoromethyl)benzaldehyde (4.45 g, 17.3 mmol, 1.00 eq.) in anhydrous dichloromethane (24.3 mL, 0.75 M) was added boron tribromide (1.00 M in dichloromethane, 26.0 mL, 26.0 mmol, 1.50 eq.) dropwise. Following complete addition, the dark solution was warmed to room temperature, stirred for 30 minutes and then poured onto ice. The mixture was diluted with water (100 mL) and extracted with dichloromethane (3 x 100 mL). The combined organic extracts were washed with hydrochloric acid (1.00 M in water, 100 mL), brine (100 mL), dried (magnesium sulfate), filtered and dry loaded onto Celite under reduced pressure. The residue was purified by flash column chromatography (120 g silica, 0-20% ethyl acetate/hexanes gradient) to give the title compound as a light yellow oil (3.85 g). ¹H NMR (400 MHz, Chloroform-d) δ 12.27 (s, 1H), 10.19 - 10.15 (m, 1H), 6.98 - 6.93 (m, 1H), 6.84 - 6.79 (m, 1H), 1.94 (tt, 1H), 1.19 - 1.12 (m, 2H), 0.88 - 0.83 (m, 2H); LC-MS (method 5): Rₜ = 2.95 min; MS (ESlneg): m/z = 229 [M-H]⁻

### Intermediate 59

### 6-Cyclopropyl-4-(trifluoromethyl)benzofuran-2-carboxylic acid

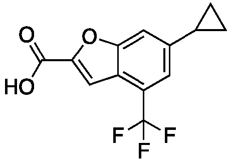

To a room temperature stirred solution of 4-cyclopropyl-2-hydroxy-6-(trifluoromethyl)benzaldehyde (3.85 g, 16.7 mmol, 1.00 eq.) in anhydrous N,N-dimethylformamide (112 mL, 0.15 M) was sequentially added potassium carbonate (3.47 g, 25.1 mmol, 1.50 eq.) and ethyl bromoacetate (2.03 mL, 18.4 mmol, 1.10 eq.). The resulting yellow suspension was heated at 160 °C for 2 h, after which sodium hydroxide (2.00 M in water, 20.9 mL, 41.8 mmol, 2.50 eq.) was added. After cooling to room temperature and concentration under reduced pressure, the residue was acidified to pH 2.0 with hydrochloric acid (1.00 M in water, - 70.0 mL) and extracted with ethyl acetate (3 x 100 mL). The combined organic extracts were washed with brine (100 mL), dried (magnesium sulfate), filtered and concentrated under reduced pressure. The reside was purified by reverse phase flash column chromatography (275 g HP C₁₈, 10-100% acetonitrile/ water gradient buffered with 0.1% formic acid) to give the title compound as a white solid (3.74 g). ¹H NMR (400 MHz, DMSO-d₆) δ 13.84 (s, 1H), 7.75 (s, 1H), 7.56 - 7.54 (m, 1H), 7.53 - 7.51 (m, 1H), 2.20 (tt, 1H), 1.12 - 1.04 (m, 2H), 0.91 - 0.84 (m, 2H); LC-MS (method 5): Rₜ = 2.72 min; MS (ESlneg): m/z = 269 [M-H]⁻

### Intermediate 60

### 6-(Propan-2-yl)-1-benzofuran-2-carboxylic acid

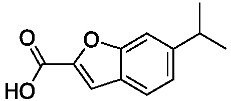

To a 0 °C stirred suspension of 2-hydroxy-4-(propan-2-yl)benzaldehyde (1.00 g, 6.08 mmol, 1.00 eq.) and potassium carbonate (1.26 g, 9.12 mmol, 1.50 eq.) in anhydrous N,N-dimethylformamide (12.1 mL, 0.50 M) was added ethyl bromoacetate (803 µL, 7.29 mmol, 1.20 eq.). Following complete addition, the mixture was heated at 160 °C for 17 h. After cooling to room temperature, the mixture was diluted with water (20 mL), neutralized with 1.0 M hydrochloric acid (10 mL) and then extracted with dichloromethane (3 x 20 mL). The combined organic extracts were dried (magnesium sulfate), filtered, dry loaded onto Celite and then purified by reverse phase flash column chromatography (80 g HP C₁₈, 40-100% water/acetonitrile gradient buffered with 0.1% formic acid) to give the title compound as a beige solid (950 mg). ¹H NMR (400 MHz, Chloroform-d) δ 7.67 - 7.59 (m, 2H), 7.46 (s, 1H), 7.25 - 7.21 (m, 1H), 3.06 (hept, 1H), 1.32 (d, 6H); LC-MS (Method 4): Rt = 1.40 min; MS (ESlpos): m/z = 205 [M+H]⁺

### Intermediate 61

### 4-Cyclopropyl-2-fluoro-6-methoxy-benzaldehyde

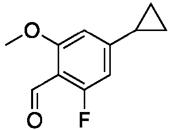

To a stirred suspension of 4-bromo-2-fluoro-6-methoxy-benzaldehyde (4.66 g, 20.0 mmol, 1.00 eq.) and tripotassium phosphate (8.49 g, 40.0 mmol, 2.00 eq.) in argon degassed tetrahydrofuran (20.0 mL) and water (5.00 mL) was added cyclopropylboronic acid (2.58 mg, 30.0 mmol, 1.50 eq.), tricyclohexylphosphine tetrafluoroborate (736 mg, 2.00 mmol, 10.0 mol%) and palladium(ll) acetate (225 mg, 1.00 mmol, 5.00 mol%). The resulting dark mixture was heated at 80 °C for 18 h and then cooled to room temperature. The mixture was diluted with water (200 mL) and extracted with ethyl acetate (3 x 100 mL). The combined organic extracts were washed with water (100 mL), brine (100 mL), dried (magnesium sulfate), filtered and dry loaded onto Celite under reduced pressure. The residue was purifeid by flash column chromatography (330 g Silica, 0-40% ehtyl acetate/hexanes) to give the title compound as yellow solid (3.00 g). ¹H NMR (400 MHz, Chloroform-d) δ 10.34 (d, 1H), 6.49 (t, 1H), 6.34 (dd, 1H), 3.92 (s, 3H), 1.96 - 1.85 (m, 1H), 1.17 - 1.03 (m, 2H), 0.79 (dt, 2H); LC-MS (method 5): Rt = 2.38 min; MS (ESlpos): m/z = 195 [M+H]⁺

### Intermediate 62

### 4-Cyclopropyl-2-imidazol-1-yl-6-methoxy-benzaldehyde

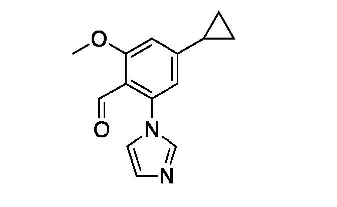

To a stirred suspension of 4-cyclopropyl-2-fluoro-6-methoxy-benzaldehyde (3.24 g, 16.7 mmol, 1.00 eq.) and potassium carbonate (4.61 g, 33.4 mmol, 2.00 eq.) in anhydrous dimethylsulfoxide (33.4 mL, 0.50 M) was added imidazole (1.70 g, 25.0 mmol, 1.50 eq.). The resulitng mixture was heated at 80 °C for 14 h and then cooled to room temperature. The mixture was diluted with water (200 mL) and extracted with ethyl acetate (3 x 100 mL). The combined organic extracts were washed with water (2 x 100 mL), brine (100 mL), dried (magnesium sulfate), filterd and concentrated under reduced pressure. The residue was purified by reverse phase flash column chromatgraphy (275 g HP C₁₈, 10-100% acetonitrile/water gradient buffered with 0.1% formic acid to give the title compound as a light yellow solid (2.18 g). ¹H NMR (400 MHz, Chloroform-d) δ 10.08 (s, 1H), 7.61 (s, 1H), 7.18 (s, 1H), 7.09 - 7.03 (m, 1H), 6.78 (d, 1H), 6.55 (d, 1H), 3.97 (s, 3H), 2.01 - 1.91 (m, 1H), 1.21 - 1.08 (m, 2H), 0.83 (dt, 2H); LC-MS (method 5): Rₜ = 1.33 min; MS (ESlpos): m/z = 243 [M+H]⁺

### Intermediate 63

### 4-Cyclopropyl-2-hydroxy-6-imidazol-1-yl-benzaldehyde

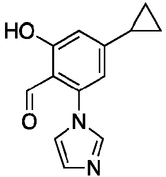

To a -5 °C stirred solution of 4-cyclopropyl-2-imidazol-1-yl-6-methoxy-benzaldehyde (2.09 g, 8.63 mmol, 1.00 eq.) in anhydrous dichloromethane (11.5 mL, 0.75 M) was added boron tribromide (1.00 M in dichloromethane, 21.6 mL, 21.6 mmol, 2.50 eq.) dropwise. Following complete addition, the solution was warmed to room temperature, stirred for 4 h and then poured onto ice. The mixture was diluted with saturated aqueous sodium bicarbonate (100 mL) and extracted with dichloromethane (3 x 100 mL). The combined organic extracts were dried (magnesium sulfate), filtered and dry loaded onto Celite under reduced pressure. The residue was purified by flash column chromatography (80 g HP Silica, 0-20% methanol/dichloromethane gradient) to give the title compound as a off white solid (1.33 g). ¹H NMR (400 MHz, Chloroform-d) δ 11.83 (s, 1H), 9.51 (s, 1H), 7.76 - 7.70 (m, 1H), 7.28 - 7.23 (m, 1H), 7.23 - 7.16 (m, 1H), 6.77 - 6.69 (m, 1H), 6.60 - 6.55 (m, 1H), 1.98 - 1.87 (m, 1H), 1.23 - 1.10 (m, 2H), 0.93 - 0.79 (m, 2H); LC-MS (method 5): Rt =1.46 min; MS (ESlpos): m/z = 229 [M+H]⁺

### Intermediate 64

### 6-Cyclopropyl-4-imidazol-1-yl-benzofuran-2-carboxylic acid

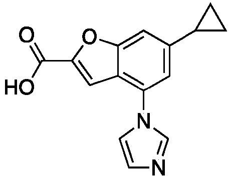

To a room temperature stirred solution of 4-cyclopropyl-2-hydroxy-6-imidazol-1-yl-benzaldehyde (1.33 g, 5.54 mmol, 1.00 eq.) in anhydrous N,N-dimethylformamide (31.6 mL, 0.15 M) was sequentially added potassium carbonate (1.15 g, 8.30 mmol, 1.50 eq.) and ethyl bromoacetate (0.67 mL, 6.09 mmol, 1.10 eq.). The resulting yellow suspension was heated at 160 °C for 2 h, after which sodium hydroxide (2.00 M in water, 6.92 mL, 13.8 mmol, 2.50 eq.) was added. After cooling to room temperature, the mixture was acidified to pH 2.0 with hydrochloric acid (1.00 M in water) and dry loeaded onto Celite under reduced pressure. The residue was purified by reverse phase flash column chromatography (275 g HP C18, 10-100% acetonitrile/water gradient buffered with 0.1% formic acid to give the title compound as a tan solid (832 mg, 98% purity). ¹H NMR (400 MHz, DMSO-d₆) δ 13.26 (s, 1H), 8.26 (s, 1H), 8.13 (s, 1H), 7.80 (d, 1H), 7.60 (s, 1H), 7.49 (d, J = 1.2 Hz, 1H), 7.26 (d, 1H), 7.19 (d, J = 1.1 Hz, 1H), 2.15 (tt, 1H), 1.12 - 1.00 (m, 2H), 0.97 - 0.86 (m, 2H); LC-MS (method 5): Rt = 1.24 min; MS (ESlpos): m/z = 269 [M+H]⁺

### Intermediate 65

### 4-(tert-Butyl)-1-cyclopropoxy-2-iodobenzene

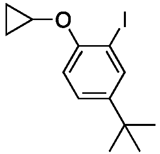

To a stirred solution of 4-tert-butyl-2-iodo-phenol (2.00 g, 7.24 mmol, 1.00 eq.) in anhdyrous DMF (24.1 mL, 0.30 M) was sequentially added cesium carbonate (4.72 g, 14.5 mmol, 2.00 eq.) and bromocyclopropane (1.45 mL, 18.1 mmol, 2.50 eq.). The resulting mixture was heated at 150 °C for 24 h and then cooled to room temperature. The reaction mixture was diluted with water (30 mL), and extracted with ethyl acetate (3 x 30 mL). The combined organic extracts were washed with brine (30 mL), dried (magnesium sulfate), filtered, and dry loaded onto Celite under reduced pressure. The residue was purified by flash column chromatography (120 g Silica, 0-20 %, ethyl acetate/hexanes gradient) to give the title compound as an orange oil (819 mg). ¹H NMR (400 MHz, Chloroform-d) δ 7.74 (d, 1H), 7.32 (dd, 1H), 7.13 - 7.06 (m, 1H), 3.83 - 3.74 (m, 1H), 1.31 - 1.26 (m, 9H), 0.91 - 0.74 (m, 4H); LC-MS (method 4): Rₜ = 1.79 min; MS (ESlpos): m/z = not detected

### Intermediate 66

### 5-(tert-Butyl)-2-cyclopropoxybenzenesulfonamide

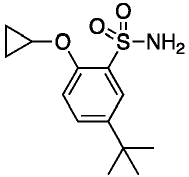

To a 40 mL vial was added 4-tert-butyl-1-(cyclopropoxy)-2-iodo-benzene (819 mg, 2.59 mmol, 1.00 eq), palladium(ll) acetate (29.1 mg, 0.13 mmol, 5.00 mol%), CataCXium^{®} A (74.3 mg, 0.21 mmol, 7.50 mol%), and 1,4-Diazabicyclo[2.2.2]octane bis(sulfur dioxide) adduct (373 mg, 1.55 mmol, 0.60 eq.) sequentially followed by the addition of degassed anhydrous isopropanol (13.0 mL, 0.20 M) and triethylamine (1.08 mL, 7.77 mmol, 3.00 eq.). The resulting red solution was heated at 75 °C for 17 h, cooled to room temperature and concentrated under reduced pressure. The residue was resuspended in anhydrous tetrahydrofuran (13.0 mL) and cooled to 0 °C in a ice/water bath. To this mixture was then added sulfuryl chloride (630 µL, 7.77 mmol, 3.00 eq.) dropwise, after which the resulting mixture was warmed to room temperature and stirred for a further 30 minutes. The reaction was quenched with ammonium chloride (20 mL), and the aqueous layer was extracted with dichloromethane (3 x 20 mL). The combined organics were washed with brine (20 mL), dried over magnesium sulfate, filtered, and dry loaded onto Celite under reduced pressure. The crude was purified by flash column chromatography (40 g HP Silica, 0-20%, ethyl acetate/hexanes) to afford 5-*tert*-butyl-2-(cyclopropoxy)benzenesulfonyl chloride as an oil, which was suspended in anhydrous tetrahydrofuran (13.0 mL). To this solution was added ammonium hydroxide solution (28.0-30.0% NH₃ basis, 7.37 mL, 51.8 mmol, 20.0 eq.) and stirred at room temperature for 30 minutes. The reaction was quenched with aqueous hydrochloric acid (1.00 M, 20 mL) and extracted with ethyl acetate (3 x 20 mL). The combined organics were washed with brine (20 mL), dried over magnesium sulfate, filtered, and dry loaded onto Celite under reduced pressure. The crude was purified by flash column chromatography (40 g HP Silica, 0-60 %, ethyl acetate/hexanes) to afford the title compound as a white solid (162 mg). ¹H NMR (400 MHz, Chloroform-d) δ 7.91 (d, 1H), 7.55 (dd, 1H), 7.36 - 7.29 (m, 1H), 4.93 (s, 2H), 3.98 - 3.88 (m, 1H), 1.32 (s, 9H), 0.96 - 0.80 (m, 4H); LC-MS (method 4): Rₜ = 1.41 min; MS (ESlpos): m/z = 270 [M+H]⁺

### Intermediate 67

### 1-(Benzyloxy)-4-(tert-butyl)-2-iodobenzene

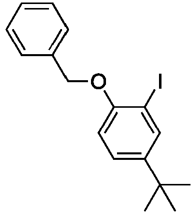

To a stirred suspension of 4-tert-butyl-2-iodo-phenol (1.50 g, 5.43 mmol, 1.00 eq.) and potassium carbonate (1.50 g, 10.9 mmol, 2.00 eq.) in anhydrous N,N-dimethylformamide (2.72 mL, 2.00 M) was added benzyl bromide (1.29 mL, 10.9 mmol, 2.00 eq.). The resulting brown suspension was heated at 60 °C for 48 h and then cooled to room temperature. The mixture was diluted with water (50 mL) and extracted with dichloromethane (3 x 50 mL). The combined organic extracts were washed with brine (50 mL), dried (magnesium sulfate), filtered and dry loaded onto Celite. The residue was purified by reverse phase flash column chromatography (275 g HP C₁₈, 60-100% acetonitrile/water gradient buffered with 0.1% formic acid) to give the title compound as a colourless oil (1.42 g). ¹H NMR (400 MHz, Chloroform-d) δ 7.79 (d, 1H), 7.50 (ddt, 2H), 7.44 - 7.35 (m, 2H), 7.37 - 7.24 (m, 2H), 6.79 (d, 1H), 5.13 (s, 2H), 1.28 (s, 9H); LC-MS (method 5): Rₜ = 3.77 min; MS (ESlneg): m/z = 365 [M-H]⁻

### Intermediate 68

### 2-(Benzyloxy)-5-(tert-butyl)benzenesulfonamide

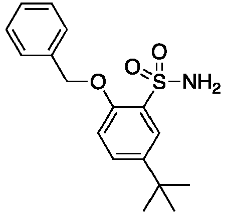

To a 40 mL vial under an argon atmosphere was added 1-benzyloxy-4-tert-butyl-2-iodobenzene (1.43 g, 3.89 mmol, 1.00 eq), palladium(ll) acetate (43.7 mg, 0.19 mmol, 5.00 mol%), CataCXium^{®} A (112 mg, 0.31 mmol, 7.50 mol%), and 1,4-Diazabicyclo[2.2.2]octane bis(sulfur dioxide) adduct (561 mg, 2.33 mmol, 0.60 eq.) followed by the addition of degassed anhydrous isopropanol (19.5 mL, 0.20 M) and triethylamine (1.62 mL, 11.7 mmol, 3.00 eq.). The resulting red solution was heated at 75 °C for 16 h, cooled to room temperature and concentrated under reduced pressure. The residue was resuspended in anhydrous tetrahydrofuran (19.5 mL) and cooled to 0 °C in a ice/water bath. To this mixture was then added sulfuryl chloride (0.94 mL, 11.7 mmol, 3.00 eq.) dropwise, after which the resulting mixture was warmed to room temperature and stirred for a further 30 minutes. The reaction was quenched with ammonium chloride (30 mL), and the aqueous layer was extracted with dichloromethane (3 x 30 mL). The combined organics were washed with brine (30 mL), dried (magnesium sulfate), filtered, and dry loaded onto Celite under reduced pressure. The crude was purified by flash column chromatography (80 g HP Silica, 0-20%, ethyl acetate/hexanes) to afford 2-benzyloxy-5-tert-butyl-benzenesulfonyl chloride as an oil, which was suspended in anhydrous tetrahydrofuran (19.5 mL). To this solution was added ammonium hydroxide solution (28.0-30.0% NH₃ basis, 11.1 mL, 76.8 mmol, 20.0 eq.) and stirred at room temperature for 30 minutes. The reaction was quenched with aqueous hydrochloric acid (1.00 M, 30 mL) and extracted with ethyl acetate (3 x 30 mL). The combined organics were washed with brine (30 mL), dried over magnesium sulfate, filtered, and dry loaded onto Celite under reduced pressure. The crude was purified by flash column chromatography (80 g HP Silica, 0-60 %, ethyl acetate/hexanes) to afford the title compound as a white solid (457 mg). ¹H NMR (400 MHz, Chloroform-d) δ 7.98 - 7.92 (m, 1H), 7.57 - 7.46 (m, 3H), 7.46 - 7.32 (m, 3H), 7.07 - 7.00 (m, 1H), 5.23 (s, 2H), 4.95 (s, 2H), 1.32 (s, 9H); LC-MS (method 4): Rt = 1.50 min; MS (ESlpos): m/z = 320 [M+H]⁺

### Intermediate 69

### 4-(tert-Butyl)-1-(cyclopropylmethoxy)-2-iodobenzene

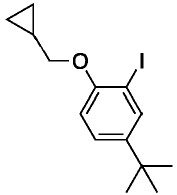

To a stirred suspension of 4-tert-butyl-2-iodo-phenol (1.50 g, 5.43 mmol, 1.00 eq.) and potassium carbonate (1.50 g, 10.9 mmol, 2.00 eq.) in anhydrous N,N-dimethylformamide (2.72 mL, 2.00 M) was added bromomethylcyclopropane (1.01 mL, 10.9 mmol, 2.00 eq.). The resulting brown suspension was heated at 60 °C for 48 h and then cooled to room temperature. The mixture was diluted with water (50 mL) and extracted with dichloromethane (3 x 50 mL). The combined organic extracts were washed with brine (50 mL), dried (magnesium sulfate), filtered and dry loaded onto Celite. The residue was purified by flash column chromatography (80 g HP Silica, 0-20% ethyl acetate/hexanes gradient) to give the title compound as a colourless oil (1.27 g). ¹H NMR (400 MHz, Chloroform-d) δ 7.76 (d, 1H), 7.31 - 7.24 (m, 1H), 6.73 (d, 1H), 3.86 (d, 2H), 1.37 - 1.22 (m, 1H), 1.28 (s, 9H), 0.69 - 0.56 (m, 2H), 0.47 - 0.34 (m, 2H); LC-MS (method 5): Rₜ = 3.72 min; MS (ESlpos): m/z = not detected

### Intermediate 70

### 5-(tert-Butyl)-2-(cyclopropylmethoxy)benzenesulfonamide

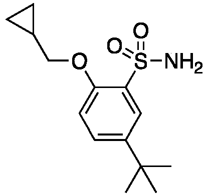

To a 40 mL vial under an argon atmosphere was added 4-tert-butyl-1-(cyclopropylmethoxy)-2-iodo-benzene (1.27 g, 3.84 mmol, 1.00 eq), palladium(ll) acetate (43.1 mg, 0.19 mmol, 5.00 mol%), CataCXium^{®} A (110 mg, 0.31 mmol, 7.50 mol%), and 1,4-Diazabicyclo[2.2.2]octane bis(sulfur dioxide) adduct (554 mg, 2.30 mmol, 0.60 eq.) followed by the addition of degassed anhydrous isopropanol (19.2 mL, 0.20 M) and triethylamine (1.60 mL, 11.5 mmol, 3.00 eq.). The resulting red solution was heated at 75 °C for 16 h, cooled to room temperature and concentrated under reduced pressure. The residue was resuspended in anhydrous tetrahydrofuran (19.2 mL) and cooled to 0 °C in a ice/water bath. To this mixture was then added sulfuryl chloride (0.93 mL, 11.5 mmol, 3.00 eq.) dropwise, after which the resulting mixture was warmed to room temperature and stirred for a further 30 minutes. The reaction was quenched with ammonium chloride (30 mL), and the aqueous layer was extracted with dichloromethane (3 x 30 mL). The combined organics were washed with brine (30 mL), dried (magnesium sulfate), filtered, and dry loaded onto Celite under reduced pressure. The crude was purified by flash column chromatography (80 g HP silica, 0-20%, ethyl acetate/hexanes gradient) to give 5-tert-butyl-2-(cyclopropylmethoxy)benzenesulfonyl chloride as an oil, which was suspended in anhydrous tetrahydrofuran (19.2 mL). To this solution was added ammonium hydroxide solution (28.0-30.0% NH₃ basis, 10.9 mL, 76.8 mmol, 20.0 eq.) and stirred at room temperature for 30 minutes. The reaction was quenched with aqueous hydrochloric acid (1.00 M, 20 mL) and extracted with ethyl acetate (3 x 20 mL). The combined organics were washed with brine (20 mL), dried (magnesium sulfate), filtered, and dry loaded onto Celite under reduced pressure. The crude was purified by flash column chromatography (80 g HP Silica, 0-60 %, ethyl acetate/hexanes gradient) to afford the title compound as a white solid (580 mg). ¹H NMR (400 MHz, Chloroform-d) δ 7.95 - 7.89 (m, 1H), 7.51 (dd, 1H), 6.96 - 6.89 (m, 1H), 5.11 (s, 2H), 3.98 (d, 2H), 1.46 - 1.31 (m, 1H), 1.33 - 1.28 (m, 9H), 0.76 - 0.63 (m, 2H), 0.46 - 0.32 (m, 2H); LC-MS (method 4): Rt = 1.46 min; MS (ESlpos): m/z = 284 [M+H]⁺

### Intermediate 71

### 4-(tert-Butyl)-2-iodo-1-(2,2,2-trifluoroethoxy)benzene

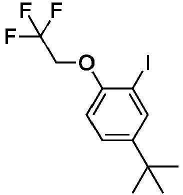

To a stirred suspension of 4-tert-butyl-2-iodo-phenol (1.50 g, 5.43 mmol, 1.00 eq.) and potassium carbonate (3.00 g, 21.7 mmol, 4.00 eq.) in anhydrous N,N-dimethylformamide (2.72 mL, 2.00 M) was added 1,1,1-trifluoro-2-iodo-ethane (2.12 mL, 21.7 mmol, 4.00 eq.). The resulting brown suspension was heated at 130 °C for 24 h and then cooled to room temperature. The mixture was diluted with water (50 mL) and extracted with dichloromethane (3 x 50 mL). The combined organic extracts were washed with brine (50 mL), dried (magnesium sulfate), filtered and dry loaded onto Celite. The residue was purified by flash column chromatography (80 g HP Silica, 0-20% ethyl acetate/hexanes gradient) to give the title compound as a colourless oil (1.15 g). ¹H NMR (400 MHz, Chloroform-d) δ 7.79 (d, 1H), 7.32 (dd, 1H), 6.78 (d, 1H), 4.36 (q, 2H), 1.29 (s, 9H); LC-MS (method 5): Rt = 3.49 min; MS (ESlpos): m/z = not detected

### Intermediate 72

### 5-(tert-Butyl)-2-(2,2,2-trifluoroethoxy)benzenesulfonamide

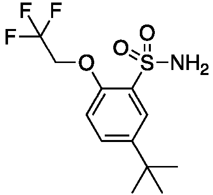

To a 40 mL vial under an argon atmosphere was added 4-tert-butyl-2-iodo-1-(2,2,2-trifluoroethoxy)benzene (1.15 g, 3.21 mmol, 1.00 eq), palladium(ll) acetate (36.1 mg, 0.16 mmol, 5.00 mol%), CataCXium^{®} A (92.1 mg, 0.26 mmol, 7.50 mol%), and 1,4-Diazabicyclo[2.2.2]octane bis(sulfur dioxide) adduct (463 mg, 1.93 mmol, 0.60 eq.) followed by the addition of degassed anhydrous isopropanol (16.1 mL, 0.20 M) and triethylamine (1.34 mL, 9.64 mmol, 3.00 eq.). The resulting red solution was heated at 75 °C for 16 h, cooled to room temperature and concentrated under reduced pressure. The residue was resuspended in anhydrous tetrahydrofuran (16.1 mL) and cooled to 0 °C in a ice/water bath. To this mixture was then added sulfuryl chloride (0.78 mL, 9.64 mmol, 3.00 eq.) dropwise, after which the resulting mixture was warmed to room temperature and stirred for a further 30 minutes. The reaction was quenched with ammonium chloride (30 mL), and the aqueous layer was extracted with dichloromethane (3 x 30 mL). The combined organics were washed with brine (30 mL), dried (magnesium sulfate), filtered, and dry loaded onto Celite under reduced pressure. The crude was purified by flash column chromatography (80 g HP Silica, 0-20%, ethyl acetate/hexanes gradient) to afford 5-tert-butyl-2-(2,2,2-trifluoroethoxy)benzenesulfonyl chloride as an oil, which was suspended in anhydrous tetrahydrofuran (16.1 mL). To this solution was added ammonium hydroxide solution (28.0-30.0% NH₃ basis, 9.14 mL, 64.3 mmol, 20.0 eq.) and stirred at room temperature for 30 minutes. The reaction was quenched with aqueous hydrochloric acid (1.00 M, 20 mL) and extracted with ethyl acetate (3 x 20 mL). The combined organics were washed with brine (20 mL), dried (magnesium sulfate), filtered, and dry loaded onto Celite under reduced pressure. The residue was purified by flash column chromatography (80 g HP Silica, 0-60 %, ethyl acetate/hexanes gradient) to give the title compound as a white solid (324 mg). ¹H NMR (400 MHz, Chloroform-d) δ 7.97 (d, 1H), 7.57 (dd, 1H), 7.02 - 6.95 (m, 1H), 4.98 (s, 2H), 4.53 (q, 2H), 1.32 (s, 9H); LC-MS (method 4): Rₜ = 1.46 min; MS (ESlpos): m/z = 312 [M+H]⁺

### Intermediate 73

### 4-(tert-Butyl)-1-cyclobutoxy-2-iodobenzene

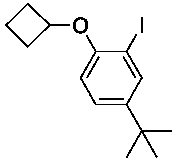

To a stirred suspension of 4-tert-butyl-2-iodo-phenol (1.50 g, 5.43 mmol, 1.00 eq.) and potassium carbonate (2.25 g, 16.3 mmol, 3.00 eq.) in anhydrous N,N-dimethylformamide (2.72 mL, 2.00 M) was added bromocyclobutane (1.53 mL, 16.3 mmol, 3.00 eq.). The resulting brown suspension was heated at 60 °C for 3 days and then cooled to room temperature. The mixture was diluted with water (50 mL) and extracted with dichloromethane (3 x 50 mL). The combined organic extracts were washed with brine (50 mL), dried (magnesium sulfate), filtered and dry loaded onto Celite. The residue was purified by flash column chromatography (80 g HP Silica, 0-20% ethyl acetate/hexanes gradient) to give the title compound as a colourless oil (1.44 g). ¹H NMR (400 MHz, Chloroform-d) δ 7.75 (d, 1H), 7.25 (dd, 1H), 6.60 (d, 1H), 4.71 - 4.58 (m, 1H), 2.52 - 2.38 (m, 2H), 2.32 - 2.16 (m, 2H), 1.94 - 1.80 (m, 1H), 1.76 - 1.59 (m, 1H), 1.27 (s, 9H); LC-MS (method 5): Rt = 3.81 min; MS (ESlpos): m/z = not detected

### Intermediate 74

### 5-(tert-Butyl)-2-cyclobutoxybenzenesulfonamide

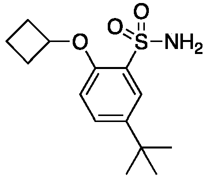

To a 40 mL vial under an argon atmosphere was added 4-tert-butyl-1-(cyclobutoxy)-2-iodo-benzene (1.44 g, 4.37 mmol, 1.00 eq), palladium(ll) acetate (49.0 mg, 0.22 mmol, 5.00 mol%), CataCXium^{®} A (125 mg, 0.35 mmol, 7.50 mol%), and 1,4-Diazabicyclo[2.2.2]octane bis(sulfur dioxide) adduct (630 mg, 2.62 mmol, 0.60 eq.) followed by the addition of degassed anhydrous isopropanol (14.6 mL, 0.30 M) and triethylamine (1.82 mL, 13.1 mmol, 3.00 eq.). The resulting red solution was heated at 75 °C for 16 h, cooled to room temperature and concentrated under reduced pressure. The residue was resuspended in anhydrous tetrahydrofuran (14.6 mL) and cooled to 0 °C in a ice/water bath. To this mixture was then added sulfuryl chloride (1.06 mL, 13.1 mmol, 3.00 eq.) dropwise, after which the resulting mixture was warmed to room temperature and stirred for a further 30 minutes. The reaction was quenched with ammonium chloride (30 mL), and the aqueous layer was extracted with dichloromethane (3 x 30 mL). The combined organics were washed with brine (30 mL), dried (magnesium sulfate), filtered, and dry loaded onto Celite under reduced pressure. The residue was purified by flash column chromatography (80 g HP Silica, 0-20%, ethyl acetate/hexanes) to give 5-tert-butyl-2-(cyclobutoxy)benzenesulfonyl chloride as an oil, which was suspended in anhydrous tetrahydrofuran (14.6 mL). To this solution was added ammonium hydroxide solution (28.0-30.0% NH₃ basis, 12.4 mL, 87.3 mmol, 20.0 eq.) and stirred at room temperature for 30 minutes. The reaction was quenched with aqueous hydrochloric acid (1.00 M, 20 mL) and extracted with ethyl acetate (3 x 20 mL). The combined organic layers were washed with brine (20 mL), dried (magnesium sulfate), filtered, and dry loaded onto Celite under reduced pressure. The residue was purified by flash column chromatography (80 g HP Silica, 0-60 %, ethyl acetate/hexanes) to give the title compound as a white solid (746 mg). ¹H NMR (400 MHz, Chloroform-d) δ 7.94 - 7.87 (m, 1H), 7.48 (dd, 1H), 6.86 - 6.77 (m, 1H), 5.03 (s, 2H), 4.87 - 4.74 (m, 1H), 2.57 - 2.44 (m, 2H), 2.33 - 2.18 (m, 2H), 1.99 - 1.84 (m, 1H), 1.81 - 1.65 (m, 1H), 1.33 - 1.28 (m, 9H); LC-MS (method 4): Rt = 1.51 min; MS (ESlpos): m/z = 284 [M+H]⁺

### Intermediate 75

### 4-(tert-Butyl)-2-iodo-1-isopropoxybenzene

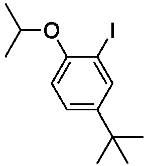

To a stirred suspension of 4-tert-butyl-2-iodo-phenol (1.50 g, 5.43 mmol, 1.00 eq.) and potassium carbonate (1.50 g, 10.9 mmol, 2.00 eq.) in anhydrous N,N-dimethylformamide (2.72 mL, 2.00 M) was added 2-bromopropane (1.02 mL, 10.9 mmol, 2.00 eq.). The resulting brown suspension was heated at 60 °C for 48 h and then cooled to room temperature. The mixture was diluted with water (50 mL) and extracted with dichloromethane (3 x 50 mL). The combined organic extracts were washed with brine (50 mL), dried (magnesium sulfate), filtered and dry loaded onto Celite. The residue was purified by flash column chromatography (80 g HP Silica, 0-20% ethyl acetate/hexanes gradient) to give the title compound as a colourless oil (1.63 g). ¹H NMR (400 MHz, Chloroform-d) δ 7.75 (d, 1H), 7.27 (dd, 1H), 6.75 (d, 1H), 4.51 (p, J = 6.1 Hz, 1H), 1.38 (d, 6H), 1.28 (s, 9H); LC-MS (method 5): Rₜ = 3.71 min; MS (ESlpos): m/z = not detected

### Intermediate 76

### 5-(tert-Butyl)-2-isopropoxybenzenesulfonamide

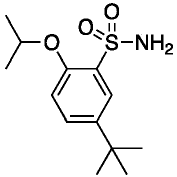

To a 40 mL vial under an argon atmosphere was added 4-tert-butyl-2-iodo-1-isopropoxy-benzene (1.63 g, 5.13 mmol, 1.00 eq), palladium(ll) acetate (57.6 mg, 0.26 mmol, 5.00 mol%), CataCXium^{®} A (147 mg, 0.41 mmol, 7.50 mol%), and 1,4-Diazabicyclo[2.2.2]octane bis(sulfur dioxide) adduct (740 mg, 3.08 mmol, 0.60 eq.) followed by the addition of degassed anhydrous isopropanol (17.1 mL, 0.30 M) and triethylamine (2.13 mL, 15.4 mmol, 3.00 eq.). The resulting red solution was heated at 75 °C for 16 h, cooled to room temperature and concentrated under reduced pressure. The residue was resuspended in anhydrous tetrahydrofuran (17.1 mL) and cooled to 0 °C in a ice/water bath. To this mixture was then added sulfuryl chloride (1.24 mL, 15.4 mmol, 3.00 eq.) dropwise, after which the resulting mixture was warmed to room temperature and stirred for a further 30 minutes. The reaction was quenched with ammonium chloride (30 mL), and extracted with dichloromethane (3 x 30 mL). The combined organic extracts were washed with brine (30 mL), dried (magnesium sulfate), filtered, and dry loaded onto Celite under reduced pressure. The residue was purified by flash column chromatography (80 g HP Silica, 0-20%, ethyl acetate/hexanes gradient) to give 5-tert-butyl-2-isopropoxy-benzenesulfonyl chloride as an oil, which was suspended in anhydrous tetrahydrofuran (17.1 mL). To this solution was added ammonium hydroxide solution (28.0-30.0% NH₃ basis, 14.6 mL, 103 mmol, 20.0 eq.) and stirred at room temperature for 30 minutes. The reaction was quenched with aqueous hydrochloric acid (1.00 M, 30 mL) and extracted with ethyl acetate (3 x 30 mL). The combined organic extracts were washed with brine (30 mL), dried (magnesium sulfate), filtered, and dry loaded onto Celite under reduced pressure. The residue was purified by flash column chromatography (80 g HP Silica, 0-60 %, ethyl acetate/hexanes) to give the title compound as a white solid (720 mg). ¹H NMR (400 MHz, Chloroform-d) δ 7.92 (d, 1H), 7.55 - 7.47 (m, 1H), 7.00 - 6.93 (m, 1H), 5.01 (s, 2H), 4.76 (hept, 1H), 1.44 (d, 6H), 1.33 - 1.28 (m, 9H); LC-MS (method 4): Rt = 1.48 min; MS (ESlpos): m/z = 272 [M+H]⁺

### Intermediate 77

### 1-Cyclobutoxy-2-iodo-4-isopropylbenzene

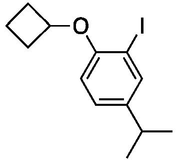

To a stirred solution of 2-iodo-4-isopropyl-phenol (2.00 g, 7.63 mmol, 1.00 eq.) in anhydrous N,N-dimethylformamide (15.3 mL, 0.50 M) was added potassium carbonate (2.11 g, 15.3 mmol, 2.00 eq.), and the reaction stirred for 5 minutes at room temperature. Bromocyclobutane (790 µL, 8.39 mmol, 1.10 eq.) was added, and the reaction was left stirring at 80 °C for 40 h. Additional bromocyclobutane (790 µL, 8.39 mmol, 1.10 eq.) and potassium carbonate (1.06 g, 7.65 mmol, 1.00 eq.) were added and the mixture was stirred at 80 °C for a further 24 h. The reaction was cooled to room temperature, diluted with water (30 mL) and extracted with dichloromethane (3 x 30 mL). The combined organic extracts were washed with brine (30 mL), dried (magnesium sulfate), filtered, and dry loaded onto Celite under reduced pressure. The residue was purified by flash column chromatography (80 g HP Silica, 0-10% ethyl acetate/hexanes gradient) to give the title compound as colorless oil (2.25 g). ¹H NMR (400 MHz, Chloroform-d) δ 7.65 - 7.59 (m, 1H), 7.13 - 7.05 (m, 1H), 6.63 - 6.56 (m, 1H), 4.64 (p, 1H), 2.88 - 2.73 (m, 1H), 2.51 - 2.39 (m, 2H), 2.32 - 2.17 (m, 2H), 1.94 - 1.80 (m, 1H), 1.76 - 1.59 (m, 1H), 1.24 - 1.17 (m, 6H); LC-MS (method 4): Rₜ = 1.87 min; MS (ESlpos): m/z = not detected.

### Intermediate 78

### 2-Cyclobutoxy-5-isopropylbenzenesulfonyl chloride

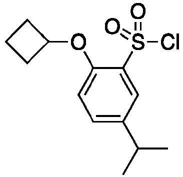

To an oven dried 40 mL vial under an argon atmosphere was added palladium(ll) acetate (80.0 mg, 0.36 mmol, 5.00 mol%), CataCXium^{®} A (204 mg, 0.57 mmol, 7.50 mol%), and 1,4-Diazabicyclo[2.2.2]octane bis(sulfur dioxide) adduct (1.03 g, 4.27 mmol, 0.60 eq.) followed by the addition of degassed anhydrous isopropanol (17.8 mL, 0.20 M), 1-(cyclobutoxy)-2-iodo-4-isopropyl-benzene (2.25 g, 7.12 mmol, 1.00 eq.), and triethylamine (2.96 mL, 21.4 mmol, 3.00 eq.). The resulting red solution was heated at 75 °C for 17 h, cooled to room temperature and concentrated under reduced pressure. The residue was resuspended in anhydrous tetrahydrofuran (17.8 mL) and cooled to 0 °C in a ice/water bath. To this mixture was then added sulfuryl chloride (1.73 mL, 21.4 mmol, 3.00 eq.) dropwise, after which the resulting mixture was warmed to room temperature and stirred for a further 30 minutes. The reaction was quenched with ammonium chloride (40 mL), and extracted with dichloromethane (3 x 40 mL). The combined organic extracts were washed with brine (40 mL), dried (magnesium sulfate), filtered, and dry loaded onto Celite under reduced pressure. The residue was purified by flash column chromatography (120 g Silica, 0-20 %, ethyl acetate/hexanes gradient) to give the title compound as an oil (1.74 g). ¹H NMR (400 MHz, Chloroform-d) δ 7.80 - 7.75 (m, 1H), 7.50 - 7.42 (m, 1H), 6.90 - 6.83 (m, 1H), 4.83 (p, 1H), 2.98 - 2.83 (m, 1H), 2.57 - 2.46 (m, 1H), 2.41 - 2.26 (m, 2H), 2.02 - 1.64 (m, 3H), 1.28 - 1.19 (m, 6H); LC-MS (method 4): Rt = 1.76 min; MS (ESlneg): m/z = not detected

### Intermediate 79

### 2-Cyclobutoxy-5-isopropylbenzenesulfonamide

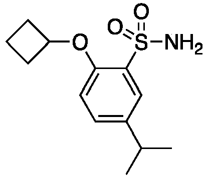

To a stirred solution of 2-(cyclobutoxy)-5-isopropyl-benzenesulfonyl chloride (1.74 g, 6.01 mmol, 1.00 eq.) in anhydrous tetrahydrofuran (10.0 mL, 0.60 M) was added ammonium hydroxide solution (28.0-30.0% NH₃ basis, 16.7 mL, 120 mmol, 20.0 eq.) and stirred at room temperature for 30 minutes. The reaction was quenched with hydrochloric acid (1.00 M, 20 mL), and the aqueous layer was extracted with ethyl acetate (3 x 20 mL). The combined organic extracts were washed with brine (20 mL), dried (magnesium sulfate), filtered, and dry loaded onto Celite under reduced pressure. The crude was purified by flash column chromatography (80 g HP Silica, 0-60 %, ethyl acetate/hexanes gradient) to afford the title compound as a white solid (536 mg). ¹H NMR (400 MHz, Chloroform-d) δ 7.80 - 7.74 (m, 1H), 7.37 - 7.28 (m, 1H), 6.85 - 6.77 (m, 1H), 5.02 (s, 2H), 4.80 (p, 1H), 2.98 - 2.83 (m, 1H), 2.57 - 2.43 (m, 2H), 2.34 - 2.19 (m, 2H), 1.99 - 1.85 (m, 1H), 1.81 - 1.64 (m, 1H), 1.27 - 1.20 (m, 6H); LC-MS (method 4): Rₜ = 1.47 min; MS (ESlpos): m/z = 270 [M+H]⁺

### Intermediate 80

### 5-(tert-Butyl)-2-methoxybenzenesulfonamide

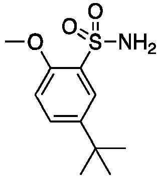

To a stirred solution of 5-tert-butyl-2-methoxy-benzenesulfonyl chloride (1.00 g, 3.81 mmol, 1.00 eq.) in anhydrous tetrahydrofuran (6.34 mL, 0.60 M) was added ammonium hydroxide solution (28.0-30.0% NH₃ basis, 10.6 mL, 76.1 mmol, 20.0 eq.) and stirred at room temperature for 30 minutes. The reaction was quenched with hydrochloric acid (1.00 M, 20 mL), and extracted with ethyl acetate (3 x 20 mL). The combined organic extracts were washed with brine (20 mL), dried (magnesium sulfate), filtered, and dry loaded onto Celite under reduced pressure. The residue was purified by flash column chromatography (80 g HP Silica, 0-60 %, ethyl acetate/hexnaes gradient) to give the title compound as a white solid (678 mg). ¹H NMR (400 MHz, Chloroform-d) δ 7.96 - 7.90 (m, 1H), 7.59 - 7.51 (m, 1H), 6.98 (d, 1H), 5.01 (s, 2H), 4.02 - 3.97 (m, 3H), 1.34 - 1.30 (m, 9H); LC-MS (method 4): Rₜ = 1.37 min; MS (ESlpos): m/z = 244 [M+H]⁺

### Intermediate 81

### 1-((2,2-Difluorocyclopropyl)methoxy)-2-iodo-4-isopropylbenzene

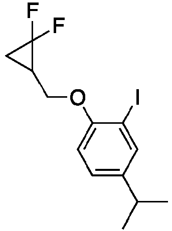

To a stirred solution of 2-iodo-4-isopropyl-phenol (1.28 g, 4.87 mmol, 1.00 eq) in anhydrous N,N-dimethylformamide (9.74 mL, 0.50 M) was added potassium carbonate (1.35 g, 9.74 mmol, 2.00 eq), and the reaction stirred for 5 minutes at room temperature. 2-(Bromomethyl)-1,1-difluoro-cyclopropane (1.00 g, 5.84 mmol, 1.20 eq) was added, and the reaction was heated at 70 °C for 20 h. The reaction was cooled to room temperature, diluted with water (20 mL) and extracted with dichloromethane (3 x 20 mL). The combined organic extracts were washed with brine (20 mL), dried (magnesium sulfate), filtered and dry loaded onto Celite under reduced pressure. The residue was purified by flash column chromatography (80 g HP silica, 0-20% ethyl acetate/hexanes gradient) to give the title compound as a clear oil (938 mg). ¹H NMR (400 MHz, Chloroform-d) δ 7.67 - 7.61 (m, 1H), 7.18 - 7.10 (m, 1H), 6.78 - 6.71 (m, 1H), 4.22 - 4.12 (m, 1H), 4.04 - 3.94 (m, 1H), 2.82 (hept, 1H), 2.20 - 2.03 (m, 1H), 1.67 - 1.54 (m, 1H), 1.42 - 1.29 (m, 1H), 1.25 - 1.18 (m, 6H); LC-MS (method 4): Rₜ = 1.77 min; MS (ESlpos): m/z = not detected

### Intermediate 82

### 2-[(2,2-Difluorocyclopropyl)methoxy]-5-isopropylbenzenesulfonyl chloride

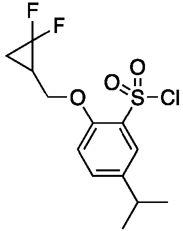

To an oven dried 40 mL vial under an argon atmosphere was added palladium(ll) acetate (29.9 mg, 0.13 mmol, 5.00 mol%), CataCXium^{®} A (76.4 mg, 0.21 mmol, 7.50 mol%), and 1,4-Diazabicyclo[2.2.2]octane bis(sulfur dioxide) adduct (384 mg, 1.60 mmol, 0.60 eq.) followed by the addition of degassed anhydrous isopropanol (13.3 mL, 0.20 M), 1-[(2,2-difluorocyclopropyl)methoxy]-2-iodo-4-isopropyl-benzene (938 mg, 2.66 mmol, 1.00 eq.), and triethylamine (1.11 mL, 7.99 mmol, 3.00 eq.). The resulting red solution was heated at 75 °C for 88 h, cooled to room temperature and concentrated under reduced pressure. The residue was resuspended in anhydrous tetrahydrofuran (13.3 mL) and cooled to 0 °C in a ice/water bath. To this mixture was then added sulfuryl chloride (650 µL, 7.99 mmol, 3.00 eq.) dropwise, after which the resulting mixture was warmed to room temperature and stirred for a further 30 minutes. The reaction was quenched with ammonium chloride (30 mL) and extracted with dichloromethane (3 x 30 mL). The combined organics were washed with brine (30 mL), dried (magnesium sulfate), filtered, and dry loaded onto Celite under reduced pressure. The residue was purified by flash column chromatography (80 g HP Silica, 0-20 %, ethyl acetate/hexanes gradient) to give the title compound as an oil (227 mg). ¹H NMR (400 MHz, Chloroform-d) δ 7.81 (d, 1H), 7.55 - 7.49 (m, 1H), 7.05 - 6.98 (m, 1H), 4.43 - 4.31 (m, 1H), 4.22 - 4.12 (m, 1H), 3.04 - 2.86 (m, 1H), 2.29 - 2.10 (m, 1H), 1.75 - 1.60 (m, 1H), 1.51 - 1.35 (m, 1H), 1.28 - 1.24 (m, 6H); LC-MS (method 4): Rₜ = 1.68 min; MS (ESlpos): m/z = not detected

### Intermediate 83

### 2-[(2,2-Difluorocyclopropyl)methoxy]-5-isopropylbenzenesulfonamide

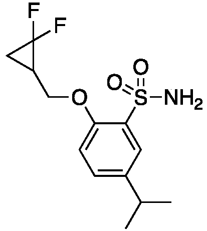

To a stirred solution of 2-[(2,2-difluorocyclopropyl)methoxy]-5-isopropyl-benzenesulfonyl chloride (227 mg, 0.70 mmol, 1.00 eq) in anhydrous tetrahydrofuran (1.16 mL, 0.60 mmol) was added ammonium hydroxide solution (20 % in water, 2.72 mL, 14.0 mmol, 20.0 eq) and stirred at room temperature for 30 minutes. The reaction was quenched with hydrochloric acid (1.00 M, 10 mL), and extracted with ethyl acetate (3 x 10 mL). The combined organics were washed with brine (10 mL), dried (magnesium sulfate), filtered, and dry loaded onto Celite under reduced pressure. The residue was purified by flash column chromatography (24 g HP Silica, 0-60 %, ethyl acetate/hexnaes gradient) to give the title compound as a white solid (156 mg). ¹H NMR (400 MHz, Chloroform-d) δ 7.82 - 7.76 (m, 1H), 7.42 - 7.34 (m, 1H), 6.95 (d, 1H), 4.99 (s, 2H), 4.35 - 4.25 (m, 1H), 4.27 - 4.17 (m, 1H), 3.00 - 2.85 (m, 1H), 2.25 - 2.09 (m, 1H), 1.72 - 1.58 (m, 1H), 1.52 - 1.39 (m, 1H), 1.30 - 1.21 (m, 6H); LC-MS (method 4): Rt = 1.45 min; MS (ESlpos): m/z = 306 [M+H]⁺

### Intermediate 84

### 4,5-Difluoro-2-iodophenol

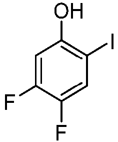

To a 0 °C solution of iodine (4.88 g, 19.2 mmol, 1.00 eq.), potassium iodide (3.19 g, 19.2 mmol, 1.00 eq.), and 3,4-difluorophenol (2.50 g, 19.2 mmol, 1.00 eq.) in water (64.1 mL, 0.30 M) was added sodium hydroxide solution (2.00 M, 19.22 mL, 38.4 mmol, 2.00 eq.). The resulting mixture was warmed to room temperature and stirred for 16 h. The reaction mixture was diluted with saturated ammonium chloride (50 mL) followed by the addition of aqueous sodium thiosulfate (50 % w/w) and stirred at room temperature for 20 minutes. The mixture was extracted with diethyl ether (3 x 40 mL), and the combined organic extracts were washed with brine (40 mL), dried (magnesium sulfate), filtered, and dry loaded onto Celite under reduced pressure. The residue was purified by flash column chromatography (120 g Silica, 0-20 %, ethyl acetate/hexanes gradient) to give the title compound as a colorless oil (2.72 g). ¹H NMR (400 MHz, Chloroform-d) δ 7.52 - 7.40 (m, 1H), 6.91 - 6.80 (m, 1H), 5.17 (s, 1H); LC-MS (Method 4): Rt = 1.39 min; MS (ESlneg): m/z = 254 [M-H]⁻

### Intermediate 85

### 1-Ethoxy-4,5-difluoro-2-iodobenzene

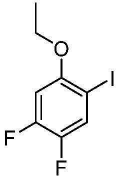

To a room temperature stirred solution of 4,5-difluoro-2-iodophenol (2.72 g, 10.63 mmol, 1.00 eq.) in anhydrous N,N-dimethylformamide (35.4 mL, 0.20 M) was added potassium carbonate (2.94 g, 21.3 mmol, 2.00 eq.) and iodoethane (1.28 mL, 15.9 mmol, 1.50 eq.). The resulting mixture was heated at 60 °C for 64 h and then cooled to room temperature. The mixture was diluted with water (30 mL) and extracted with ethyl acetate (3 x 30 mL). The combined organic extracts were washed with brine (30 mL), dried (magnesium sulfate), filtered, and dry loaded onto Celite under reduced pressure. The residue was purified by flash column chromatrography (120 g Silica, 0-20 %, ethyl acetate/hexanes gradient) to give the title compound as an oil (1.40 g). ¹H NMR (400 MHz, Chloroform-d) δ 7.62 - 7.53 (m, 1H), 6.70 - 6.60 (m, 1H), 4.02 (q, 2H), 1.47 (t, 3H); LC-MS (Method 4): Rt = 1.62 min; MS (ESlpos): m/z = not detected

### Intermediate 86

### 2-Ethoxy-4,5-difluorobenzenesulfonamide

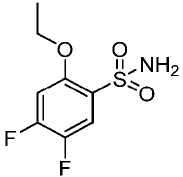

To a 40 mL vial was added 1-ethoxy-4,5-difluoro-2-iodo-benzene (1.40 g, 4.94 mmol, 1.00 eq), palladium(ll) acetate (55.4 mg, 0.25 mmol, 5.00 mol%), CataCXium^{®} A (142 mg, 0.39 mmol, 7.50 mol%), and 1,4-Diazabicyclo[2.2.2]octane bis(sulfur dioxide) adduct (712 mg, 2.96 mmol, 0.60 eq.) sequentially followed by the addition of degassed anhydrous isopropanol (24.7 mL, 0.20 M) and triethylamine (2.05 mL, 14.8 mmol, 3.00 eq.). The resulting red solution was heated at 75 °C for 16 h, cooled to room temperature and concentrated under reduced pressure. The residue was resuspended in anhydrous tetrahydrofuran (25.0 mL) and cooled to 0 °C in a ice/water bath. To this mixture was then added sulfuryl chloride (1.20 mL, 14.8 mmol, 3.00 eq.) dropwise, after which the resulting mixture was warmed to room temperature and stirred for a further 30 minutes. The reaction was quenched with ammonium chloride (30 mL), and the aqueous layer was extracted with ethyl acetate (3 x 30 mL). The combined organics were washed with brine (30 mL), dried (magnesium sulfate), filtered, and concentrated down under reduced pressure. The residue was dissolved in tetrahydrofuran (25.0 mL) and to this solution was added ammonium hydroxide solution (28.0-30.0% NH₃ basis, 14.0 mL, 98.7 mmol, 20.0 eq.) and and the resulting mixture was stirred at room temperature for 30 minutes. The reaction was quenched with aqueous hydrochloric acid (1.00 M, 30 mL) and extracted with ethyl acetate (3 x 30 mL). The combined organics were washed with brine (30 mL), dried (magnesium sulfate), filtered, and dry loaded onto Celite under reduced pressure. The residue was purified by flash column chromatography (40 g HP Silica, 0-60 %, ethyl acetate/hexanes gradient) to give the title compound as a white solid (262 mg). ¹H NMR (400 MHz, Chloroform-d) δ 7.83 - 7.74 (m, 1H), 6.91 - 6.82 (m, 1H), 5.00 (s, 2H), 4.21 (q, 2H), 1.57 - 1.49 (m, 3H); LC-MS (Method 4): Rₜ = 1.22 min; MS (ESlpos): m/z = 238 [M+H]⁺

### Intermediate 87

### 4-(Hydroxymethyl)-2-iodophenol

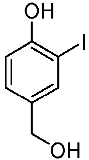

To a stirred suspension of 4-(hydroxymethyl)phenol (1.24 g, 10.0 mmol, 1.00 eq.) in acetonitrile (20.0 mL, 0.50 M) was added N-iodosuccinimide (2.25 g, 10.0 mmol, 1.00 eq.). The resulting mixture was stirred at room temperature for 30 minutes. The reaction was quenched with aqueous sodium thiosulfate (10 % w/w, 20 mL) and extracted with ethyl acetate (3 x 20 mL). The combined organic extracts were washed with brine (20 mL), dried (magnesium sulfate), filtered, and dry loaded onto Celite under reduced pressure. The residue was purified by flash column chromatography (80 g HP Silica, 0-100 %, ethyl acetate/hexanes gradient) to give the title compound as a solid (197 mg). ¹H NMR (400 MHz, DMSO-d₆) δ 10.19 (s, 1H), 7.63 - 7.57 (m, 1H), 7.15 - 7.07 (m, 1H), 6.86 - 6.79 (m, 1H), 5.08 - 5.00 (m, 1H), 4.37 - 4.31 (m, 2H); LC-MS (Method 4): Rt = 1.10 min; MS (ESlneg): m/z = 249 [M-H]⁻

### Intermediate 88

### (3-Iodo-4-methoxyphenyl)methanol

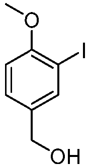

To a stirred solution of 4-(hydroxymethyl)-2-iodo-phenol (190 mg, 0.76 mmol, 1.00 eq.) in tetrahydrofuran (3.80 mL, 0.20 M) was added potassium carbonate (420 mg, 3.04 mmol, 4.00 eq.) and iodomethane (140 µL, 2.28 mmol, 3.00 eq.). The resulting mixture was heated at 50 °C for 16 h and then cooled to room temperature. The reaction mixture was diluted with water (10 mL) and extracted with ethyl acetate (3 x 10 mL). The combined organic extracts were washed with brine (10 mL), dried (magnesium sulfate), filtered, and dry loaded onto Celite under reduced pressure. The residue was purified by flash column chromatography (12 g HP Silica, 0-100, ethyl acetate/hexanes gradient) to give the title compound as a yellow solid (147 mg). ¹H NMR (400 MHz, DMSO-d₆) δ 7.73 - 7.67 (m, 1H), 7.32 - 7.24 (m, 1H), 6.99 - 6.92 (m, 1H), 5.15 (t, 1H), 4.42 - 4.36 (m, 2H), 3.80 (s, 3H); LC-MS (Method 4): Rt = 1.27 min; MS (ESlneg): m/z = not detected

### Intermediate 89

### 2-Iodo-1-methoxy-4-(methoxymethyl)benzene

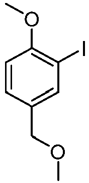

To a stirred suspension of (3-iodo-4-methoxy-phenyl)methanol (142 mg, 0.54 mmol, 1.00 eq.) in anhydrous tetrahydrofuran (2.69 mL, 0.20 M) was added sodium hydride (60 % dispersion in mineral oil, 43.0 mg, 1.08 mmol, 2.00 eq.) and iodomethane (50.0 µL, 0.81 mmol, 1.50 eq.). The resulting mixture was stirred at room temperature for 16 h. The reaction mixture was diluted with water (10 mL) and extracted with ethyl acetate (3 x 10 mL). The combined organics were washed with brine (10 mL), dried (magnesium sulfate), filtered, and dry loaded onto Celite under reduced pressure. The residue was purified by flash column chromatography (12 g HP Silica, 0-100, ethyl acetate/hexanes gradient) to give the title compound as a colorless oil (110 mg). ¹H NMR (400 MHz, Chloroform-d) δ 7.79 - 7.73 (m, 1H), 7.31 - 7.23 (m, 1H), 6.83 - 6.76 (m, 1H), 4.35 (s, 2H), 3.88 (s, 3H), 3.36 (s, 3H); LC-MS (Method 4): Rₜ = 1.47 min; MS (ESlpos): m/z = not detected

### Intermediate 90

### 2-Methoxy-5-(methoxymethyl)benzenesulfonamide

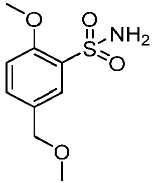

To a 20 mL vial was added 2-iodo-1-methoxy-4-(methoxymethyl)benzene (110 mg, 0.40 mmol, 1.00 eq.), palladium(ll) acetate (4.44 mg, 0.02 mmol, 5.00 mol%), CataCXium^{®} A (11.4 mg, 0.03 mmol, 7.50 mol%), and 1,4-Diazabicyclo[2.2.2]octane bis(sulfur dioxide) adduct (57.0 mg, 0.24 mmol, 0.60 eq.) sequentially followed by the addition of degassed anhydrous isopropanol (1.98 mL, 0.20 M) and triethylamine (160 µL, 1.19 mmol, 3.00 eq.). The resulting red solution was heated at 75 °C for 16 h, cooled to room temperature and concentrated under reduced pressure. The residue was resuspended in anhydrous tetrahydrofuran (2.00 mL) and cooled to 0 °C in a ice/water bath. To this mixture was then added sulfuryl chloride (100 µL, 1.19 mmol, 3.00 eq.) dropwise, after which the resulting mixture was warmed to room temperature and stirred for a further 30 minutes. The reaction was quenched with ammonium chloride (10 mL), and the aqueous layer was extracted with ethyl acetate (3 x 10 mL). The combined organic extracts were washed with brine (10 mL), dried (magnesium sulfate), filtered, and concentrated under reduced pressure. The residue was suspended in anhydrous tetrahydrofuran (2.00 mL). To this solution was then added ammonium hydroxide solution (28.0-30.0% NH₃ basis, 1.13 mL, 7.91 mmol, 20.0 eq.) and and the resulting mixture was stirred at room temperature for 30 minutes. The reaction was diluted with aqueous hydrochloric acid (1.00 M in water, 10 mL) and extracted with ethyl acetate (3 x 10 mL). The combined organic extracts were washed with brine (10 mL), dried (magnesium sulfate), filtered, and dry loaded onto Celite under reduced pressure. The residue was purified by flash column chromatography (12 g HP Silica, 0-80 %, ethyl acetate/hexanes) to afford the title compound as a white solid (12 mg). ¹H NMR (400 MHz, Chloroform-d) δ 7.92 - 7.87 (m, 1H), 7.58 - 7.50 (m, 1H), 7.04 (d, 1H), 5.00 (s, 2H), 4.42 (s, 2H), 4.02 (s, 3H), 3.38 (s, 3H); LC-MS (Method 4): Rt = 1.06 min; MS (ESlpos): m/z = 232 [M+H]⁺

### Intermediate 91

### 1-Cyclopropyl-4-(methoxymethoxy)benzene

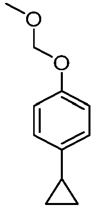

To a 0 °C stirred solution of 4-cyclopropylphenol (4.95 g, 36.9 mmol, 1.00 eq.) in anhydrous dichloromethane (123 mL, 0.30 M) was added chloromethyl methyl ether (4.20 mL, 55.3 mmol, 1.50 eq.) dropwise followed by N-ethyldiisopropylamine (12.6 mmol, 73.8 mmol, 2.00 eq). The resulting solution was warmed to room temperature and stirred for 40 h, after which additional chloromethyl methyl ether (2.8 mL, 36.9 mmol, 1.00 eq) and N-ethyldiisopropylamine (6.30 mL, 36.9 mmol, 1.00 eq) were added and stirred at room temperature. After 24 h, the mixture was diluted with water (200 mL) and extracted with dichloromethane (3 x 100 mL). The combined organic extracts were washed with hydrochloric acid (1.00 M in water, 200 mL), aqueous sodium hydroxide (1.00 M, 200 mL), brine (200 mL), dried (magnesium sulfate), filtered, and dry loaded onto Celite under reduced pressure. The residue was purified by flash column chromatography (330 g Silica, 0-20% ethyl acetate/hexanes gradient) to give the title compound as a colourless oil (6.76 g). ¹H NMR (400 MHz, Chloroform-d) δ 7.01 (d, 2H), 6.97 - 6.91 (m, 2H), 5.17 - 5.12 (m, 2H), 3.51 - 3.45 (m, 3H), 1.92 - 1.80 (m, 1H), 0.95 - 0.84 (m, 2H), 0.70 - 0.56 (m, 2H); LC-MS (Method 4): Rₜ = 1.55 min; MS (ESlpos): m/z = not detected

### Intermediate 92

### 4-Cyclopropyl-2-iodo-1-(methoxymethoxy)benzene

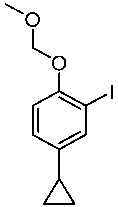

To a stirred solution of 1-cyclopropyl-4-(methoxymethoxy)benzene (5.67 g, 31.8 mmol, 1.00 eq.) in anhydrous tetrahydrofuran (106 mL, 0.30 M) was added n-butyllithium solution (2.50 M in hexanes, 14.6 mL, 0.75 mmol, 1.15 eq.) dropwise, afterwhich the solution was warmed to room temperature and stirred for 30 minutes. The mixture was then cooled back to -78 °C and a solution of iodine (9.37 g, 36.9 mmol, 1.16 eq.) in anhydrous tetrahydrofuran (110 mL) was added dropwsie *via* cannula. After complete addition, the mixture was warmed to room temperature and stirred for 30 minutes. The mixture was then quenched with sodium thiosulfate (10 wt.% in water, 100 mL), partially concentrated under reduced pressure and extracted with diethyl ether (3 x 100 mL). The combined organic extracts were washed with brine (100 mL), dried (magnesium sulfate), filtered, and concentrated under reduced pressure. The residue was purified by flash reverse phase column chromatography (330 g Silica, 40-100% acetonitrile/water bufferd with 0.1% formic acid gradient) to give the title compound as a brown oil (1.96 g). ¹H NMR (400 MHz, Chloroform-d) δ 7.53 - 7.48 (m, 1H), 7.03 - 6.91 (m, 2H), 5.19 (s, 2H), 3.54 - 3.48 (m, 3H), 1.87 - 1.75 (m, 1H), 0.98 - 0.85 (m, 2H), 0.69 - 0.55 (m, 2H); LC-MS (Method 4): Rₜ = 1.63 min; MS (ESlpos): m/z = not detected

### Intermediate 93

### 4-Cyclopropyl-2-iodophenol

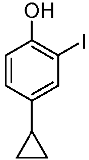

To a 0 °C stirred solution of sodium iodide (1.93 g, 12.9 mmol, 2.00 eq.) and 4-cyclopropyl-2-iodo-1-(methoxymethoxy)benzene (1.96 g, 6.43 mmol, 1.00 eq.) in anhydrous acetonitrile (32.1 mL, 0.20 M) was added trimethylsilyl chloride (1.22 mL, 9.64 mmol, 1.50 eq.) dropwise. After stirring at 0 °C for a further 10 minutes, the mixture was warmed to room temperature and stirred for 16 h. The reaction mixture was diluted with aqueous sodium thiosulfate (10 % w/w, 30 mL) and extracted with diethyl ether (3 x 30 mL). The combined organic extracts were dried (sodium sulfate), filtered and dry loaded onto Celite under reduced pressure. The residue was purified using flash column chromatography (80 g HP Silica, 0-100 %, ethyl acetate/hexanes gradient) to give the title compound as an off-white solid (1.46 g). ¹H NMR (400 MHz, Chloroform-d) δ 7.41 - 7.35 (m, 1H), 6.96 (dd, 1H), 6.90 - 6.85 (m, 1H), 5.07 (s, 1H), 1.86 - 1.75 (m, 1H), 0.97 - 0.84 (m, 2H), 0.67 - 0.53 (m, 2H); LC-MS (Method 4): Rₜ = 1.46 min; MS (ESlneg): m/z = 259 [M-H]⁻

### Intermediate 94

### 4-Cyclopropyl-2-iodo-1-methoxybenzene

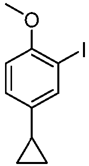

To a stirred suspension of 4-cyclopropyl-2-iodo-phenol (730 mg, 2.81 mmol, 1.00 eq.) in anhydrous DMF (14.0 mL, 0.20 M) was added potassium carbonate (776 mg, 5.61 mmol, 2.00 eq.) and iodomethane (260 µL, 4.21 mmol, 1.20 eq.). The resulting mixture was heated at 70 °C for 16 h and then cooled to room temperature. The reaction mixture was diluted with water (20 mL) and extracted with ethyl acetate (3 x 20 mL). The combined organic extracts were washed with brine (20 mL), dried (magnesium sulfate), filtered, and dry loaded onto Celite under reduced pressure. The residue was purified by flash column chromatography (40 g HP Silica, 0-30 %, ethyl acetate/hexanes gradient) to give the title compound as a colorless oil (365 mg). ¹H NMR (400 MHz, Chloroform-d) δ 7.52 - 7.47 (m, 1H), 7.06 - 6.99 (m, 1H), 6.75 - 6.68 (m, 1H), 3.84 (s, 3H), 1.87 - 1.75 (m, 1H), 0.97 - 0.84 (m, 2H), 0.68 - 0.54 (m, 2H); LC-MS (Method 4): Rₜ = 1.64 min; MS (ESlpos): m/z = not detected

### Intermediate 95

### 5-Cyclopropyl-2-methoxybenzenesulfonamide

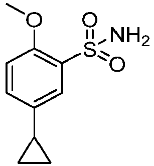

To a 20 mL vial was added 4-cyclopropyl-2-iodo-1-methoxy-benzene (359 mg, 1.31 mmol, 1.00 eq.), palladium(ll) acetate (14.7 mg, 0.07 mmol, 5.00 mol%), CataCXium^{®} A (37.6 mg, 0.10 mmol, 7.50 mol%), and 1,4-Diazabicyclo[2.2.2]octane bis(sulfur dioxide) adduct (189 mg, 0.79 mmol, 0.60 eq.) sequentially followed by the addition of degassed anhydrous isopropanol (6.55 mL, 0.20 M) and triethylamine (540 µL, 3.93 mmol, 3.00 eq.). The resulting red solution was heated at 75 °C for 16 h, cooled to room temperature and concentrated under reduced pressure. The residue was resuspended in anhydrous tetrahydrofuran (7.00 mL) and cooled to 0 °C in a ice/water bath. To this mixture was then added sulfuryl chloride (320 µL, 3.93 mmol, 3.00 eq.) dropwise, after which the resulting mixture was warmed to room temperature and stirred for a further 30 minutes. The reaction was quenched with ammonium chloride (20 mL), and the aqueous layer was extracted with ethyl acetate (3 x 20 mL). The combined organic extracts were washed with brine (20 mL), dried (magnesium sulfate), filtered and concentrated under reduced pressure. The residue was suspended in anhydrous tetrahydrofuran (7.00 mL). To this solution was then added ammonium hydroxide solution (28.0-30.0% NH₃ basis, 3.73 mL, 26.2 mmol, 20.0 eq.) and and the resulting mixture stirred at room temperature for 30 minutes. The reaction was quenched with aqueous hydrochloric acid (1.00 M, 20 mL) and extracted with ethyl acetate (3 x 20 mL). The combined organic extracts were washed with brine (20 mL), dried (magnesium sulfate), filtered and dry loaded onto Celite under reduced pressure. The residue was purified by flash column chromatography (24 g HP Silica, 0-80 %, ethyl acetate/hexanes gradient) to give the title compound as a white solid (96.0 mg). ¹H NMR (400 MHz, Chloroform-d) δ 7.64 - 7.59 (m, 1H), 7.28 (d, 1H), 6.98 - 6.91 (m, 1H), 4.99 (s, 2H), 3.98 (s, 3H), 1.95 - 1.84 (m, 1H), 1.03 - 0.89 (m, 2H), 0.73 - 0.59 (m, 2H); LC-MS (Method 4): Rt = 1.23 min; MS (ESlpos): m/z = 228 [M+H]⁺

### Intermediate 96

### 4-Cyclopropyl-1-ethoxy-2-iodobenzene

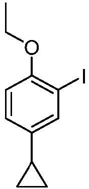

To a stirred solution of 4-cyclopropyl-2-iodo-phenol (730 mg, 2.81 mmol, 1.00 eq.) in anhydrous N,N-dimethylformamide (14.0 mL, 0.20 M) was added potassium carbonate (776 mg, 5.61 mmol, 2.00 eq.) and iodoethane (270 µL, 3.37 mmol, 1.20 eq.). The resulting mixture was heated at 70 °C for 16 h and then cooled to room temperature. The reaction mixture was diluted with water (20 mL) and extracted with ethyl acetate (3 x 20 mL). The combined organic extracts were washed with brine (20 mL), dried (magnesium sulfate), filtered and dry loaded onto Celite under reduced pressure. The residue was purified by flash column chromatography (40 g HP Silica, 0-30 %, ethyl acetate/hexanes gradient) to give the title compound as a colorless oil (760 mg). ¹H NMR (400 MHz, Chloroform-d) δ 7.52 - 7.46 (m, 1H), 7.03 - 6.96 (m, 1H), 6.73 - 6.66 (m, 1H), 4.05 (q, J = 7.0 Hz, 2H), 1.86 - 1.74 (m, 1H), 1.45 (t, 3H), 0.96 - 0.83 (m, 2H), 0.67 - 0.54 (m, 2H); LC-MS (Method 4): Rt = 1.71 min; MS (ESlpos): m/z = not detected

### Intermediate 97

### 5-Cyclopropyl-2-ethoxybenzenesulfonamide

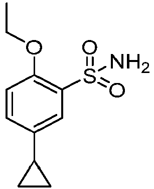

To a 20 mL vial was added 4-cyclopropyl-1-ethoxy-2-iodo-benzene (756 mg, 2.62 mmol, 1.00 eq.), palladium(ll) acetate (29.5 mg, 0.13 mmol, 5.00 mol%), CataCXium^{®} A (75.3 mg, 0.21 mmol, 7.50 mol%), and 1,4-Diazabicyclo[2.2.2]octane bis(sulfur dioxide) adduct (378 mg, 1.57 mmol, 0.60 eq.) sequentially followed by the addition of degassed anhydrous isopropanol (13.1 mL, 0.20 M) and triethylamine (1.09 mL, 7.87 mmol, 3.00 eq.). The resulting red solution was heated at 75 °C for 16 h, cooled to room temperature and concentrated under reduced pressure. The residue was resuspended in anhydrous tetrahydrofuran (13.0 mL) and cooled to 0 °C in a ice/water bath. To this mixture was then added sulfuryl chloride (640 µL, 7.87 mmol, 3.00 eq.) dropwise, after which the resulting mixture was warmed to room temperature and stirred for a further 30 minutes. The reaction mixture was diluted with ammonium chloride (20 mL) and extracted with ethyl acetate (3 x 20 mL). The combined organic extracts were washed with brine (20 mL), dried (magnesium sulfate), filtered and concentrated down under reduced pressure. The residue was resuspended in anhydrous tetrahydrofuran (13.0 mL). To this solution was then added ammonium hydroxide solution (28.0-30.0% NH₃ basis, 7.46 mL, 52.5 mmol, 20.0 eq.) and the resulting mixture was stirred at room temperature for 30 minutes. The reaction was quenched with aqueous hydrochloric acid (1.00 M, 30 mL) and extracted with ethyl acetate (3 x 20 mL). The combined organic extracts were washed with brine (20 mL), dried (magnesium sulfate), filtered and dry loaded onto Celite under reduced pressure. The reside was purified by flash column chromatography (40 g HP Silica, 0-80 %, ethyl acetate/hexanes gradient) to give the title compound as a white solid (159 mg). ¹H NMR (400 MHz, Chloroform-d) δ 7.64 - 7.58 (m, 1H), 7.28 - 7.20 (m, 1H), 6.96 - 6.89 (m, 1H), 4.99 (s, 2H), 4.21 (q, 2H), 1.95 - 1.83 (m, 1H), 1.50 (t, 3H), 1.00 - 0.89 (m, 2H), 0.69 - 0.61 (m, 2H); LC-MS (Method 4): Rₜ = 1.30 min; MS (ESlpos): m/z = 242 [M+H]⁺

### Intermediate 98

### tert-Butyl 4-((4-(tert-butyl)-2-iodophenoxy)methyl)piperidine-1-carboxylate

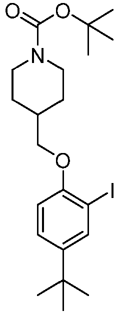

To a stirred solution of 4-tert-butyl-2-iodo-phenol (1.50 g, 5.43 mmol, 1.00 eq.) in anhydrous *N,N*-dimethylformamide (18.1 mL, 0.30 M) was added potassium carbonate (1.50 g, 10.9 mmol, 2.00 eq.) and tert-butyl 4-(bromomethyl)piperidine-1-carboxylate (2.27 g, 8.15 mmol, 1.50 eq.). The resulting mixture was heated at 75 °C for 64 h and then cooled to room temperature. The reaction mixture was diluted with water (30 mL) and extracted with dichloromethane (3 x 30 mL). The combind organic extracts were washed with brine (30 mL), dried (magnesium sulfate), filtered and dry loaded onto Celite under reduce pressure. The reside was purified by flash column chromatography (80 g HP Silica, 0-20 %, ethyl acetate/hexanes gradient) to give the title compound as a white solid (2.35 g). ¹H NMR (400 MHz, Chloroform-d) δ 7.76 (d, 1H), 7.32 - 7.24 (m, 1H), 6.74 - 6.67 (m, 1H), 4.18 - 4.13 (m, 2H), 3.82 (d, 2H), 2.83 - 2.64 (m, 2H), 1.92 - 1.84 (m, 2H), 1.49 - 1.43 (m, 10H), 1.38 - 1.20 (m, 11 H); LC-MS (Method 4): Rₜ = 1.79 min; MS (ESlpos): m/z = not detected

### Intermediate 99

### tert-Butyl 4-((4-(tert-butyl)-2-sulfamoylphenoxy)methyl)piperidine-1-carboxylate

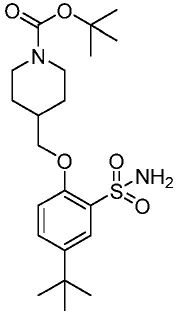

To a 40 mL vial was added tert-butyl 4-[(4-tert-butyl-2-iodo-phenoxy)methyl]piperidine-1-carboxylate (1.08 g, 2.29 mmol, 1.00 eq.), palladium(ll) acetate (25.7 mg, 0.11 mmol, 5.00 mol%), CataCXium^{®} A (65.6 mg, 0.18 mmol, 7.50 mol%), and 1,4-Diazabicyclo[2.2.2]octane bis(sulfur dioxide) adduct (330 mg, 1.37 mmol, 0.60 eq.) sequentially followed by the addition of degassed anhydrous isopropanol (11.4 mL, 0.20 M) and triethylamine (0.95 mL, 6.86 mmol, 3.00 eq.). The resulting red solution was heated at 75 °C for 16 h, cooled to room temperature and concentrated under reduced pressure. The residue was resuspended in anhydrous tetrahydrofuran (12.0 mL) and cooled to 0 °C in a ice/water bath. To this mixture was then added sulfuryl chloride (550 µL, 6.86 mmol, 3.00 eq.) dropwise, after which the resulting mixture was warmed to room temperature and stirred for a further 30 minutes. The reaction was quenched with ammonium chloride (20 mL) and extracted with dichloromethane (3 x 20 mL). The combined organic extracts were washed with brine (20 mL), dried (magnesium sulfate), filtered and concentrated under reduced pressure. The residue was was resuspended in anhydrous tetrahydrofuran (12.0 mL) and to this solution was added ammonium hydroxide solution (28.0-30.0% NH₃ basis, 6.50 mL, 45.7 mmol, 20.0 eq.). After stirring for a further 30 minutes, the mixture was diluted with aqueous hydrochloric acid (1.00 M, 30 mL) and extracted with ethyl acetate (3 x 20 mL). The combined organic extracts were washed with brine (20 mL), dried (magnesium sulfate), filtered, and dry loaded onto Celite under reduced pressure. The residue was purified by flash column chromatography (80 g HP Silica, 0-100 %, ethyl acetate/hexanes gradient) to give the title compound as a white solid (198 mg). ¹H NMR (400 MHz, Chloroform-d) δ 7.97 - 7.90 (m, 1H), 7.57 - 7.49 (m, 1H), 6.99 - 6.90 (m, 1H), 4.94 (s, 2H), 4.03 - 3.96 (m, 2H), 2.81 - 2.70 (m, 2H), 2.09 - 2.00 (m, 1H), 1.90 - 1.82 (m, 2H), 1.49 - 1.42 (m, 10H), 1.36 - 1.21 (m, 12H); LC-MS (Method 4): Rₜ = 1.57; MS (ESlpos): m/z = 427 [M+H]⁺

### EXPERIMENTAL SECTION - EXAMPLES

### Example 1

### N-([1,1'-biphenyl]-2-sulfonyl)-6-(4-fluorophenyl)-1-benzofuran-2-carboxamide

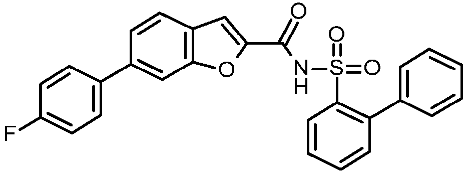

To a stirring suspension of [1,1'-biphenyl]-2-sulfonamide (100 mg, 429 µmol), 6-(4-fluorophenyl)-1-benzofuran-2-carboxylic acid (91.5 mg, 357 µmol) and PyBOP (223 mg, 429 µmol) in dichloromethane (1.8 ml) was added N,N-diisopropylethylamine (250 µl, 1.4 mmol) and the resulting reaction mixture was stirred at room temperature overnight. The mixture was diluted with water and extracted with dichloromethane three times. The combined organic layers were washed with brine, dried over sodium sulfate, concentrated in vacuo and purified by preparative HPLC to give 42.0 mg (90yes% purity, 19% yield) of the title compound as a yellow solid. ¹H-NMR (400 MHz, DMSO-d₆) δ [ppm]: 2.075 (16.00), 2.518 (1.39), 2.523 (1.15), 2.539 (1.32), 7.261 (3.86), 7.264 (5.53), 7.270 (1.67), 7.282 (9.13), 7.285 (8.11), 7.290 (1.38), 7.297 (0.90), 7.306 (9.76), 7.311 (3.18), 7.324 (15.23), 7.328 (15.59), 7.333 (2.82), 7.339 (6.99), 7.343 (8.92), 7.350 (6.78), 7.360 (2.66), 7.364 (4.38), 7.367 (2.19), 7.374 (1.47), 7.381 (4.04), 7.389 (0.84), 7.396 (0.79), 7.399 (1.24), 7.403 (0.64), 7.658 (5.14), 7.661 (4.67), 7.675 (4.49), 7.679 (7.94), 7.682 (5.57), 7.695 (3.33), 7.699 (2.99), 7.724 (8.77), 7.727 (4.40), 7.742 (4.47), 7.746 (4.24), 7.761 (1.71), 7.765 (1.60), 7.792 (0.71), 7.800 (5.98), 7.806 (2.36), 7.813 (6.32), 7.822 (5.99), 7.830 (2.19), 7.836 (5.29), 7.844 (0.53), 7.868 (6.61), 7.889 (5.37), 7.944 (6.77), 8.187 (4.43), 8.190 (4.76), 8.207 (4.04), 8.210 (3.84); LC-MS (method 1): Rt = 1.43 min; MS (ESlneg): m/z = 470 [M-H]⁻

### Example 2

### N-([1,1'-biphenyl]-2-sulfonyl)-6-methyl-1-benzofuran-2-carboxamide

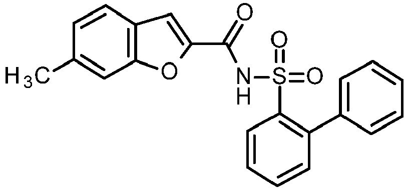

Intermediate 4 (50.0 mg, 110 µmol), methylboronic acid (32.8 mg, 548 µmol), potassium carbonate (45.4 mg, 329 µmol) and 1,1-bis(disphenylphosphino)ferrocene-palladium(ll)dichloride dichloromethane complex (8.95 mg, 11.0 µmol) were sealed in a vessel and were flushed with argon. Dioxane (2 ml) and water (1 ml) was added and the mixture was stirred at 130 °C for 1 h, filtered and concentrated in vacuo. The crude product was purified by preparative HPLC to yield 18.7 mg (41% yield) of the title compound as an offwhite solid. ¹H-NMR (400 MHz, ACETONITRILE-d₃) δ [ppm]: 1.167 (0.44), 1.268 (1.13), 2.101 (0.53), 2.107 (0.68), 2.114 (0.79), 2.146 (2.17), 2.499 (16.00), 7.197 (1.80), 7.199 (1.82), 7.219 (2.83), 7.225 (0.74), 7.241 (4.01), 7.252 (1.10), 7.258 (4.95), 7.265 (4.74), 7.269 (6.35), 7.276 (0.97), 7.282 (1.14), 7.286 (1.99), 7.290 (1.01), 7.322 (2.03), 7.325 (2.06), 7.336 (1.33), 7.341 (3.90), 7.344 (3.97), 7.349 (3.63), 7.352 (3.36), 7.358 (2.49), 7.364 (0.67), 7.371 (0.61), 7.376 (1.05), 7.380 (0.78), 7.386 (5.15), 7.388 (4.86), 7.602 (3.11), 7.616 (1.13), 7.622 (3.03), 7.635 (2.07), 7.639 (2.03), 7.655 (1.66), 7.658 (1.52), 7.697 (1.60), 7.701 (1.72), 7.716 (2.46), 7.720 (2.29), 7.735 (0.98), 7.738 (0.93), 8.246 (2.19), 8.249 (2.23), 8.266 (2.04), 8.269 (2.00); LC-MS (method 1): Rt = 1.32 min; MS (ESlpos): m/z = 392 [M+H]⁺

### Example 3

### N-([1,1'-biphenyl]-2-sulfonyl)-6-cyano-1-benzofuran-2-carboxamide

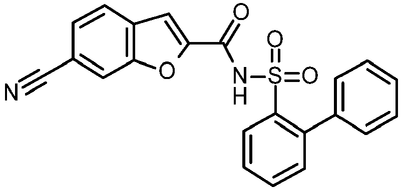

To a mixture of Intermediate 4 (50.0 mg, 110 µmol), di-mu-chloro[bis(1-phenylprop-2-en-1-yl)]dipalladium (2.84 mg, 5.48 µmol), 1,1'bis(diphenylphosphanyl)ferrocene (3.04 mg, 5.48 µmol) and zinc cyanide (15.4 mg, 131 µmol) in *N*,N-dimethylacetamide (1.0 ml) under argon was added DIPEA and the resulting mixture was stirred at 80 °C overnight.

The reaction mixture was partitioned between dichloromethane and sat., aq. NaHCO₃ solution and extracted with dichloromethane twice. The combined organic layers were dried, concentrated in vacuo and purified by preparative HPLC to give 23.2 mg (52% yield) of the title compound. ¹H-NMR (400 MHz, ACETONITRILE-d₃) δ [ppm]: 1.129 (0.86), 1.268 (0.85), 1.768 (0.52), 1.915 (0.59), 1.964 (2.72), 1.975 (0.56), 1.982 (0.51), 1.989 (0.49), 2.095 (1.99), 2.101 (2.43), 2.107 (2.83), 2.114 (2.98), 2.149 (4.27), 2.503 (1.96), 7.215 (3.32), 7.234 (11.12), 7.247 (3.45), 7.252 (12.95), 7.262 (12.53), 7.266 (16.00), 7.282 (5.79), 7.304 (0.43), 7.327 (5.41), 7.330 (5.66), 7.333 (3.75), 7.338 (4.21), 7.345 (6.50), 7.349 (7.37), 7.355 (6.15), 7.368 (1.48), 7.373 (2.22), 7.377 (1.18), 7.483 (13.32), 7.485 (13.19), 7.623 (2.29), 7.626 (2.41), 7.642 (5.46), 7.645 (5.47), 7.653 (7.39), 7.656 (7.48), 7.662 (4.51), 7.665 (4.16), 7.673 (8.47), 7.677 (8.33), 7.705 (3.92), 7.709 (4.13), 7.724 (5.96), 7.727 (5.69), 7.743 (2.44), 7.746 (2.26), 7.887 (8.43), 7.908 (7.11), 7.967 (10.12), 8.253 (5.81), 8.256 (5.83), 8.273 (5.50), 8.276 (5.36); LC-MS (method 2): Rt = 1.16 min; MS (ESlpos): m/z = 403 [M+H]⁺

### Example 4

### N-([1,1'-biphenyl]-2-sulfonyl)-6-(pyridin-3-yl)-1-benzofuran-2-carboxamide

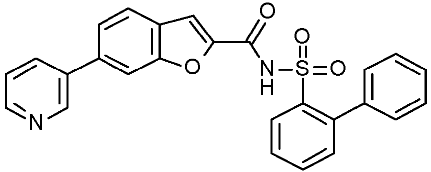

A stirring mixture of Intermediate 4 (100 mg, 219 µmol), pyridin-3-ylboronic acid (33.7 mg, 274 µmol), potassium carbonate (90.9 mg, 657 µmol) and (1,1-bis(diphenylphosphino)ferrocene)-dichloropalladium(II) (16.0 mg, 21.9 µmol) in a mixture of THF (2 ml) and water (1 ml) under argon was heated at 75 °C for 3 h. After adding additional water and extracting with ethyl acetate twice, the combined organic layers were washed with brine, dried over sodium sulfate and concentrated in vacuo. The residue was purified by preparative HPLC to give 42.0 mg (90 % purity, 38% yield) of the title compound as a light brown solid. ¹H-NMR (400 MHz, DMSO-d₆) δ [ppm]: 2.074 (16.00), 2.518 (2.72), 2.522 (1.76), 7.273 (2.05), 7.277 (3.07), 7.283 (1.34), 7.294 (9.34), 7.298 (11.97), 7.308 (4.23), 7.312 (4.76), 7.317 (8.62), 7.322 (2.72), 7.326 (4.49), 7.330 (4.49), 7.336 (3.21), 7.348 (2.11), 7.354 (3.41), 7.359 (1.74), 7.363 (1.30), 7.371 (2.75), 7.378 (0.71), 7.383 (0.64), 7.387 (0.87), 7.392 (0.48), 7.549 (2.23), 7.560 (2.33), 7.568 (2.36), 7.580 (2.36), 7.640 (1.41), 7.643 (1.57), 7.659 (3.33), 7.662 (3.27), 7.682 (8.66), 7.705 (2.54), 7.708 (2.79), 7.723 (3.70), 7.727 (3.74), 7.737 (3.80), 7.741 (4.55), 7.745 (1.68), 7.758 (4.22), 7.761 (4.45), 7.917 (5.87), 7.937 (4.68), 8.076 (6.22), 8.181 (3.85), 8.184 (4.06), 8.200 (3.41), 8.204 (3.45), 8.224 (1.77), 8.228 (2.47), 8.234 (1.98), 8.244 (1.76), 8.250 (2.27), 8.254 (1.74), 8.618 (3.80), 8.622 (3.79), 8.630 (3.79), 8.634 (3.62), 9.020 (4.67), 9.024 (4.71); LC-MS (method 1): Rₜ = 1.03 min; MS (ESlpos): m/z = 455 [M+H]⁺

### Example 5

### 2-[([1,1'-biphenyl]-2-sulfonyl)carbamoyl]-1-benzofuran-6-carboxylic acid

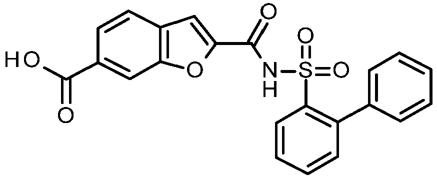

To a stirring solution of Intermediate 4 (100 mg, 219 µmol) in DMSO (3 ml) in an autoclave was added 1,1'-bis(diphenylphosphino)ferrocene (25.0 mg, 97 % purity, 43.8 µmol), palladium(ll) acetate (2.46 mg, 11.0 µmol) and potassium acetate (86.0 mg, 877 µmol). The resulting mixture was flushed with carbon monoxide and stirred overnight at 105 °C at a maximum pressure of 20 bar. The reaction mixture was purified by preparative HPLC to give 8.00 mg (90 % purity, 8% yield) of the title compound as an ochre solid. ¹H-NMR (600 MHz, DMSO-d₆) δ [ppm]: -0.006 (2.30), 0.005 (2.36), 1.910 (0.53), 2.076 (1.36), 2.386 (1.30), 2.389 (1.71), 2.391 (1.27), 2.519 (7.14), 2.523 (5.96), 2.525 (4.58), 2.544 (16.00), 2.614 (1.33), 2.617 (1.71), 2.619 (1.30), 6.997 (8.74), 7.083 (9.54), 7.168 (8.83), 7.251 (5.17), 7.261 (5.28), 7.340 (4.07), 7.485 (0.59), 7.497 (0.62), 7.533 (1.39), 7.552 (1.62), 7.565 (1.48), 7.825 (1.56), 7.873 (3.10), 7.886 (2.18), 8.095 (4.87), 8.133 (2.33), 8.145 (2.15), 8.318 (1.86), 13.112 (0.62); LC-MS (method 1): Rₜ = 1.05 min; MS (ESlneg): m/z = 420 [M-H]⁻

### Example 6

### 4,6-Dimethyl-N-(2'-methyl[1,1'-biphenyl]-2-sulfonyl)-1-benzofuran-2-carboxamide

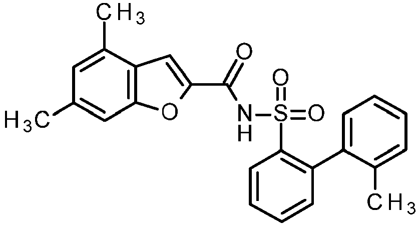

Synthesized analogously to Example 1 using 4,6-dimethyl-1-benzofuran-2-carboxylic acid (100 mg, 526 µmol) and 2'-methyl[1,1'-biphenyl]-2-sulfonamide (156 mg, 631 µmol) instead of 6-(4-fluorophenyl)-1-benzofuran-2-carboxylic acid and [1,1'-biphenyl]-2-sulfonamide to give 120 mg (49% yield) of the title compound as a white solid. ¹H-NMR (400 MHz, DMSO-d₆) δ [ppm]: 1.888 (16.00), 2.409 (14.75), 2.460 (15.33), 2.518 (3.14), 2.523 (1.95), 6.992 (1.88), 6.995 (1.99), 7.010 (4.37), 7.096 (1.02), 7.114 (1.82), 7.133 (0.90), 7.169 (1.62), 7.188 (2.26), 7.199 (0.41), 7.224 (1.45), 7.241 (1.55), 7.272 (3.70), 7.287 (1.86), 7.289 (1.76), 7.305 (0.73), 7.638 (0.61), 7.655 (1.45), 7.674 (1.10), 7.702 (0.98), 7.720 (1.31), 7.738 (0.55), 7.815 (0.64), 8.185 (2.12), 8.189 (2.15), 8.206 (1.95), 8.209 (1.88); LC-MS (method 3): Rt = 1.43 min; MS (ESlpos): m/z = 420 [M+H]⁺

### Example 7

### 4,6-Dimethyl-N-(5-methyl[1,1'-biphenyl]-2-sulfonyl)-1-benzofuran-2-carboxamide

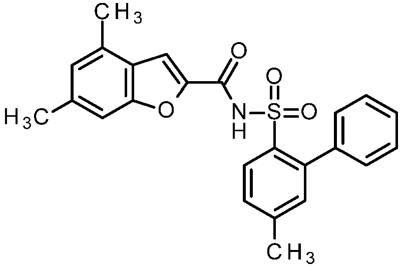

Synthesized analogously to Example 6 using 5-methyl[1,1'-biphenyl]-2-sulfonamide (156 mg, 631 µmol) instead of 2'-methyl[1,1'-biphenyl]-2-sulfonamide to give 85.0 mg (95 % purity, 37 % yield) of the title compound as a white solid. ¹H-NMR (500 MHz, DMSO-d₆) δ [ppm]: 2.357 (1.14), 2.361 (1.70), 2.374 (14.32), 2.400 (15.80), 2.445 (16.00), 2.517 (6.46), 2.521 (4.10), 2.631 (0.94), 2.635 (1.31), 2.638 (0.96), 3.077 (7.49), 6.913 (2.68), 7.007 (2.36), 7.156 (2.88), 7.227 (1.18), 7.239 (3.23), 7.254 (4.56), 7.270 (1.33), 7.287 (1.18), 7.303 (1.03), 7.367 (2.60), 7.381 (2.34), 8.014 (2.97), 8.030 (2.77); LC-MS (method 1): Rt = 1.45 min; MS (ESlneg): m/z = 418 [M-H]⁻

### Example 8

### N²-([1,1'-biphenyl]-2-sulfonyl)-N⁶,N⁶-dimethyl-1-benzofuran-2,6-dicarboxamide

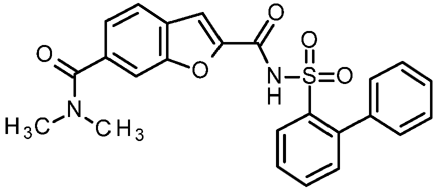

Synthesized analogously to **Example 1** using Intermediate 7 (120 mg, 228 µmol) and N-methylmethanamine (140 µL, 2.0 M in THF, 270 µmol) instead of 6-(4-fluorophenyl)-1-benzofuran-2-carboxylic acid and [1,1'-biphenyl]-2-sulfonamide to give 59.0 mg (90% purity, 52% yield) of the title compound as a white solid. ¹H-NMR (400 MHz, DMSO-d₆) δ [ppm]: 1.237 (0.69), 1.253 (0.46), 2.074 (5.75), 2.331 (0.83), 2.518 (3.77), 2.523 (2.29), 2.539 (0.40), 2.673 (0.81), 2.912 (10.60), 3.009 (10.15), 3.399 (2.33), 6.639 (0.85), 7.255 (4.77), 7.260 (6.54), 7.266 (2.63), 7.277 (13.00), 7.280 (11.75), 7.291 (5.71), 7.294 (7.65), 7.299 (2.46), 7.312 (16.00), 7.316 (9.50), 7.331 (10.35), 7.335 (6.69), 7.345 (3.62), 7.349 (5.58), 7.354 (3.06), 7.359 (8.69), 7.362 (8.15), 7.367 (5.56), 7.374 (1.81), 7.379 (7.67), 7.382 (8.23), 7.645 (2.19), 7.649 (2.37), 7.664 (4.98), 7.667 (4.75), 7.684 (4.42), 7.688 (4.42), 7.700 (6.54), 7.711 (11.88), 7.718 (4.65), 7.733 (5.12), 7.737 (5.12), 7.752 (2.15), 7.755 (1.85), 7.847 (8.29), 7.867 (7.48), 8.154 (0.48), 8.176 (6.33), 8.179 (6.50), 8.196 (5.37), 8.199 (5.35); LC-MS (method 1): Rt = 1.07 min; MS (ESlneg): m/z = 447 [M-H]⁻

### Example 9

### N-([1,1'-biphenyl]-2-sulfonyl)-4,6-dimethyl-1-benzofuran-2-carboxamide

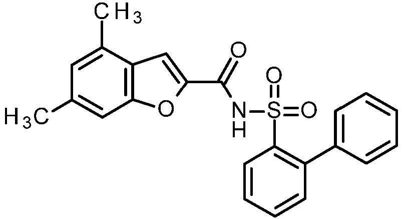

Synthesized analogously to Example 1 using 4,6-dimethyl-1-benzofuran-2-carboxylic acid (100 mg, 526 µmol) instead of 6-(4-fluorophenyl)-1-benzofuran-2-carboxylic acid to give 150 mg (85 % purity, 60 % yield) as a white solid. ¹H-NMR (400 MHz, DMSO-d₆) δ [ppm]: 1.636 (0.60), 1.645 (0.67), 1.653 (1.65), 1.661 (0.66), 1.669 (0.62), 2.406 (15.19), 2.452 (16.00), 2.518 (4.53), 2.522 (2.80), 2.892 (0.44), 2.903 (0.69), 2.909 (1.29), 2.920 (1.31), 2.926 (0.67), 2.936 (0.42), 7.001 (3.63), 7.259 (5.98), 7.275 (5.74), 7.279 (4.63), 7.289 (2.72), 7.292 (3.67), 7.297 (1.07), 7.310 (7.52), 7.313 (2.48), 7.325 (3.33), 7.328 (4.21), 7.354 (1.17), 7.358 (1.93), 7.362 (0.99), 7.369 (0.75), 7.376 (1.97), 7.382 (0.52), 7.389 (0.42), 7.393 (0.64), 7.636 (0.73), 7.640 (0.79), 7.655 (1.81), 7.659 (1.79), 7.675 (1.93), 7.678 (2.06), 7.706 (1.31), 7.709 (1.33), 7.725 (1.65), 7.728 (1.65), 7.743 (0.67), 8.159 (2.50), 8.162 (2.64), 8.179 (2.28), 8.182 (2.18); LC-MS (method 1): Rt = 1.38 min; MS (ESlpos): m/z = 406 [M+H]⁺

### Example 10

### N-([1,1'-biphenyl]-2-sulfonyl)-4-methoxy-6-methyl-1-benzofuran-2-carboxamide

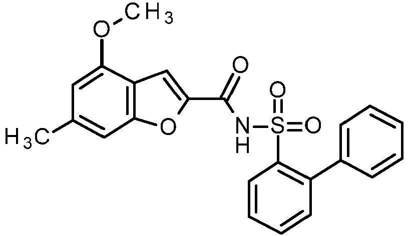

Synthesized analogously to Example 1 using Intermediate 6 (150 mg, 727 µmol) instead of 6-(4-fluorophenyl)-1-benzofuran-2-carboxylic acid to give 31.0 mg (85% purity, 9% yield) of the title compound as a yellow solid. ¹H-NMR (400 MHz, DMSO-d₆) δ [ppm]: 1.154 (0.90), 1.172 (1.76), 1.190 (0.90), 1.228 (0.53), 1.988 (3.58), 2.085 (0.48), 2.432 (10.62), 2.518 (1.84), 2.523 (1.21), 2.540 (4.42), 3.872 (0.49), 3.897 (0.40), 3.918 (16.00), 4.017 (0.78), 4.035 (0.75), 6.713 (3.34), 7.037 (2.82), 7.039 (2.93), 7.238 (1.34), 7.242 (1.98), 7.259 (3.81), 7.263 (3.18), 7.279 (1.51), 7.282 (2.17), 7.287 (0.65), 7.299 (3.83), 7.304 (2.79), 7.307 (1.75), 7.319 (1.89), 7.322 (1.90), 7.326 (1.64), 7.341 (0.86), 7.345 (1.49), 7.348 (0.77), 7.356 (0.56), 7.363 (1.50), 7.380 (0.46), 7.640 (0.59), 7.644 (0.64), 7.660 (1.63), 7.663 (1.84), 7.669 (1.36), 7.679 (1.45), 7.682 (1.19), 7.710 (0.95), 7.713 (1.00), 7.729 (1.32), 7.732 (1.32), 7.747 (0.58), 7.751 (0.51), 8.161 (1.73), 8.165 (1.80), 8.181 (1.59), 8.184 (1.58); LC-MS (method 1): Rₜ = 1.35 min; MS (ESlpos): m/z = 422 [M+H]⁺

### Example 11

### N-(3'-chloro[1,1'-biphenyl]-2-sulfonyl)-4,6-dimethyl-1-benzofuran-2-carboxamide

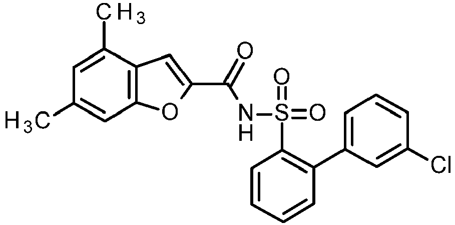

Synthesized analogously to Example 1 using 4,6-dimethyl-1-benzofuran-2-carboxylic acid (100 mg, 526 µmol) and 3'-chloro[1,1'-biphenyl]-2-sulfonamide (169 mg, 631 µmol) instead of 6-(4-fluorophenyl)-1-benzofuran-2-carboxylic acid and [1,1'-biphenyl]-2-sulfonamide to give 120 mg (49% yield) of the title compound as a white solid. ¹H-NMR (400 MHz, DMSO-d₆) δ [ppm]: 2.075 (10.71), 2.404 (15.32), 2.458 (16.00), 2.518 (4.14), 2.523 (2.87), 6.996 (3.66), 7.222 (0.96), 7.226 (2.65), 7.229 (3.20), 7.235 (2.91), 7.242 (3.64), 7.245 (3.34), 7.249 (1.39), 7.259 (3.56), 7.326 (1.77), 7.330 (1.79), 7.345 (2.07), 7.348 (2.05), 7.354 (2.49), 7.374 (4.66), 7.386 (0.46), 7.395 (2.53), 7.449 (1.67), 7.452 (1.91), 7.454 (1.83), 7.457 (1.65), 7.469 (1.09), 7.472 (1.23), 7.474 (1.19), 7.477 (1.01), 7.665 (0.62), 7.668 (0.68), 7.684 (1.69), 7.687 (1.63), 7.703 (1.45), 7.706 (1.35), 7.717 (1.27), 7.721 (1.39), 7.736 (1.79), 7.739 (1.87), 7.754 (1.63), 8.169 (2.41), 8.172 (2.69), 8.189 (2.13), 8.192 (2.21); LC-MS (method 1): Rt = 1.43 min; MS (ESlpos): m/z = 440 [M+H]⁺

### Example 12

### 4-Methoxy-6-methyl-N-(5-methyl[1,1'-biphenyl]-2-sulfonyl)-1-benzofuran-2-carboxamide

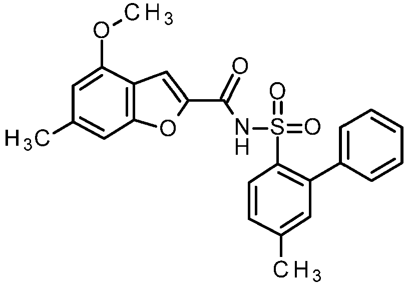

Synthesized analogously to Example 1 using Intermediate 6 (150 mg, 727 µmol) and 5-methyl[1,1'-biphenyl]-2-sulfonamide (216 mg, 873 µmol) instead of 6-(4-fluorophenyl)-1-benzofuran-2-carboxylic acid and [1,1'-biphenyl]-2-sulfonamide to give 105 mg (% yield) of the title compound as a light yellow solid. ¹H-NMR (400 MHz, DMSO-d₆) δ [ppm]: 1.172 (0.48), 1.987 (0.94), 2.403 (11.19), 2.431 (11.17), 2.518 (2.28), 2.522 (1.42), 3.917 (16.00), 6.711 (3.58), 7.037 (3.08), 7.039 (3.03), 7.128 (2.42), 7.223 (1.68), 7.227 (2.30), 7.245 (3.84), 7.272 (1.51), 7.275 (2.16), 7.279 (0.68), 7.293 (3.85), 7.296 (1.64), 7.311 (1.85), 7.336 (0.92), 7.339 (1.57), 7.343 (0.84), 7.351 (0.61), 7.358 (1.68), 7.364 (0.41), 7.376 (0.51), 7.445 (1.20), 7.466 (1.30), 7.669 (1.74), 8.041 (3.02), 8.062 (2.82); LC-MS (method 1): Rₜ = 1.41 min; MS (ESlpos): m/z = 436 [M+H]⁺

### Example 13

### N-(2-Ethoxybenzene-1-sulfonyl)-4-methoxy-6-methyl-1-benzofuran-2-carboxamide

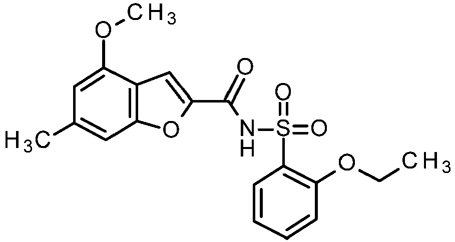

Synthesized analogously to Example 12 using 2-ethoxybenzene-1-sulfonamide (176 mg, 873 µmol) instead of 5-methyl[1,1'-biphenyl]-2-sulfonamide to give 98.0 mg (90 % purity, 31% yield) of the title compound as a light yellow solid. ¹H-NMR (400 MHz, DMSO-d₆) δ [ppm]: 1.154 (0.76), 1.172 (1.66), 1.189 (0.87), 1.213 (4.46), 1.231 (9.58), 1.248 (4.63), 1.987 (2.94), 2.083 (0.57), 2.425 (10.18), 2.518 (1.22), 2.522 (0.78), 3.920 (16.00), 4.016 (0.67), 4.034 (0.66), 4.127 (1.13), 4.145 (3.68), 4.162 (3.75), 4.180 (1.09), 6.719 (3.24), 7.046 (2.89), 7.048 (2.81), 7.113 (0.96), 7.115 (0.97), 7.134 (1.77), 7.151 (1.08), 7.154 (1.07), 7.203 (1.58), 7.222 (1.78), 7.622 (0.91), 7.627 (0.95), 7.641 (1.00), 7.645 (1.17), 7.648 (1.00), 7.662 (0.73), 7.666 (0.71), 7.900 (1.86), 7.904 (1.91), 7.920 (1.87), 7.924 (1.66), 8.080 (2.10), 12.534 (0.54); LC-MS (method 1): Rt = 1,22 min; MS (ESlpos): m/z = 390 [M+H]⁺

### Example 14

### N-([1,1'-biphenyl]-2-sulfonyl)-3,6-dimethyl-1-benzofuran-2-carboxamide

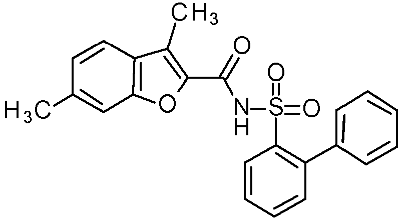

Synthesized analogously to Example 1 using 3,6-dimethyl-1-benzofuran-2-carboxylic acid (100 mg, 526 µmol) instead of 6-(4-fluorophenyl)-1-benzofuran-2-carboxylic acid to give 59.0 mg (26% yield) of the title compound as a grey solid. ¹H-NMR (400 MHz, DMSO-d₆) δ [ppm]: 2.401 (16.00), 2.485 (10.12), 2.518 (1.16), 2.523 (0.81), 7.192 (1.21), 7.193 (1.22), 7.212 (1.34), 7.214 (1.33), 7.276 (5.19), 7.287 (12.66), 7.297 (0.70), 7.319 (2.04), 7.328 (0.77), 7.330 (1.12), 7.337 (1.80), 7.341 (2.31), 7.347 (0.45), 7.357 (2.37), 7.359 (2.58), 7.637 (2.52), 7.657 (3.09), 7.673 (1.06), 7.677 (0.98), 7.703 (0.95), 7.706 (1.01), 7.721 (1.32), 7.725 (1.33), 7.740 (0.54), 7.744 (0.49), 8.140 (1.57), 8.143 (1.72), 8.159 (1.43), 8.162 (1.43); LC-MS (method 3): Rt = 1.38 min; MS (ESlpos): m/z = 406 [M+H]⁺

### Example 15

### N-([1,1'-biphenyl]-2-sulfonyl)-6-cyclopropyl-7-fluoro-1-benzofuran-2-carboxamide

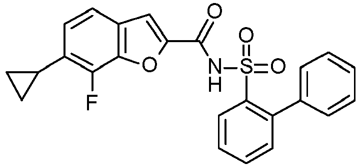

Synthesized analogously to **Example 1** using **Intermediate 8** (150 mg, 681 µmol) instead of 6-(4-fluorophenyl)-1-benzofuran-2-carboxylic acid to give 30.0 mg (95% purity, 10% yield) of the title compound as an offwhite solid. ¹H-NMR (400 MHz, DMSO-d₆) δ [ppm]: 0.801 (2.68), 0.812 (8.98), 0.817 (8.09), 0.825 (8.59), 0.830 (8.42), 0.841 (3.33), 1.047 (3.30), 1.058 (7.96), 1.063 (7.69), 1.068 (4.20), 1.074 (4.23), 1.079 (8.00), 1.084 (7.63), 1.095 (2.76), 1.169 (1.86), 1.375 (2.05), 1.409 (2.09), 2.163 (1.03), 2.176 (2.08), 2.184 (2.29), 2.197 (3.94), 2.210 (2.12), 2.218 (1.89), 2.230 (0.83), 2.334 (2.39), 2.338 (1.01), 2.520 (11.31), 2.525 (7.83), 2.542 (0.70), 2.676 (2.33), 2.680 (1.00), 3.639 (0.42), 6.915 (4.26), 6.931 (4.66), 6.935 (4.86), 6.951 (4.55), 7.256 (4.39), 7.260 (6.41), 7.266 (2.46), 7.277 (16.00), 7.281 (14.44), 7.285 (10.05), 7.288 (10.52), 7.292 (2.79), 7.304 (15.45), 7.309 (10.07), 7.324 (7.03), 7.328 (7.48), 7.331 (6.73), 7.337 (4.25), 7.341 (6.02), 7.346 (3.00), 7.351 (2.21), 7.358 (5.27), 7.366 (1.21), 7.375 (1.47), 7.380 (0.87), 7.491 (9.71), 7.512 (9.03), 7.645 (2.38), 7.648 (2.64), 7.667 (8.52), 7.683 (5.01), 7.686 (4.31), 7.714 (3.56), 7.717 (3.78), 7.732 (4.98), 7.735 (4.92), 7.751 (2.00), 8.175 (7.04), 8.178 (7.33), 8.194 (6.37), 8.198 (6.21); LC-MS (method 1): Rt = 1.39 min; MS (ESlpos): m/z = 436 [M+H]⁺

### Example 16

### N-([1,1'-biphenyl]-2-sulfonyl)-6-cyclopropyl-1-benzofuran-2-carboxamide

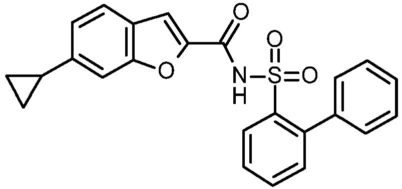

Synthesized analogously to **Intermediate 5** using **Intermediate 4** (50.0 mg, 110 µmol) instead of **Intermediate 3** to yield 35.5 mg (78 % yield) of the title compound. ¹H-NMR (400 MHz, ACETONITRILE-d₃) δ [ppm]: 0.771 (2.78), 0.782 (8.51), 0.788 (7.08), 0.795 (7.97), 0.800 (7.80), 0.811 (3.44), 1.044 (3.17), 1.054 (6.61), 1.060 (7.60), 1.065 (3.70), 1.071 (3.62), 1.076 (7.41), 1.081 (7.14), 1.092 (2.83), 1.129 (1.09), 1.168 (0.42), 1.268 (0.70), 1.421 (0.68), 1.456 (0.71), 1.768 (0.62), 1.775 (0.45), 1.915 (0.72), 1.964 (0.53), 2.042 (1.19), 2.055 (2.29), 2.063 (2.37), 2.067 (1.57), 2.075 (4.24), 2.084 (1.76), 2.088 (2.57), 2.096 (2.70), 2.101 (1.52), 2.109 (2.37), 2.113 (2.23), 2.144 (13.14), 2.501 (2.39), 7.097 (4.94), 7.101 (5.03), 7.118 (5.27), 7.121 (5.46), 7.215 (8.22), 7.222 (3.22), 7.226 (1.49), 7.238 (4.20), 7.241 (9.37), 7.253 (2.53), 7.258 (12.53), 7.263 (12.60), 7.268 (16.00), 7.275 (1.88), 7.280 (2.46), 7.284 (4.89), 7.288 (2.00), 7.321 (4.84), 7.324 (4.70), 7.329 (0.78), 7.335 (3.00), 7.339 (8.40), 7.344 (6.24), 7.351 (2.58), 7.352 (2.63), 7.356 (6.15), 7.359 (3.04), 7.363 (13.50), 7.366 (12.31), 7.373 (2.50), 7.378 (1.15), 7.587 (7.63), 7.606 (7.09), 7.614 (2.66), 7.618 (2.47), 7.633 (4.97), 7.636 (4.65), 7.653 (4.46), 7.656 (3.93), 7.695 (4.18), 7.699 (4.12), 7.714 (5.99), 7.717 (6.06), 7.732 (2.45), 7.736 (2.34), 8.242 (5.15), 8.245 (5.33), 8.262 (4.86), 8.265 (4.64); LC-MS (method 3): Rt = 1.36 min; MS (ESlpos): m/z = 418 [M+H]⁺

### Example 17

### N-([1,1'-Biphenyl]-2-sulfonyl)-6-(difluoromethyl)-1-benzofuran-2-carboxamide

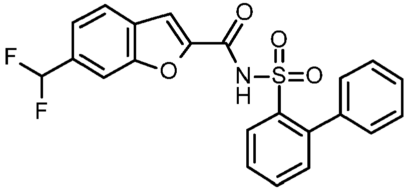

**Intermediate 4** (50.0 mg, 110 µmol), XPhos (5.22 mg, 11.0 µmol), XPhos Pd G3 (9.27 mg, 11.0 µmol) and [1,3-Bis[2,6-bis(i-propyl)phenyl]-2-imidazolidinylidene]difluoromethylsilver(I) (78.3 mg, 142 µmol) were sealed in a vessel flushed with argon. Toluene (1.0 ml) was added and the mixture was stirred at 100 °C overnight. The mixture was filtered, the filtrate was concentrated in vacuo and the residue was purified by preparative HPLC to give 1.00 mg (100 % purity, 2 % yield) of the title compound. ¹H-NMR (400 MHz, ACETONITRILE-d₃) δ [ppm]: 1.084 (0.44), 1.099 (0.40), 1.763 (0.68), 1.769 (0.91), 1.775 (0.68), 1.915 (1.03), 1.965 (2.46), 2.095 (2.31), 2.102 (3.04), 2.107 (3.91), 2.114 (4.51), 2.151 (16.00), 2.502 (1.94), 6.791 (2.20), 6.932 (4.45), 7.072 (2.15), 7.215 (1.40), 7.218 (2.25), 7.222 (0.91), 7.234 (3.93), 7.236 (4.93), 7.239 (3.63), 7.250 (1.71), 7.255 (5.45), 7.290 (5.28), 7.294 (7.27), 7.300 (3.55), 7.304 (2.99), 7.307 (2.32), 7.311 (5.50), 7.313 (4.26), 7.315 (4.12), 7.320 (3.42), 7.323 (3.04), 7.326 (1.47), 7.332 (3.02), 7.338 (0.72), 7.346 (0.70), 7.350 (1.17), 7.353 (0.59), 7.402 (5.08), 7.514 (2.04), 7.533 (2.31), 7.593 (1.28), 7.597 (1.33), 7.612 (2.76), 7.616 (2.48), 7.632 (2.43), 7.635 (2.17), 7.665 (2.03), 7.668 (2.17), 7.684 (3.06), 7.687 (2.92), 7.703 (1.15), 7.706 (1.08), 7.737 (3.55), 7.839 (2.86), 7.859 (2.50), 8.240 (2.90), 8.243 (3.00), 8.259 (2.53), 8.263 (2.64); LC-MS (method 3 ): Rt = 1.73 min; MS (ESlpos): m/z = 428 [M+H]⁺

### Example 18

### N-(2-Ethoxybenzene-1-sulfonyl)-3,6-dimethyl-1-benzofuran-2-carboxamide

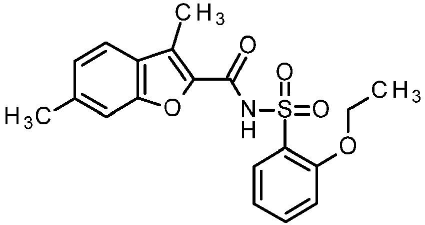

Synthesized analogously to Example 1 using 3,6-dimethyl-1-benzofuran-2-carboxylic acid (100 mg, 526 µmol) and 2-ethoxybenzene-1-sulfonamide (127 mg, 631 µmol) instead of 6-(4-fluorophenyl)-1-benzofuran-2-carboxylic acid and [1,1'-biphenyl]-2-sulfonamide to yield 60.0 mg (29% yield) of the title compound as a white solid. ¹H-NMR (400 MHz, DMSO-d₆) δ [ppm]: 1.234 (3.73), 1.251 (8.38), 1.269 (3.80), 2.074 (5.33), 2.398 (16.00), 2.421 (0.50), 2.477 (10.79), 2.518 (1.77), 2.522 (1.25), 4.120 (0.87), 4.137 (2.71), 4.155 (2.68), 4.172 (0.81), 7.112 (0.68), 7.130 (1.32), 7.150 (0.73), 7.191 (1.25), 7.201 (1.26), 7.210 (1.39), 7.212 (1.38), 7.222 (1.24), 7.408 (2.34), 7.633 (2.33), 7.653 (1.86), 7.888 (1.41), 7.892 (1.43), 7.908 (1.33), 7.912 (1.26); LC-MS (method 3): Rt = 1.25 min; MS (ESlpos): m/z = 374 [M+H]⁺

### Example 19

### N-(2-Methylquinoline-8-sulfonyl)-6-(trifluoromethyl)-1-benzofuran-2-carboxamide

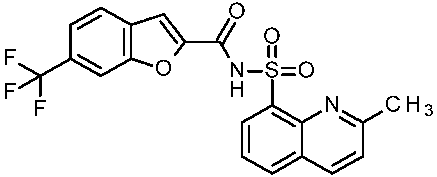

Synthesized analogously to Example 1 using 6-(trifluoromethyl)-1-benzofuran-2-carboxylic acid (150 mg, 652 µmol) and 2-methylquinoline-8-sulfonamide (174 mg, 782 µmol) instead of 6-(4-fluorophenyl)-1-benzofuran-2-carboxylic acid and [1,1'-biphenyl]-2-sulfonamide to yield 151 mg (95 % purity, 51% yield) of the title compound as a white solid. ¹H-NMR (400 MHz, DMSO-d₆) δ [ppm]: 2.331 (0.50), 2.527 (0.99), 2.673 (0.66), 2.695 (16.00), 7.544 (3.04), 7.565 (3.08), 7.671 (1.37), 7.674 (1.37), 7.692 (1.48), 7.695 (1.47), 7.761 (1.50), 7.781 (2.41), 7.800 (1.58), 8.078 (1.91), 8.098 (1.69), 8.119 (2.67), 8.211 (2.00), 8.314 (1.55), 8.317 (1.60), 8.334 (1.48), 8.338 (1.36), 8.432 (2.87), 8.454 (2.71), 8.491 (1.93), 8.494 (1.94), 8.510 (1.90), 8.513 (1.61); LC-MS (method 1): Rt = 1.10 min; MS (ESlpos): m/z = 435 [M+H]⁺

### Example 20

### N-([1,1'-Biphenyl]-2-sulfonyl)-6-(pyridin-4-yl)-1-benzofuran-2-carboxamide

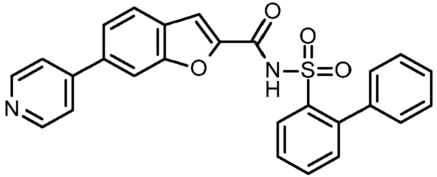

Synthesized analogously to Example 4 using pyridin-4-ylboronic acid (33.7 mg, 274 µmol) instead of pyridin-3-ylboronic acid to yield 8.00 mg (7% yield) of the title compound as a light brown solid. ¹H-NMR (400 MHz, DMSO-d₆) δ [ppm]: 0.852 (0.59), 1.233 (1.78), 1.986 (0.44), 2.332 (3.26), 2.518 (16.00), 2.523 (10.96), 3.348 (11.48), 7.281 (1.93), 7.301 (4.81), 7.309 (14.89), 7.322 (2.30), 7.575 (0.89), 7.624 (1.48), 7.642 (1.19), 7.663 (1.11), 7.683 (1.33), 7.814 (1.48), 7.834 (2.15), 7.909 (2.52), 7.922 (5.33), 7.943 (2.15), 8.168 (4.44), 8.184 (2.00), 8.700 (3.41), 8.715 (3.11); LC-MS (method 1): Rt = 0.92 min; MS (ESlpos): m/z = 455 [M+H]⁺

### Example 21

### 4,6-Dimethyl-N-(2-methylquinoline-8-sulfonyl)-1-benzofuran-2-carboxamide

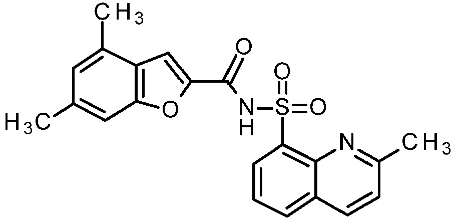

Synthesized analogously to Example 1 using 4,6-dimethyl-1-benzofuran-2-carboxylic acid (100 mg, 526 µmol) and 2-methylquinoline-8-sulfonamide (140 mg, 631 µmol) instead of 6-(4-fluorophenyl)-1-benzofuran-2-carboxylic acid and [1,1'-biphenyl]-2-sulfonamide to yield 80.0 mg (95 % purity, 37 % yield) of the title compound as light red solid. ¹H-NMR (400 MHz, DMSO-d₆) δ [ppm]: 2.322 (0.62), 2.326 (0.81), 2.331 (0.60), 2.378 (12.67), 2.470 (13.58), 2.518 (3.03), 2.522 (1.92), 2.664 (0.70), 2.669 (0.98), 2.673 (0.88), 2.687 (16.00), 2.966 (0.74), 3.347 (0.90), 6.989 (2.94), 7.225 (2.91), 7.505 (2.80), 7.527 (2.84), 7.731 (1.04), 7.750 (1.91), 7.770 (1.19), 8.205 (0.55), 8.285 (1.15), 8.305 (1.07), 8.385 (2.44), 8.406 (2.37), 8.456 (1.55), 8.459 (1.62), 8.474 (1.55), 8.477 (1.50); LC-MS (method 1): Rt = 1.21 min; MS (ESlpos): m/z = 395 [M+H]⁺

### Example 22

### 6-Cyclopropyl-4-fluoro-N-(2-methylquinoline-8-sulfonyl)-1-benzofuran-2-carboxamide

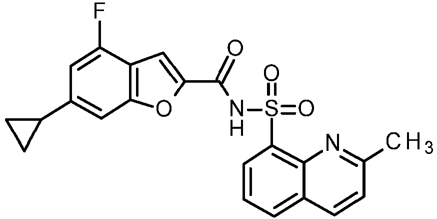

Synthesized analogously to **Example 21** using Intermediate 5 (200 mg, 908 µmol) instead of 4,6-dimethyl-1-benzofuran-2-carboxylic acid to yield 30.0 mg (7% yield) of the title compound as an offwhite solid. ¹H-NMR (400 MHz, DMSO-d₆) δ [ppm]: 0.759 (0.77), 0.770 (2.68), 0.775 (2.34), 0.782 (2.49), 0.788 (2.48), 0.798 (0.96), 0.995 (0.93), 1.005 (2.30), 1.010 (2.25), 1.016 (1.22), 1.022 (1.24), 1.026 (2.41), 1.031 (2.17), 1.043 (0.80), 2.044 (0.62), 2.052 (0.69), 2.065 (1.16), 2.077 (0.64), 2.085 (0.60), 2.321 (0.41), 2.325 (0.90), 2.330 (1.23), 2.334 (0.90), 2.339 (0.42), 2.521 (4.17), 2.525 (2.64), 2.542 (5.36), 2.662 (0.53), 2.667 (1.07), 2.672 (1.55), 2.676 (1.62), 2.684 (16.00), 6.928 (1.67), 6.955 (1.67), 7.246 (3.95), 7.524 (2.95), 7.545 (3.06), 7.743 (1.35), 7.763 (2.29), 7.783 (1.44), 8.194 (1.01), 8.299 (1.42), 8.319 (1.35), 8.402 (2.79), 8.424 (2.60), 8.468 (1.80), 8.472 (1.75), 8.487 (1.74), 8.490 (1.59); LC-MS (method 3): Rt = 1.21 min; MS (ESlpos): m/z = 425 [M+H]⁺

### Example 23

### N-([1,1'-biphenyl]-2-sulfonyl)-6-(prop-1-en-2-yl)-1-benzofuran-2-carboxamide

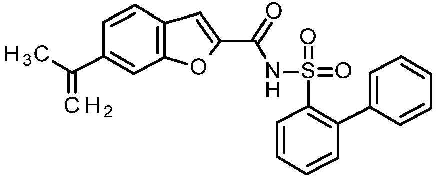

Synthesized analogously to **Example 4** using 4,4,5,5-tetramethyl-2-(prop-1-en-2-yl)-1,3,2-dioxaborolane (46.0 mg, 274 µmol) instead of pyridin-3-ylboronic acid to yield 18.0 mg (19% yield) of the title compound as a white solid. ¹H-NMR (400 MHz, DMSO-d₆) δ [ppm]: 1.234 (0.66), 1.874 (0.42), 1.886 (0.42), 2.175 (16.00), 2.327 (1.82), 2.331 (1.37), 2.518 (6.20), 2.523 (3.81), 2.539 (2.53), 2.669 (1.82), 2.673 (1.34), 5.207 (3.04), 5.210 (4.44), 5.214 (2.98), 5.574 (4.74), 7.253 (2.44), 7.258 (3.22), 7.274 (7.09), 7.277 (5.93), 7.287 (3.43), 7.290 (4.53), 7.308 (8.55), 7.312 (4.95), 7.327 (5.21), 7.343 (1.76), 7.347 (2.65), 7.351 (1.40), 7.357 (1.04), 7.365 (2.50), 7.372 (0.66), 7.382 (0.74), 7.546 (2.68), 7.550 (2.74), 7.567 (3.16), 7.571 (3.31), 7.646 (2.18), 7.662 (3.01), 7.681 (1.91), 7.714 (1.94), 7.730 (7.27), 7.743 (4.77), 7.764 (3.49), 8.171 (3.19), 8.174 (3.25), 8.191 (2.89), 8.194 (2.77); LC-MS (method 1): Rt = 1.39 min; MS (ESlpos): m/z = 417 [M+H]⁺

### Example 24

### 6-Cyclopropyl-N-[2-(cyclopropyloxy)benzene-1-sulfonyl]-4-fluoro-1-benzofuran-2-carboxamide

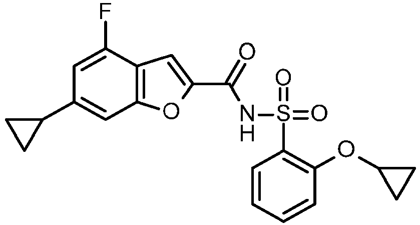

Synthesized analogously to Example 1 using Intermediate 5 (200 mg, 908 µmol) and 2-(cyclopropyloxy)benzene-1-sulfonamide (232 mg, 1.09 mmol) instead of 6-(4-fluorophenyl)-1-benzofuran-2-carboxylic acid and [1,1'-biphenyl]-2-sulfonamide to yield 10.0 mg (3 % yield) of the title compound. ¹H-NMR (400 MHz, DMSO-d₆) δ [ppm]: -0.149 (0.53), 0.146 (0.54), 0.509 (1.49), 0.517 (1.87), 0.523 (4.97), 0.529 (7.96), 0.534 (5.15), 0.547 (2.05), 0.707 (1.64), 0.721 (6.05), 0.737 (4.85), 0.741 (5.48), 0.756 (1.26), 0.790 (2.31), 0.801 (7.88), 0.806 (6.78), 0.813 (7.29), 0.819 (7.24), 0.829 (2.84), 1.016 (2.81), 1.026 (6.45), 1.032 (6.60), 1.036 (3.52), 1.043 (3.52), 1.047 (6.82), 1.053 (6.19), 1.064 (2.27), 1.241 (0.56), 1.715 (0.66), 1.724 (0.71), 1.732 (1.80), 1.739 (0.74), 1.748 (0.70), 2.061 (0.82), 2.074 (1.76), 2.077 (2.26), 2.082 (1.88), 2.094 (3.24), 2.107 (1.73), 2.115 (1.59), 2.128 (0.70), 2.334 (2.25), 2.339 (1.00), 2.521 (11.29), 2.525 (7.24), 2.542 (16.00), 2.676 (2.33), 2.681 (1.03), 3.067 (0.51), 3.073 (0.98), 3.083 (1.00), 3.089 (0.58), 4.056 (0.84), 4.062 (1.64), 4.070 (2.36), 4.077 (3.17), 4.084 (2.28), 4.092 (1.60), 6.949 (4.43), 6.977 (4.37), 7.169 (2.23), 7.187 (4.48), 7.206 (2.48), 7.303 (10.47), 7.469 (3.97), 7.489 (4.71), 7.683 (1.77), 7.701 (2.84), 7.720 (1.44), 7.896 (4.53), 7.900 (4.66), 7.916 (4.47), 7.920 (4.17), 8.082 (1.57), 12.687 (0.48); LC-MS (method 3): Rₜ = 1.33 min; MS (ESlpos): m/z = 416 [M+H]⁺

### Example 25

### 6-Cyclopropyl-N-(2-ethoxybenzene-1-sulfonyl)-4-fluoro-1-benzofuran-2-carboxamide

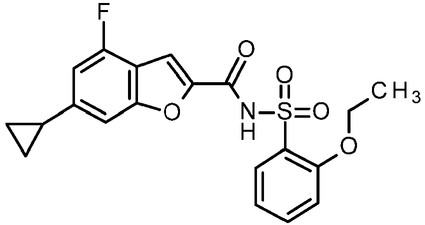

Synthesized analogously to Example 24 using 2-ethoxybenzene-1-sulfonamide (49.4 mg, 245 µmol) instead of 2-(cyclopropyloxy)benzene-1-sulfonamide to yield 38.0 mg (44% yield) of the title compound. ¹H-NMR (400 MHz, DMSO-d₆) δ [ppm]: -0.008 (0.49), 0.000 (11.78), 0.784 (1.29), 0.796 (4.28), 0.802 (3.65), 0.809 (3.79), 0.814 (3.76), 0.824 (1.40), 1.013 (1.53), 1.023 (3.59), 1.029 (3.50), 1.034 (1.87), 1.044 (3.66), 1.050 (3.25), 1.061 (1.17), 1.225 (7.19), 1.242 (16.00), 1.259 (7.20), 2.057 (0.51), 2.069 (1.00), 2.077 (1.98), 2.090 (1.78), 2.102 (0.95), 2.110 (0.85), 2.326 (0.62), 2.330 (0.84), 2.334 (0.61), 2.521 (2.36), 2.526 (1.71), 2.668 (0.62), 2.672 (0.84), 2.676 (0.58), 4.139 (1.85), 4.156 (5.84), 4.173 (5.66), 4.191 (1.72), 6.946 (2.63), 6.948 (2.56), 6.974 (2.60), 6.976 (2.47), 7.121 (1.50), 7.141 (2.98), 7.159 (1.66), 7.212 (2.62), 7.233 (2.88), 7.300 (5.86), 7.631 (1.19), 7.636 (1.23), 7.653 (1.88), 7.670 (0.98), 7.675 (0.94), 7.908 (3.01), 7.912 (2.94), 7.927 (2.85), 7.932 (2.59), 8.119 (1.55); LC-MS (method 3): Rₜ = 1.26 min; MS (ESlpos): m/z = 404 [M+H]⁺

### Example 26

### N-([1,1'-biphenyl]-2-sulfonyl)-6-(2-methoxyethyl)-1-benzofuran-2-carboxamide

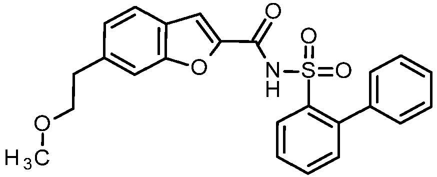

**Intermediate 4** (100 mg, 219 µmol), 2,6-dimethylpyridine (150 µL, 1.3 mmol; CAS-RN:[108-48-5]) and [lr(dF(CF3)ppy2)2(dtbbpy)]PF6 (4.92 mg, 4.38 µmol) were dissolved in the reaction vial in 1,2-dimethoxyethane (2.0 ml). In a separate vial, the Ni-catalyst was prepared by dissolving nickel(ll) chloride dimethoxyethane adduct (2.41 mg, 11.0 µmol) and 4,4'-di-tert-butyl-2,2'-bipyridine (2.94 mg, 11.0 µmol) in 1,2-dimethoxyethane (2.0 ml) followed by stirring for 5 min. The catalyst solution was syringed to the sealed reaction vial and degassed for 5 minutes with argon. TTMS and 1-bromo-2-methoxyethane (93 µl, 990 µmol) was added subsequently. The reaction vial was placed in a water bath and was irradiated by two 40 W Kessil LED Aquarium lamps for 16 h. Water was added and the mixture was extracted two times with ethyl acetate. The combined organic layers were filtered over a hydrophobic filter, the solvent was evaporated under reduced pressure and the residue was purified by preparative HPLC to yield 8.0 mg (92 % purity, 8 % yield) of the title compound. ¹H-NMR (400 MHz, DMSO-d₆) δ [ppm]: 2.540 (16.00), 2.927 (0.85), 2.944 (1.85), 2.961 (0.94), 3.239 (13.18), 3.564 (1.30), 3.581 (2.73), 3.597 (1.18), 7.223 (0.71), 7.226 (0.70), 7.244 (0.78), 7.247 (0.79), 7.277 (2.02), 7.282 (2.83), 7.292 (1.03), 7.300 (2.01), 7.314 (1.00), 7.317 (0.88), 7.508 (1.46), 7.668 (1.20), 7.689 (1.23), 8.160 (0.82), 8.164 (0.88), 8.180 (0.78), 8.183 (0.77); LC-MS (method 2): Rt = 0.74 min; MS (ESlneg): m/z = 434 [M-H]⁻

### Example 27

### N-([1,1'-biphenyl]-2-sulfonyl)-6-(butan-2-yl)-1-benzofuran-2-carboxamide

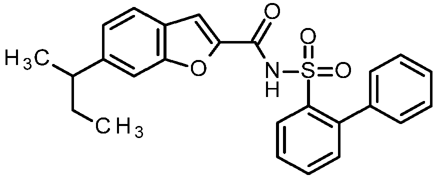

Synthesized analogously to **Example 26** using **Intermediate 4** (100 mg, 219 µmol) and 2-bromobutane (110 µl, 990 µmol) instead of 1-bromo-2-methoxyethane to yield 7.0 mg (, 7 % yield) of the title compound. ¹H-NMR (400 MHz, DMSO-d₆) δ [ppm]: 0.296 (0.66), 0.750 (6.35), 0.769 (16.00), 0.787 (6.80), 0.900 (0.57), 1.156 (0.69), 1.226 (13.99), 1.232 (3.30), 1.243 (13.90), 1.568 (0.90), 1.586 (3.39), 1.605 (5.12), 1.623 (3.42), 1.642 (0.84), 2.337 (0.54), 2.518 (7.34), 2.523 (4.91), 2.540 (0.96), 2.679 (0.57), 2.722 (0.96), 2.739 (1.89), 2.757 (1.83), 2.775 (0.87), 3.193 (0.69), 3.246 (0.51), 3.299 (1.29), 7.212 (2.43), 7.215 (2.40), 7.232 (2.55), 7.235 (2.55), 7.257 (1.92), 7.262 (2.58), 7.278 (6.20), 7.282 (5.51), 7.290 (3.78), 7.292 (4.49), 7.299 (3.00), 7.310 (7.13), 7.314 (3.30), 7.321 (2.94), 7.324 (2.67), 7.328 (3.15), 7.343 (1.56), 7.347 (2.13), 7.352 (1.26), 7.358 (0.99), 7.365 (2.10), 7.466 (4.97), 7.632 (1.14), 7.650 (1.89), 7.669 (1.53), 7.682 (3.72), 7.703 (4.16), 7.719 (1.68), 7.736 (0.72), 8.089 (0.42), 8.161 (3.00), 8.165 (3.24), 8.181 (2.85), 8.184 (2.79); LC-MS (method 1): Rₜ = 1.50 min; MS (ESlpos): m/z = 434 [M+H]⁺

### Example 28

### 6-Cyclopropyl-N-(2-ethoxy-3-fluoro-phenyl)sulfonyl-4-fluoro-benzofuran-2-carboxamide

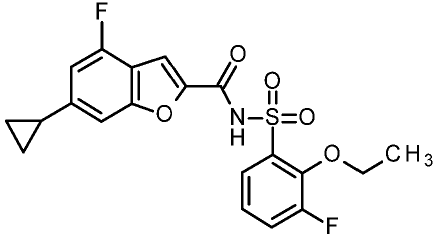

To a room temperature stirred solution of 6-cyclopropyl-4-fluoro-benzofuran-2-carboxylic acid (74.9 mg, 0.34 mmol, 1.00 eq.) in anhydrous tetrahydrofuran (1.70 mL, 0.20 M) was added carbonyldiimidazole (66.2 mg, 0.41 mmol, 1.20 eq.). After stirring for 1 hour, 2-ethoxy-3-fluoro-benzenesulfonamide (82.0 mg, 0.37 mmol, 1.10 eq.) and 1,8-diazabicyclo[5.4.0]undec-7-ene (70.0 µL, 0.48 mmol, 1.40 eq.) were sequentially added. The resulting mixture was stirred at room temperature for 16 h and then concentrated down under reduced pressure. The residue was purified by reverse phase preparative column chromatography (Waters XBridge PREP C₁₈, 5µM OBD, 30 x 150 mm, 58-66 % acetonitrile/water gradient buffered with 0.1% formic acid) to give the title compound as a white solid (97.0 mg). ¹H NMR (400 MHz, DMSO-d₆) δ 12.91 (s, 1H), 8.07 (s, 1H), 7.82 - 7.75 (m, 1H), 7.70 - 7.60 (m, 1H), 7.37 - 7.28 (m, 1H), 7.32 - 7.27 (m, 1H), 6.95 (d, 1H), 4.27 - 4.17 (m, 2H), 2.15 - 2.03 (m, 1H), 1.33 (t, 3H), 1.08 - 0.97 (m, 2H), 0.85 - 0.73 (m, 2H); LC-MS (method 5): Rt = 3.08 min; MS (ESlpos): m/z = 422 [M+H]⁺

### Example 29

### 6-Cyclopropyl-N-(2-ethoxy-5-sec-butyl-phenyl)sulfonyl-4-fluoro-benzofuran-2-carboxamide

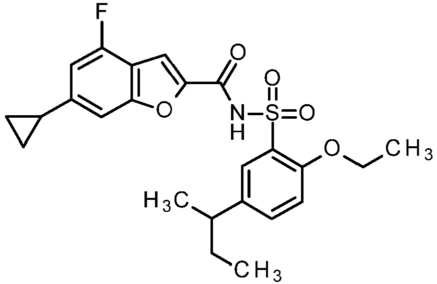

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-ethoxy-4-fluoro-benzenesulfonamide (96.5 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for 16 h and then concentrated down under reduced pressure. The residue was purified by reverse phase preparative column chromatography (Waters XBridge PREP C₁₈, 5 µM OBD, 30 x 150 mm, 68-76 % acetonitrile/water gradient buffered with 0.1% formic acid) to give the title compound as a white solid (128 mg). ¹H NMR (400 MHz, DMSO-d₆) δ 12.62 (s, 1H), 8.11 (s, 1H), 7.73 - 7.67 (m, 1H), 7.52 - 7.44 (m, 1H), 7.28 (s, 1H), 7.14 (d, 1H), 6.95 (d, 1H), 4.13 (q, 2H), 2.74 - 2.61 (m, 1H), 2.15 - 2.03 (m, 1H), 1.62 - 1.48 (m, 2H), 1.28 - 1.17 (m, 6H), 1.08 - 0.98 (m, 2H), 0.84 - 0.74 (m, 5H); LC-MS (method 5): Rt = 3.46 min; MS (ESlpos): m/z = 460 [M+H]⁺

### Example 30

### 6-Cyclopropyl-N-(2-ethoxy-4-fluoro-phenyl)sulfonyl-4-fluoro-benzofuran-2-carboxamide

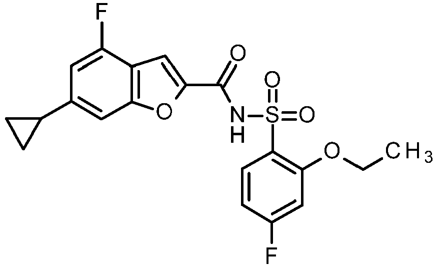

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-ethoxy-4-fluoro-benzenesulfonamide (96.5 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for 16 h and then concentrated down under reduced pressure. The residue was purified by reverse phase preparative column chromatography (Waters XBridge PREP C₁₈, 5µM OBD, 30 x 150 mm, 57-65 % acetonitrile/water gradient buffered with 0.1% formic acid) to give the title compound as a white solid (137 mg). ¹H NMR (400 MHz, DMSO-d₆) δ 12.74 (s, 1H), 8.10 (s, 1H), 8.02 - 7.94 (m, 1H), 7.29 (s, 1H), 7.21 - 7.13 (m, 1H), 7.04 - 6.91 (m, 2H), 4.18 (q, 2H), 2.15 - 2.03 (m, 1H), 1.25 (t, 3H), 1.07 - 0.98 (m, 2H), 0.84 - 0.76 (m, 2H); LC-MS (method 5): Rt = 3.02 min; MS (ESlpos): m/z = 422 [M+H]⁺

### Example 31

### 6-Cyclopropyl-N-[2-ethoxy-5-(trifluoromethyl)phenyl]sulfonyl-4-fluoro-benzofuran-2-carboxamide

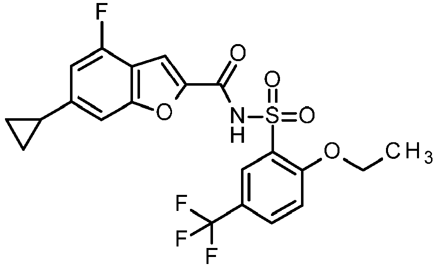

To a room temperature stirred solution of 6-cyclopropyl-4-fluoro-benzofuran-2-carboxylic acid (11.0 mg, 0.05 mmol, 1.00 eq.) in anhydrous tetrahydrofuran (0.25 mL, 0.20 M) was added carbonyldiimidazole (9.73 mg, 0.06 mmol, 1.20 eq.). After stirring for 1 hour, 2-ethoxy-5-(trifluoromethyl)benzenesulfonamide (14.8 mg, 0.05 mmol, 1.10 eq.) and 1,8-diazabicyclo[5.4.0]undec-7-ene (10.0 µL, 0.07 mmol, 1.40 eq.) were sequentially added. The resulting mixture was stirred at room temperature for 16 h and then concentrated down under reduced pressure. The residue was purified by reverse phase preparative column chromatography (Waters XBridge PREP C₁₈, 5 µM OBD, 19 x 250 mm, 61-69 % acetonitrile/water gradient buffered with 0.1% formic acid) to give the title compound as a white solid (16.0 mg). ¹H NMR (400 MHz, DMSO-d₆) δ 8.17 - 8.11 (m, 1H), 8.10 - 8.00 (m, 2H), 7.45 (d, 1H), 7.29 (s, 1H), 6.95 (d, 1H), 4.29 (q, 2H), 2.15 - 2.04 (m, 1H), 1.27 (t, 3H), 1.07 - 0.99 (m, 2H), 0.85 - 0.76 (m, 2H); LC-MS (method 5): Rt = 3.20 min; MS (ESlpos): m/z = 472 [M+H]⁺

### Example 32

### 6-Cyclopropyl-N-(2-ethoxy-5-fluoro-phenyl)sulfonyl-4-fluoro-benzofuran-2-carboxamide

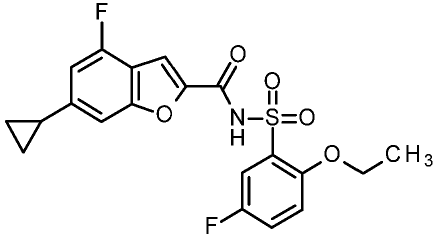

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 h, 2-ethoxy-5-fluoro-benzenesulfonamide (96.5 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for 1 h and then concentrated down under reduced pressure. The residue was purified by reverse phase preparative column chromatography (Waters XBridge PREP C18, 5µM OBD, 30 x 150 mm, 56-64 % acetonitrile/water gradient buffered with 0.1% formic acid) to give the title compound as a yellow solid (110 mg). ¹H NMR (400 MHz, DMSO-d₆) δ 12.86 (s, 1H), 8.12 - 8.07 (m, 1H), 7.68 (dd, 1H), 7.60 - 7.50 (m, 1H), 7.32 - 7.24 (m, 2H), 6.95 (d, 1H), 4.15 (q, 2H), 2.15 - 2.03 (m, 1H), 1.23 (t, 3H), 1.08 - 1.00 (m, 2H), 0.85 - 0.76 (m, 2H); LC-MS (method 5): Rₜ = 3.00 min; MS (ESlpos): m/z = 422 [M+H]⁺

### Example 33

### 6-Cyclopropyl-N-(2-ethoxy-5-phenoxy-phenyl)sulfonyl-4-fluoro-benzofuran-2-carboxamide

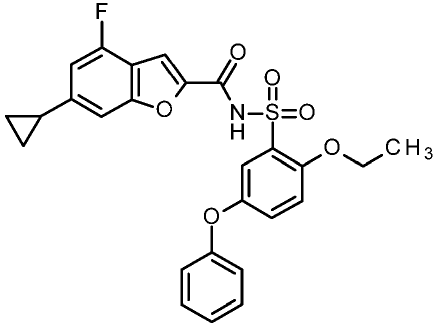

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-ethoxy-5-phenoxy-benzenesulfonamide (129 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for one hour and then concentrated under reduced pressure. The residue was purified by reverse phase preparative column chromatography (Waters XBridge PREP C₁₈, 5 µM OBD, 30 x 150 mm, 66-74% acetonitrile/water gradient buffered with 0.1% formic acid) to give the title compound as a white solid (109 mg). ¹H NMR (400 MHz, DMSO-d₆) δ 12.80 (s, 1H), 8.12 (s, 1H), 7.51 (d, 1H), 7.48 - 7.35 (m, 3H), 7.33 - 7.24 (m, 2H), 7.21 - 7.13 (m, 1H), 7.08 - 7.01 (m, 2H), 6.96 (d, 1H), 4.17 (q, 2H), 2.16 - 2.05 (m, 1H), 1.26 (t, 3H), 1.10 - 0.98 (m, 2H), 0.85 - 0.77 (m, 2H); LC-MS (method 5): Rt = 3.39 min; MS (ESlpos): m/z = 496 [M+H]⁺

### Example 34

### 6-Cyclopropyl-N-[2-ethoxy-5-(trifluoromethoxy)phenyl]sulfonyl-4-fluoro-benzofuran-2-carboxamide

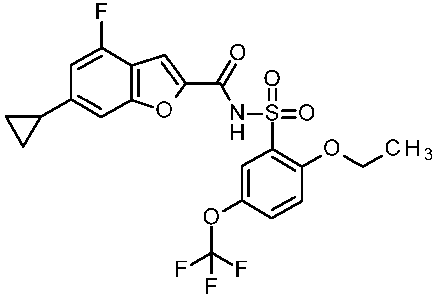

To a room temperature stirred solution of 6-cyclopropyl-4-fluoro-benzofuran-2-carboxylic acid (8.81 mg, 0.04 mmol, 1.00 eq.) in anhydrous tetrahydrofuran (0.200 mL, 0.20 M) was added carbonyldiimidazole (7.78 mg, 0.05 mmol, 1.20 eq.). After stirring for 1 hour, 2-ethoxy-5-(trifluoromethoxy)benzenesulfonamide (12.6 mg, 0.04 mmol, 1.10 eq.) and 1,8-diazabicyclo[5.4.0]undec-7-ene (10.0 µL, 0.06 mmol, 1.40 eq.) were sequentially added. The resulting mixture was stirred at room temperature overnight and then concentrated under reduced pressure. The residue was purified by reverse phase preparative column chromatography (Waters XBridge PREP C₁₈, 5 µM OBD, 30 x 150 mm, 63-71% acetonitrile/water gradient buffered with 0.1% formic acid) to give the title compound as a white solid (12 mg). ¹H NMR (400 MHz, DMSO-d₆) δ 8.07 - 8.02 (m, 1H), 7.84 - 7.78 (m, 1H), 7.74 - 7.66 (m, 1H), 7.34 (d, 1H), 7.28 (s, 1H), 6.95 (d, 1H), 4.20 (q, 2H), 2.15 - 2.03 (m, 1H), 1.24 (t, 3H), 1.08 - 0.98 (m, 2H), 0.84 - 0.75 (m, 2H); LC-MS (method 5): Rt = 3.27 min; MS (ESlpos): m/z = 488 [M+H]⁺

### Example 35

### N-(2-Benzyloxy-5-isopropyl-phenyl)sulfonyl-6-cyclopropyl-4-fluoro-benzofuran-2-carboxamide

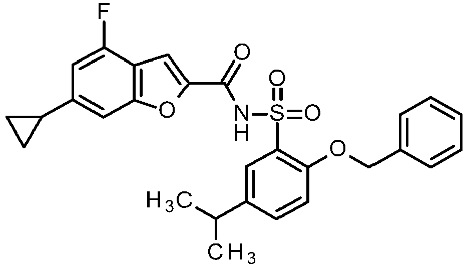

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-benzyloxy-5-isopropyl-benzenesulfonamide (134 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for 2 h and then concentrated under reduced pressure. The residue was purified by reverse phase preparative column chromatography (Waters XBridge PREP C₁₈, 5 µM OBD, 30 x 150 mm, 70-78% acetonitrile/water gradient buffered with 0.1% formic acid) to give the title compound as a white solid (134 mg). ¹H NMR (400 MHz, DMSO-d₆) δ 12.77 (s, 1H), 8.02 (s, 1H), 7.78 (d, 1H), 7.49 (dd, 1H), 7.39 - 7.32 (m, 2H), 7.29 (s, 1H), 7.23 - 7.07 (m, 4H), 6.96 (d, 1H), 5.28 (s, 2H), 2.94 (hept, 1H), 2.16 - 2.05 (m, 1H), 1.20 (d, 6H), 1.09 - 1.00 (m, 2H), 0.86 - 0.77 (m, 2H); LC-MS (method 5): Rt = 3.51 min; MS (ESlpos): m/z = 508 [M+H]⁺

### Example 36

### 6-Cyclopropyl-N-[2-(cyclopropylmethoxy)-5-isopropyl-phenyl]sulfonyl-4-fluoro-benzofuran-2-carboxamide

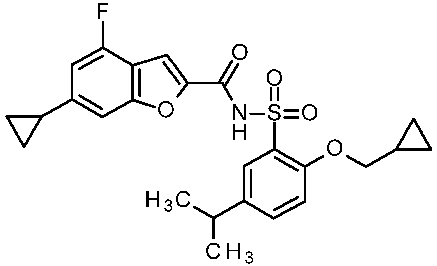

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-(cyclopropylmethoxy)-5-isopropyl-benzenesulfonamide (119 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature over four nights and then concentrated under reduced pressure. The residue was purified by reverse phase preparative column chromatography (Waters XBridge PREP C₁₈, 5 µM OBD, 30 x 150 mm, 68-76% acetonitrile/water gradient buffered with 0.1% formic acid) to give the title compound as a white solid (117 mg). ¹H NMR (400 MHz, DMSO-d₆) δ 12.64 - 12.59 (m, 1H), 8.11 (s, 1H), 7.75 (d, 1H), 7.52 (dd, 1H), 7.31 - 7.26 (m, 1H), 7.15 (d, 1H), 6.98 - 6.90 (m, 1H), 3.94 (d, 2H), 2.96 (hept, 1H), 2.15 - 2.03 (m, 1H), 1.25 - 1.11 (m, 7H), 1.08 - 0.97 (m, 2H), 0.85 - 0.73 (m, 2H), 0.37 - 0.26 (m, 2H), 0.22 - 0.11 (m, 2H); LC-MS (method 5): Rt = 3.45 min; MS (ESlpos): m/z = 472 [M+H]⁺

### Example 37

### 6-Cyclopropyl-N-(2,4-dichlorophenyl)sulfonyl-4-fluoro-benzofuran-2-carboxamide

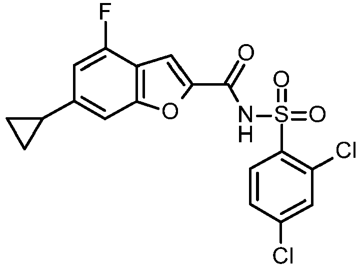

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2,4-dichlorobenzenesulfonamide (99.5 mg, 0.44 mmol, 1.10 eq.) and 1,8-diazabicyclo[5.4.0]undec-7-ene (84.8 µL, 0.56 mmol, 1.40 eq.) were sequentially added. The resulting mixture was stirred at room temperature for a further 18 h and then concentrated under reduced pressure. The residue was purified by reverse phase preparative column chromatography (Waters XBridge PREP C18, 5µM OBD, 30 x 150 mm, 64-72% acetonitrile/water gradient buffered with 0.1% formic acid) to give the title compound as a white solid (129 mg). 1H NMR (400 MHz, DMSO-d₆) δ 8.17 (d, 1H), 7.99 (s, 1H), 7.88 (d, 1H), 7.71 (dd, 1H), 7.28 (d, 1H), 6.94 (dd, 1H), 2.15 - 2.03 (m, 1H), 1.08 - 0.97 (m, 2H), 0.85 - 0.73 (m, 2H); LC-MS (method 5): Rt = 3.38 min; MS (ESlpos): m/z = 428 [M+H]⁺

### Example 38

### 6-Cyclopropyl-4-fluoro-N-(2-phenylphenyl)sulfonyl-benzofuran-2-carboxamide

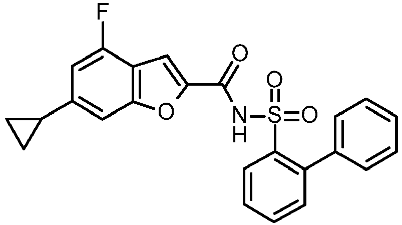

To a 0°C stirred solution of 6-cyclopropyl-4-fluoro-benzofuran-2-carboxamide (79.1 mg, 0.36 mmol, 1.00 eq.) in anhydrous tetrahydrofuran (3.61 mL, 0.10 mL) was carfefully added sodium hydride (60% dispersion in mineral oil, 31.8 mg, 0.79 mmol, 2.20 eq.). The resulting mixture was stirred at 0 °C for 30 minutes, after which 2-phenylbenzenesulfonyl chloride (100 mg, 0.40 mmol, 1.10 eq.) was added. The mixture was warmed to room temperature, stirred for 4 h adn then diluted with water. The mixture was acidifed to pH 2.0 with hydrochloric acid (1.00 M in water) and extracted with ethyl acetate (3 x 10 mL). The combined organic extracts were washed with brine (10 mL), dried (magnesium sulfate), filtered and concentrated under reduced pressure. The residue was purified by reverse phase preparative column chromatography (Waters XBridge PREP C₁₈, 5 µM OBD, 30 x 150 mm, 63-71% acetonitrile/water gradient buffered with 0.1% formic acid) to give the title compound as a white solid (111 mg). ¹H NMR (400 MHz, DMSO-d₆) δ 12.23 (s, 1H), 8.18 (dd, 1H), 7.79 - 7.62 (m, 3H), 7.41 - 7.22 (m, 7H), 6.95 (dd, 1H), 2.09 (tt, 1H), 1.11 - 0.98 (m, 2H), 0.85 - 0.74 (m, 2H); LC-MS (method 5): Rt = 3.26 min; MS (ESlpos): m/z = 436 [M+H]⁺

### Example 39

### N-(Benzenesulfonyl)-6-cyclopropyl-4-fluoro-benzofuran-2-carboxamide

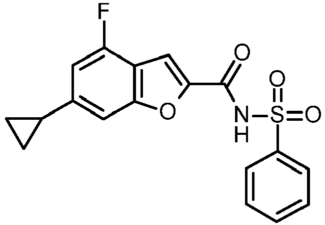

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, benzenesulfonamide (69.2 mg, 0.44 mmol, 1.10 eq.) and 1,8-diazabicyclo[5.4.0]undec-7-ene (84.8 µL, 0.56 mmol, 1.40 eq.) were sequentially added. The resulting mixture was stirred at room temperature for a further 18 h and then concentrated under reduced pressure. The residue was purified by reverse phase preparative column chromatography (Waters XBridge PREP C₁₈, 5µM OBD, 30 x 150 mm, 52-60% acetonitrile/water gradient buffered with 0.1% formic acid) to give the title compound as a white solid (89.3 mg). ¹H NMR (400 MHz, DMSO-d₆) δ 12.79 (s, 1H), 8.06 - 7.98 (m, 2H), 7.95 (d1H), 7.78 - 7.69 (m, 1H), 7.70 - 7.60 (m, 2H), 7.29 (s, 1H), 6.94 (dd, 1H), 2.09 (tt, 1H), 1.10 - 0.97 (m, 2H), 0.84 - 0.73 (m, 2H); LC-MS (method 5): Rₜ = 2.84 min; MS (ESlpos): m/z = 360 [M+H]⁺

### Example 40

### 6-Cyclopropyl-4-fluoro-N-(2-propoxyphenyl)sulfonyl-benzofuran-2-carboxamide

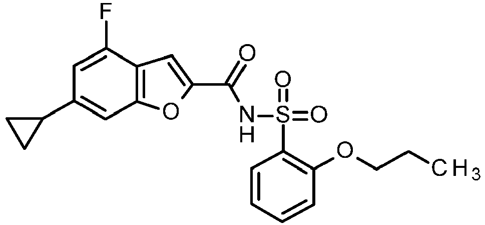

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-propoxybenzenesulfonamide (94.7 mg, 0.44 mmol, 1.10 eq.) and 1,8-diazabicyclo[5.4.0]undec-7-ene (83.8 µL, 0.56 mmol, 1.40 eq.) were sequentially added. The resulting mixture was stirred at room temperature for a further 20 h and then concentrated under reduced pressure. The residue was purified by reverse phase preparative column chromatography (Waters XBridge PREP C₁₈, 5 µM OBD, 30 x 150 mm, 59-67% acetonitrile/water gradient buffered with 0.1% formic acid) to give the title compound as a white solid (98.2 mg). ¹H NMR (400 MHz, DMSO-d₆) δ 12.67 (s, 1H), 8.07 (s, 1H), 7.92 (dd, 1H), 7.72 - 7.59 (m, 1H), 7.28 (s, 1H), 7.21 (d, 1H), 7.13 (t, 1H), 6.94 (d, 1H), 4.06 (t, 2H), 2.09 (tt, 1H), 1.70 (h, 2H), 1.07 - 0.99 (m, 2H), 0.87 (t, 3H), 0.83 - 0.76 (m, 2H); LC-MS (method 5): Rt = 3.12 min; MS (ESlpos): m/z = 418 [M+H]⁺

### Example 41

### 6-Cyclopropyl-4-fluoro-N-[5-isopropyl-2-(2,2,2-trifluoroethoxy)phenyl]sulfonyl-benzofuran-2-carboxamide

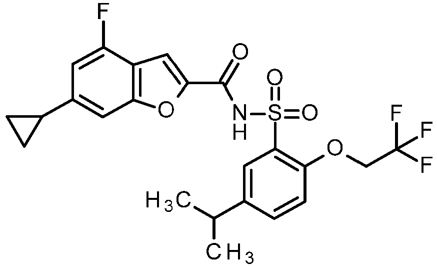

To a room temperature stirred solution of 6-cyclopropyl-4-fluoro-benzofuran-2-carboxylic acid (44.0 mg, 0.20 mmol, 1.00 eq.) in anhydrous tetrahydrofuran (1.00 mL, 0.20 M) was added carbonyldiimidazole (38.9 mg, 0.24 mmol, 1.20 eq.). After stirring for 1 hour, 5-isopropyl-2-(2,2,2-trifluoroethoxy)benzenesulfonamide (65.4 mg, 0.22 mmol, 1.10 eq.) and 1,8-diazabicyclo[5.4.0]undec-7-ene (40.0 µL, 0.28 mmol, 1.40 eq.) were sequentially added. The resulting mixture was stirred at room temperature for 18 h and then concentrated under reduced pressure. The residue was purified by reverse phase preparative column chromatography (Waters XBridge PREP C₁₈, 5 µM OBD, 30 x 150 mm, 65-73% acetonitrile/water gradient buffered with 0.1% formic acid) to give the title compound as a white solid (44.8 mg). ¹H NMR (400 MHz, DMSO-d₆) δ 12.72 (s, 1H), 8.03 (s, 1H), 7.80 (d, 1H), 7.64 - 7.56 (m, 1H), 7.32 - 7.25 (m, 2H), 6.94 (d, 1H), 4.88 (q, 2H), 3.07 - 2.92 (m, 1H), 2.15 - 2.03 (m, 1H), 1.23 (d, 6H), 1.08 - 0.97 (m, 2H), 0.87 - 0.73 (m, 2H); LC-MS (method 5): Rₜ = min; MS (ESlpos): m/z = 500 [M+H]⁺

### Example 42

### N-(Benzenesulfonyl)-6-cyclopropyl-4-(dimethylamino)benzofuran-2-carboxamide

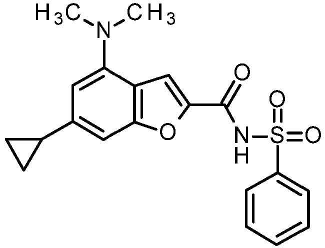

To a room temperature stirred solution of 6-cyclopropyl-4-(dimethylamino)benzofuran-2-carboxylic acid (98.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, benzenesulfonamide (69.2 mg, 0.44 mmol, 1.10 eq.) and 1,8-diazabicyclo[5.4.0]undec-7-ene (84.8 µL, 0.56 mmol, 1.40 eq.) were sequentially added. The resulting mixture was stirred at room temperature for a further 2 h and then concentrated under reduced pressure. The residue was purified by reverse phase preparative column chromatography (Waters XBridge PREP C₁₈, 5 µM OBD, 30 x 150 mm, 52-60% acetonitrile/water gradient buffered with 0.1% formic acid) to give the title compound as a yellow solid (110 mg). ¹H NMR (400 MHz, DMSO-d₆) δ 12.53 (s, 1H), 8.21 (s, 1H), 8.04 - 7.96 (m, 2H), 7.73 (t, 1H), 7.65 (t, 2H), 6.59 (s, 1H), 6.23 (s, 1H), 3.09 (s, 6H), 1.96 (tt, 1H), 1.00 - 0.90 (m, 2H), 0.77 - 0.68 (m, 2H); LC-MS (method 5): Rₜ = 2.83 min; MS (ESlpos): m/z = 385 [M+H]⁺

### Example 43

### 6-Cyclopropyl-4-(dimethylamino)-N-[(2-methyl-8-quinolyl)sulfonyl]benzofuran-2-carboxamide

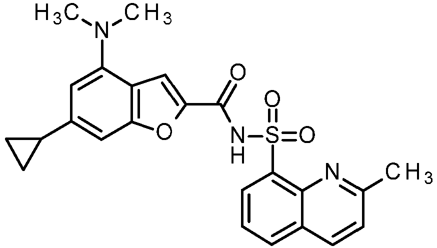

To a room temperature stirred solution of 6-cyclopropyl-4-(dimethylamino)benzofuran-2-carboxylic acid (98.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 h, 2-methylquinoline-8-sulfonamide (98.8 mg, 0.44 mmol, 1.10 eq.) and 1,8-diazabicyclo[5.4.0]undec-7-ene (84.8 µL, 0.56 mmol, 1.40 eq.) were sequentially added. The resulting mixture was stirred at room temperature for a further 2 h and then concentrated under reduced pressure. The residue was purified by reverse phase preparative column chromatography (Waters XBridge PREP C₁₈, 5 µM OBD, 30 x 150 mm, 52-60% acetonitrile/water gradient buffered with 0.1% formic acid) to give the title compound as a yellow solid (149 mg). ¹H NMR (400 MHz, DMSO-d₆) δ 12.36 (s, 1H), 8.54 (s, 1H), 8.46 (dd, 1H), 8.38 (d, 1H), 8.28 (dd1H), 7.74 (t, 1H), 7.50 (d, 1H), 6.52 (d, 1H), 6.22 (d, 1H), 3.12 (s, 6H), 2.68 (s, 3H), 1.93 (tt, 1H), 0.97 - 0.88 (m, 2H), 0.74 - 0.65 (m, 2H); LC-MS (method 5): Rt = 2.85 min; MS (ESlpos): m/z = 450 [M+H]⁺

### Example 44

### 6-Cyclopropyl-4-(dimethylamino)-N-(2-phenylphenyl)sulfonyl-benzofuran-2-carboxamide

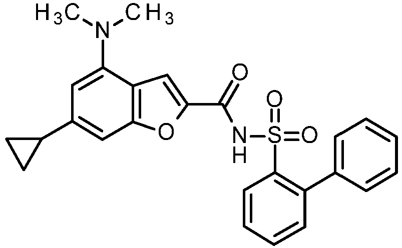

To a room temperature stirred solution of 6-cyclopropyl-4-(dimethylamino)benzofuran-2-carboxylic acid (98.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-phenylbenzenesulfonamide (103 mg, 0.44 mmol, 1.10 eq.) and 1,8-diazabicyclo[5.4.0]undec-7-ene (84.8 µL, 0.56 mmol, 1.40 eq.) were sequentially added. The resulting mixture was stirred at room temperature for a further 2 h and then concentrated under reduced pressure. The residue was purified by reverse phase preparative column chromatography (Waters XBridge PREP C₁₈, 5 µM OBD, 30 x 150 mm, 64-72% acetonitrile/water gradient buffered with 0.1% formic acid) to give the title compound as a yellow solid (150 mg). 1H NMR (400 MHz, DMSO-d6) δ 11.90 (s, 1H), 8.17 (dd, 1H), 7.96 (s, 1H), 7.73 (td, 1H), 7.66 (td, 1H), 7.41 - 7.26 (m, 4H), 7.28 - 7.21 (m, 2H), 6.59 - 6.57 (m, 1H), 6.23 (d, 1H), 3.08 (s, 6H), 1.96 (tt, 1H), 1.00 - 0.90 (m, 2H), 0.78 - 0.69 (m, 2H); LC-MS (method 5): Rt = 3.28 min; MS (ESlpos): m/z = 461 [M+H]⁺

### Example 45

### 6-Cyclopropyl-4-(dimethylamino)-N-(2-ethoxyphenyl)sulfonyl-benzofuran-2-carboxamide

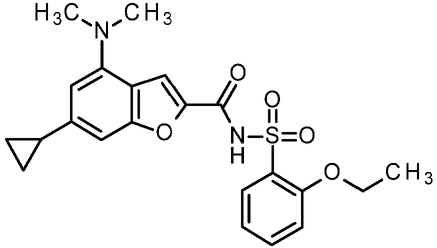

To a room temperature stirred solution of 6-cyclopropyl-4-(dimethylamino)benzofuran-2-carboxylic acid (98.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-ethoxybenzenesulfonamide (88.6 mg, 0.44 mmol, 1.10 eq.) and 1,8-diazabicyclo[5.4.0]undec-7-ene (84.8 µL, 0.56 mmol, 1.40 eq.) were sequentially added. The resulting mixture was stirred at room temperature for a further 2 h and then concentrated under reduced pressure. The residue was purified by reverse phase preparative column chromatography (Waters XBridge PREP C₁₈, 5 µM OBD, 30 x 150 mm, 56-64% acetonitrile/water gradient buffered with 0.1% formic acid) to give the title compound as a yellow solid (144 mg). ¹H NMR (400 MHz, DMSO-d₆) δ 12.27 (s, 1H), 8.43 (s, 1H), 7.91 (dd, 1H), 7.64 (ddd, 1H), 7.21 (d, 1H), 7.16 - 7.08 (m, 1H), 6.59 (d1H), 6.24 (d, 1H), 4.15 (q, 2H), 3.11 (s, 6H), 1.96 (tt, 1H), 1.25 (t, 3H), 1.00 - 0.90 (m, 2H), 0.77 - 0.68 (m, 2H); LC-MS (method 5): Rt = 3.00 min; MS (ESlpos): m/z = 429 [M+H]⁺

### Example 46

### N-(benzenesulfonyl)-6-cyclopropyl-4-(difluoromethoxy)benzofuran-2-carboxamide

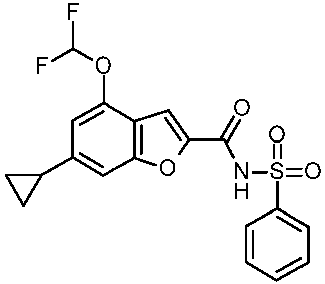

To a room temperature stirred solution of 6-cyclopropyl-4-(difluoromethoxy)benzofuran-2-carboxylic acid (53.6 mg, 0.20 mmol, 1.00 eq.) in anhydrous tetrahydrofuran (1.00 mL, 0.20 M) was added carbonyldiimidazole (38.9 mg, 0.24 mmol, 1.20 eq.). After stirring for 1 h, benzenesulfonamide (34.6 mg, 0.22 mmol, 1.10 eq.) and 1,8-diazabicyclo[5.4.0]undec-7-ene (41.9 µL, 0.28 mmol, 1.40 eq.) were sequentially added. The resulting mixture was stirred at room temperature for a further 18 h and then concentrated under reduced pressure. The residue was purified by reverse phase preparative column chromatography (Waters XBridge PREP C₁₈, 5µM OBD, 30 x 150 mm, 53-61% acetonitrile/water gradient buffered with 0.1% formic acid) to give the title compound as a white solid (64.3 mg). ¹H NMR (400 MHz, DMSO-d₆) δ 12.84 (s, 1H), 8.06 - 7.95 (m, 3H), 7.79 - 7.62 (m, 3H), 7.41 (t, 1H), 7.33 - 7.28 (m, 1H), 6.91 (s, 1H), 2.16 - 2.04 (m, 1H), 1.11 - 0.98 (m, 2H), 0.86 - 0.77 (m, 2H); LC-MS (method 5): Rt = 2.89 min; MS (ESlpos): m/z = 408 [M+H]⁺

### Example 47

### 6-Cyclopropyl-4-(difluoromethoxy)-N-[(2-methyl-8-quinolyl)sulfonyl]benzofuran-2-carboxamide

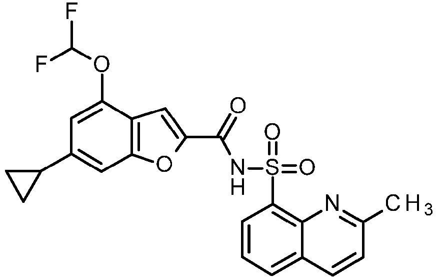

To a room temperature stirred solution of 6-cyclopropyl-4-(difluoromethoxy)benzofuran-2-carboxylic acid (53.6 mg, 0.20 mmol, 1.00 eq.) in anhydrous tetrahydrofuran (1.00 mL, 0.20 M) was added carbonyldiimidazole (38.9 mg, 0.24 mmol, 1.20 eq.). After stirring for 1 hour, 2-methylquinoline-8-sulfonamide (48.9 mg, 0.22 mmol, 1.10 eq.) and 1,8-diazabicyclo[5.4.0]undec-7-ene (41.9 µL, 0.28 mmol, 1.40 eq.) were sequentially added. The resulting mixture was stirred at room temperature for a further 18 h and then concentrated under reduced pressure. The residue was purified by reverse phase preparative column chromatography (Waters XBridge PREP C₁₈, 5µM OBD, 30 x 150 mm, 49-57% acetonitrile/water gradient buffered with 0.1% formic acid) to give the title compound as a white solid (73.6 mg). ¹H NMR (400 MHz, DMSO-d₆) δ 8.47 (dd, 1H), 8.40 (d, 1H), 8.30 (dd, 1H), 8.24 (s, 1H), 7.76 (t, 1H), 7.52 (d, 1H), 7.41 (t, 1H), 7.24 (d, 1H), 6.89 (d, 1H), 2.68 (s, 3H), 2.06 (tt, 1H), 1.07 - 0.97 (m, 2H), 0.82 - 0.73 (m, 2H); LC-MS (method 5): Rt = 2.74 min; MS (ESlpos): m/z = 4.73 [M+H]⁺

### Example 48

### 6-Cyclopropyl-4-(difluoromethoxy)-N-(2-phenylphenyl)sulfonyl-benzofuran-2-carboxamide

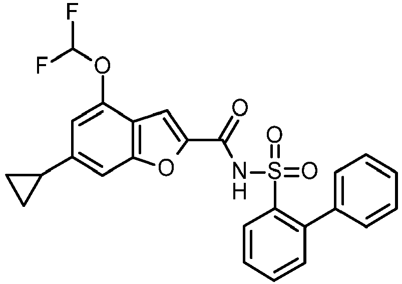

To a room temperature stirred solution of 6-cyclopropyl-4-(difluoromethoxy)benzofuran-2-carboxylic acid (53.6 mg, 0.20 mmol, 1.00 eq.) in anhydrous tetrahydrofuran (1.00 mL, 0.20 M) was added carbonyldiimidazole (38.9 mg, 0.24 mmol, 1.20 eq.). After stirring for 1 hour, 2-phenylbenzenesulfonamide (51.3 mg, 0.22 mmol, 1.10 eq.) and 1,8-diazabicyclo[5.4.0]undec-7-ene (41.9 µL, 0.28 mmol, 1.40 eq.) were sequentially added. The resulting mixture was stirred at room temperature for a further 18 h and then concentrated under reduced pressure. The residue was purified by reverse phase preparative column chromatography (Waters XBridge PREP C₁₈, 5 µM OBD, 30 x 150 mm, 63-71% acetonitrile/water gradient buffered with 0.1% formic acid) to give the title compound as a white solid (82.9 mg). ¹H NMR (400 MHz, DMSO-d₆) δ 12.30 (s, 1H), 8.18 (dd, 1H), 7.78 - 7.63 (m, 3H), 7.40 (t, 1H), 7.39 - 7.22 (m, 7H), 6.91 (s, 1H), 2.15 - 2.04 (m, 1H), 1.09 - 0.98 (m, 2H), 0.86 - 0.74 (m, 2H); LC-MS (method 5): Rt = 3.27 min; MS (ESlpos): m/z = 484 [M+H]⁺

### Example 49

### 6-Cyclopropyl-4-(difluoromethoxy)-N-(2-ethoxyphenyl)sulfonyl-benzofuran-2-carboxamide

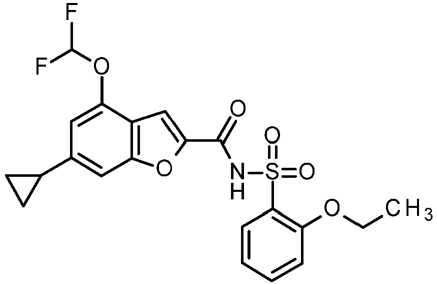

To a room temperature stirred solution of 6-cyclopropyl-4-(difluoromethoxy)benzofuran-2-carboxylic acid (53.6 mg, 0.20 mmol, 1.00 eq.) in anhydrous tetrahydrofuran (1.00 mL, 0.20 M) was added carbonyldiimidazole (38.9 mg, 0.24 mmol, 1.20 eq.). After stirring for 1 hour, 2-ethoxybenzenesulfonamide (44.3 mg, 0.22 mmol, 1.10 eq.) and 1,8-diazabicyclo[5.4.0]undec-7-ene (41.9 µL, 0.28 mmol, 1.40 eq.) were sequentially added. The resulting mixture was stirred at room temperature for a further 2 h and then concentrated under reduced pressure. The residue was purified by reverse phase preparative column chromatography (Waters XBridge PREP C18, 5µM OBD, 30 x 150 mm, 56-64% acetonitrile/water gradient buffered with 0.1% formic acid) to give the title compound as a white solid (74.5 mg). ¹H NMR (400 MHz, DMSO-d₆) δ 12.66 (s, 1H), 8.16 (s, 1H), 7.92 (dd, 1H), 7.65 (ddd, 1H), 7.41 (t, 1H), 7.32 - 7.26 (m, 1H), 7.24 - 7.20 (m, 1H), 7.17 - 7.10 (m, 1H), 6.91 (s, 1H), 4.16 (q, 2H), 2.15 - 2.03 (m, 1H), 1.25 (t, 3H), 1.07 - 1.00 (m, 2H), 0.84 - 0.77 (m, 2H); LC-MS (method 5): Rt = 2.98 min; MS (ESlpos): m/z = 452 [M+H]⁺

### Example 50

### 6-Cyclopropyl-N-[(2-methyl-8-quinolyl)sulfonyl]-4-(trifluoromethyl)benzofuran-2-carboxamide

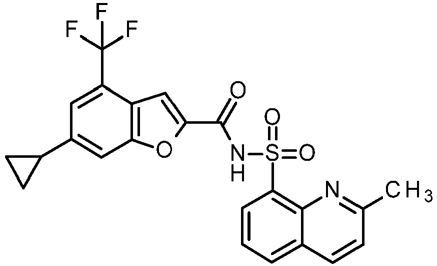

To a room temperature stirred solution of 6-cyclopropyl-4-(trifluoromethyl)benzofuran-2-carboxylic acid (108 mg, 0.40 mmol, 1.00 eq.) in anhydrous tetrahydrofuran (1.33 mL, 0.30 M) was added carbonyldiimidazole (77.8 mg, 0.48 mmol, 1.20 eq.). After stirring for 1 hour, 2-methylquinoline-8-sulfonamide (108 mg, 0.44 mmol, 1.10 eq.) and 1,8-diazabicyclo[5.4.0]undec-7-ene (83.8 µL, 0.56 mmol, 1.40 eq.) were sequentially added. The resulting mixture was stirred at room temperature for a further 16 h and then concentrated under reduced pressure. The residue was purified by reverse phase preparative column chromatography (Waters XBridge PREP C₁₈, 5 µM OBD, 30x150 mm, 49-57% acetonitrile/water gradient buffered with 0.1% formic acid) to give the title compound as a white solid (161 mg). ¹H NMR (400 MHz, DMSO-d₆) δ 8.49 (dd, 1H), 8.46 - 8.34 (m, 2H), 8.30 (dd, 1H), 7.76 (t, 1H), 7.63 (s, 1H), 7.55 - 7.48 (m, 2H), 2.67 (s, 3H), 2.16 (tt, 1H), 1.09 - 0.99 (m, 2H), 0.86 - 0.78 (m, 2H); LC-MS (method 6): Rt = 5.83 min; MS (ESlpos): m/z = 475 [M+H]⁺

### Example 51

### 6-Cyclopropyl-N-(2-ethoxyphenyl)sulfonyl-4-(trifluoromethyl)benzofuran-2-carboxamide

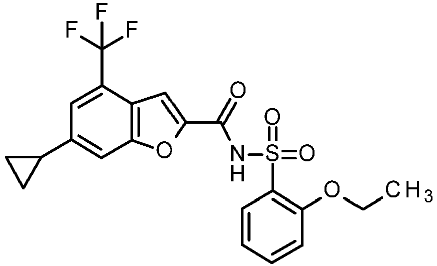

To a room temperature stirred solution of 6-cyclopropyl-4-(trifluoromethyl)benzofuran-2-carboxylic acid (108 mg, 0.40 mmol, 1.00 eq.) in anhydrous tetrahydrofuran (1.33 mL, 0.30 M) was added carbonyldiimidazole (77.8 mg, 0.48 mmol, 1.20 eq.). After stirring for 1 hour, 2-ethoxybenzenesulfonamide (88.6 mg, 0.44 mmol, 1.10 eq.) and 1,8-diazabicyclo[5.4.0]undec-7-ene (83.8 µL, 0.56 mmol, 1.40 eq.) were sequentially added. The resulting mixture was stirred at room temperature for a further 16 h and then concentrated under reduced pressure. The residue was purified by reverse phase preparative column chromatography (Waters XBridge PREP C₁₈, 5 µM OBD, 30x150 mm, 56-64% acetonitrile/water gradient buffered with 0.1% formic acid) to give the title compound as a white solid (122 mg). ¹H NMR (400 MHz, DMSO-d₆) δ 12.78 (s, 1H), 8.31 (s, 1H), 7.92 (dd, 1H), 7.70 (s, 1H), 7.65 (ddd, 1H), 7.53 (s, 1H), 7.21 (d, 1H), 7.14 (t, 1H), 4.15 (q, 2H), 2.20 (tt, 1H), 1.25 (t, 3H), 1.12 - 1.02 (m, 2H), 0.90 - 0.82 (m, 2H); LC-MS (method 6): Rt = 6.60 min; MS (ESlpos): m/z = 454 [M+H]⁺

### Example 52

### N-(Benzenesulfonyl)-6-cyclopropyl-4-(trifluoromethyl)benzofuran-2-carboxamide

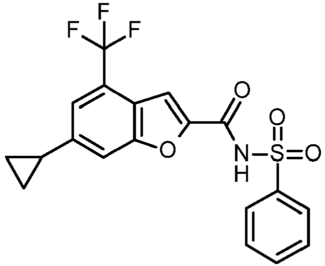

To a room temperature stirred solution of 6-cyclopropyl-4-(trifluoromethyl)benzofuran-2-carboxylic acid (108 mg, 0.40 mmol, 1.00 eq.) in anhydrous tetrahydrofuran (1.33 mL, 0.30 M) was added carbonyldiimidazole (77.8 mg, 0.48 mmol, 1.20 eq.). After stirring for 1 hour, benzenesulfonamide (69.1 mg, 0.44 mmol, 1.10 eq.) and 1,8-diazabicyclo[5.4.0]undec-7-ene (83.8 µL, 0.56 mmol, 1.40 eq.) were sequentially added. The resulting mixture was stirred at room temperature for a further 16 h and then concentrated under reduced pressure. The residue was purified by reverse phase preparative column chromatography (Waters XBridge PREP C18, 5µM OBD, 30x150 mm, 57-65% acetonitrile/water gradient buffered with 0.1% formic acid) to give the title compound as a white solid (127 mg). ¹H NMR (400 MHz, DMSO-d₆) δ 12.92 (s, 1H), 8.14 - 8.05 (m, 1H), 8.05 - 7.96 (m, 2H), 7.77 - 7.69 (m, 2H), 7.69 - 7.62 (m, 2H), 7.56 - 7.50 (m, 1H), 2.19 (tt, 1H), 1.11 - 1.04 (m, 2H), 0.89 - 0.83 (m, 2H); LC-MS (method 6): Rt = 6.31 min; MS (ESlpos): m/z = 410 [M+H]⁺

### Example 53

### 6-Cyclopropyl-N-(2-phenylphenyl)sulfonyl-4-(trifluoromethyl)benzofuran-2-carboxamide

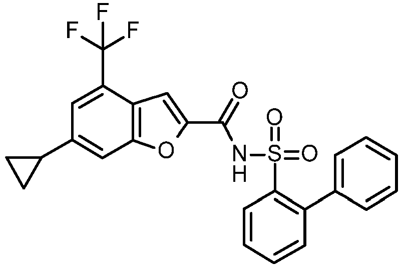

To a room temperature stirred solution of 6-cyclopropyl-4-(trifluoromethyl)benzofuran-2-carboxylic acid (108 mg, 0.40 mmol, 1.00 eq.) in anhydrous tetrahydrofuran (1.33 mL, 0.30 M) was added carbonyldiimidazole (77.8 mg, 0.48 mmol, 1.20 eq.). After stirring for 1 hour, 2-phenylbenzenesulfonamide (103 mg, 0.44 mmol, 1.10 eq.) and 1,8-diazabicyclo[5.4.0]undec-7-ene (83.8 µL, 0.56 mmol, 1.40 eq.) were sequentially added. The resulting mixture was stirred at room temperature for a further 16 h and then concentrated under reduced pressure. The residue was purified by reverse phase preparative column chromatography (Waters XBridge PREP C₁₈, 5 µM OBD, 30x150 mm, 67-75% acetonitrile/water gradient buffered with 0.1% formic acid) to give the title compound as a white solid (167 mg). ¹H NMR (400 MHz, DMSO-d₆) δ 12.42 (s, 1H), 8.19 (dd, 1H), 7.79 - 7.62 (m, 4H), 7.52 (s, 1H), 7.39 - 7.20 (m, 6H), 2.20 (tt, 1H), 1.11 - 1.04 (m, 2H), 0.90 - 0.84 (m, 2H). LC-MS (method 6): Rt = 7.42 min; MS (ESlpos): m/z = 486 [M+H]⁺

### Example 54

### N-{[1,1'-Biphenyl]-2-sulfonyl}-6-(propan-2-yl)-1-benzofuran-2-carboxamide

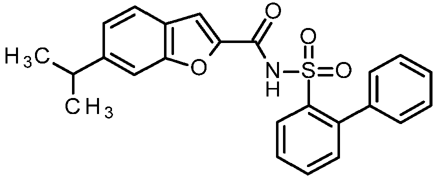

To a room temperature stirred solution of 6-(propan-2-yl)-1-benzofuran-2-carboxylic acid (40.0 mg, 0.19 mmol, 1.00 eq.), [1,1'-biphenyl]-2- sulfonamide (45.6 mg, 0.20 mmol, 1.00 eq.) and PyBOP (122 mg, 0.24 mmol, 1.20 eq.) in anhydrous dichloromethane (979 µL, 0.20 M) was added N,N-diisopropylethylamine (136 µL, 0.78 mmol, 4.00 eq.). The resulting solution was stirred at room temperature for 2 h and then concentrated under reduced pressure. The residue was purified by reverse phase column chromatography (30 g HP C18, 40-100% acetonitrile/water gradient buffered with 0.1% formic acid) to give the title compound as a white solid (66.2 mg). 1H NMR (400 MHz, DMSO-d₆) δ 12.31 (s, 1H), 8.18 (dd, 1H), 7.79 - 7.56 (m, 4H), 7.50 (s, 1H), 7.40 - 7.24 (m, 7H), 3.04 (p, 1H), 1.25 (d, 6H); LC-MS (Method 2): Rₜ = 1.68 min, MS (ESlpos): m/z = 420 [M+H]⁺

### Example 55

### N-(2-Ethoxybenzenesulfonyl)-6-(propan-2-yl)-1-benzofuran-2-carboxamide

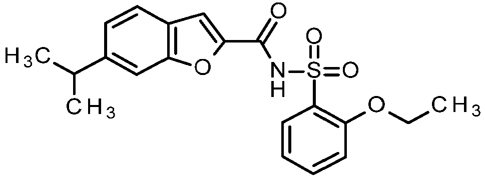

To a room temperature stirred solution of 6-(propan-2-yl)-1-benzofuran-2-carboxylic acid (40.0 mg, 0.19 mmol, 1.00 eq.), 2-ethoxybenzene-1- sulfonamide (39.4 mg, 0.20 mmol, 1.00 eq.) and PyBOP (122 mg, 0.24 mmol, 1.20 eq.) in anhydrous dichloromethane (979 µL, 0.20 M) was added N,N-diisopropylethylamine (136 µL, 0.78 mmol, 4.00 eq.). The resulting solution was stirred at room temperature for 20 h and then concentrated under reduced pressure. The residue was purified by reverse phase column chromatography (30 g HP C₁₈, 40-100% acetonitrile/water gradient buffered with 0.1% formic acid) to give the title compound as a white solid (64.1 mg). ¹H NMR (400 MHz, DMSO-d₆) δ 12.61 (s, 1H), 8.03 (s, 1H), 7.92 (dd1H), 7.73 (d, 1H), 7.68 - 7.62 (m, 1H), 7.51 (s, 1H), 7.28 (dd, 1H), 7.22 (d, 1H), 7.17 - 7.11 (m, 1H), 4.16 (q, 2H), 3.04 (hept, 1H), 1.28 - 1.20 (m, 9H); LC-MS (Method 2.0 min): Rt = 1.56 min, MS (ESlpos): m/z = 388 [M+H]⁺

### Example 56

### N-(Benzenesulfonyl)-6-(propan-2-yl)-1-benzofuran-2-carboxamide

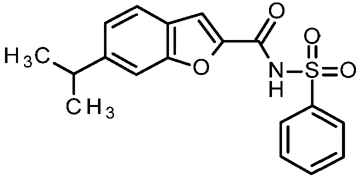

To a room temperature stirred solution of 6-(propan-2-yl)-1-benzofuran-2-carboxylic acid (40.0 mg, 0.19 mmol, 1.00 eq.), benzenesulfonamide (30.7 mg, 0.20 mmol, 1.00 eq.) and PyBOP (122 mg, 0.24 mmol, 1.20 eq.) in anhydrous dichloromethane (979 µL, 0.20 M) was added N,N-diisopropylethylamine (136 µL, 0.78 mmol, 4.00 eq.). The resulting solution was stirred at room temperature for 20 h and then concentrated under reduced pressure. The residue was purified by reverse phase column chromatography (30 g HP C₁₈, 40-100% acetonitrile/water gradient buffered with 0.1% formic acid) to give the title compound as a white solid (57.4 mg). ¹H NMR (400 MHz, DMSO-d₆) δ 8.04 - 7.99 (m, 2H), 7.85 (s, 1H), 7.76 - 7.69 (m, 2H), 7.68 - 7.62 (m, 2H), 7.51 (s, 1H), 7.27 (dd, 1H), 3.05 (hept, 1H), 1.24 (d, 6H); LC-MS (Method 2.0 min): Rt = 1.53 min, MS (ESlpos): m/z = 344 [M+H]⁺

### Example 57

### N-(2,4-Dichlorobenzenesulfonyl)-6-(propan-2-yl)-1-benzofuran-2-carboxamide

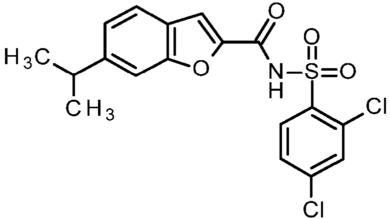

To a room temperature stirred solution of 6-(propan-2-yl)-1-benzofuran-2-carboxylic acid (40.0 mg, 0.19 mmol, 1.00 eq.), 2,4-dichlorobenzene-1- sulfonamide (44.2 mg, 0.20 mmol, 1.00 eq.) and PyBOP (122 mg, 0.24 mmol, 1.20 eq.) in anhydrous dichloromethane (979 µL, 0.20 M) was added N,N-diisopropylethylamine (136 µL, 0.78 mmol, 4.00 eq.). The resulting solution was stirred at room temperature for 20 h and then concentrated under reduced pressure. The residue was purified by reverse phase column chromatography (30 g HP C₁₈, 40-100% acetonitrile/water gradient buffered with 0.1% formic acid) to give the title compound as a white solid (68.0 mg). ¹H NMR (400 MHz, DMSO-d₆) δ 8.15 (d, 1H), 7.85 (d, 2H), 7.74 - 7.68 (m, 2H), 7.50 (s, 1H), 7.27 (dd, 1H), 3.04 (hept, 1H), 1.25 (d, 6H); LC-MS (Method 2): Rt = 1.81 min, MS (ESlpos): m/z = 413 [M+H]⁺

### Example 58

### 6-Cyclopropyl-N-(2-ethoxyphenyl)sulfonyl-4-imidazol-1-yl-benzofuran-2-carboxamide

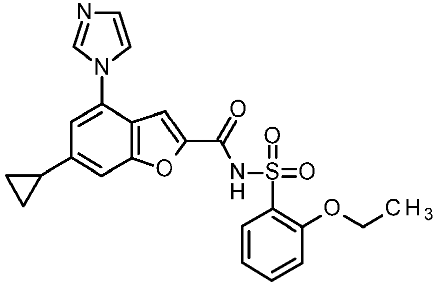

To a room temperature stirred solution of 6-cyclopropyl-4-imidazol-1-yl-benzofuran-2-carboxylic acid (107 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 h, 2-ethoxybenzenesulfonamide (88.6 mg, 0.44 mmol, 1.10 eq.) and 1,8-diazabicyclo[5.4.0]undec-7-ene (84.8 µL, 0.56 mmol, 1.40 eq.) were sequentially added. The resulting mixture was stirred at room temperature for a further 2 h and then concentrated under reduced pressure. The residue was purified by reverse phase preparative column chromatography (Waters XBridge PREP C₁₈, 5 µM OBD, 30 x 150 mm, 30-38% acetonitrile/water gradient buffered with 0.1% formic acid) to give the title compound as a white solid (4.1 mg, 95% purity). 1H NMR (400 MHz, DMSO-d₆) δ 9.90 (s, 2H), 8.18 (s, 1H), 7.81 (d, 1H), 7.71 (s, 1H), 7.40 (d, 2H), 7.17 (d, 2H), 7.07 - 6.93 (m, 2H), 4.03 (q, 2H), 2.11 (td, 1H), 1.26 - 1.12 (m, 3H), 1.02 (dt, 2H), 0.91 - 0.82 (m, 2H); LC-MS (method 5): Rt = 2.00 min; MS (ESlpos): m/z = 452 [M+H]⁺

### Example 59

### N-((5-(tert-butyl)-2-cyclopropoxyphenyl)sulfonyl)-6-cyclopropyl-4-fluorobenzofuran-2-carboxamide

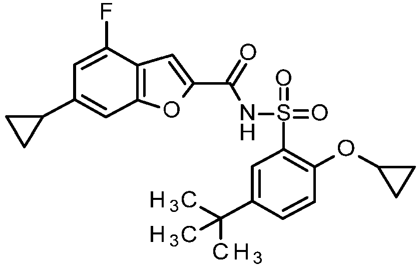

To a room temperature stirred solution of 6-cyclopropyl-4-fluoro-benzofuran-2-carboxylic acid (59.5 mg, 0.27 mmol, 1.00 eq.) in anhydrous tetrahydrofuran (1.35 mL, 0.20 M) was added carbonyldiimidazole (52.5 mg, 0.32 mmol, 1.20 eq.). After stirring for 1 hour, 5-tert-butyl-2-(cyclopropoxy)benzenesulfonamide (80.0 mg, 0.30 mmol, 1.10 eq.) and 1,8-diazabicyclo[5.4.0]undec-7-ene (60.0 µL, 0.38 mmol, 1.40 eq.) were sequentially added. The resulting mixture was stirred at room temperature for 15 h and then concentrated under reduced pressure. The residue was purified by reverse phase preparative column chromatography (Waters XBridge PREP C₁₈, 5 µM OBD, 30 x 150 mm, 65-73 % acetonitrile/water gradient buffered with 0.1% formic acid) to give the title compound as a white solid (79 mg). ¹H NMR (400 MHz, DMSO-d₆) δ 12.61 (s, 1H), 8.06 (s, 1H), 7.85 (d, 1H), 7.77 - 7.69 (m, 1H), 7.40 (d, 1H), 7.29 (s, 1H), 6.99 - 6.91 (m, 1H), 4.08 - 3.99 (m, 1H), 2.16 - 2.04 (m, 1H), 1.31 (s, 9H), 1.09 - 0.99 (m, 2H), 0.85 - 0.76 (m, 2H), 0.74 - 0.66 (m, 2H), 0.56 - 0.50 (m, 2H); LC-MS (method 5): Rt = 3.35 min; MS (ESlpos): m/z = 472 [M+H]⁺

### Example 60

### N-[(2-(Benzyloxy)-5-(tert-butyl)phenyl)sulfonyl]-6-cyclopropyl-4-fluorobenzofuran-2-carboxamide

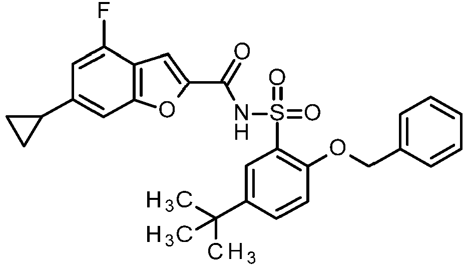

To a room temperature stirred solution of 6-cyclopropyl-4-fluoro-benzofuran-2-carboxylic acid (99.1 mg, 0.45 mmol, 1.00 eq.) in anhydrous tetrahydrofuran (2.25 mL, 0.20 M) was added carbonyldiimidazole (87.6 mg, 0.54 mmol, 1.20 eq.). After stirring for 1 h, 2-benzyloxy-5-tert-butyl-benzenesulfonamide (158 mg, 0.49 mmol, 1.10 eq.) and 1,8-diazabicyclo[5.4.0]undec-7-ene (90.0 µL, 0.63 mmol, 1.40 eq.) were sequentially added. The resulting mixture was stirred at room temperature for 15 h and then concentrated under reduced pressure. The residue was purified by reverse phase preparative column chromatography (Waters XBridge PREP C₁₈, 5 µM OBD, 30 x 150 mm, 69-77 % acetonitrile/water gradient buffered with 0.1% formic acid) to give the title compound as a white solid (134 mg). 1H NMR (400 MHz, DMSO-d₆) δ 12.84 (s, 1H), 8.03 (s, 1H), 7.89 (d, 1H), 7.68 - 7.60 (m, 1H), 7.39 - 7.32 (m, 2H), 7.30 (s, 1H), 7.23 - 7.13 (m, 2H), 7.15 - 7.06 (m, 2H), 6.96 (d, 1H), 5.29 (s, 2H), 2.16 - 2.05 (m, 1H), 1.28 (s, 9H), 1.10 - 0.98 (m, 2H), 0.86 - 0.77 (m, 2H); LC-MS (method 5): Rt = 3.51 min; MS (ESlpos): m/z = 522 [M+H]⁺

### Example 61

### N-((5-(tert-butyl)-2-(cyclopropylmethoxy)phenyl)sulfonyl)-6-cyclopropyl-4-fluorobenzofuran-2-carboxamide

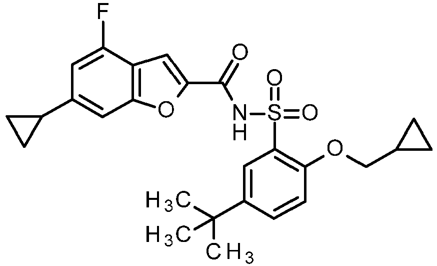

To a room temperature stirred solution of 6-cyclopropyl-4-fluoro-benzofuran-2-carboxylic acid (99.1 mg, 0.45 mmol, 1.00 eq.) in anhydrous tetrahydrofuran (2.25 mL, 0.20 M) was added carbonyldiimidazole (87.6 mg, 0.54 mmol, 1.20 eq.). After stirring for 1 h, 5-tert-butyl-2-(cyclopropylmethoxy)benzenesulfonamide (140 mg, 0.49 mmol, 1.10 eq.) and 1,8-diazabicyclo[5.4.0]undec-7-ene (90.0 µL, 0.63 mmol, 1.40 eq.) were sequentially added. The resulting mixture was stirred at room temperature for 15 h and then concentrated under reduced pressure. The residue was purified by reverse phase preparative column chromatography (Waters XBridge PREP C₁₈, 5µM OBD, 30 x 150 mm, 68-76 % acetonitrile/water gradient buffered with 0.1% formic acid) to give the title compound as a white solid (151 mg). ¹H NMR (400 MHz, DMSO-d₆) δ 12.68 (s, 1H), 8.13 (s, 1H), 7.88 (d, 1H), 7.67 (dd, 1H), 7.32 - 7.27 (m, 1H), 7.20 - 7.13 (m, 1H), 6.99 - 6.91 (m, 1H), 3.95 (d, 2H), 2.16 - 2.04 (m, 1H), 1.31 (s, 9H), 1.27 - 1.10 (m, 1H), 1.09 - 0.99 (m, 2H), 0.86 - 0.77 (m, 2H), 0.38 - 0.28 (m, 2H), 0.20 - 0.11 (m, 2H); LC-MS (method 5): Rt = 3.45 min; MS (ESlpos): m/z = 486 [M+H]⁺

### Example 62

### N-((5-(tert-butyl)-2-(2,2,2-trifluoroethoxy)phenyl)sulfonyl)-6-cyclopropyl-4-fluorobenzofuran-2-carboxamide

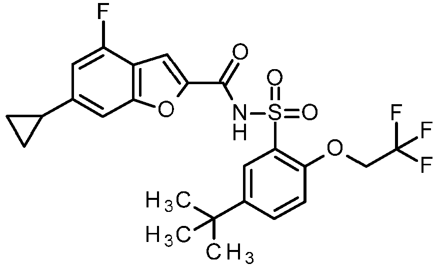

To a room temperature stirred solution of 6-cyclopropyl-4-fluoro-benzofuran-2-carboxylic acid (92.5 mg, 0.42 mmol, 1.00 eq.) in anhydrous tetrahydrofuran (2.10 mL, 0.20 M) was added carbonyldiimidazole (81.7 mg, 0.50 mmol, 1.20 eq.). After stirring for 1 hour, 5-tert-butyl-2-(2,2,2-trifluoroethoxy)benzenesulfonamide (144 mg, 0.46 mmol, 1.10 eq.) and 1,8-diazabicyclo[5.4.0]undec-7-ene (90.0 µL, 0.59 mmol, 1.40 eq.) were sequentially added. The resulting mixture was stirred at room temperature for 15 h and then concentrated under reduced pressure. The residue was purified by reverse phase preparative column chromatography (Waters XBridge PREP C₁₈, 5µM OBD, 30 x 150 mm, 65-73 % acetonitrile/water gradient buffered with 0.1% formic acid) to give the title compound as a white solid (180 mg). ¹H NMR (400 MHz, DMSO-d₆) δ 12.81 (s, 1H), 8.05 (s, 1H), 7.92 (d, 1H), 7.75 (dd, 1H), 7.32 - 7.25 (m, 2H), 6.98 - 6.90 (m, 1H), 4.90 (q, 2H), 2.14 - 2.02 (m, 1H), 1.31 (s, 9H), 1.08 - 0.96 (m, 2H), 0.84 - 0.75 (m, 2H); LC-MS (method 5): Rt = 3.34 min; MS (ESlpos): m/z = 514 [M+H]⁺

### Example 63

### N-((5-(tert-Butyl)-2-cyclobutoxyphenyl)sulfonyl)-6-cyclopropyl-4-fluorobenzofuran-2-carboxamide

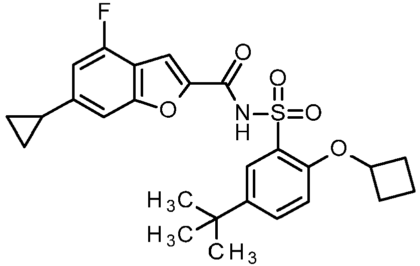

To a room temperature stirred solution of 6-cyclopropyl-4-fluoro-benzofuran-2-carboxylic acid (99.1 mg, 0.45 mmol, 1.00 eq.) in anhydrous tetrahydrofuran (2.25 mL, 0.20 M) was added carbonyldiimidazole (87.6 mg, 0.54 mmol, 1.20 eq.). After stirring for 1 hour, 5-tert-butyl-2-(cyclobutoxy)benzenesulfonamide (140 mg, 0.49 mmol, 1.10 eq.) and 1,8-diazabicyclo[5.4.0]undec-7-ene (90.0 µL, 0.63 mmol, 1.40 eq.) were sequentially added. The resulting mixture was stirred at room temperature for 15 h and then concentrated under reduced pressure. The residue was purified by reverse phase preparative column chromatography (Waters XBridge PREP C₁₈, 5 µM OBD, 30 x 150 mm, 69-77 % acetonitrile/water gradient buffered with 0.1% formic acid) to give the title compound as a white solid (183 mg). ¹H NMR: ¹H NMR (400 MHz, DMSO-d₆) δ 12.69 (s, 1H), 8.14 (s, 1H), 7.85 (d, 1H), 7.64 (dd, 1H), 7.33 - 7.27 (m, 1H), 6.99 - 6.91 (m, 2H), 4.82 (p, 1H), 2.36 - 2.23 (m, 2H), 2.15 - 2.03 (m, 1H), 2.04 - 1.89 (m, 2H), 1.57 - 1.38 (m, 2H), 1.29 (s, 9H), 1.08 - 0.99 (m, 2H), 0.85 - 0.76 (m, 2H); LC-MS (method 5): Rt = 3.48 min; MS (ESlpos): m/z = 486 [M+H]⁺

### Example 64

### N-((5-(tert-Butyl)-2-isopropoxyphenyl)sulfonyl)-6-cyclopropyl-4-fluorobenzofuran-2-carboxamide

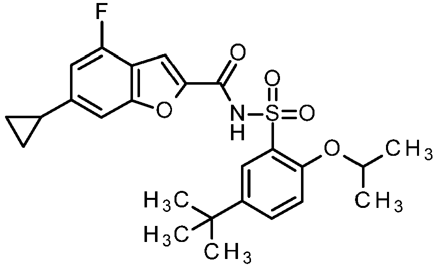

To a room temperature stirred solution of 6-cyclopropyl-4-fluoro-benzofuran-2-carboxylic acid (99.1 mg, 0.45 mmol, 1.00 eq.) in anhydrous tetrahydrofuran (2.25 mL, 0.20 M) was added carbonyldiimidazole (87.6 mg, 0.54 mmol, 1.20 eq.). After stirring for 1 hour, 5-tert-butyl-2-isopropoxy-benzenesulfonamide (134 mg, 0.49 mmol, 1.10 eq.) and 1,8-diazabicyclo[5.4.0]undec-7-ene (90.0 µL, 0.63 mmol, 1.40 eq.) were sequentially added. The resulting mixture was stirred at room temperature for 15 h and then concentrated under reduced pressure. The residue was purified by reverse phase preparative column chromatography (Waters XBridge PREP C₁₈, 5µM OBD, 30 x 150 mm, 68-76 % acetonitrile/water gradient buffered with 0.1% formic acid) to give the title compound as a white solid (169 mg). ¹H NMR (400 MHz, DMSO-d₆) δ 12.58 (s, 1H), 8.13 (s, 1H), 7.86 (d, 1H), 7.66 (dd, 1H), 7.29 (s, 1H), 7.16 (d, 1H), 6.99 - 6.91 (m, 1H), 4.83 - 4.69 (m, 1H), 2.15 - 2.03 (m, 1H), 1.30 (s, 9H), 1.16 (d, 6H), 1.08 - 0.98 (m, 2H), 0.85 - 0.76 (m, 2H); LC-MS (method 5): Rt = 3.42 min; MS (ESlpos): m/z = 474 [M+H]⁺

### Example 65

### N-[(2-Cyclobutoxy-5-isopropylphenyl)sulfonyl]-6-cyclopropyl-4-fluorobenzofuran-2-carboxamide

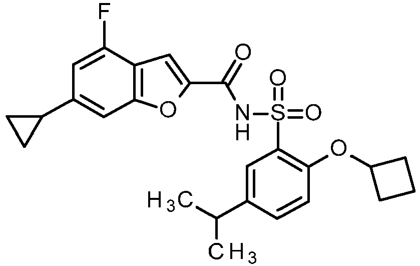

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-(cyclobutoxy)-5-isopropyl-benzenesulfonamide (119 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for one hour and then concentrated under reduced pressure. The residue was purified by reverse phase preparative column chromatography (Waters XBridge PREP C₁₈, 5µM OBD, 30 x 150 mm, 68-76 % acetonitrile/water gradient buffered with 0.1% formic acid) to give the title compound as a white solid (142 mg). ¹H NMR (400 MHz, DMSO-d₆) δ 12.69 (s, 1H), 8.19 - 8.14 (m, 1H), 7.75 (d, 1H), 7.50 (dd, 1H), 7.34 - 7.28 (m, 1H), 7.00 - 6.92 (m, 2H), 4.82 (p1H), 2.96 (hept, 1H), 2.36 - 2.23 (m, 2H), 2.16 - 2.04 (m, 1H), 2.05 - 1.90 (m, 2H), 1.57 - 1.38 (m, 2H), 1.21 (d, 6H), 1.09 - 0.99 (m, 2H), 0.85 - 0.76 (m, 2H); LC-MS (method 5): Rt = 3.42 min; MS (ESlpos): m/z = 472 [M+H]⁺

### Example 66

### N-((5-(tert-butyl)-2-methoxyphenyl)sulfonyl)-6-cyclopropyl-4-fluorobenzofuran-2-carboxamide

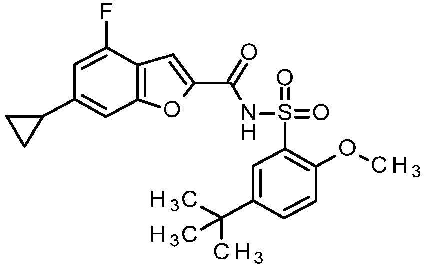

To a room temperature stirred solution of 6-cyclopropyl-4-fluoro-benzofuran-2-carboxylic acid (99.1 mg, 0.45 mmol, 1.00 eq.) in anhydrous tetrahydrofuran (2.25 mL, 0.20 M) was added carbonyldiimidazole (87.6 mg, 0.54 mmol, 1.20 eq.). After stirring for 1 hour, 5-tert-butyl-2-methoxy-benzenesulfonamide (120 mg, 0.49 mmol, 1.10 eq.) and 1,8-diazabicyclo[5.4.0]undec-7-ene (90.0 µL, 0.63 mmol, 1.40 eq.) were sequentially added. The resulting mixture was stirred at room temperature for 19 h and then concentrated under reduced pressure. The residue was purified by reverse phase preparative column chromatography (Waters XBridge PREP C₁₈, 5 µM OBD, 30 x 150 mm, 62-70 % acetonitrile/water gradient buffered with 0.1% formic acid) to give the title compound as a white solid (165 mg). ¹H NMR (400 MHz, DMSO-d₆) δ 12.76 (s, 1H), 8.09 (s, 1H), 7.87 (d, 1H), 7.71 (dd, 1H), 7.31 - 7.25 (m, 1H), 7.16 (d, 1H), 6.99 - 6.91 (m, 1H), 3.83 (s, 3H), 2.14 - 2.02 (m, 1H), 1.30 (s, 9H), 1.08 - 0.96 (m, 2H), 0.84 - 0.75 (m, 2H); LC-MS (method 5): Rₜ = 3.21 min; MS (ESlpos): m/z = 446 [M+H]⁺

### Example 67

### 6-Cyclopropyl-N-{(2-[(2,2-difluorocyclopropyl)methoxy)-5-isopropylphenyl]sulfonyl}-4-fluorobenzofuran-2-carboxamide

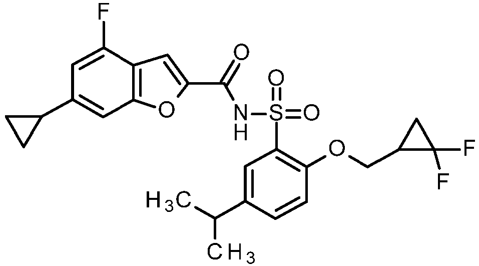

To a room temperature stirred solution of 6-cyclopropyl-4-fluoro-benzofuran-2-carboxylic acid (13.2 mg, 0.06 mmol, 1.00 eq.) in anhydrous tetrahydrofuran (0.30 mL, 0.20 M) was added carbonyldiimidazole (11.7 mg, 0.07 mmol, 1.20 eq.). After stirring for 1 hour, 2-[(2,2-difluorocyclopropyl)methoxy]-5-isopropyl-benzenesulfonamide (20.2 mg, 0.07 mmol, 1.10 eq.) and 1,8-diazabicyclo[5.4.0]undec-7-ene (10.0 µL, 0.08 mmol, 1.40 eq.) were sequentially added. The resulting mixture was stirred at room temperature for one hour and then concentrated under reduced pressure. The residue was purified by reverse phase preparative column chromatography (Waters XBridge PREP C₁₈, 5 µM OBD, 30 x 150 mm, 66-74 % acetonitrile/water gradient buffered with 0.1% formic acid) to give the title compound as a white solid (17.3 mg). ¹H NMR (400 MHz, DMSO-d₆) δ 12.67 (s, 1H), 8.06 (s, 1H), 7.80 - 7.74 (m, 1H), 7.59 - 7.51 (m, 1H), 7.27 (s, 1H), 7.20 (d, 1H), 6.93 (d, 1H), 4.21 - 4.14 (m, 2H), 3.05 - 2.92 (m, 1H), 2.27 - 2.03 (m, 2H), 1.61 - 1.47 (m, 1H), 1.41 - 1.28 (m, 1H), 1.22 (d, 6H), 1.08 - 0.99 (m, 2H), 0.84 - 0.75 (m, 2H); LC-MS (method 5): Rₜ = 3.38 min; MS (ESlpos): m/z = 508 [M+H]⁺

### Example 68

### 6-Cyclopropyl-N-[(2-ethoxy-4,5-difluorophenyl)sulfonyl]-4-fluorobenzofuran-2-carboxamide

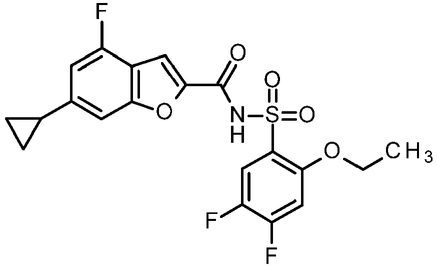

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 2-ethoxy-4,5-difluoro-benzenesulfonamide (104 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for 15 h and then concentrated under reduced pressure. The residue was purified by reverse phase preparative column chromatography (Waters XBridge PREP C₁₈, 5 µM OBD, 30 x 150 mm, 56-64 % acetonitrile/water gradient buffered with 0.1% formic acid) to give the title compound as a white solid (131 mg). ¹H NMR (400 MHz, DMSO-d₆) δ 12.89 (s, 1H), 8.10 (s, 1H), 7.94 (t, 1H), 7.47 (dd, 1H), 7.29 (s, 1H), 6.94 (d, 1H), 4.17 (q, 2H), 2.13 - 2.04 (m, 1H), 1.23 (t, 3H), 1.08 - 0.97 (m, 2H), 0.84 - 0.75 (m, 2H); LC-MS (Method 5): Rₜ = 2.98 min; MS (ESlpos): m/z = 440 [M+H]⁺

### Example 69

### 6-Cyclopropyl-N-[(5-cyclopropyl-2-methoxyphenyl)sulfonyl]-4-fluorobenzofuran-2-carboxamide

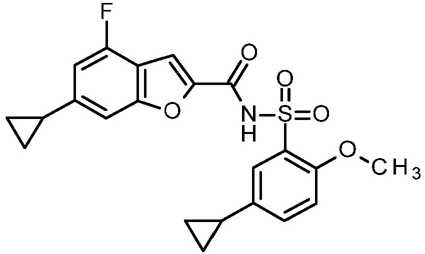

To a room temperature stirred solution of 6-cyclopropyl-4-fluoro-benzofuran-2-carboxylic acid (37.4 mg, 0.17 mmol, 1.00 eq.) in anhydrous tetrahydrofuran (0.85 mL, 0.20 M) was added carbonyldiimidazole (33.1 mg, 0.20 mmol, 1.20 eq.). After stirring for 1 hour, 5-cyclopropyl-2-methoxy-benzenesulfonamide (42.5 mg, 0.19 mmol, 1.10 eq.) and 1,8-diazabicyclo[5.4.0]undec-7-ene (40.0 µL, 0.24 mmol, 1.40 eq.) were sequentially added. The resulting mixture was stirred at room temperature for one hour and then concentrated under reduced pressure. The residue was purified by reverse phase preparative column chromatography (Waters XBridge PREP C₁₈, 5µM OBD, 30 x 150 mm, 56-64 % acetonitrile/water gradient buffered with 0.1% formic acid) to give the title compound as a white solid (32 mg). ¹H NMR (400 MHz, DMSO-d₆) δ 12.77 (s, 1H), 8.08 (s, 1H), 7.67 - 7.61 (m, 1H), 7.38 - 7.31 (m, 1H), 7.30 - 7.28 (m, 1H), 7.15 - 7.08 (m, 1H), 6.99 - 6.91 (m, 1H), 3.82 (s, 3H), 2.14 - 1.96 (m, 2H), 1.08 - 0.90 (m, 4H), 0.84 - 0.75 (m, 2H), 0.69 - 0.58 (m, 2H); LC-MS (method 5): Rt = 2.99 min; MS (ESlpos): m/z = 430 [M+H]⁺

### Example 70

### 6-Cyclopropyl-N-((5-cyclopropyl-2-ethoxyphenyl)sulfonyl)-4-fluorobenzofuran-2-carboxamide

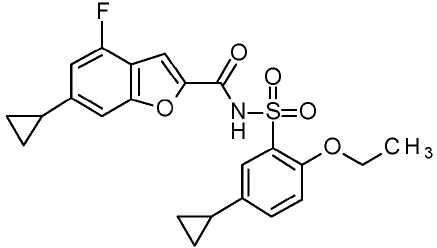

To a room temperature stirred solution of 6-cyclopropyl-4-fluoro-benzofuran-2-carboxylic acid (59.5 mg, 0.27 mmol, 1.00 eq.) in anhydrous tetrahydrofuran (1.35 mL, 0.20 M) was added carbonyldiimidazole (52.5 mg, 0.32 mmol, 1.20 eq.). After stirring for 1 hour, 5-cyclopropyl-2-ethoxy-benzenesulfonamide (71.7 mg, 0.30 mmol, 1.10 eq.) and 1,8-diazabicyclo[5.4.0]undec-7-ene (60.0 µL, 0.38 mmol, 1.40 eq.) were sequentially added. The resulting mixture was stirred at room temperature for 16 h and then concentrated down reduced pressure. The residue was purified by reverse phase preparative column chromatography (Waters XBridge PREP C₁₈, 5 µM OBD, 30 x 150 mm, 60-68 % acetonitrile/water gradient buffered with 0.1% formic acid) to give the title compound as a yellow solid (64 mg). ¹H NMR (400 MHz, DMSO-d₆) δ 12.67 (s, 1H), 8.10 (s, 1H), 7.66 - 7.61 (m, 1H), 7.34 - 7.27 (m, 2H), 7.14 - 7.07 (m, 1H), 6.99 - 6.91 (m, 1H), 4.11 (q, 2H), 2.15 - 1.95 (m, 2H), 1.21 (t, 3H), 1.08 - 0.90 (m, 4H), 0.85 - 0.76 (m, 2H), 0.68 - 0.60 (m, 2H); LC-MS (Method 5): Rt = 3.13 min; MS (ESlpos): m/z = 444 [M+H]⁺

### Example 71

### 6-Cyclopropyl-4-fluoro-N-{[2-methoxy-5-(2-oxopropyl)phenyl]sulfonyl}benzofuran-2-carboxamide

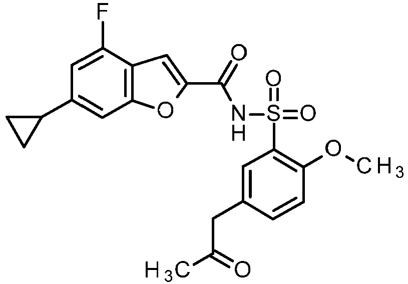

To a room temperature stirred solution of 6-cyclopropyl-4-fluoro-benzofuran-2-carboxylic acid (88.1 mg, 0.40 mmol, 1.00 eq.) in anhydrous tetrahydrofuran (2.00 mL, 0.20 M) was added carbonyldiimidazole (77.8 mg, 0.48 mmol, 1.20 eq.). After stirring for 1 hour, 5-acetonyl-2-methoxy-benzenesulfonamide (107 mg, 0.44 mmol, 1.10 eq.) and 1,8-diazabicyclo[5.4.0]undec-7-ene (80.0 µL, 0.56 mmol, 1.40 eq.) were sequentially added. The resulting mixture was stirred at room temperature for one hour and then concentrated under reduced pressure. The residue was purified by reverse phase preparative column chromatography (Waters XBridge PREP C₁₈, 5 µM OBD, 30 x 150 mm, 47-55 % acetonitrile/water gradient buffered with 0.1% formic acid) to give the title compound as a white solid (109 mg). ¹H NMR (400 MHz, DMSO-d₆) δ 12.77 (s, 1H), 8.10 (s, 1H), 7.77 - 7.72 (m, 1H), 7.46 (dd, 1H), 7.29 (s, 1H), 7.22 - 7.15 (m, 1H), 6.95 (d, 1H), 3.90 - 3.82 (m, 5H), 2.16 (s, 3H), 2.14 - 2.02 (m, 1H), 1.10 - 0.97 (m, 2H), 0.87 - 0.75 (m, 2H); LC-MS (Method 5): Rₜ = 2.63 min; MS (ESlpos): m/z = 446 [M+H]⁺

### Example 72

### 6-Cyclopropyl-N-(2-ethoxy-5-isopropyl-phenyl)sulfonyl-4-fluoro-benzofuran-2-carboxamide

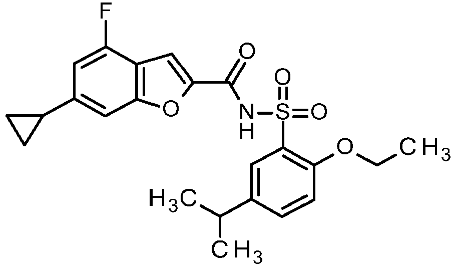

To a room temperature stirred solution of 6-cyclopropyl-4-fluoro-benzofuran-2-carboxylic acid (96.9 mg, 0.44 mmol, 1.00 eq.) in anhydrous tetrahydrofuran (2.20 mL, 0.20 M) was added carbonyldiimidazole (85.6 mg, 0.53 mmol, 1.20 eq.). After stirring for 1 hour, 2-ethoxy-5-isopropyl-benzenesulfonamide (118 mg, 0.48 mmol, 1.10 eq.) and 1,8-diazabicyclo[5.4.0]undec-7-ene (90.0 µL, 0.62 mmol, 1.40 eq.) were sequentially added. The resulting mixture was stirred at room temperature for 16 h and then concentrated under reduced pressure. The residue was purified by reverse phase preparative column chromatography (Waters XBridge PREP C₁₈, 5 µM OBD, 30 x 150 mm, 65-73% acetonitrile/water gradient buffered with 0.1% formic acid) to give the title compound as a white solid (84 mg). ¹H NMR (400 MHz, DMSO-d₆) δ 12.64 - 12.59 (m, 1H), 8.10 (s, 1H), 7.77 - 7.72 (m, 1H), 7.53 (dd, 1H), 7.28 (s, 1H), 7.14 (d, 1H), 6.95 (d, 1H), 4.13 (q, 2H), 2.96 (hept, 1H), 2.15 - 2.03 (m, 1H), 1.27 - 1.18 (m, 9H), 1.08 - 0.97 (m, 2H), 0.84 - 0.75 (m, 2H); LC-MS (method 5): Rₜ = 3.33 min; MS (ESlpos): m/z = 446 [M+H]⁺

### Example 73

### N-(2-chlorobenzenesulfonyl)-6-(propan-2-yl)-1-benzofuran-2-carboxamide

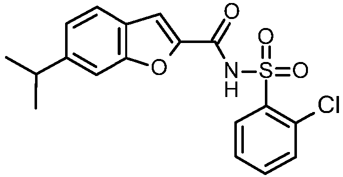

To a room temperature stirred solution of 6-(propan-2-yl)-1-benzofuran-2-carboxylic acid (40.0 mg, 0.19 mmol, 1.00 eq.), 2-chlorobenzene-1-sulfonamide (37.5 mg, 0.20 mmol, 1.00 eq.) and PyBOP (122 mg, 0.24 mmol, 1.20 eq.) in anhydrous dichloromethane (979 µL, 0.20 M) was added *N,N*-diisopropylethylamine (136 µL, 0.78 mmol, 4.00 eq.). The resulting solution was stirred at room temperature for 20 h and then concentrated under reduced pressure. The residue was purified by reverse phase column chromatography (30 g HP C₁₈, 40-100% acetonitrile/water gradient buffered with 0.1% formic acid) to give the title compound as a white solid (68.2 mg). ¹H NMR (400 MHz, DMSO-d₆) δ 8.18 (dd, 1H), 7.96 (s, 1H), 7.75 - 7.65 (m, 3H), 7.62 (td, 1H), 7.51 (s, 1H), 7.28 (dd, 1H), 3.04 (hept, 1H), 1.25 (d, 6H); LC-MS (Method 2): Rt = 1.58 min, MS (ESlpos): m/z = 378 [M+H]⁺

### EXPERIMENTAL SECTION - BIOLOGICAL ASSAYS

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

An empty field in any of the following tables means that the respective compound has not been tested in that Assay.

The *in vitro* activity of the compounds of the present invention can be demonstrated in the following assays:

### Kat6a Activity Assay

Kat6a inhibitory activities of the compounds described in the present invention were quantified using a Fluorescence Resonance Energy Transfer (TR-FRET) assay which measures acetylation of a synthetic, biotinylated Histone-H4-derived peptide by the enzyme.

Recombinant human His-tagged Kat6a protein (amino acids 194-810), was purified in-house from Baculovirus- infected insect cells (Sf9). Histone H4 peptide (amino acids 1-24, SGRGKGGKGLGKGGAKRHRK-VLRD-K(Btn)-amide), which was used as substrate, was synthesized by Biosyntan GmbH, Berlin, Germany. Acetyl Coenzyme A was purchased from Sigma-Aldrich (#A-2056).

Kat6a was incubated for 60 mins at 22°C in the presence of different concentrations of test substances (0 µM, and within the range 0.01 - 20 µM) in assay buffer [25 mM Tris/HCl pH 8, 1 mM EGTA, 2.5 mM Glutathion, 0.02% Chicken Albumin, 0.05% Pluronic F127, 25 mM NaCl, 220 nM H4 peptide and 600 nM Acetyl Coenzyme A].

The reaction was stopped by addition of Detection Solution (25mM HEPES pH 7.5, 0.1% BSA, 22nM SAXL665 (Cisbio #610SAXLE), 100µM Anacardic Acid (Enzo #ALX-270-381), 1nM Anti-Histone H4 (ACETYL K8) Antibody (ABCAM #AB15823) and 0.5 nM Anti-Rabbit IgG Eu (Perkin Elmer #AD0083).

### Kat6b Activity Assay

Kat6b inhibitory activities of the compounds described in the present invention were quantified using a Fluorescence Resonance Energy Transfer (TR-FRET) assay which measures acetylation of a synthetic, biotinylated Histone-H4-derived peptide by the enzyme.

Recombinant human GST-tagged Kat6b protein (431-end, N-terminal GST-tag), purified from Baculovirus- infected insect cells (Sf9), was purchased from SignalChem (#K315-381BG). Histone H4 peptide (amino acids 1-24, SGRGKGGKGLGKGGAKRHRK-VLRD-K(Btn)-amide), which was used as substrate, was synthesized by Biosyntan GmbH, Berlin, Germany. Acetyl Coenzyme A was purchased from Sigma-Aldrich (#A-2056).

Kat6b was incubated for 30 mins at 22°C in the presence of different concentrations of test substances (0 µM, and within the range 0.01 - 20 µM) in assay buffer [25 mM Tris/HCl pH 8, 1 mM EGTA, 2.5 mM Glutathion, 0.02% Chicken Albumin, 0.05% Pluronic F127, 25 mM NaCl, 500 nM H4 peptide and 600 nM Acetyl Coenzyme A].

The reaction was stopped by addition of Detection Solution (25mM HEPES pH 7.5, 0.1% BSA, 22nM SAXL665 (Cisbio #610SAXLE), 100µM Anacardic Acid (Enzo #ALX-270-381), 1nM Anti-Histone H4 (ACETYL K8) Antibody (ABCAM #AB15823), 0.5 nM Anti-Rabbit IgG Eu (Perkin Elmer #AD0083).

The fluorescence emission at 620 nm and 665 nm after excitation at 330-350 nm was measured in a TR-FRET measuring instrument, for instance a Rubystar or a Pherastar (both from BMG Lab Technologies, Offenburg, Germany) or a Viewlux (PerkinElmer). The ratio of the emission at 665 nm and at 622 nm was used as indicator of the amount of acetylated peptide.

The resulting data (ratio) were normalized, taking 0% inhibition as the mean value of control measurements where all reagents were included. In this case 50 nl DMSO were used instead of compounds. A 100% inhibition corresponded to the mean value of control measurements where all reagents except enzyme were included. IC50 values were determined by regression analysis based on a 4 parameter equation (minimum, maximum, ICM, Hill; Y = Max + (Min - Max) / (1 + (X/ICso) Hill) using the Screener Software (Genedata).

Table 2 depicts the IC50 values of selected examples in biochemical KAT6A and KAT6B assays.

**Table 2: IC₅₀ values of selected examples in biochemical KAT6A and KAT6B assays.**

| **Example** | **KAT6A - DPF/HAT domain** | **KAT6B activity assay** |
|---|---|---|
| | **IC₅₀ [mol/l] (arithmetic mean)** | **IC₅₀ [mol/l] (arithmetic mean)** |
| 1 | 3.38E-6 | 4.79E-6 |
| 2 | 8.82E-7 | 2.22E-6 |
| 3 | >2.00E-5 | |
| 4 | 1.52E-5 | >2.00E-5 |
| 5 | 5.75E-6 | > 2.00E-5 |
| 6 | 1.28E-7 | 2.45E-7 |
| 7 | 8.65E-8 | 1.14E-7 |
| 8 | 1.73E-5 | > 2.00E-5 |
| 9 | 9.05E-8 | 2.20E-7 |
| 10 | 9.33E-7 | 1.12E-6 |
| 11 | 6.35E-8 | 2.99E-7 |
| 12 | 4.01E-7 | 3.72E-7 |
| 13 | 1.13E-6 | 1.73E-6 |
| 14 | 4.59E-7 | |
| 15 | 9.08E-7 | |
| 16 | 6.24E-8 | 1.04E-7 |
| 17 | 1.30E-6 | 2.58E-6 |
| 18 | 8.27E-7 | |
| 19 | 7.29E-7 | |
| 20 | >2.00E-5 | |
| 21 | 2.71E-8 | 5.31E-8 |
| 22 | 9.48E-9 | 2.25E-8 |
| 23 | 2.15E-7 | 1.55E-7 |
| 24 | 1.41E-8 | 1.90E-8 |
| 25 | 3.50E-8 | 2.84E-8 |
| 26 | 1.13E-6 | 2.85E-6 |
| 27 | 2.03E-6 | 9.21E-6 |
| 28 | 1.06E-7 | |
| 29 | 1.84E-9 | |
| 30 | 3.73E-8 | |
| 31 | 8.99E-9 | |
| 32 | 5.56E-8 | |
| 33 | 6.32E-9 | |
| 34 | 2.08E-8 | |
| 35 | 5.99E-9 | |
| 36 | 3.01E-9 | |
| 37 | 7.80E-8 | |
| 38 | 1.73E-8 | |
| 39 | 7.07E-8 | |
| 40 | 2.48E-8 | |
| 41 | 4.99E-9 | |
| 42 | 4.17E-7 | |
| 43 | 1.30E-8 | |
| 44 | 5.20E-8 | |
| 45 | 7.99E-8 | |
| 46 | 1.48E-7 | |
| 47 | 4.78E-9 | |
| 48 | 2.67E-8 | |
| 49 | 2.86E-8 | |
| 50 | 5.55E-9 | |
| 51 | 2.56E-8 | |
| 52 | 1.38E-7 | |
| 53 | 4.30E-8 | |
| 54 | 4.64E-7 | 6.67E-7 |
| 55 | 5.96E-7 | 5.38E-7 |
| 56 | 1.56E-6 | 3.94E-6 |
| 57 | 6.11E-7 | 5.25E-6 |
| 58 | 3.95E-8 | |
| 59 | 3.58E-9 | |
| 60 | 2.67E-9 | |
| 61 | 2.92E-9 | |
| 62 | 4.16E-9 | |
| 63 | 2.46E-9 | |
| 64 | 4.33E-9 | |
| 65 | 2.09E-9 | |
| 66 | 3.31E-9 | |
| 67 | 4.50E-9 | |
| 68 | | |
| 69 | | |
| 70 | | |
| 71 | | |
| 72 | 4.79E-9 | |
| 73 | 1.35E-6 | 1.78E-6 |

### Repression of ESR1 transcription upon treatment of cells with compounds

KAT6A controls transcription of the ESR1 gene in breast cancer cells. Compound treatment that interferes with this mechanism was quantified using MVLN cells. These cells constitutively express the ER and are stably transfected with the luciferase (LUC) reporter gene and the corresponding hormone responsive element derived from the 5'-flanking region of the Xenopus Vitellogenin A2 gene (Pons et al., 1990, Demirpense et al., 1993, Joyeux et al., 1993). The repressive effect on ERα transcription activity of a test chemical is directly related to the luciferase measured in the lysate of treated MVLN cells. For measurements the MVLN cells were plated (20,000 cells per well in a 96-well format) in 100 µl culture medium (RPMI 1640 Medium without phenol red (Life Technologies; 11835-063), Pen Strep (Gibco, 15140-122), 10% FCS (Sigma; F2442)) for 24h in humidified incubator at 37 C and 5 % CO₂. Then medium was replaced by 100 µl assay medium (RPMI 1640 Medium without phenol red (Life Technologies; 11835-063), Pen Strep (Gibco, 15140-122), 2.5% FCS (Sigma; F2442)) and cells were treated with compounds (stock solution, 10mM in DMSO) using the HP D300 Digital Dispenser in a concentration range of 2x10E-5 M to 4.69x10E-9 M in a single-dot curve with 8 dilutions and a DMSO concentration of 0.2%. Each sample was prepared as duplicate and rim wells were excluded. For luciferase signal determination 100 µl of a 1:1 mixture of Steadylite plus (Perkin Elmer; 6016759) and assay medium was added after 24h for 15 minutes and measured in Victor X3 (Perkin Elmer). To exclude any effects caused by cell loss during the treatment, cell density was determined using Alamar Blue (Invitrogen; DAL1100). Prior to the luciferase measurement 10 µl Alamar Blue were added to each well and the plates were incubated for 2h in the incubator. Then fluorescence was measured with Victor X3 (Perkin Elmer) at 530 nm /590 nm.

For the evaluation of the results, the values were normalized to DMSO-only treated cells and the Bella DRC Master Sheet was used to calculate EC50s (EC₅₀ is the concentration for 50% of maximal reduction of luciferase signal achieved by Fulvestrant).

Table 3 shows the results of the inhibition of luciferase signal in MVLN cells upon treatment with the compounds of the invention.

### Proliferation Assays for compound characterization

For compound characterization cell survival assays were routinely run in two cell lines. ZR-75-1 is a breast carcinoma cell line containing a focal KAT6A amplification and high KAT6A protein expression. Proliferation of this cell line is inhibited upon treatment with KAT6A inhibitors. As a negative control cell line MDA-MB-436 were used. These cells carry a heterogeneous deletion of the KAT6A gene, have a low KAT6A protein expression and are not growth inhibited upon treatment with KAT6A inhibitors.

For measurements cell lines were plated at the below indicated concentration per 100 mL in 96-well plates in a humidified incubator at 37°C and 5% CO2.

ZR-75-1 (ATCC, CRL-1500; breast carcinoma); 3000 cells per well; culture medium: RPMI 1640 (Biochrom #FG 1215) medium containing 2.5 % FBS (Biochrom # S0615). LCLC-97TM1 (DSMZ, ACC 388; large cell lung carcinoma); 6000 cells per well; culture medium: RPMI 1640 (Biochrom #FG 1215) medium containing 2.5 % FBS (Biochrom # S0615). MDA-MB-436 (CLS, 300278; breast adenocarcinoma); 3000 cells per well; culture medium: DMEM/Ham's F12 (Biochrom # FG 4815) medium containing 2.5 % FBS (Biochrom # S0615).

24 hours later, cells were treated with compounds (stock solution, 10mM in DMSO) using the HP D300 Digital Dispenser in a concentration range of 2.0x10E-5 M to 2.0x10E-9 M in a single-dot curve with 9 dilutions and a DMSO concentration of 0.2%. Each sample was prepared as triplicate and rim wells were excluded. Plates were incubated at 37°C and 5% CO₂ for 144h. Cell viability was measured by adding 10 µL of Alamar Blue Reagent (Invitrogen # DAL 1100) and plates were incubated for 2h at 37°C and 5% CO2. Fluorescence was measured at 590 nM wavelength with a Tecan Spark or Victor X3 MTP-Reader (PerkinElmer).

For the evaluation of the results, the values were normalized to DMSO-only treated cells and the Bella DRC Master Sheet was used to calculate EC50s (EC₅₀ is the concentration for 50% of maximal inhibition of cell proliferation).

Table 3 shows the results of the inhibition in proliferation assays performed in ZR-75-1 and MDA-MB-436 cells upon treatment with the compounds of the invention.

**Table 3: IC₅₀ values of selected examples in proliferation assays performed in MVLN, ZR-75-1, LCLC97TM1 and MDA-MB-436 cells.**

| Example | MVLN reporter assay; IC50 [M] (arithmetic mean) | ZR75-1 proliferation assay; EC50 [M] (arithmetic mean) | LCLC97TM1 proliferation assay; EC50 [M] (arithmetic mean) | MDA-MB-436 proliferation assay; EC50 [M] (arithmetic mean) |
|---|---|---|---|---|
| 1 | 1.31E-5 | | | |
| 2 | 1.11E-5 | >2.00E-5 | | >2.00E-5 |
| 3 | | >2.00E-5 | | >2.00E-5 |
| 4 | 1.98E-5 | >2.00E-5 | > 2.00E-5 | >2.00E-5 |
| 5 | >2.00E-5 | > 2.00E-5 | | > 2.00E-5 |
| 6 | 5.55E-6 | > 2.00E-5 | | >2.00E-5 |
| 7 | 4.54E-6 | > 2.00E-5 | | > 2.00E-5 |
| 8 | >2.00E-5 | > 2.00E-5 | | > 2.00E-5 |
| 9 | 2.50E-6 | 1.81E-5 | | >2.00E-5 |
| 10 | 1.00E-5 | 1.39E-5 | | >2.00E-5 |
| 11 | 5.85E-6 | 1.17E-5 | | 1.42E-5 |
| 12 | 6.05E-6 | 1.03E-5 | | > 2.00E-5 |
| 13 | 5.08E-6 | 1.02E-5 | | > 2.00E-5 |
| 14 | 4.11E-6 | 8.70E-6 | 9.97E-6 | > 2.00E-5 |
| 15 | 6.75E-6 | 8.42E-6 | > 2.00E-5 | > 2.00E-5 |
| 16 | 1.02E-6 | 5.93E-6 | 1.14E-5 | >2.00E-5 |
| 17 | | 6.12E-6 | | > 2.00E-5 |
| 18 | 5.56E-6 | 5.01E-6 | 1.06E-5 | > 2.00E-5 |
| 19 | 3.63E-6 | 4.94E-6 | > 2.00E-5 | > 2.00E-5 |
| 20 | | 3.49E-6 | | > 2.00E-5 |
| 21 | 1.17E-6 | 1.61E-6 | 3.47E-6 | >2.00E-5 |
| 22 | 2.81E-7 | 1.26E-6 | | |
| 23 | | 1.15E-6 | | > 2.00E-5 |
| 24 | 2.47E-7 | 6.74E-7 | | |
| 25 | 5.42E-7 | 6.44E-7 | > 2.00E-5 | > 2.00E-5 |
| 26 | 1.56E-5 | | | |
| 27 | >2.00E-5 | > 2.00E-5 | | > 2.00E-5 |
| 28 | 1.70E-6 | | | |
| 29 | 7.41E-8 | 8.86E-8 | 2.77E-7 | > 2.00E-5 |
| 30 | 2.84E-7 | | | |
| 31 | 2.31E-7 | | | |
| 32 | 4.23E-7 | | | |
| 33 | 1.74E-6 | | | |
| 34 | 1.42E-6 | | | |
| 35 | 2.19E-7 | | | |
| 36 | 1.41E-7 | | | |
| 37 | 1.06E-5 | 6.28E-6 | > 2.00E-5 | > 2.00E-5 |
| 38 | 1.00E-6 | 9.57E-7 | 3.21E-6 | > 2.00E-5 |
| 39 | 4.57E-6 | 3.21E-6 | > 2.00E-5 | > 2.00E-5 |
| 40 | 6.63E-7 | | | |
| 41 | 2.98E-7 | | | |
| 42 | >2.00E-5 | > 2.00E-5 | > 2.00E-5 | > 2.00E-5 |
| 43 | 6.92E-6 | 5.64E-6 | > 2.00E-5 | > 2.00E-5 |
| 44 | 1.16E-5 | 8.67E-6 | > 2.00E-5 | > 2.00E-5 |
| 45 | 1.68E-5 | > 2.00E-5 | > 2.00E-5 | > 2.00E-5 |
| 46 | 1.77E-5 | | | |
| 47 | 4.65E-6 | 3.24E-6 | >2.00E-5 | >2.00E-5 |
| 48 | 5.54E-6 | | | |
| 49 | 6.97E-6 | | | |
| 50 | 1.95E-6 | 1.38E-5 | > 2.00E-5 | > 2.00E-5 |
| 51 | 2.98E-6 | 9.14E-6 | | |
| 52 | 1.88E-5 | 7.32E-6 | | |
| 53 | 6.16E-6 | > 2.00E-5 | | |
| 54 | 3.02E-6 | 5.75E-6 | > 2.00E-5 | >2.00E-5 |
| 55 | 1.34E-6 | 1.27E-5 | > 2.00E-5 | >2.00E-5 |
| 56 | 1.52E-5 | > 2.00E-5 | | > 2.00E-5 |
| 57 | 1.21E-5 | > 2.00E-5 | | > 2.00E-5 |
| 58 | >2.00E-5 | | | |
| 59 | 1.04E-7 | | | |
| 60 | 8.24E-8 | | | |
| 61 | 1.11E-7 | | | |
| 62 | 2.40E-7 | | | |
| 63 | 2.24E-7 | | | |
| 64 | 1.76E-7 | | | |
| 65 | 1.49E-7 | | | |
| 66 | 4.96E-8 | 1.76E-7 | 2.64E-7 | > 2.00E-5 |
| 67 | 8.29E-8 | | | |
| 68 | | | | |
| 69 | | | | |
| 70 | | | | |
| 71 | | | | |
| 72 | 1.23E-7 | | | |
| 73 | 8.69E-6 | 7.08E-6 | > 2.00E-5 | >2.00E-5 |

### Determination of mutagenic activities by a bacterial reverse gene mutation test (Ames test)

The employed commercial assay Ames MPF Penta I comprises the histidine auxotrophic Salmonella tester strains TA98, TA100, TA1535, TA1537 and two tryptophan auxotrophic tester strains E.coli wp2 (E.coli wp2 [pKM101] and E.coli wp2 uvrA, combined exposure). These strains meet the requirements of the OECD guideline 471 for testing chemicals. Bacterical strains were exposed to various concentrations of the test compounds in a histidine (His) or tryptophane (Trp) deficient medium. As recommended by ICH S2(R1) guideline, the test was conducted in two assay conditions, with and without addition of microsomal enzymes (S9 mix), taking the potential formation of DNA reactive metabolites via xenobiotic biotransformation into account.

His-or Trp-independent bacterial colonies may arise from spontaneous reversions (backward mutations) or chemically induced reversions. The potential of the test compound to induce gene mutations is assessed by comparing the control with the test compounds treated bacterial cultures.

**Table 4: Results of the bacterial reverse gene mutation test Ames MPF Penta I**

| **Bacterial strains** | **TA98** | **TA100** | **TA1535** | **TA1537** | **E.coli wp2** |
|---|---|---|---|---|---|
| ***Testing without addition* of *S9 mix*** | | | | | |
| Example 25 _{BAY 3081560} | negative | negative | negative | negative | negative |
| Example 16 _{BAY 2882800} | negative | negative | negative | negative | negative |
| Example 55 _{BAY 2863017} | negative | negative | negative | negative | negative |

| ***Testing with addition* of *S9 mix*** | | | | | |
|---|---|---|---|---|---|
| Example 25 _{BAY 3081560} | negative | negative | negative | negative | negative |
| Example 16 _{BAY 2882800} | negative | negative | negative | negative | negative |
| Example 55 _{BAY 2863017} | negative | negative | negative | negative | negative |

## Claims

1. A compound of general formula **(I)**, 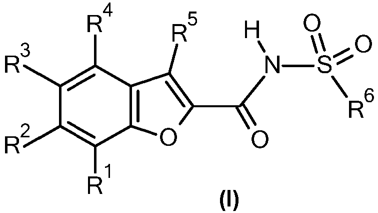 wherein
R¹ is selected from a hydrogen atom and a halogen atom;
R² is selected from a cyano group, a C₁-C₆-alkyl group, a C₃-C₈-cycloalkyl group, a C₁-C₆-haloalkyl group, a (C₁-C₃-alkyl)-O-(C₁-C₂-alkyl)- group, a C₂-C₆-alkenyl group, a R⁹OOC- group, a (R⁷R⁸N)-(C=O)- group, a phenyl group and a heteroaryl group,
wherein said phenyl or heteroaryl group is each optionally substituted with one or more halogen atoms;
R³ is a hydrogen atom;
R⁴ is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N- group and an imidazole group,
wherein said imidazole group is connected to the rest of the molecule via a nitrogen atom of said imidazole group;
R⁵ is selected from a hydrogen atom and a methyl group;
R⁶ is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
wherein said phenyl group is optionally substituted with one or more substituents independently selected from a halogen atom and a C₁-C₄-alkoxy group;
R⁷ and R⁸ are each independently selected from a hydrogen atom, a C₁-C₄-alkyl group and a C₃-C₈-cycloalkyl group;
R⁹ is selected from a hydrogen atom, a C₁-C₃-alkyl group and a C₃-C₅-cycloalkyl group;
or a tautomer, or an N-oxide, or a salt thereof, or a salt of a tautomer, or a salt of an N-oxide, or a mixture of same.

2. The compound according to claim 1, wherein:
R¹ is selected from a hydrogen atom and a halogen atom;
R² is selected from a C₁-C₄-alkyl group, a C₃-C₅-cycloalkyl group, and a C₁-C₄-haloalkyl group;
R³ is a hydrogen atom;
R⁴ is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N- group and an imidazole group,
wherein said imidazole group is connected to the rest of the molecule via a nitrogen atom of said imidazole group;
R⁵ is selected from a hydrogen atom and a methyl group;
R⁶ is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
wherein said phenyl group is optionally substituted with one or more substituents independently selected from a halogen atom and a C₁-C₄-alkoxy group;
or a tautomer, or an N-oxide, or a salt thereof, or a salt of a tautomer, or a salt of an N-oxide, or a mixture of same.

3. The compound according to any of claims 1 or 2, wherein:
R¹ is selected from a hydrogen atom and a halogen atom;
R² is a C₃-C₅-cycloalkyl group;
R³ is a hydrogen atom;
R⁴ is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N- group and an imidazole group,
wherein said imidazole group is connected to the rest of the molecule via a nitrogen atom of said imidazole group;
R⁵ is selected from a hydrogen atom and a methyl group;
R⁶ is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group, a C₃-C₈-cycloalkoxy group, a phenyl group and a (phenyl)-O- group,
wherein said phenyl group is optionally substituted with one or more substituents independently selected from a halogen atom and a C₁-C₄-alkoxy group;
or a tautomer, or an N-oxide, or a salt thereof, or a salt of a tautomer, or a salt of an N-oxide, or a mixture of same.

4. The compound according to any of claims 1 to 3, wherein:
R¹ is selected from a hydrogen atom and a halogen atom;
R² is a C₃-C₅-cycloalkyl group;
R³ is a hydrogen atom;
R⁴ is selected from a hydrogen atom, a halogen atom, a C₁-C₆-alkyl group, a C₁-C₆-haloalkyl group, a C₁-C₆-alkoxy group, a C₁-C₆-haloalkoxy group, a (H₃C)₂N- group and an imidazole group,
wherein said imidazole group is connected to the rest of the molecule via a nitrogen atom of said imidazole group;
R⁵ is selected from a hydrogen atom and a methyl group;
R⁶ is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

5. The compound according to any of claims 1 to 4, wherein:
R¹ is selected from a hydrogen atom and a halogen atom;
R² is a C₃-C₅-cycloalkyl group;
R³ is a hydrogen atom;
R⁴ is selected from a hydrogen atom, a halogen atom and a C₁-C₆-haloalkyl group;
R⁵ is a hydrogen atom;
R⁶ is selected from a phenyl group and a heteroaryl group,
wherein said phenyl or heteroaryl group is each optionally substituted with one or more substituents independently selected from a halogen atom, a C₁-C₆-alkyl group, a C₁-C₆-haloalkyl group, a C₁-C₆-alkoxy group, a (C₃-C₈-cycloalkyl)-(C₁-C₂-alkyl)-O- group, a (C₃-C₈-halocycloalkyl)-(C₁-C₂-alkyl)-O- group, a (phenyl)-(C₁-C₂-alkyl)-O- group, a C₁-C₆-haloalkoxy group and a C₃-C₈-cycloalkoxy group;
or a tautomer, or an N-oxide, or a salt thereof, or a salt of a tautomer, or a salt of an N-oxide, or a mixture of same.

6. A compound of general formula **(I)** according to any of claims 1 to 5, which is selected from
N-([1,1'-biphenyl]-2-sulfonyl)-6-(4-fluorophenyl)-1-benzofuran-2-carboxamide,
N-([1,1'-biphenyl]-2-sulfonyl)-6-methyl-1-benzofuran-2-carboxamide,
N-([1,1'-biphenyl]-2-sulfonyl)-6-cyano-1-benzofuran-2-carboxamide,
N-([1,1'-biphenyl]-2-sulfonyl)-6-(pyridin-3-yl)-1-benzofuran-2-carboxamide,
2-[([1,1'-biphenyl]-2-sulfonyl)carbamoyl]-1-benzofuran-6-carboxylic acid,
4,6-Dimethyl-N-(2'-methyl[1,1'-biphenyl]-2-sulfonyl)-1-benzofuran-2-carboxamide,
4,6-Dimethyl-N-(5-methyl[1,1'-biphenyl]-2-sulfonyl)-1-benzofuran-2-carboxamide,
N2-([1,1'-biphenyl]-2-sulfonyl)-N6,N6-dimethyl-1-benzofuran-2,6-dicarboxamide,
N-([1,1'-biphenyl]-2-sulfonyl)-4,6-dimethyl-1-benzofuran-2-carboxamide,
N-([1,1'-biphenyl]-2-sulfonyl)-4-methoxy-6-methyl-1-benzofuran-2-carboxamide,
N-(3'-chloro[1,1'-biphenyl]-2-sulfonyl)-4,6-dimethyl-1-benzofuran-2-carboxamide,
4-Methoxy-6-methyl-N-(5-methyl[1,1'-biphenyl]-2-sulfonyl)-1-benzofuran-2-carboxamide,
N-(2-Ethoxybenzene-1-sulfonyl)-4-methoxy-6-methyl-1-benzofuran-2-carboxamide,
N-([1,1'-biphenyl]-2-sulfonyl)-3,6-dimethyl-1-benzofuran-2-carboxamide,
N-([1,1'-biphenyl]-2-sulfonyl)-6-cyclopropyl-7-fluoro-1-benzofuran-2-carboxamide,
N-([1,1'-biphenyl]-2-sulfonyl)-6-cyclopropyl-1-benzofuran-2-carboxamide,
N-([1,1'-Biphenyl]-2-sulfonyl)-6-(difluoromethyl)-1-benzofuran-2-carboxamide,
N-(2-Ethoxybenzene-1-sulfonyl)-3,6-dimethyl-1-benzofuran-2-carboxamide,
N-(2-Methylquinoline-8-sulfonyl)-6-(trifluoromethyl)-1-benzofuran-2-carboxamide,
N-([1,1'-Biphenyl]-2-sulfonyl)-6-(pyridin-4-yl)-1-benzofuran-2-carboxamide,
4,6-Dimethyl-N-(2-methylquinoline-8-sulfonyl)-1-benzofuran-2-carboxamide,
6-Cyclopropyl-4-fluoro-N-(2-methylquinoline-8-sulfonyl)-1-benzofuran-2-carboxamide,
N-([1,1'-biphenyl]-2-sulfonyl)-6-(prop-1-en-2-yl)-1-benzofuran-2-carboxamide,
6-Cyclopropyl-N-[2-(cyclopropyloxy)benzene-1-sulfonyl]-4-fluoro-1-benzofuran-2-carboxamide,
6-Cyclopropyl-N-(2-ethoxybenzene-1-sulfonyl)-4-fluoro-1-benzofuran-2-carboxamide,
N-([1,1'-biphenyl]-2-sulfonyl)-6-(2-methoxyethyl)-1-benzofuran-2-carboxamide,
N-([1,1'-biphenyl]-2-sulfonyl)-6-(butan-2-yl)-1-benzofuran-2-carboxamide,
6-Cyclopropyl-N-(2-ethoxy-3-fluoro-phenyl)sulfonyl-4-fluoro-benzofuran-2-carboxamide,
6-Cyclopropyl-N-(2-ethoxy-5-sec-butyl-phenyl)sulfonyl-4-fluoro-benzofuran-2-carboxamide,
6-Cyclopropyl-N-(2-ethoxy-4-fluoro-phenyl)sulfonyl-4-fluoro-benzofuran-2-carboxamide,
6-Cyclopropyl-N-[2-ethoxy-5-(trifluoromethyl)phenyl]sulfonyl-4-fluoro-benzofuran-2-carboxamide,
6-Cyclopropyl-N-(2-ethoxy-5-fluoro-phenyl)sulfonyl-4-fluoro-benzofuran-2-carboxamide,
6-Cyclopropyl-N-(2-ethoxy-5-phenoxy-phenyl)sulfonyl-4-fluoro-benzofuran-2-carboxamide,
6-Cyclopropyl-N-[2-ethoxy-5-(trifluoromethoxy)phenyl]sulfonyl-4-fluoro-benzofuran-2-carboxamide,
N-(2-Benzyloxy-5-isopropyl-phenyl)sulfonyl-6-cyclopropyl-4-fluoro-benzofuran-2-carboxamide,
6-Cyclopropyl-N-[2-(cyclopropylmethoxy)-5-isopropyl-phenyl]sulfonyl-4-fluoro-benzofuran-2-carboxamide,
6-Cyclopropyl-N-(2,4-dichlorophenyl)sulfonyl-4-fluoro-benzofuran-2-carboxamide,
6-Cyclopropyl-4-fluoro-N-(2-phenylphenyl)sulfonyl-benzofuran-2-carboxamide,
N-(Benzenesulfonyl)-6-cyclopropyl-4-fluoro-benzofuran-2-carboxamide,
6-Cyclopropyl-4-fluoro-N-(2-propoxyphenyl)sulfonyl-benzofuran-2-carboxamide,
6-Cyclopropyl-4-fluoro-N-[5-isopropyl-2-(2,2,2-trifluoroethoxy)phenyl]sulfonyl-benzofuran-2-carboxamide,
N-(Benzenesulfonyl)-6-cyclopropyl-4-(dimethylamino)benzofuran-2-carboxamide,
6-Cyclopropyl-4-(dimethylamino)-N-[(2-methyl-8-quinolyl)sulfonyl]benzofuran-2-carboxamide,
6-Cyclopropyl-4-(dimethylamino)-N-(2-phenylphenyl)sulfonyl-benzofuran-2-carboxamide,
6-Cyclopropyl-4-(dimethylamino)-N-(2-ethoxyphenyl)sulfonyl-benzofuran-2-carboxamide,
N-(benzenesulfonyl)-6-cyclopropyl-4-(difluoromethoxy)benzofuran-2-carboxamide,
6-Cyclopropyl-4-(difluoromethoxy)-N-[(2-methyl-8-quinolyl)sulfonyl]benzofuran-2-carboxamide,
6-Cyclopropyl-4-(difluoromethoxy)-N-(2-phenylphenyl)sulfonyl-benzofuran-2-carboxamide,
6-Cyclopropyl-4-(difluoromethoxy)-N-(2-ethoxyphenyl)sulfonyl-benzofuran-2-carboxamide,
6-Cyclopropyl-N-[(2-methyl-8-quinolyl)sulfonyl]-4-(trifluoromethyl)benzofuran-2-carboxamide,
6-Cyclopropyl-N-(2-ethoxyphenyl)sulfonyl-4-(trifluoromethyl)benzofuran-2-carboxamide,
N-(Benzenesulfonyl)-6-cyclopropyl-4-(trifluoromethyl)benzofuran-2-carboxamide,
6-Cyclopropyl-N-(2-phenylphenyl)sulfonyl-4-(trifluoromethyl)benzofuran-2-carboxamide,
N-{[1,1'-Biphenyl]-2-sulfonyl}-6-(propan-2-yl)-1-benzofuran-2-carboxamide,
N-(2-Ethoxybenzenesulfonyl)-6-(propan-2-yl)-1-benzofuran-2-carboxamide,
N-(Benzenesulfonyl)-6-(propan-2-yl)-1-benzofuran-2-carboxamide,
N-(2,4-Dichlorobenzenesulfonyl)-6-(propan-2-yl)-1-benzofuran-2-carboxamide,
6-Cyclopropyl-N-(2-ethoxyphenyl)sulfonyl-4-imidazol-1-yl-benzofuran-2-carboxamide,
N-((5-(tert-butyl)-2-cyclopropoxyphenyl)sulfonyl)-6-cyclopropyl-4-fluorobenzofuran-2-carboxamide,
N-[(2-(Benzyloxy)-5-(tert-butyl)phenyl)sulfonyl]-6-cyclopropyl-4-fluorobenzofuran-2-carboxamide,
N-((5-(tert-butyl)-2-(cyclopropylmethoxy)phenyl)sulfonyl)-6-cyclopropyl-4-fluorobenzofuran-2-carboxamide,
N-((5-(tert-butyl)-2-(2,2,2-trifluoroethoxy)phenyl)sulfonyl)-6-cyclopropyl-4-fluorobenzofuran-2-carboxamide,
N-((5-(tert-Butyl)-2-cyclobutoxyphenyl)sulfonyl)-6-cyclopropyl-4-fluorobenzofuran-2-carboxamide,
N-((5-(tert-Butyl)-2-isopropoxyphenyl)sulfonyl)-6-cyclopropyl-4-fluorobenzofuran-2-carboxamide,
N-[(2-Cyclobutoxy-5-isopropylphenyl)sulfonyl]-6-cyclopropyl-4-fluorobenzofuran-2-carboxamide,
N-((5-(tert-butyl)-2-methoxyphenyl)sulfonyl)-6-cyclopropyl-4-fluorobenzofuran-2-carboxamide,
6-Cyclopropyl-N-{(2-[(2,2-difluorocyclopropyl)methoxy)-5-isopropylphenyl]sulfonyl}-4-fluorobenzofuran-2-carboxamide,
6-Cyclopropyl-N-[(2-ethoxy-4,5-difluorophenyl)sulfonyl]-4-fluorobenzofuran-2-carboxamide,
6-Cyclopropyl-N-[(5-cyclopropyl-2-methoxyphenyl)sulfonyl]-4-fluorobenzofuran-2-carboxamide,
6-Cyclopropyl-N-((5-cyclopropyl-2-ethoxyphenyl)sulfonyl)-4-fluorobenzofuran-2-carboxamide,
6-Cyclopropyl-4-fluoro-N-{[2-methoxy-5-(2-oxopropyl)phenyl]sulfonyl}benzofuran-2-carboxamide,
6-Cyclopropyl-N-(2-ethoxy-5-isopropyl-phenyl)sulfonyl-4-fluoro-benzofuran-2-carboxamide and
N-(2-chlorobenzenesulfonyl)-6-(propan-2-yl)-1-benzofuran-2-carboxamide.

7. A method of preparing a compound of general formula **(I)** according to any of claims 1 to 6, said method comprising the reaction of an intermediate compound of formula **(Ila)** with an R²-containing coupling partner suitable for C-C bond formation, preferably wherein the coupling partner is a boron-containing compound, a Zinc organometallic compound or an alkyl halide, 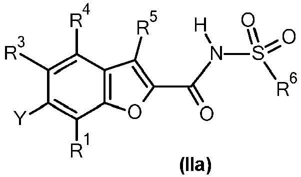 wherein R¹, R², R³, R⁴, R⁵ and R⁶ are as defined for the compound of general formula **(I)** according to any of claims 1 to 6 or a tautomer, or an N-oxide, or a salt thereof, or a salt of a tautomer, or a salt of an N-oxide, or a mixture of same and Y is a halogen atom selected from chlorine and bromine.

8. A compound of general formula **(I)** according to any of claims 1 to 6 for use in the treatment or prophylaxis of a disease.

9. A compound for use according to claim 8, wherein the disease is a hyperproliferative disease.

10. A compound for use according to claim 9, wherein the hyperproliferative disease is cancer.

11. A compound for use according to claim 10, wherein the cancer is selected from lung cancer, breast cancer, bladder cancer, uterine cancer, endometrial cancer, prostate cancer and leukemia.

12. A pharmaceutical composition comprising a compound of general formula **(I)** according to any of claims 1 to 6 and one or more pharmaceutically acceptable excipients.

13. A pharmaceutical composition according to claim 12 for use in the treatment or prophylaxis of a disease, wherein the disease is cancer selected from lung cancer, breast cancer, bladder cancer, uterine cancer, endometrial cancer, prostate cancer and leukemia.

14. A pharmaceutical composition comprising one or more compounds of general formula (I) according to any of claims 1 to 6 and one or more further anti-cancer agents.

## Patentansprüche

1. Verbindung der allgemeinen Formel (**I**), 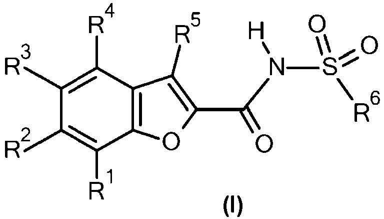 wobei
R¹ ausgewählt ist aus einem Wasserstoffatom und einem Halogenatom;
R² ausgewählt ist aus einer Cyanogruppe, einer C₁-C₆-Alkylgruppe, einer C₃-C₈-Cycloalkylgruppe, einer C₁-C₆-Haloalkylgruppe, einer (C₁-C₃-Alkyl)-O-(C₁-C₂-Alkyl)-Gruppe, einer C₂-C₆-Alkenylgruppe, einer R⁹OOC-Gruppe, einer (R⁷R⁸N)-(C=O)-Gruppe, einer Phenylgruppe und einer Heteroarylgruppe,
wobei die Phenyl- oder Heteroarylgruppe jeweils optional substituiert ist mit einem oder mehreren Halogenatomen;
R³ ein Wasserstoffatom ist;
R⁴ ausgewählt ist aus einem Wasserstoffatom, einem Halogenatom, einer C₁-C₆-Alkylgruppe, einer C₁-C₆-Haloalkylgruppe, einer C₁-C₆-Alkoxygruppe, einer C₁-C₆-Haloalkoxygruppe, einer (H₃C)₂N-Gruppe und einer Imidazolgruppe,
wobei die Imidazolgruppe mit dem Rest des Moleküls über ein Stickstoffatom der Imidazolgruppe verbunden ist;
R⁵ ausgewählt ist aus einem Wasserstoffatom und einer Methylgruppe;
R⁶ ausgewählt ist aus einer Phenylgruppe und einer Heteroarylgruppe,
wobei die Phenyl- oder Heteroarylgruppe jeweils optional substituiert ist mit einem oder mehreren Substituenten unabhängig ausgewählt aus einem Halogenatom, einer C₁-C₆-Alkylgruppe, einer C₁-C₆-Haloalkylgruppe, einer C₁-C₆-Alkoxygruppe, einer (C₃-C₈-Cycloalkyl)-(C₁-C₂-Alkyl)-O-Gruppe, einer (C₃-C₈-Halocycloalkyl)-(C₁-C₂-Alkyl)-O-Gruppe, einer (Phenyl)-(C₁-C₂-Alkyl)-O-Gruppe, einer C₁-C₆-Haloalkoxygruppe, einer C₃-C₈-Cycloalkoxygruppe, einer Phenylgruppe und einer (Phenyl)-O-Gruppe,
wobei die Phenylgruppe optional substituiert ist mit einem oder mehreren Substituenten unabhängig ausgewählt aus einem Halogenatom und einer C₁-C₄-Alkoxygruppe;
R⁷ und R⁸ jeweils unabhängig ausgewählt sind aus einem Wasserstoffatom, einer C₁-C₄-Alkylgruppe und einer C₃-C₈-Cycloalkylgruppe;
R⁹ ausgewählt ist aus einem Wasserstoffatom, einer C₁-C₃-Alkylgruppe und einer C₃-C₅-Cycloalkylgruppe;
oder ein Tautomer oder ein N-Oxid oder ein Salz davon oder ein Salz eines Tautomers oder ein Salz eines N-Oxids oder eine Mischung derselben.

2. Verbindung nach Anspruch 1, wobei:
R¹ ausgewählt ist aus einem Wasserstoffatom und einem Halogenatom;
R² ausgewählt ist aus einer C₁-C₄-Alkylgruppe, einer C₃-C₅-Cycloalkylgruppe und einer C₁-C₄-Haloalkylgruppe;
R³ ein Wasserstoffatom ist;
R⁴ ausgewählt ist aus einem Wasserstoffatom, einem Halogenatom, einer C₁-C₆-Alkylgruppe, einer C₁-C₆-Haloalkylgruppe, einer C₁-C₆-Alkoxygruppe, einer C₁-C₆-Haloalkoxygruppe, einer (H₃C)₂N-Gruppe und einer Imidazolgruppe,
wobei die Imidazolgruppe mit dem Rest des Moleküls über ein Stickstoffatom der Imidazolgruppe verbunden ist;
R⁵ ausgewählt ist aus einem Wasserstoffatom und einer Methylgruppe;
R⁶ ausgewählt ist aus einer Phenylgruppe und einer Heteroarylgruppe,
wobei die Phenyl- oder Heteroarylgruppe jeweils optional substituiert ist mit einem oder mehreren Substituenten unabhängig ausgewählt aus einem Halogenatom, einer C₁-C₆-Alkylgruppe, einer C₁-C₆-Haloalkylgruppe, einer C₁-C₆-Alkoxygruppe, einer (C₃-C₈-Cycloalkyl)-(C₁-C₂-Alkyl)-O-Gruppe, einer (C₃-C₈-Halocycloalkyl)-(C₁-C₂-Alkyl)-O-Gruppe, einer (Phenyl)-(C₁-C₂-Alkyl)-O-Gruppe, einer C₁-C₆-Haloalkoxygruppe, einer C₃-C₈-Cycloalkoxygruppe, einer Phenylgruppe und einer (Phenyl)-O-Gruppe,
wobei die Phenylgruppe optional substituiert ist mit einem oder mehreren Substituenten unabhängig ausgewählt aus einem Halogenatom und einer C₁-C₄-Alkoxygruppe;
oder ein Tautomer oder ein N-Oxid oder ein Salz davon oder ein Salz eines Tautomers oder ein Salz eines N-Oxids oder eine Mischung derselben.

3. Verbindung nach einem der Ansprüche 1 oder 2, wobei:
R¹ ausgewählt ist aus einem Wasserstoffatom und einem Halogenatom;
R² eine C₃-C₅-Cycloalkylgruppe ist;
R³ ein Wasserstoffatom ist;
R⁴ ausgewählt ist aus einem Wasserstoffatom, einem Halogenatom, einer C₁-C₆-Alkylgruppe, einer C₁-C₆-Haloalkylgruppe, einer C₁-C₆-Alkoxygruppe, einer C₁-C₆-Haloalkoxygruppe, einer (H₃C)₂N-Gruppe und einer Imidazolgruppe,
wobei die Imidazolgruppe mit dem Rest des Moleküls über ein Stickstoffatom der Imidazolgruppe verbunden ist;
R⁵ ausgewählt ist aus einem Wasserstoffatom und einer Methylgruppe;
R⁶ ausgewählt ist aus einer Phenylgruppe und einer Heteroarylgruppe,
wobei die Phenyl- oder Heteroarylgruppe jeweils optional substituiert ist mit einem oder mehreren Substituenten unabhängig ausgewählt aus einem Halogenatom, einer C₁-C₆-Alkylgruppe, einer C₁-C₆-Haloalkylgruppe, einer C₁-C₆-Alkoxygruppe, einer (C₃-C₈-Cycloalkyl)-(C₁-C₂-Alkyl)-O-Gruppe, einer (C₃-C₈-Halocycloalkyl)-(C₁-C₂-Alkyl)-O-Gruppe, einer (Phenyl)-(C₁-C₂-Alkyl)-O-Gruppe, einer C₁-C₆-Haloalkoxygruppe, einer C₃-C₈-Cycloalkoxygruppe, einer Phenylgruppe und einer (Phenyl)-O-Gruppe,
wobei die Phenylgruppe optional substituiert ist mit einem oder mehreren Substituenten unabhängig ausgewählt aus einem Halogenatom und einer C₁-C₄-Alkoxygruppe;
oder ein Tautomer oder ein N-Oxid oder ein Salz davon oder ein Salz eines Tautomers oder ein Salz eines N-Oxids oder eine Mischung derselben.

4. Verbindung nach einem der Ansprüche 1 bis 3, wobei:
R¹ ausgewählt ist aus einem Wasserstoffatom und einem Halogenatom;
R² eine C₃-C₅-Cycloalkylgruppe ist;
R³ ein Wasserstoffatom ist;
R⁴ ausgewählt ist aus einem Wasserstoffatom, einem Halogenatom, einer C₁-C₆-Alkylgruppe, einer C₁-C₆-Haloalkylgruppe, einer C₁-C₆-Alkoxygruppe, einer C₁-C₆-Haloalkoxygruppe, einer (H₃C)₂N-Gruppe und einer Imidazolgruppe,
wobei die Imidazolgruppe mit dem Rest des Moleküls über ein Stickstoffatom der Imidazolgruppe verbunden ist;
R⁵ ausgewählt ist aus einem Wasserstoffatom und einer Methylgruppe;
R⁶ ausgewählt ist aus einer Phenylgruppe und einer Heteroarylgruppe,
wobei die Phenyl- oder Heteroarylgruppe jeweils optional substituiert ist mit einem oder mehreren Substituenten unabhängig ausgewählt aus einem Halogenatom, einer C₁-C₆-Alkylgruppe, einer C₁-C₆-Haloalkylgruppe, einer C₁-C₆-Alkoxygruppe, einer (C₃-C₈-Cycloalkyl)-(C₁-C₂-Alkyl)-O-Gruppe, einer (C₃-C₈-Halocycloalkyl)-(C₁-C₂-Alkyl)-O-Gruppe, einer (Phenyl)-(C₁-C₂-Alkyl)-O-Gruppe, einer C₁-C₆-Haloalkoxygruppe und einer C₃-C₈-Cycloalkoxygruppe;
oder ein Tautomer oder ein N-Oxid oder ein Salz davon oder ein Salz eines Tautomers oder ein Salz eines N-Oxids oder eine Mischung derselben.

5. Verbindung nach einem der Ansprüche 1 bis 4, wobei:
R¹ ausgewählt ist aus einem Wasserstoffatom und einem Halogenatom;
R² eine C₃-C₅-Cycloalkylgruppe ist;
R³ ein Wasserstoffatom ist;
R⁴ ausgewählt ist aus einem Wasserstoffatom, einem Halogenatom und einer C₁-C₆-Haloalkylgruppe;
R⁵ ein Wasserstoffatom ist;
R⁶ ausgewählt ist aus einerPhenylgruppe und einer Heteroarylgruppe,
wobei die Phenyl- oder Heteroarylgruppe jeweils optional substituiert ist mit einem oder mehreren Substituenten unabhängig ausgewählt aus einem Halogenatom, einer C₁-C₆-Alkylgruppe, einer C₁-C₆-Haloalkylgruppe, einer C₁-C₆-Alkoxygruppe, einer (C₃-C₈-Cycloalkyl)-(C₁-C₂-Alkyl)-O-Gruppe, einer (C₃-C₈-Halocycloalkyl)-(C₁-C₂-Alkyl)-O-Gruppe, einer (Phenyl)-(C₁-C₂-Alkyl)-O-Gruppe, einer C₁-C₆-Haloalkoxygruppe und einer C₃-C₈-Cycloalkoxygruppe;
oder ein Tautomer oder ein N-Oxid oder ein Salz davon oder ein Salz eines Tautomers oder ein Salz eines N-Oxids oder eine Mischung derselben.

6. Verbindung der allgemeinen Formel **(I)** nach einem der Ansprüche 1 bis 5, die ausgewählt ist aus
N-([1,1'-Biphenyl]-2-sulfonyl)-6-(4-fluorphenyl)-1-benzofuran-2-carboxamid,
N-([1,1'-Biphenyl]-2-sulfonyl)-6-methyl-1-benzofuran-2-carboxamid,
N-([1,1'-Biphenyl]-2-sulfonyl)-6-cyano-1-benzofuran-2-carboxamid,
N-([1,1'-Biphenyl]-2-sulfonyl)-6-(pyridin-3-yl)-1-benzofuran-2-carboxamid,
2-[([1,1'-Biphenyl]-2-sulfonyl)carbamoyl]-1-benzofuran-6-carbonsäure,
4,6-Dimethyl-N-(2'-methyl[1,1'-biphenyl]-2-sulfonyl)-1-benzofuran-2-carboxamid,
4,6-Dimethyl-N-(5-methyl[1,1'-biphenyl]-2-sulfonyl)-1-benzofuran-2-carboxamid,
N2-([1,1'-Biphenyl]-2-sulfonyl)-N6,N6-dimethyl-1-benzofuran-2,6-dicarboxamid,
N-([1,1'-Biphenyl]-2-sulfonyl)-4,6-dimethyl-1-benzofuran-2-carboxamid,
N-([1,1'-Biphenyl]-2-sulfonyl)-4-methoxy-6-methyl-1-benzofuran-2-carboxamid,
N-(3'-Chlor[1,1'-biphenyl]-2-sulfonyl)-4,6-dimethyl-1-benzofuran-2-carboxamid,
4-Methoxy-6-methyl-N-(5-methyl[1,1'-biphenyl]-2-sulfonyl)-1-benzofuran-2-carboxamid,
N-(2-Ethoxybenzol-1-sulfonyl)-4-methoxy-6-methyl-1-benzofuran-2-carboxamid,
N-([1,1'-Biphenyl]-2-sulfonyl)-3,6-dimethyl-1-benzofuran-2-carboxamid,
N-([1,1'-Biphenyl]-2-sulfonyl)-6-cyclopropyl-7-fluor-1-benzofuran-2-carboxamid,
N-([1,1'-Biphenyl]-2-sulfonyl)-6-cyclopropyl-1-benzofuran-2-carboxamid,
N-([1,1'-Biphenyl]-2-sulfonyl)-6-(difluormethyl)-1-benzofuran-2-carboxamid,
N-(2-Ethoxybenzol-1-sulfonyl)-3,6-dimethyl-1-benzofuran-2-carboxamid,
N-(2-Methylchinolin-8-sulfonyl)-6-(trifluormethyl)-1-benzofuran-2-carboxamid,
N-([1,1'-Biphenyl]-2-sulfonyl)-6-(pyridin-4-yl)-1-benzofuran-2-carboxamid,
4,6-Dimethyl-N-(2-methylchinolin-8-sulfonyl)-1-benzofuran-2-carboxamid,
6-Cyclopropyl-4-fluor-N-(2-methylchinolin-8-sulfonyl)-1-benzofuran-2- carboxamid,
N-([1,1'-Biphenyl]-2-sulfonyl)-6-(prop-1-en-2-yl)-1-benzofuran-2-carboxamid,
6-Cyclopropyl-N-[2-(cyclopropyloxy)benzol-1-sulfonyl]-4-fluor-1-benzofuran-2-carboxamid,
6-Cyclopropyl-N-(2-ethoxybenzol-1-sulfonyl)-4-fluor-1-benzofuran-2-carboxamid,
N-([1,1'-Biphenyl]-2-sulfonyl)-6-(2-methoxyethyl)-1-benzofuran-2-carboxamid,
N-([1,1'-Biphenyl]-2-sulfonyl)-6-(butan-2-yl)-1-benzofuran-2-carboxamid,
6-Cyclopropyl-N-(2-ethoxy-3-fluor-phenyl)sulfonyl-4-fluor-benzofuran-2-carboxamid,
6-Cyclopropyl-N-(2-ethoxy-5-sec-butyl-phenyl)sulfonyl-4-fluor-benzofuran-2-carboxamid,
6-Cyclopropyl-N-(2-ethoxy-4-fluor-phenyl)sulfonyl-4-fluor-benzofuran-2-carboxamid,
6-Cyclopropyl-N-[2-ethoxy-5-(trifluormethyl)phenyl]sulfonyl-4-fluor-benzofuran-2-carboxamid,
6-Cyclopropyl-N-(2-ethoxy-5-fluor-phenyl)sulfonyl-4-fluor-benzofuran-2-carboxamid,
6-Cyclopropyl-N-(2-ethoxy-5-phenoxy-phenyl)sulfonyl-4-fluor-benzofuran-2-carboxamid,
6-Cyclopropyl-N-[2-ethoxy-5-(trifluormethoxy)phenyl]sulfonyl-4-fluor-benzofuran-2-carboxamid,
N-(2-Benzyloxy-5-isopropyl-phenyl)sulfonyl-6-cyclopropyl-4-fluor-benzofuran-2-carboxamid,
6-Cyclopropyl-N-[2-(cyclopropylmethoxy)-5-isopropyl-phenyl]sulfonyl-4-fluor-benzofuran-2-carboxamid,
6-Cyclopropyl-N-(2,4-dichlorophenyl)sulfonyl-4-fluor-benzofuran-2-carboxamid,
6-Cyclopropyl-4-fluor-N-(2-phenylphenyl)sulfonyl-benzofuran-2-carboxamid,
N-(Benzolsulfonyl)-6-cyclopropyl-4-fluor-benzofuran-2-carboxamid,
6-Cyclopropyl-4-fluor-N-(2-propoxyphenyl)sulfonyl-benzofuran-2-carboxamid,
6-Cyclopropyl-4-fluor-N-[5-isopropyl-2-(2,2,2-trifluorethoxy)phenyl]sulfonyl-benzofuran-2-carboxamid,
N-(Benzolsulfonyl)-6-cyclopropyl-4-(dimethylamino)benzofuran-2-carboxamid,
6-Cyclopropyl-4-(dimethylamino)-N-[(2-methyl-8-chinolyl)sulfonyl]benzofuran-2-carboxamid,
6-Cyclopropyl-4-(dimethylamino)-N-(2-phenylphenyl)sulfonyl-benzofuran-2-carboxamid,
6-Cyclopropyl-4-(dimethylamino)-N-(2-ethoxyphenyl)sulfonyl-benzofuran-2-carboxamid,
N-(Benzolsulfonyl)-6-cyclopropyl-4-(difluormethoxy)benzofuran-2-carboxamid,
6-Cyclopropyl-4-(difluormethoxy)-N-[(2-methyl-8-chinolyl)sulfonyl]benzofuran-2-carboxamid,
6-Cyclopropyl-4-(difluormethoxy)-N-(2-phenylphenyl)sulfonyl-benzofuran-2-carboxamid,
6-Cyclopropyl-4-(difluormethoxy)-N-(2-ethoxyphenyl)sulfonyl-benzofuran-2-carboxamid,
6-Cyclopropyl-N-[(2-methyl-8-chinolyl)sulfonyl]-4-(trifluormethyl)benzofuran-2-carboxamid,
6-Cyclopropyl-N-(2-ethoxyphenyl)sulfonyl-4-(trifluormethyl)benzofuran-2-carboxamid,
N-(Benzolsulfonyl)-6-cyclopropyl-4-(trifluormethyl)benzofuran-2-carboxamid,
6-Cyclopropyl-N-(2-phenylphenyl)sulfonyl-4-(trifluormethyl)benzofuran-2-carboxamid,
N-{[1,1'-Biphenyl]-2-sulfonyl}-6-(propan-2-yl)-1-benzofuran-2-carboxamid,
N-(2-Ethoxybenzolsulfonyl)-6-(propan-2-yl)-1-benzofuran-2-carboxamid,
N-(Benzolsulfonyl)-6-(propan-2-yl)-1-benzofuran-2-carboxamid,
N-(2,4-Dichlorbenzolsulfonyl)-6-(propan-2-yl)-1-benzofuran-2-carboxamid,
6-Cyclopropyl-N-(2-ethoxyphenyl)sulfonyl-4-imidazol-1-yl-benzofuran-2-carboxamid,
N-((5-(tert-Butyl)-2-cyclopropoxyphenyl)sulfonyl)-6-cyclopropyl-4-fluorbenzofuran-2-carboxamid,
N-[(2-(Benzyloxy)-5-(tert-butyl)phenyl)sulfonyl]-6-cyclopropyl-4-fluorbenzofuran-2-carboxamid,
N-((5-(tert-Butyl)-2-(cyclopropylmethoxy)phenyl)sulfonyl)-6-cyclopropyl-4-fluorbenzofuran-2-carboxamid,
N-((5-(tert-Butyl)-2-(2,2,2-trifluorethoxy)phenyl)sulfonyl)-6-cyclopropyl-4-fluorbenzofuran-2-carboxamid,
N-((5-(tert-Butyl)-2-cyclobutoxyphenyl)sulfonyl)-6-cyclopropyl-4-fluorbenzofuran-2-carboxamid,
N-((5-(tert-Butyl)-2-isopropoxyphenyl)sulfonyl)-6-cyclopropyl-4-fluorbenzofuran-2-carboxamid,
N-[(2-Cyclobutoxy-5-isopropylphenyl)sulfonyl]-6-cyclopropyl-4-fluorbenzofuran-2-carboxamid,
N-((5-(tert-Butyl)-2-methoxyphenyl)sulfonyl)-6-cyclopropyl-4-fluorbenzofuran-2-carboxamid,
6-Cyclopropyl-N-{(2-[(2,2-difluorcyclopropyl)methoxy)-5-isopropylphenyl]sulfonyl}-4-fluorbenzofuran-2-carboxamid,
6-Cyclopropyl-N-[(2-ethoxy-4,5-difluorphenyl)sulfonyl]-4-fluorbenzofuran-2-carboxamid,
6-Cyclopropyl-N-[(5-cyclopropyl-2-methoxyphenyl)sulfonyl]-4-fluorbenzofuran-2-carboxamid,
6-Cyclopropyl-N-((5-cyclopropyl-2-ethoxyphenyl)sulfonyl)-4-fluorbenzofuran-2-carboxamid,
6-Cyclopropyl-4-fluor-N-{[2-methoxy-5-(2-oxopropyl)phenyl]sulfonyl}benzofuran-2-carboxamid,
6-Cyclopropyl-N-(2-ethoxy-5-isopropyl-phenyl)sulfonyl-4-fluor-benzofuran-2-carboxamid, und
N-(2-Chlorobenzolsulfonyl)-6-(propan-2-yl)-1-benzofuran-2-carboxamid.

7. Verfahren zum Herstellen einer Verbindung der allgemeinen Formel **(I)** nach einem der Ansprüche 1 bis 6, wobei das Verfahren die Reaktion einer Zwischenverbindung der Formel (**IIa**) mit einem R²-enthaltenden Kopplungspartner, der für C-C-Bindungsbildung geeignet ist, umfasst, wobei bevorzugt der Kopplungspartner eine Bor-enthaltende Verbindung, eine Zinkorganometallverbindung oder ein Alkylhalogenid ist, 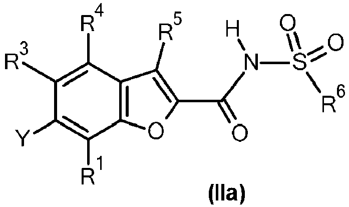 wobei R¹, R², R³, R⁴, R⁵ und R⁶ wie für die Verbindung der allgemeinen Formel **(I)** nach einem der Ansprüche 1 bis 6 definiert oder ein Tautomer oder ein N-Oxid oder ein Salz davon oder ein Salz eines Tautomers oder ein Salz eines N-Oxids oder eine Mischung derselben sind, und Y ein Halogenatom ausgewählt aus Chlor und Brom ist.

8. Verbindung der allgemeinen Formel **(I)** nach einem der Ansprüche 1 bis 6 zur Verwendung bei der Behandlung oder Prophylaxe einer Krankheit.

9. Verbindung zur Verwendung nach Anspruch 8, wobei die Krankheit eine hyperproliferative Krankheit ist.

10. Verbindung zur Verwendung nach Anspruch 9, wobei die hyperproliferative Krankheit Krebs ist.

11. Verbindung zur Verwendung nach Anspruch 10, wobei der Krebs ausgewählt ist aus Lungenkrebs, Brustkrebs, Blasenkrebs, Gebärmutterkrebs, Endometriumkrebs, Prostatakrebs und Leukämie.

12. Pharmazeutische Zusammensetzung, umfassend eine Verbindung der allgemeinen Formel **(I)** nach einem der Ansprüche 1 bis 6 und einen oder mehrere pharmazeutisch annehmbare Hilfsstoffe.

13. Pharmazeutische Zusammensetzung nach Anspruch 12 zur Verwendung bei der Behandlung oder Prophylaxe einer Krankheit, wobei die Krankheit Krebs ausgewählt aus Lungenkrebs, Brustkrebs, Blasenkrebs, Gebärmutterkrebs, Endometriumkrebs, Prostatakrebs und Leukämie ist.

14. Pharmazeutische Zusammensetzung, die eine oder mehrere Verbindungen der allgemeinen Formel **(I)** nach einem der Ansprüche 1 bis 6 und ein oder mehrere weitere Antikrebsmittel umfasst.

## Revendications

1. Composé de formule générale **(I),** 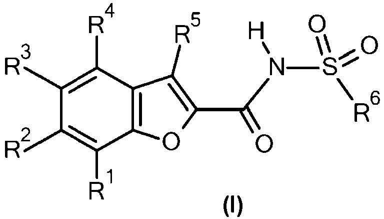 dans lequel
R¹ est choisi parmi un atome d'hydrogène et un atome d'halogène ;
R² est choisi parmi un groupe cyano, un groupe alkyle en C₁-C₆, un groupe cycloalkyle en C₃-C₈, un groupe halogénoalkyle en C₁-C₆, un groupe (alkyle en C₁-C₃)-O-(alkyle en C₁-C₂), un groupe alcényle en C₂-C₆, un groupe R⁹OOC-, un groupe (R⁷R⁸N)-(C=O)-, un groupe phényle et un groupe hétéroaryle,
dans lequel ledit groupe phényle ou hétéroaryle est chacun éventuellement substitué par un ou plusieurs atomes d'halogène ;
R³ est un atome d'hydrogène ;
R⁴ est choisi parmi un atome d'hydrogène, un atome d'halogène, un groupe alkyle en C₁-C₆, un groupe halogénoalkyle en C₁-C₆, un groupe alcoxy en C₁-C₆, un groupe halogénoalcoxy en C₁-C₆, un groupe (H₃C)₂N- et un groupe imidazole,
dans lequel ledit groupe imidazole est lié au reste de la molécule par l'intermédiaire d'un atome d'azote dudit groupe imidazole ;
R⁵ est choisi parmi un atome d'hydrogène et un groupe méthyle ;
R⁶ est choisi parmi un groupe phényle et un groupe hétéroaryle,
dans lequel ledit groupe phényle ou hétéroaryle est chacun éventuellement substitué par un ou plusieurs substituants choisis indépendamment parmi un atome d'halogène, un groupe alkyle en C₁-C₆, un groupe halogénoalkyle en C₁-C₆, un groupe alcoxy en C₁-C₆, un groupe (cycloalkyle en C₃-C₈)-(alkyle en C₁-C₂)-O-, un groupe (halogénocycloalkyle en C₃-C₈)-(alkyle en C₁-C₂)-O-, un groupe (phényle)-(alkyle en C₁-C₂)-O-, un groupe halogénoalcoxy en C₁-C₆, un groupe cycloalcoxy en C₃-C₈, un groupe phényle et un groupe (phényle)-O-,
dans lequel ledit groupe phényle est éventuellement substitué par un ou plusieurs substituants choisis indépendamment parmi un atome d'halogène et un groupe alcoxy en C₁-C₄ ;
R⁷ et R⁸ sont chacun indépendamment choisis parmi un atome d'hydrogène, un groupe alkyle en C₁-C₄ et un groupe cycloalkyle en C₃-C₈ ;
R⁹ est choisi parmi un atome d'hydrogène, un groupe alkyle en C₁-C₃ et un groupe cycloalkyle en C₃-C₅ ;
ou un tautomère, ou un N-oxyde, ou un sel de celui-ci, ou un sel d'un tautomère, ou un sel d'un N-oxyde, ou un mélange de ceux-ci.

2. Composé selon la revendication 1, dans lequel :
R¹ est choisi parmi un atome d'hydrogène et un atome d'halogène ;
R² est choisi parmi un groupe alkyle en C₁-C₄, un groupe cycloalkyle en C₃-C₅ et un groupe halogénoalkyle en C₁-C₄ ;
R³ est un atome d'hydrogène ;
R⁴ est choisi parmi un atome d'hydrogène, un atome d'halogène, un groupe alkyle en C₁-C₆, un groupe halogénoalkyle en C₁-C₆, un groupe alcoxy en C₁-C₆, un groupe halogénoalcoxy en C₁-C₆, un groupe (H₃C)₂N- et un groupe imidazole,
dans lequel ledit groupe imidazole est lié au reste de la molécule par l'intermédiaire d'un atome d'azote dudit groupe imidazole ;
R⁵ est choisi parmi un atome d'hydrogène et un groupe méthyle ;
R⁶ est choisi parmi un groupe phényle et un groupe hétéroaryle,
dans lequel ledit groupe phényle ou hétéroaryle est chacun éventuellement substitué par un ou plusieurs substituants choisis indépendamment parmi un atome d'halogène, un groupe alkyle en C₁-C₆, un groupe halogénoalkyle en C₁-C₆, un groupe alcoxy en C₁-C₆, un groupe (cycloalkyle en C₃-C₈)-(alkyle en C₁-C₂)-O-, un groupe (halogénocycloalkyle en C₃-C₈)-(alkyle en C₁-C₂)-O-, un groupe (phényle)-(alkyle en C₁-C₂)-O-, un groupe halogénoalcoxy en C₁-C₆, un groupe cycloalcoxy en C₃-C₈, un groupe phényle et un groupe (phényle)-O-,
dans lequel ledit groupe phényle est éventuellement substitué par un ou plusieurs substituants choisis indépendamment parmi un atome d'halogène et un groupe alcoxy en C₁-C₄ ;
ou un tautomère, ou un N-oxyde, ou un sel de celui-ci, ou un sel d'un tautomère, ou un sel d'un N-oxyde, ou un mélange de ceux-ci.

3. Composé selon l'une quelconque des revendications 1 ou 2, dans lequel :
R¹ est choisi parmi un atome d'hydrogène et un atome d'halogène ;
R² est un groupe cycloalkyle en C₃-C₅ ;
R³ est un atome d'hydrogène ;
R⁴ est choisi parmi un atome d'hydrogène, un atome d'halogène, un groupe alkyle en C₁-C₆, un groupe halogénoalkyle en C₁-C₆, un groupe alcoxy en C₁-C₆, un groupe halogénoalcoxy en C₁-C₆, un groupe (H₃C)₂N- et un groupe imidazole,
dans lequel ledit groupe imidazole est lié au reste de la molécule par l'intermédiaire d'un atome d'azote dudit groupe imidazole ;
R⁵ est choisi parmi un atome d'hydrogène et un groupe méthyle ;
R⁶ est choisi parmi un groupe phényle et un groupe hétéroaryle,
dans lequel ledit groupe phényle ou hétéroaryle est chacun éventuellement substitué par un ou plusieurs substituants choisis indépendamment parmi un atome d'halogène, un groupe alkyle en C₁-C₆, un groupe halogénoalkyle en C₁-C₆, un groupe alcoxy en C₁-C₆, un groupe (cycloalkyle en C₃-C₈)-(alkyle en C₁-C₂)-O-, un groupe (halogénocycloalkyle en C₃-C₈)-(alkyle en C₁-C₂)-O-, un groupe (phényle)-(alkyle en C₁-C₂)-O-, un groupe halogénoalcoxy en C₁-C₆, un groupe cycloalcoxy en C₃-C₈, un groupe phényle et un groupe (phényle)-O-,
dans lequel ledit groupe phényle est éventuellement substitué par un ou plusieurs substituants choisis indépendamment parmi un atome d'halogène et un groupe alcoxy en C₁-C₄ ;
ou un tautomère, ou un N-oxyde, ou un sel de celui-ci, ou un sel d'un tautomère, ou un sel d'un N-oxyde, ou un mélange de ceux-ci.

4. Composé selon l'une quelconque des revendications 1 à 3, dans lequel :
R¹ est choisi parmi un atome d'hydrogène et un atome d'halogène ;
R² est un groupe cycloalkyle en C₃-C₅ ;
R³ est un atome d'hydrogène ;
R⁴ est choisi parmi un atome d'hydrogène, un atome d'halogène, un groupe alkyle en C₁-C₆, un groupe halogénoalkyle en C₁-C₆, un groupe alcoxy en C₁-C₆, un groupe halogénoalcoxy en C₁-C₆, un groupe (H₃C)₂N- et un groupe imidazole,
dans lequel ledit groupe imidazole est lié au reste de la molécule par l'intermédiaire d'un atome d'azote dudit groupe imidazole ;
R⁵ est choisi parmi un atome d'hydrogène et un groupe méthyle ;
R⁶ est choisi parmi un groupe phényle et un groupe hétéroaryle,
dans lequel ledit groupe phényle ou hétéroaryle est chacun éventuellement substitué par un ou plusieurs substituants choisis indépendamment parmi un atome d'halogène, un groupe alkyle en C₁-C₆, un groupe halogénoalkyle en C₁-C₆, un groupe alcoxy en C₁-C₆, un groupe (cycloalkyle en C₃-C₈)-(alkyle en C₁-C₂)-O-, un groupe (halogénocycloalkyle en C₃-C₈)-(alkyle en C₁-C₂)-O-, un groupe (phényle)-(alkyle en C₁-C₂)-O-, un groupe halogénoalcoxy en C₁-C₆ et un groupe cycloalcoxy en C₃-C₈ ;
ou un tautomère, ou un N-oxyde, ou un sel de celui-ci, ou un sel d'un tautomère, ou un sel d'un N-oxyde, ou un mélange de ceux-ci.

5. Composé selon l'une quelconque des revendications 1 à 4, dans lequel :
R¹ est choisi parmi un atome d'hydrogène et un atome d'halogène ;
R² est un groupe cycloalkyle en C₃-C₅ ;
R³ est un atome d'hydrogène ;
R⁴ est choisi parmi un atome d'hydrogène, un atome d'halogène et un groupe halogénoalkyle en C₁-C₆ ;
R⁵ est un atome d'hydrogène ;
R⁶ est choisi parmi un groupe phényle et un groupe hétéroaryle,
dans lequel ledit groupe phényle ou hétéroaryle est chacun éventuellement substitué par un ou plusieurs substituants choisis indépendamment parmi un atome d'halogène, un groupe alkyle en C₁-C₆, un groupe halogénoalkyle en C₁-C₆, un groupe alcoxy en C₁-C₆, un groupe (cycloalkyle en C₃-C₈)-(alkyle en C₁-C₂)-O-, un groupe (halogénocycloalkyle en C₃-C₈)-(alkyle en C₁-C₂)-O-, un groupe (phényle)-(alkyle en C₁-C₂)-O-, un groupe halogénoalcoxy en C₁-C₆ et un groupe cycloalcoxy en C₃-C₈ ;
ou un tautomère, ou un N-oxyde, ou un sel de celui-ci, ou un sel d'un tautomère, ou un sel d'un N-oxyde, ou un mélange de ceux-ci.

6. Composé de formule générale (**I**) selon l'une quelconque des revendications 1 à 5, qui est choisi parmi
le N-([1,1'-biphényl]-2-sulfonyl)-6-(4-fluorophényl)-1-benzofurane-2-carboxamide,
le N-([1,1'-biphényl]-2-sulfonyl)-6-méthyl-1-benzofurane-2-carboxamide,
le N-([1,1'-biphényl]-2-sulfonyl)-6-cyano-1-benzofurane-2-carboxamide,
le N-([1,1'-biphényl]-2-sulfonyl)-6-(pyridin-3-yl)-1-benzofurane-2-carboxamide,
l'acide 2-[([1,1'-biphényl]-2-sulfonyl)carbamoyl]-1-benzofurane-6-carboxylique,
le 4,6-diméthyl-N-(2'-méthyl[1,1'-biphényl]-2-sulfonyl)-1-benzofurane-2-carboxamide,
le 4,6-diméthyl-N-(5-méthyl[1,1'-biphényl]-2-sulfonyl)-1-benzofurane-2-carboxamide,
le N2-([1,1'-biphényl]-2-sulfonyl)-N6,N6-diméthyl-1-benzofurane-2,6-dicarboxamide,
le N-([1,1'-biphényl]-2-sulfonyl)-4,6-diméthyl-1-benzofurane-2-carboxamide,
le N-([1,1'-biphényl]-2-sulfonyl)-4-méthoxy-6-méthyl-1-benzofurane-2-carboxamide,
le N-(3'-chloro[1,1'-biphényl]-2-sulfonyl)-4,6-diméthyl-1-benzofurane-2-carboxamide,
le 4-méthoxy-6-méthyl-N-(5-méthyl[1,1'-biphényl]-2-sulfonyl)-1-benzofurane-2-carboxamide,
le N-(2-éthoxybenzène-1-sulfonyl)-4-méthoxy-6-méthyl-1-benzofurane-2-carboxamide,
le N-([1,1'-biphényl]-2-sulfonyl)-3,6-diméthyl-1-benzofurane-2-carboxamide,
le N-([1,1'-biphényl]-2-sulfonyl)-6-cyclopropyl-7-fluoro-1-benzofurane-2-carboxamide,
le N-([1,1'-biphényl]-2-sulfonyl)-6-cyclopropyl-1-benzofurane-2-carboxamide,
le N-([1,1'-biphényl]-2-sulfonyl)-6-(difluorométhyl)-1-benzofurane-2-carboxamide,
le N-(2-éthoxybenzène-1-sulfonyl)-3,6-diméthyl-1-benzofurane-2-carboxamide,
le N-(2-méthylquinoléine-8-sulfonyl)-6-(trifluorométhyl)-1-benzofurane-2-carboxamide,
le N-([1,1'-biphényl]-2-sulfonyl)-6-(pyridin-4-yl)-1-benzofurane-2-carboxamide,
le 4,6-diméthyl-N-(2-méthylquinoléine-8-sulfonyl)-1-benzofurane-2-carboxamide,
le 6-cyclopropyl-4-fluoro-N-(2-méthylquinoléine-8-sulfonyl)-1-benzofurane-2-carboxamide,
le N-([1,1'-biphényl]-2-sulfonyl)-6-(prop-1-én-2-yl)-1-benzofurane-2-carboxamide,
le 6-cyclopropyl-N-[2-(cyclopropyloxy)benzène-1-sulfonyl]-4-fluoro-1-benzofurane-2-carboxamide,
le 6-cyclopropyl-N-(2-éthoxybenzène-1-sulfonyl)-4-fluoro-1-benzofurane-2-carboxamide,
le N-([1,1'-biphényl]-2-sulfonyl)-6-(2-méthoxyéthyl)-1-benzofurane-2-carboxamide,
le N-([1,1'-biphényl]-2-sulfonyl)-6-(butan-2-yl)-1-benzofurane-2-carboxamide,
le 6-cyclopropyl-N-(2-éthoxy-3-fluoro-phényl)sulfonyl-4-fluoro-benzofurane-2-carboxamide,
le 6-cyclopropyl-N-(2-éthoxy-5-sec-butyl-phényl)sulfonyl-4-fluoro-benzofurane-2-carboxamide,
le 6-cyclopropyl-N-(2-éthoxy-4-fluoro-phényl)sulfonyl-4-fluoro-benzofurane-2-carboxamide,
le 6-cyclopropyl-N-[2-éthoxy-5-(trifluorométhyl)phényl]sulfonyl-4-fluoro-benzofurane-2-carboxamide,
le 6-cyclopropyl-N-(2-éthoxy-5-fluoro-phényl)sulfonyl-4-fluoro-benzofurane-2-carboxamide,
le 6-cyclopropyl-N-(2-éthoxy-5-phénoxy-phényl)sulfonyl-4-fluoro-benzofurane-2-carboxamide,
le 6-cyclopropyl-N-[2-éthoxy-5-(trifluorométhoxy)phényl]sulfonyl-4-fluoro-benzofurane-2-carboxamide,
le N-(2-benzyloxy-5-isopropyl-phényl)sulfonyl-6-cyclopropyl-4-fluoro-benzofurane-2-carboxamide,
le 6-cyclopropyl-N-[2-(cyclopropylméthoxy)-5-isopropyl-phényl]sulfonyl-4-fluoro-benzofurane-2-carboxamide,
le 6-cyclopropyl-N-(2,4-dichlorophényl)sulfonyl-4-fluoro-benzofurane-2-carboxamide,
le 6-cyclopropyl-4-fluoro-N-(2-phénylphényl)sulfonyl-benzofurane-2-carboxamide,
le N-(benzènesulfonyl)-6-cyclopropyl-4-fluoro-benzofurane-2-carboxamide,
le 6-cyclopropyl-4-fluoro-N-(2-propoxyphényl)sulfonyl-benzofurane-2-carboxamide,
le 6-cyclopropyl-4-fluoro-N-[5-isopropyl-2-(2,2,2-trifluoroéthoxy)phényl]sulfonyl-benzofurane-2-carboxamide,
le N-(benzènesulfonyl)-6-cyclopropyl-4-(diméthylamino)benzofurane-2-carboxamide,
le 6-cyclopropyl-4-(diméthylamino)-N-[(2-méthyl-8-quinolyl)sulfonyl]benzofurane-2-carboxamide,
le 6-cyclopropyl-4-(diméthylamino)-N-(2-phénylphényl)sulfonyl-benzofurane-2-carboxamide,
le 6-cyclopropyl-4-(diméthylamino)-N-(2-éthoxyphényl)sulfonyl-benzofurane-2-carboxamide,
le N-(benzènesulfonyl)-6-cyclopropyl-4-(difluorométhoxy)benzofurane-2-carboxamide,
le 6-cyclopropyl-4-(difluorométhoxy)-N-[(2-méthyl-8-quinolyl)sulfonyl]benzofurane-2-carboxamide,
le 6-cyclopropyl-4-(difluorométhoxy)-N-(2-phénylphényl)sulfonyl-benzofurane-2-carboxamide,
le 6-cyclopropyl-4-(difluorométhoxy)-N-(2-éthoxyphényl)sulfonyl-benzofurane-2-carboxamide,
le 6-cyclopropyl-N-[(2-méthyl-8-quinolyl)sulfonyl]-4-(trifluorométhyl)benzofurane-2-carboxamide,
le 6-cyclopropyl-N-(2-éthoxyphényl)sulfonyl-4-(trifluorométhyl)benzofurane-2-carboxamide,
le N-(benzènesulfonyl)-6-cyclopropyl-4-(trifluorométhyl)benzofurane-2-carboxamide,
le 6-cyclopropyl-N-(2-phénylphényl)sulfonyl-4-(trifluorométhyl)benzofurane-2-carboxamide,
le N-{[1,1'-biphényl]-2-sulfonyl}-6-(propan-2-yl)-1-benzofurane-2-carboxamide,
le N-(2-éthoxybenzènesulfonyl)-6-(propan-2-yl)-1-benzofurane-2-carboxamide,
le N-(benzènesulfonyl)-6-(propan-2-yl)-1-benzofurane-2-carboxamide,
le N-(2,4-dichlorobenzènesulfonyl)-6-(propan-2-yl)-1-benzofurane-2-carboxamide,
le 6-cyclopropyl-N-(2-éthoxyphényl)sulfonyl-4-imidazol-1-yl-benzofurane-2-carboxamide,
le N-((5-(tert-butyl)-2-cyclopropoxyphényl)sulfonyl)-6-cyclopropyl-4-fluorobenzofurane-2-carboxamide,
le N-[(2-(benzyloxy)-5-(tert-butyl)phényl)sulfonyl]-6-cyclopropyl-4-fluorobenzofurane-2-carboxamide,
le N-((5-(tert-butyl)-2-(cyclopropylméthoxy)phényl)sulfonyl)-6-cyclopropyl-4-fluorobenzofurane-2-carboxamide,
le N-((5-(tert-butyl)-2-(2,2,2-trifluoroéthoxy)phényl)sulfonyl)-6-cyclopropyl-4-fluorobenzofurane-2-carboxamide,
le N-((5-(tert-butyl)-2-cyclobutoxyphényl)sulfonyl)-6-cyclopropyl-4-fluorobenzofurane-2-carboxamide,
le N-((5-(tert-butyl)-2-isopropoxyphényl)sulfonyl)-6-cyclopropyl-4-fluorobenzofurane-2-carboxamide,
le N-[(2-cyclobutoxy-5-isopropylphényl)sulfonyl]-6-cyclopropyl-4-fluorobenzofurane-2-carboxamide,
le N-((5-(tert-butyl)-2-méthoxyphényl)sulfonyl)-6-cyclopropyl-4-fluorobenzofurane-2-carboxamide,
le 6-cyclopropyl-N-{(2-[(2,2-difluorocyclopropyl)méthoxy)-5-isopropylphényl]sulfonyl}-4-fluorobenzofurane-2-carboxamide,
le 6-cyclopropyl-N-[(2-éthoxy-4,5-difluorophényl)sulfonyl]-4-fluorobenzofurane-2-carboxamide,
le 6-cyclopropyl-N-[(5-cyclopropyl-2-méthoxyphényl)sulfonyl]-4-fluorobenzofurane-2-carboxamide,
le 6-cyclopropyl-N-((5-cyclopropyl-2-éthoxyphényl)sulfonyl)-4-fluorobenzofurane-2-carboxamide,
le 6-cyclopropyl-4-fluoro-N-{[2-méthoxy-5-(2-oxopropyl)phényl]sulfonyl}benzofurane-2-carboxamide,
le 6-cyclopropyl-N-(2-éthoxy-5-isopropyl-phényl)sulfonyl-4-fluoro-benzofurane-2-carboxamide, et
le N-(2-chlorobenzènesulfonyl)-6-(propan-2-yl)-1-benzofurane-2-carboxamide.

7. Procédé de préparation d'un composé de formule générale (**I**) selon l'une quelconque des revendications 1 à 6, ledit procédé comprenant la réaction d'un composé intermédiaire de formule (**IIa**) avec un partenaire de couplage contenant R² approprié pour la formation de liaisons C-C, de préférence dans lequel le partenaire de couplage est un composé contenant du bore, un composé organométallique de zinc ou un halogénure d'alkyle, 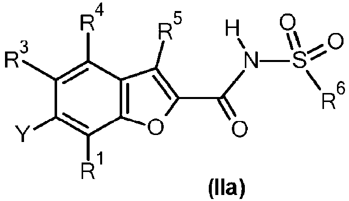 dans lequel R¹, R², R³, R⁴, R⁵ et R⁶ sont tels que définis pour le composé de formule générale (I) selon l'une quelconque des revendications 1 à 6 ou un tautomère, ou un N-oxyde, ou un sel de celui-ci, ou un sel d'un tautomère, ou un sel d'un N-oxyde, ou un mélange de ceux-ci et Y est un atome d'halogène choisi parmi le chlore et le brome.

8. Composé de formule générale (**I**) selon l'une quelconque des revendications 1 à 6 destiné à être utilisé dans le traitement ou la prophylaxie d'une maladie.

9. Composé destiné à être utilisé selon la revendication 8, ladite maladie étant une maladie hyperproliférative.

10. Composé destiné à être utilisé selon la revendication 9, ladite maladie hyperproliférative étant un cancer.

11. Composé destiné à être utilisé selon la revendication 10, dans lequel le cancer est choisi parmi le cancer du poumon, le cancer du sein, le cancer de la vessie, le cancer de l'utérus, le cancer de l'endomètre, le cancer de la prostate et la leucémie.

12. Composition pharmaceutique comprenant un composé de formule générale (**I**) selon l'une quelconque des revendications 1 à 6 et un ou plusieurs excipients acceptables pharmaceutiquement.

13. Composition pharmaceutique selon la revendication 12 destinée à être utilisée dans le traitement ou la prophylaxie d'une maladie, ladite maladie étant un cancer choisi parmi le cancer du poumon, le cancer du sein, le cancer de la vessie, le cancer de l'utérus, le cancer de l'endomètre, le cancer de la prostate et la leucémie.

14. Composition pharmaceutique comprenant un ou plusieurs composés de formule générale (**I**) selon l'une quelconque des revendications 1 à 6 et un ou plusieurs agents anticancéreux supplémentaires.
